# EUROPEAN PATENT APPLICATION

(11) **EP 4 357 343 A1**
(43) Date of publication of application: **24.04.2024**
(21) Application number: 22202598.3
(22) Date of filing: 19.10.2022
(51) Int. Cl.: C07D 417/04, C07D 401/04, C07D 401/12, C07D 403/14, C07D 413/14, C07D 417/14, C07D 495/04, A61K 31/397, A61P 35/00, A61K 31/428, A61K 31/4439, A61K 31/506, A61K 31/496, A61K 31/4365, C07D 401/14

(54) **RNA-BINDING MOLECULES WITH A SMALL CORE SCAFFOLD (R-SCORE) AND USES THEREOF**

(71) Applicant: Max-Planck-Gesellschaft zur Förderung der Wissenschaften e.V., 80539 München (DE)
(72) Inventor: WU, Peng, 44227 Dortmund (DE); JIANG, Mao, 44227 Dortmund (DE); LIU, Yang, 44227 Dortmund (DE); BORGELT, Lydia, 44227 Dortmund (DE)
(74) Representative: Arth, Hans-Lothar

(57) **Abstract**

The present invention relates to novel RNA-binding azetidine compounds of general formula (I) and stereoisomeric forms, hydrates, solvates and pharmaceutically acceptable salts thereof, as well as pharmaceutical compositions containing said azetidine compound together with at least one pharmaceutically acceptable carrier, excipient and/or diluent. Said azetidine compounds are particularly useful for the treatment and/or prophylaxis of diseases caused by and/or associated with microRNA expression or microRNA misexpression, particularly cancer, tumor, virus infections, immune-related diseases, inflammatory diseases, diabetes, cardiovascular diseases, metabolic diseases, and neurodegenerative diseases.

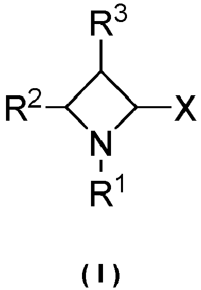

## Description

The present invention relates to novel RNA-binding azetidine compounds of general formula **(I)** and stereoisomeric forms, hydrates, solvates and pharmaceutically acceptable salts thereof, as well as pharmaceutical compositions containing said azetidine compound together with at least one pharmaceutically acceptable carrier, excipient and/or diluent. Said azetidine compounds are particularly useful for the treatment and/or prophylaxis of diseases caused by and/or associated with microRNA expression or microRNA misexpression, particularly cancer, tumor, virus infections, immune-related diseases, inflammatory diseases, diabetes, cardiovascular diseases, metabolic diseases, and neurodegenerative diseases.

### Background of the invention

RNAs play key roles in gene expression and dysregulation of RNA metabolism and biology are associated with the progression of various human diseases. Targeting human transcriptome stands for a new perspective in drug discovery. Both coding and non-coding RNAs are becoming promising targets to develop therapeutic agents of different modalities. Micro RNAs (miRNAs) are a class of small, non-coding RNA which are approximately 17 to 25 nucleotides, regulating gene expression and affect many biological processes including inflammation, cell cycle regulation, stress response, etc. Micro RNA is transcribed by RNA polymerase II, the primary transcript namely primary miRNA (pri-miRNA) contains the miRNA sequence in the arm of the local stem then undergoes the microprocessor complex (Dorsha and DGCR8) mediated process and releases the stem-loop-shaped intermediate which contains around 85 nucleotides, namely precursor miRNA (pre-miRNA), then the pre-miRNA was transported to the cytoplasm and further cleaved by Dicer process to produce the mature miRNA (Michlewski G, et al. RNA 2019, 25, 1-16).

Dysregulation of oncogenic miRNAs, such as miR-17 and miR-21, has been founded in different types of cancers. Comparing B-cell lymphoma samples and cell lines to normal tissues, the levels of primary or mature miRNAs derived from the miR17-92 cluster are often increased. In a mouse B-cell lymphoma model, enforced expression of the mir-17-92 cluster acted with c-myc expression to accelerate tumor development (He L, et al. Nature 2005, 435, 828-833). In the development of lung cancers, especially in small-cell lung cancer, the miR-17-92 cluster is markedly and frequently overexpressed and enhances lung cancer cell growth (Yoji H, et al. Cancer Res. 2005, 65, 9628-9632). miR-17 promote gastric cancer cell proliferation and inhibit cell apoptosis via post-transcriptional modulation of p21 and TP53INP1(Mei W, et al. Eur. J. Cancer 2013, 49, 2010-2021). Consistently, miR-17 has been found overexpressing in breast cancer, colon cancer, pancreas cancer, prostate cancer and high expression of miR-17-92 cluster predicated a poor overall survival in many cancers (Volinia S, et al. Proc. Natl. Acad. Sci. 2006, 103, 2257-2261; Feifei L, et al. Oncotarget. 2017, 8, 69125-69138). Similar to miR-17, miR-21 has be identified as an oncogenic miRNA (Mridul B, et al. Cell Signal. 2021, 83, 109995). The overexpression of miR-21 has been observed in breast, colon, liver, brain, pancreas, prostate cancer and other human cancers (Leigh-Ann M, et al. Current genomics. 11 (7), 537-561(2010)). Anti-miR-21 oligonucleotides can suppress cancer cell growth in vitro and in vivo, suggested that miR-21 may serve as a novel therapeutic target (M.L S, et al. Oncogene. 2007, 26, 2799-2803).

Development of small molecules targeting miR-17 and miR-21 may lead to the discovery of novel therapeutics for many related diseases. In addition to the discovery of such small molecules via screening efforts, sequence-based design of structure-specific small-molecule binders to miRNA are present in literature (Disney M, et al. ACS Chem. Biol. 2016, 11, 1720-1728; Disney M, et al. J. Am. Chem. Soc. 2020, 142, 6970-6802). To date, the set of available RNA-binding small molecules comprises aromatic hydrophobic moieties as the core scaffolds mimicking nucleobases. However, the aromatic core scaffolds limit the chemical space of potential RNA-binding molecules. Therefore, there is a huge need for the discovery of RNA-binding small molecules covering new chemical space with the hope to identify molecules with different physicochemical properties and biological performance upon RNA binding.

It is the objective of the present invention to provide RNA-binding small compounds and/or pharmaceutically acceptable salts thereof which can be used as pharmaceutically active agents, especially for the treatment of diseases caused by and/or associated with microRNA expression or microRNA misexpression, as well as compositions comprising at least one of those compounds as pharmaceutically active agents.

The objective of the present invention is solved by the teaching of the independent claims. Further advantageous features, aspects and details of the invention are evident from the dependent claims, the description, the figures, and the examples of the present application.

### Description of the invention

The present invention is directed to new RNA-binding small molecules having an azetidine core scaffold. Said small molecules having a rigid core (also denoted as "R-Score" herein) cover new unexplored chemical space associated with the steric rigid feature of the small azetidine core, and will enable systematic assembly of RNA-binding azetidine compounds by attaching RNA-binding fragments as substituents.

The inventors have shown that the RNA-binding azetidine compounds of the present invention bind to oncogenic miRNAs, inhibit colony formation, and inhibit proliferation in human cancer cells expressing oncogenic miRNAs, thereby demonstrating indicated the possibility to apply the R-Score molecules as a general strategy to target structured elements on RNA targets

Thus, the present invention is directed to a compound of general formula **(I)** 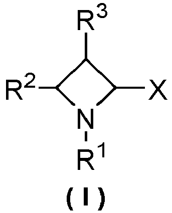 wherein
**R¹** represents -CH₃, -CH₂CH₃, -CH₂CH₂CH₃, -CH(CH₃)₂, -(CH₂)₃CH₃, -(CH₂)₄CH₃, -(CH₂)₅CH₃, -CH₂CH(CH₃)₂, -(CH₂)₂CH(CH₃)₂, -CH(CH₂CH₃)CH₃, -CH(CH₂CH₃)₂, -C(CH₃)₃, -CH₂CH=CH₂, -(CH₂)₂CH=CH₂, -(CH₂)₃CH=CH₂, -(CH₂)₄CH=CH₂, -**L¹**-**A¹**, or **-B¹** ;
**R²** represents -H, -CN, -N**R⁴R⁵**, **-L²**-**A²**, **-B²** , or **-C²**;
**R³** represents **-A³** or **-B³** with the proviso that when **R²** is -CN **R³** is **-A³** ;
**L¹** represents -CO- , or -SO₂- ;
**L²** represents -CH₂-N**R⁶**-CO-, -CH₂-N**R⁶-**SO₂- or 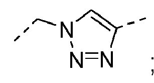 ;
**R⁴** and **R⁵** independently of each other represent -CH₃, -CH₂CH₃, -CH(CH₃)₂, -CH₂CH₂CH₃, -CH₂CH(CH₃)₂, -C(CH₃)₃, -cyclo-C₃H₅, -cyclo-C₄H₇, -cyclo-C₅H₉, -cyclo-C₆H₁₁, -CH₂-cyclo-C₃H₅, -CH₂-cyclo-C₄H₇, -CH₂-cyclo-C₅H₉, -CH₂-cyclo-C₆H₁₁, -CH₂-Ph, -CH₂OCH₃, -CH₂OCH₂CH₃, -CH₂CH₂OCH₃, or -CH₂CH₂SCH₃;
**A¹** and **A²** are independently of each other selected from 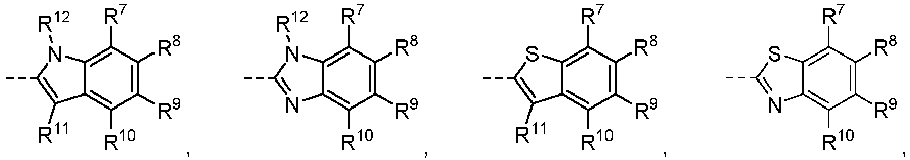 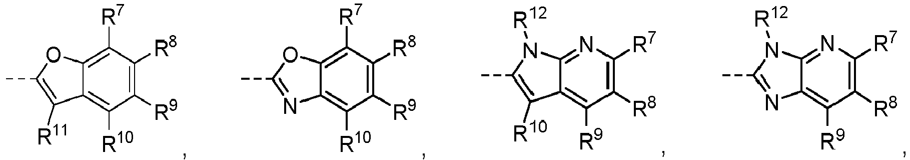 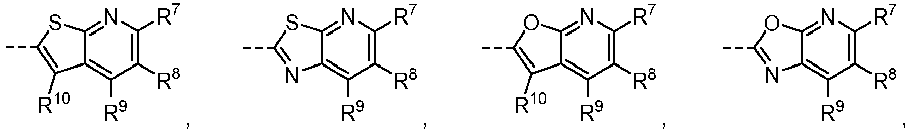 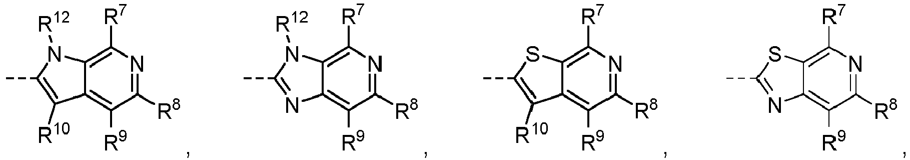 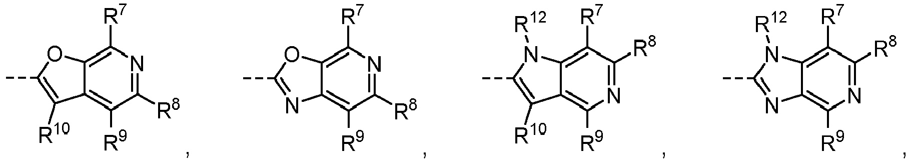 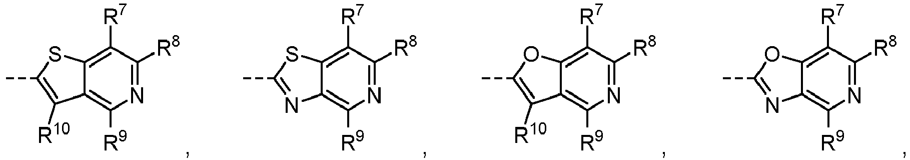 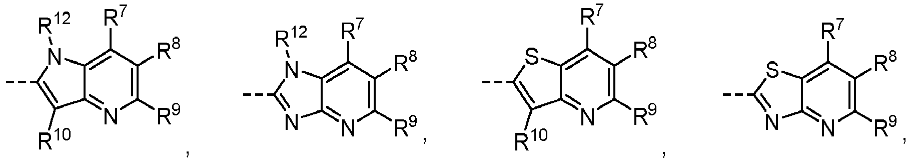 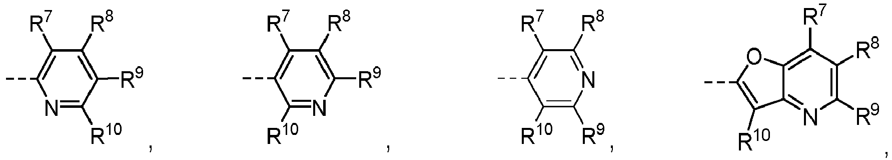 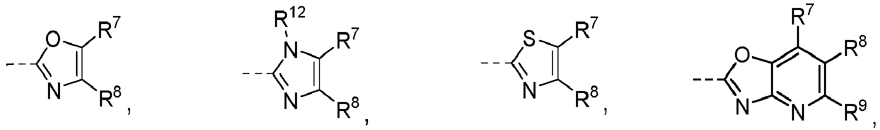 or 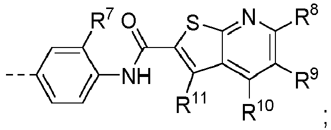
**A³** represents 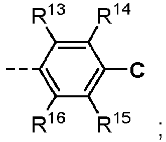 with C being selected from: 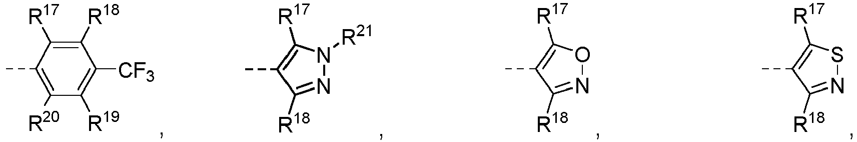 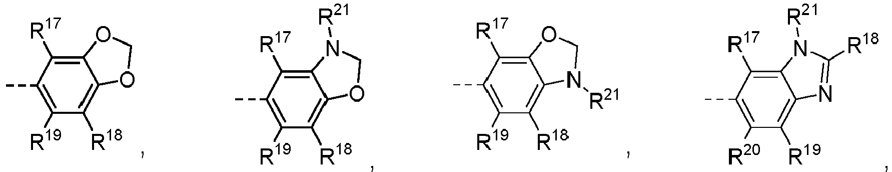 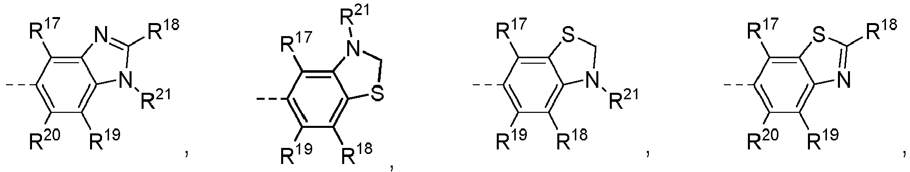 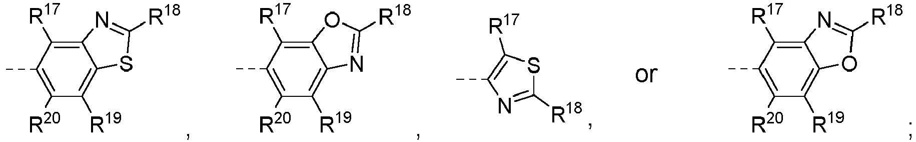
**B¹** represents **-H¹**, **-P¹**-**H¹**, or -**P¹**-**Y¹**-**H¹** ;
**B²** represents **-H²**, -**P²**-**H²**, or -**P²**-**Y²**-**H²** ;
**B³** represents **-H³**, -**P³**-**H³**, or -**P³**-**Y³**-**H³** ;
**C²** is selected from: 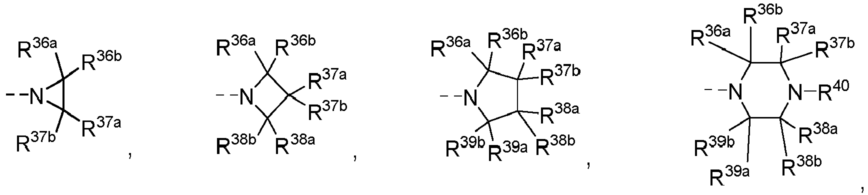 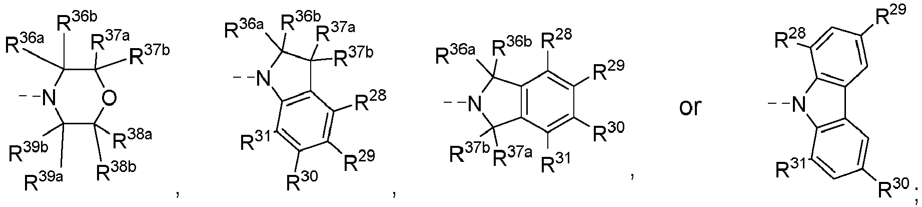
**Y¹**, **Y²**, and **Y³** are independently of each other selected from: -O-, -N**R²²**-, -CO-N**R²²**-, -N**R²²**-CO-, -SO₂-N**R²²**-, -N**R²²**-SO₂-, or -N**R²²**-CO-N**R²³**-;
**P¹**, **P²**, and **P³** are independently of each other selected from: 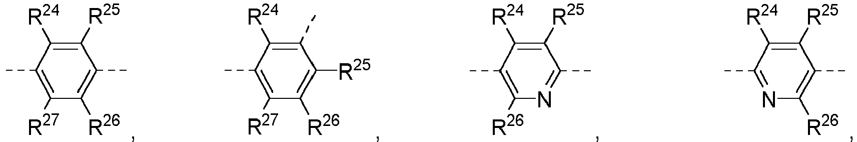 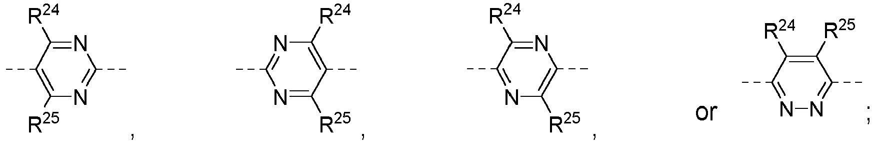
**H¹**, **H²**, and **H³** are independently of each other selected from: 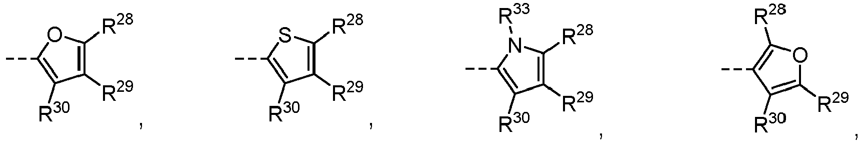 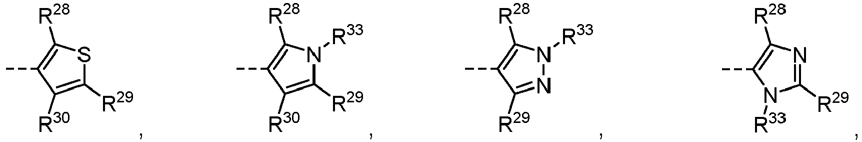 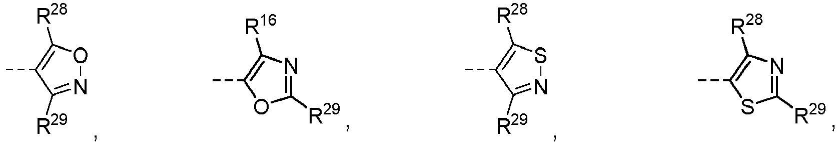 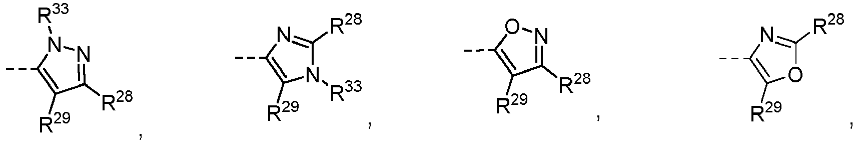 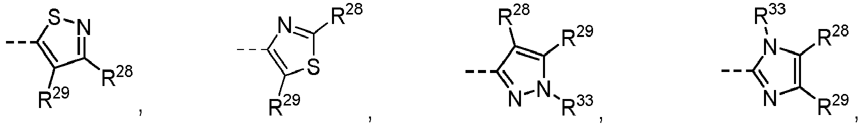 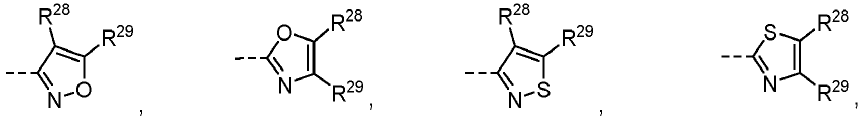 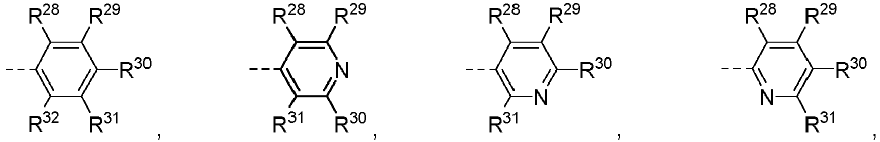 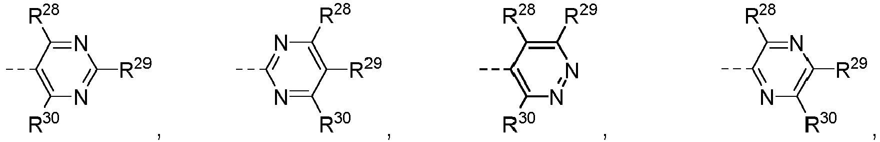 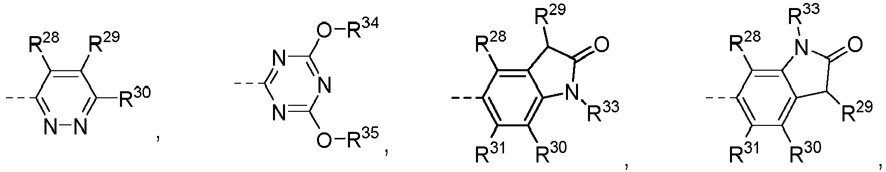 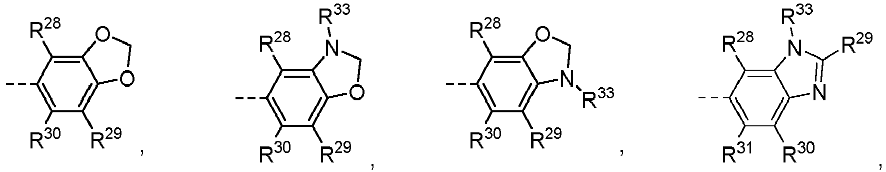 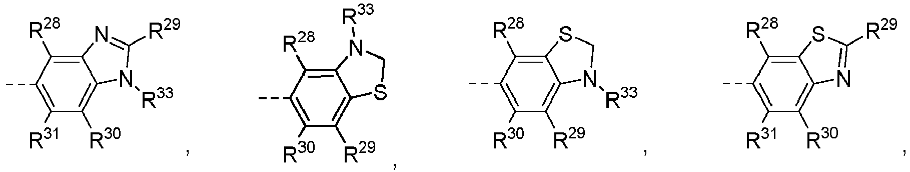 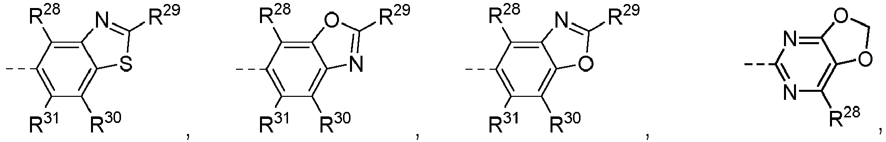 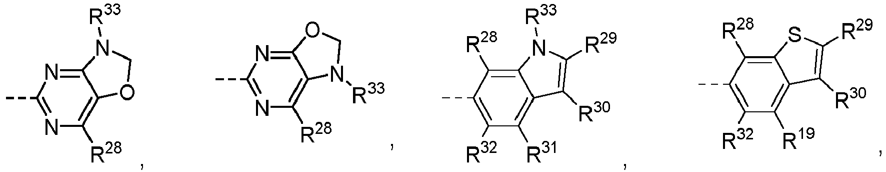 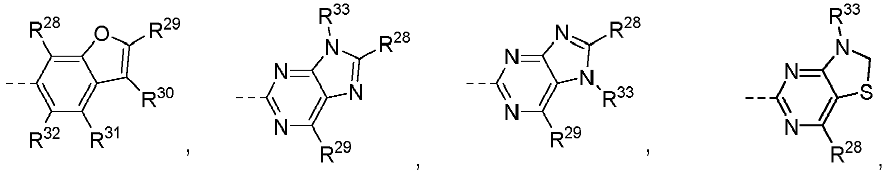 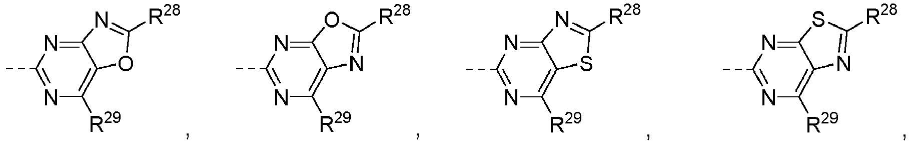 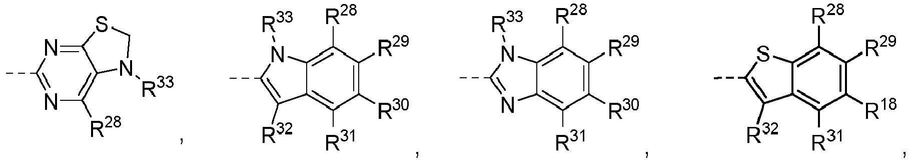 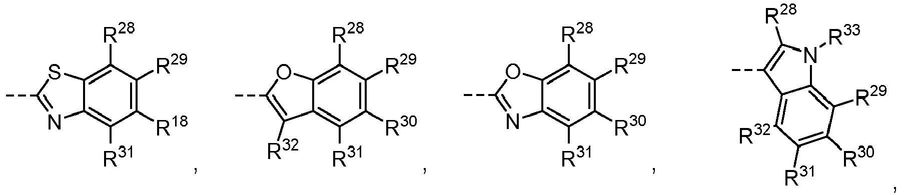 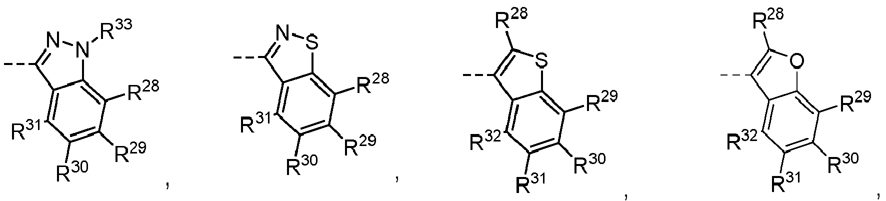 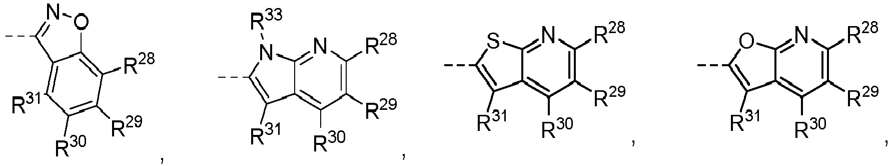 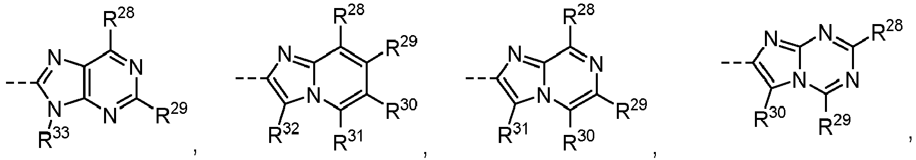 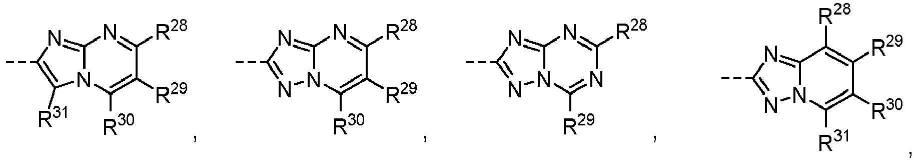 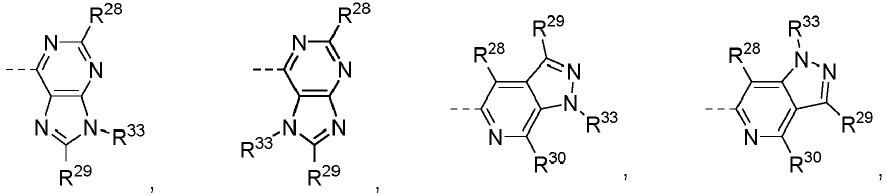 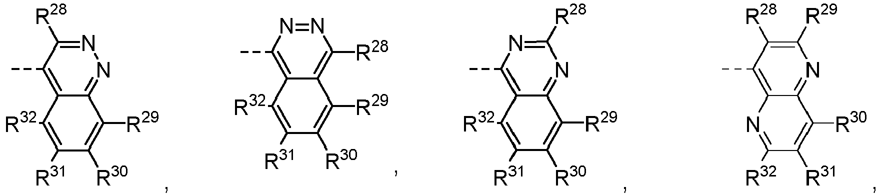 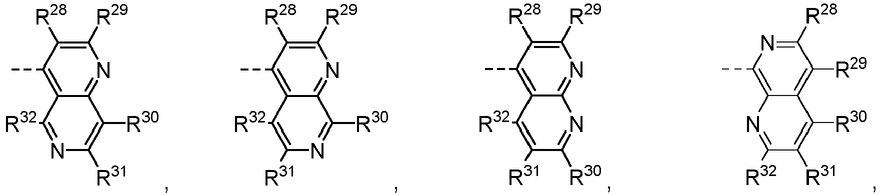 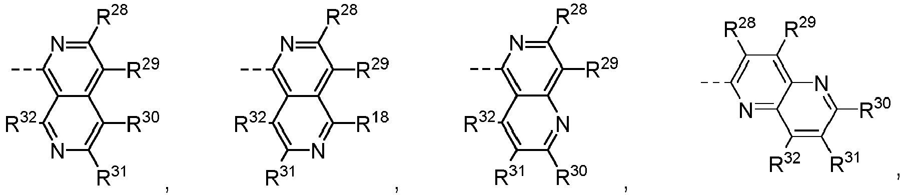 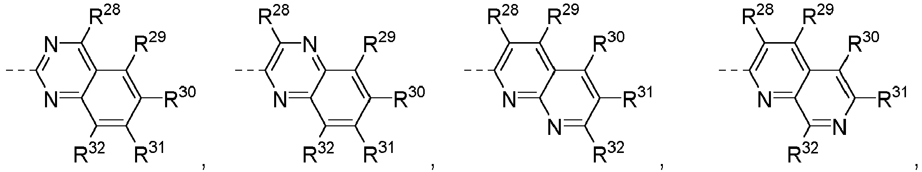 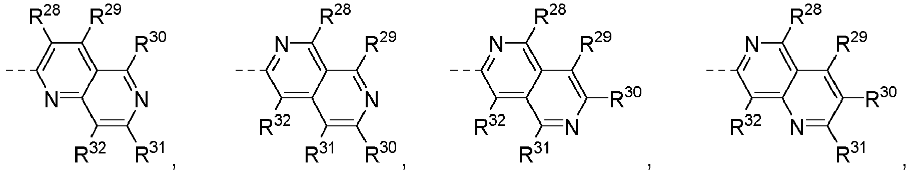 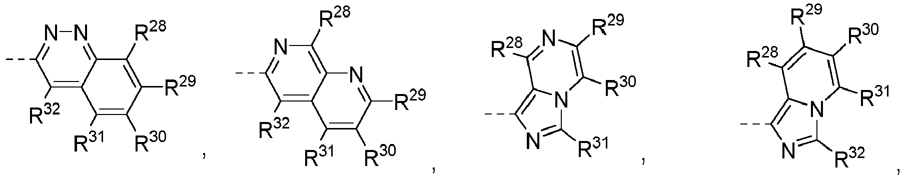 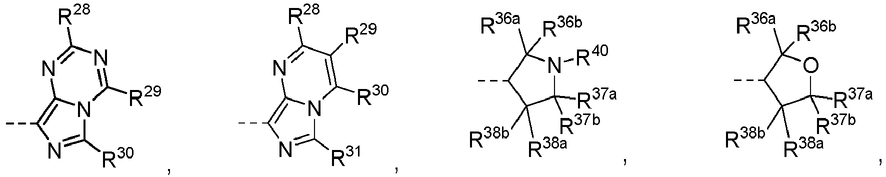 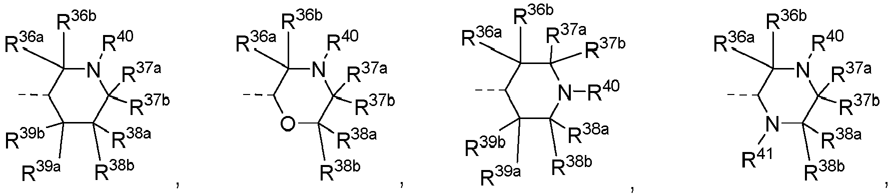 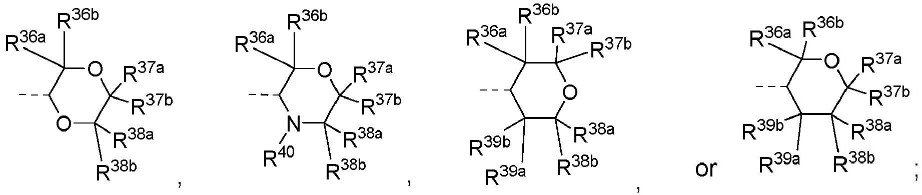
**R¹⁷** - **R²⁰** and **R²⁸-R³²** represent independently of each other -H, -F, -CI, -Br, -I, -OH, -CN, -NO₂, -CH₃, -C₂H₅, -C₃H₇, -CH(CH₃)₂, -C₄H₉, -CH₂-CH(CH₃)₂, -CH(CH₃)-C₂H₅, -C(CH₃)₃, -cyclo-C₃H₅, -CH₂-cyclo-C₃H₅, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -OCH₃, -OC₂H₅, -OC₃H₇, -OCH(CH₃)₂, -OC(CH₃)₃, -OC₄H₉, -OCHF₂, -OCF₃, -OCH₂CF₃, -OC₂F₅, -OCH₂OCH₃, -O-cyclo-C₃H₅, -OCH₂-cyclo-C₃H₅, -O-C₂H₄-cyclo-C₃H₅, -CHO, -COCH₃, -COCF₃, -COC₂H₅, -COC₃H₇, -COCH(CH₃)₂, -COC(CH₃)₃, -COOH, -COOCH₃, -COOC₂H₅, -COOC₃H₇, -COOCH(CH₃)₂, -COOC(CH₃)₃, -OOC-CH₃, -OOC-CF₃, -OOC-C₂H₅, -OOC-C₃H₇, -OOC-CH(CH₃)₂, -OOC-C(CH₃)₃, -NH₂, -NHCH₃, -NHC₂H₅, -NHC₃H₇, -NHCH(CH₃)₂, -NHC(CH₃)₃, -N(CH₃)₂, -N(C₂H₅)₂, -N(C₃H₇)₂, -N[CH(CH₃)₂]₂, -N[C(CH₃)₃]₂, -NHCOCH₃, -NHCOCF₃, -NHCOC₂H₅, -NHCOC₃H₇, -NHCOCH(CH₃)₂, -NHCOC(CH₃)₃, -CONH₂, -CONHCH₃, -CONHC₂H₅, -CONHC₃H₇, -CONHCH(CH₃)₂, -CONH-cyclo-C₃H₅, -CONHC(CH₃)₃, -CON(CH₃)₂, -CON(C₂H₅)₂, -CON(C₃H₇)₂, -CON[CH(CH₃)₂]₂, -CON[C(CH₃)₃]₂, -SO₂NH₂, -SO₂NHCH₃, -SO₂NHC₂H₅, -SO₂NHC₃H₇, -SO₂NHCH(CH₃)₂, -SO₂NH-cyclo-C₃H₅, -SO₂NHC(CH₃)₃, -SO₂N(CH₃)₂, -SO₂N(C₂H₅)₂, -SO₂N(C₃H₇)₂, -SO₂N[CH(CH₃)₂]₂, -SO₂N[C(CH₃)₃]₂, -NHSO₂CH₃, -NHSO₂CF₃, -NHSO₂C₂H₅, -NHSO₂C₃H₇, -NHSO₂CH(CH₃)₂, -NHSO₂C(CH₃)₃, -CH=CH₂, -CH₂-CH=CH₂, -C(CH₃)=CH₂, -CH=CH-CH₃, -C≡CH, -C≡C-CH₃, -CH₂-C≡CH, -Ph, -O-Ph, -O-CH₂-Ph, -C(=NH)NH₂, or 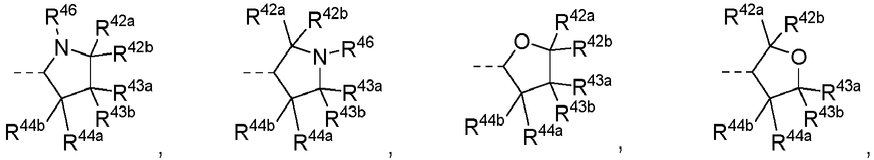 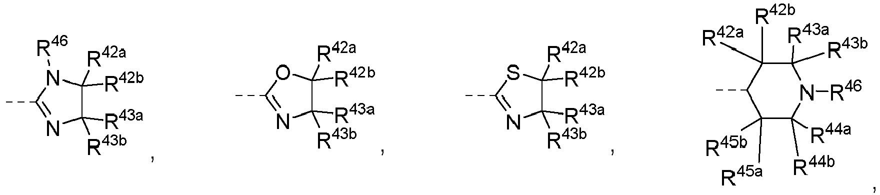 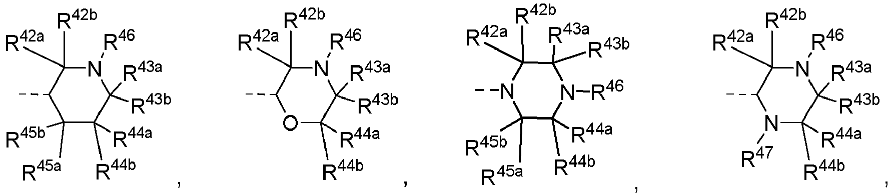 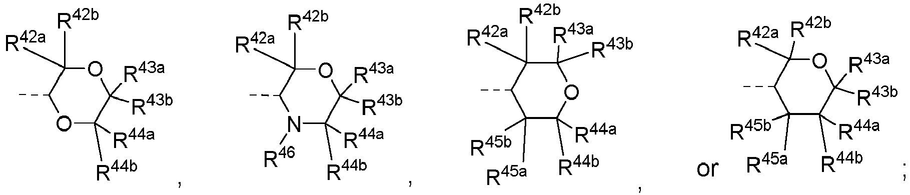
**R⁶**, **R¹¹**, **R¹²**, **R²¹** - **R²³**, **R³³** - **R³⁵**, **R⁴⁰**, **R⁴¹**, **R⁴⁶**, and **R⁴⁷** represent independently of each other -H, -CH₃, -CH₂CH₃, -CH(CH₃)₂, -CH₂CH₂CH₃, -CH₂CH(CH₃)₂, -C(CH₃)₃, -cyclo-C₃H₅, -cyclo-C₄H₇, -cyclo-C₅H₉, -cyclo-C₆H₁₁, -CH₂-cyclo-C₃H₅, -CH₂-cyclo-C₄H₇, -CH₂-cyclo-C₅H₉, -CH₂-cyclo-C₆H₁₁, -CH₂-Ph, -CH₂OCH₃, -CH₂OCH₂CH₃, -CH₂CH₂OCH₃, -COO-C(CH₃)₃, -COO-CH₂Ph, or -CH₂CH₂SCH₃;
**R²⁴ - R²⁷**, **R^{36a} - R^{39a}**, **R^{36b} - R^{39b} R^{42a}** - **R^{45a}**, and **R^{42b} - R^{45b}** represent independently of each other -H, -F, -Cl, -Br, -I, -OH, -CN, -NO₂, -CH₃, -C₂H₅, -C₃H₇, -CH(CH₃)₂, -C₄H₉, -CH₂-CH(CH₃)₂, -CH(CH₃)-C₂H₅, -C(CH₃)₃, -cyclo-C₃H₅, -CH₂-cyclo-C₃H₅, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -OCH₃, -OC₂H₅, -OC₃H₇, -OCH(CH₃)₂, -OC(CH₃)₃, -OC₄H₉, -OCHF₂, -OCF₃, -OCH₂CF₃, -OC₂F₅, -OCH₂OCH₃, -O-cyclo-C₃H₅, -OCH₂-cyclo-C₃H₅, -O-C₂H₄-cyclo-C₃H₅, -CHO, -COCH₃, -COCF₃, -COC₂H₅, -COC₃H₇, -COCH(CH₃)₂, -COC(CH₃)₃, -COOH, -COOCH₃, -COOC₂H₅, -COOC₃H₇, -COOCH(CH₃)₂, -COOC(CH₃)₃, -OOC-CH₃, -OOC-CF₃, -OOC-C₂H₅, -OOC-C₃H₇, -OOC-CH(CH₃)₂, -OOC-C(CH₃)₃, -NH₂, -NHCH₃, -NHC₂H₅, -NHC₃H₇, -NHCH(CH₃)₂, -NHC(CH₃)₃, -N(CH₃)₂, -N(C₂H₅)₂, -N(C₃H₇)₂, -N[CH(CH₃)₂]₂, -N[C(CH₃)₃]₂, -NHCOCH₃, -NHCOCF₃, -NHCOC₂H₅, -NHCOC₃H₇, -NHCOCH(CH₃)₂, -NHCOC(CH₃)₃, -CONH₂, -CONHCH₃, -CONHC₂H₅, -CONHC₃H₇, -CONHCH(CH₃)₂, -CONH-cyclo-C₃H₅, -CONHC(CH₃)₃, -CON(CH₃)₂, -CON(C₂H₅)₂, -CON(C₃H₇)₂, -CON[CH(CH₃)₂]₂, -CON[C(CH₃)₃]₂, -SO₂NH₂, -SO₂NHCH₃, -SO₂NHC₂H₅, -SO₂NHC₃H₇, -SO₂NHCH(CH₃)₂, -SO₂NH-cyclo-C₃H₅, -SO₂NHC(CH₃)₃, -SO₂N(CH₃)₂, -SO₂N(C₂H₅)₂, -SO₂N(C₃H₇)₂, -SO₂N[CH(CH₃)₂]₂, -SO₂N[C(CH₃)₃]₂, -NHSO₂CH₃, -NHSO₂CF₃, -NHSO₂C₂H₅, -NHSO₂C₃H₇, -NHSO₂CH(CH₃)₂, -NHSO₂C(CH₃)₃, -CH=CH₂, -CH₂-CH=CH₂, -C(CH₃)=CH₂, -CH=CH-CH₃, -C≡CH, -C≡C-CH₃, -CH₂-C≡CH, -Ph, -O-Ph, or -O-CH₂-Ph;
**X** represents -CN, -NH₂, or -CH₂OH;
with the proviso that **X** and **R²** cannot be simultaneously -CN;
or a regiomer, diastereomer, enantiomer, a mixture of regiomers, a mixture of diastereomers, a mixture of enantiomers, a prodrug, a solvate, a hydrate, or a pharmaceutically acceptable salt thereof.

Preferably, **L¹** represents -CO-.

Preferably, **L²** represents -CH₂-NH-CO- , or

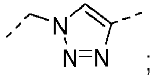

Preferably, **R⁴** and **R⁵** independently of each other represent -CH₃, -CH₂CH₃, or -CH(CH₃)₂. More preferably, **R⁴** and **R⁵** represent -CH₃, -CH₂CH₃, or -CH(CH₃)₂. In a preferred embodiment, **R⁴** and **R⁵** represent -CH₃.

Preferably, **B¹** represents **-H¹**, or **-P¹**-**H¹**. **B²** represents **-H²**, or **-P²**-**H²**. **B³** represents **-H³**, or **-P³**-**H³**.

Preferably, **Y¹**, **Y²**, and **Y³** are independently of each other selected from: -O-, -N**R²²**-, -CO-N**R²²**-, -N**R²²**-CO-. In a preferred embodiment, **Y¹**, **Y²**, and **Y³** are -N**R²²**-CO- In a preferred embodiment, **Y¹** is -NH-CO-.

Preferably, **P¹**, **P²**, and **P³** are independently of each other selected from: 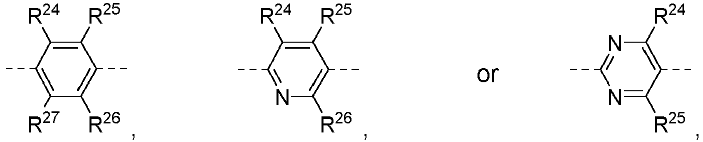 wherein **R²⁴** - **R²⁷** have the meanings as defined herein.

More preferably, **P¹**, **P²**, and **P³** represent:

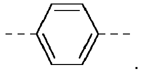

A preferred embodiment of the present invention is directed to a compound of formula (I), wherein **R¹** represents **-L¹**-**A¹**, or **-B¹**; **R²** represents **-L²**-**A²**, **-B²**, or **-C²**; and **R³** represents **-A³** or **-B³** with the proviso that when **R²** is -CN **R³** is **-A³**; wherein **A¹**, **A²**, **A³**, **B¹**, **B²**, **B³**, **C²**, **L¹**, **L²**, **R²** and **X** have the meanings as defined herein.

Preferably, **R¹⁷** - **R²⁰** and **R²⁸-R³²** represent independently of each other -H, -F, -CI, -Br, -OH, -CH₃, -C₂H₅, -C₃H₇, -CH(CH₃)₂, -C(CH₃)₃, -NH₂, -NHCH₃, -NHC₂H₅, -N(CH₃)₂, -N(C₂H₅)₂, -OCH₃, -OC₂H₅, or

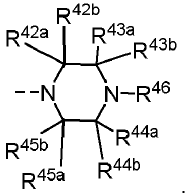

More preferably, **R¹⁷** - **R²⁰** represent -H and **R²⁸-R³²** represent independently of each other -H, -F, -Br, -OH, -CH₃, -C₂H₅, -C₃H₇, -CH(CH₃)₂, -C(CH₃)₃, -NH₂, -N(CH₃)₂, -, -OCH₃ , or

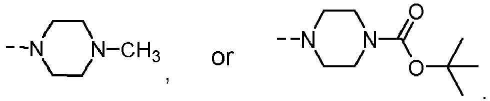

Preferably, **R⁶**, **R¹¹**, **R¹²**, **R²¹** - **R²³**, **R³³** - **R³⁵**, **R⁴⁰**, **R⁴¹**, **R⁴⁶**, and **R⁴⁷** represent independently of each other -H, -CH₃, -COO-C(CH₃)₃, or -COO-CH₂Ph. Most preferably, **R⁶**, **R**^{**1**1}, **R¹²**, **R²¹** - **R²³**, **R³³** - **R³⁵**, **R⁴⁰**, **R⁴¹**, **R⁴⁶**, and **R⁴⁷** represent independently of each other -H, -CH₃, or -COO-C(CH₃)₃. In a preferred embodiment, **R⁴⁰** represents -CH₃, or -COO-C(CH₃)₃.

Preferably, **R²⁴ - R²⁷**, **R^{36a} - R^{39a}**, **R^{36b}** - **R^{39b} R^{42a} - R^{45a}**, and **R^{42b} - R^{45b}** represent independently of each other H, -F, -CI, -Br, -OH, -CH₃, -C₂H₅, -C₃H₇, -CH(CH₃)₂, -C(CH₃)₃, -NH₂, -NHCH₃, -NHC₂H₅, -N(CH₃)₂, -N(C₂H₅)₂, -OCH₃, or -OC₂H₅. More preferably, **R²⁴** - **R²⁷** represent -H. More preferably, **R^{36a}** - **R^{39a}**, **R^{36b}** - **R^{39b} R^{42a}** - **R^{45a}**, and **R^{42b}** - **R^{45b}** represent -H. More preferably, **R²⁴ - R²⁷**, **R^{36a} - R^{39a}**, **R^{36b} - R^{39b} R^{42a} - R^{45a}**, and **R^{42b}-R^{45b}** represent -H.

In a preferred embodiment of the present invention, **R¹** represents -CH₂CH₂CH₃, -CH₂CH=CH₂, 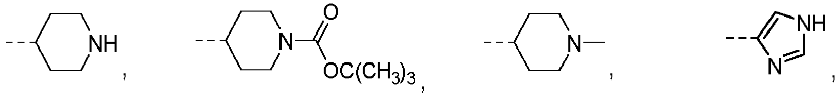 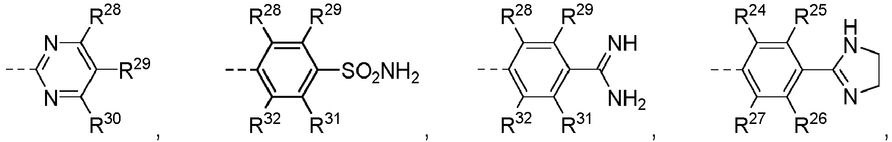 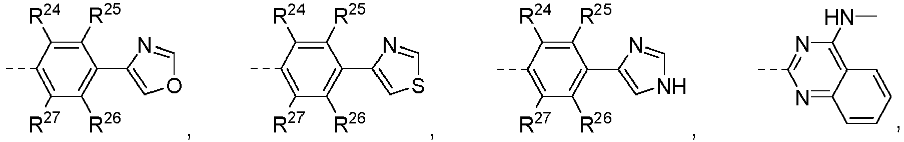 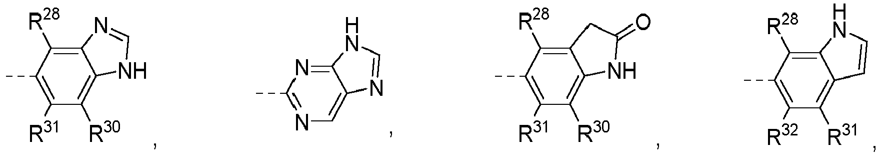 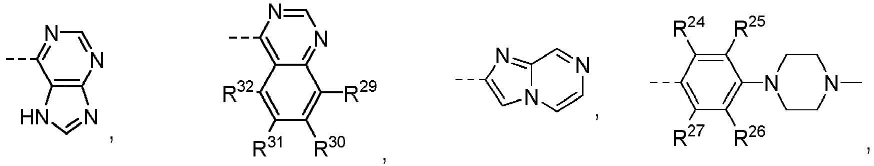 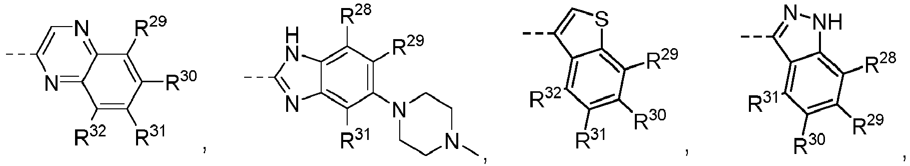 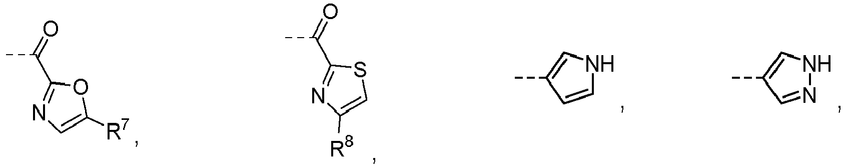 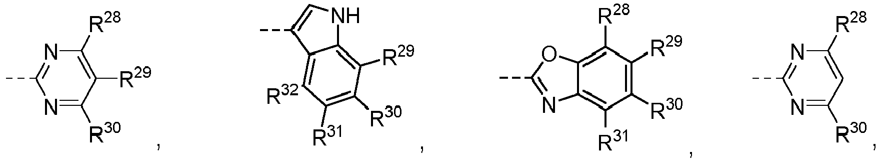 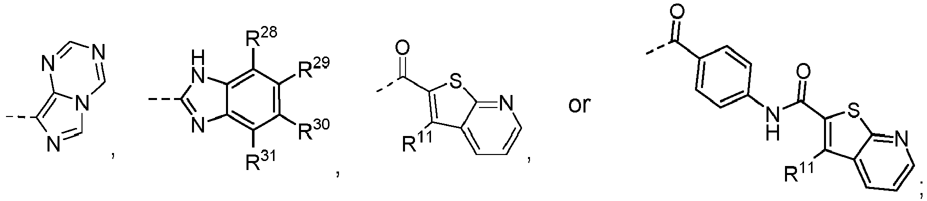 wherein **R⁷**, **R⁸**, **R¹¹** and **R**^{**24** -} **R³²** have the meanings as defined herein.

In an even more preferred embodiment of the present invention, **R¹** represents -CH₂CH₂CH₃, -CH₂CH=CH₂,

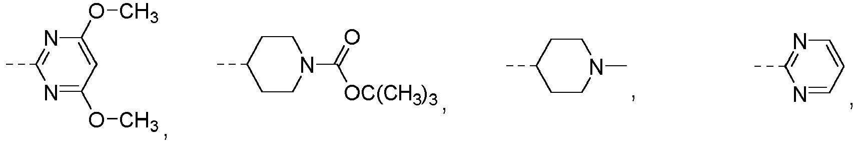

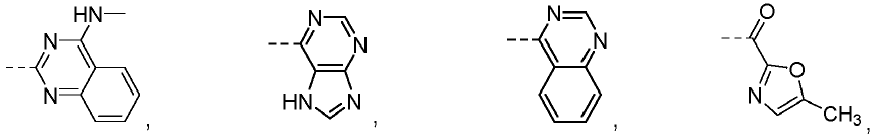

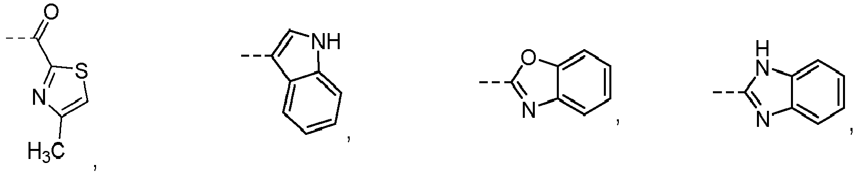

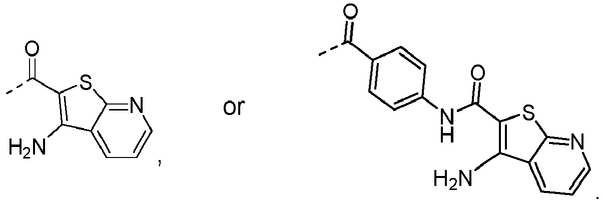

In a preferred embodiment of the present invention, **R²** represents -H, -CN, -N(CH₃)₂, 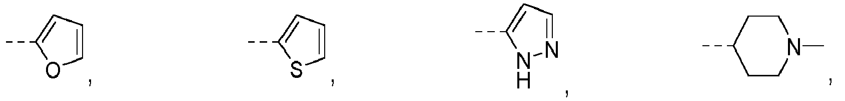 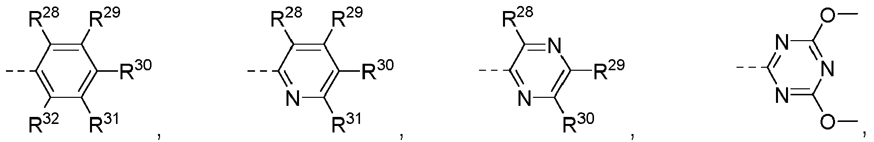 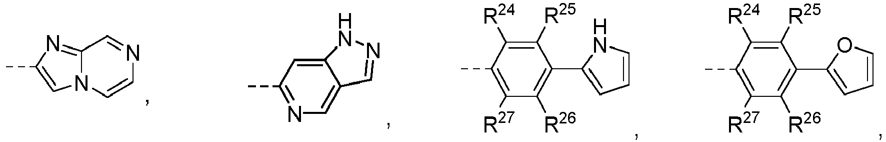 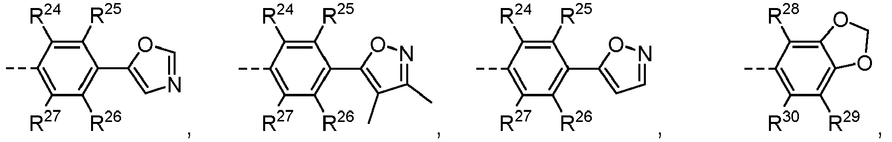 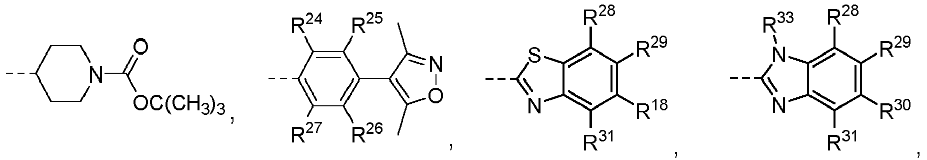 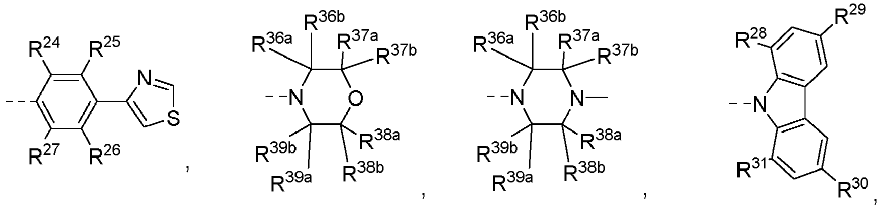 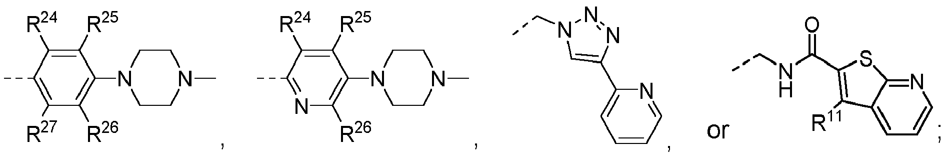 wherein **R¹¹**, **R^{24 -} R³²**, **R^{36a} - R^{39a}**, and **R^{36b}** - **R^{39b}** have the meanings as defined herein.

In an even preferred embodiment of the present invention, **R²** represents -H, -CN, 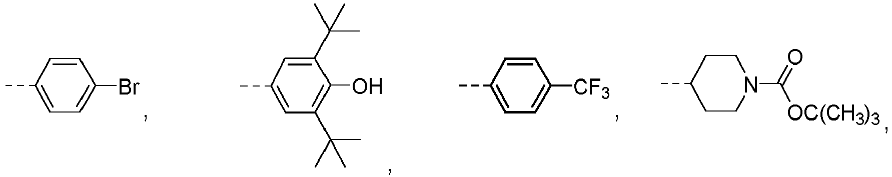 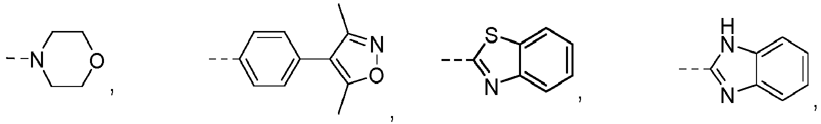 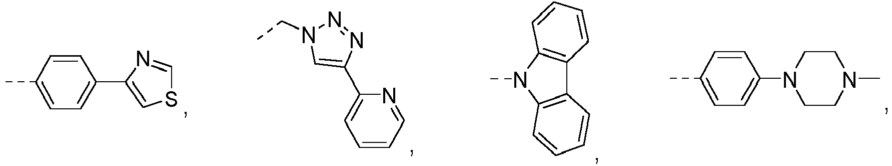 or

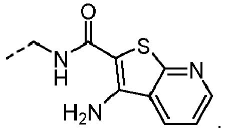

In a preferred embodiment of the present invention, **R³** represents 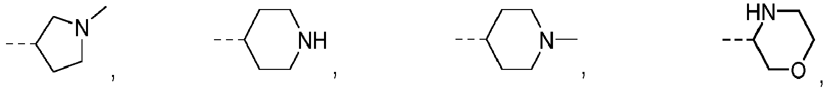 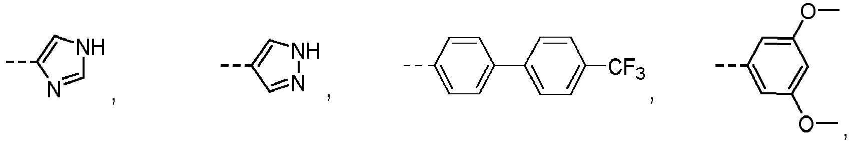 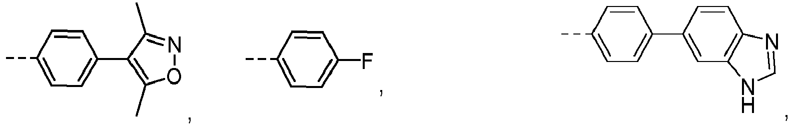 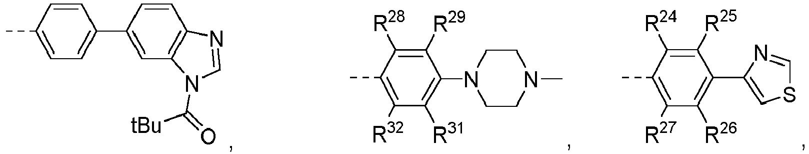 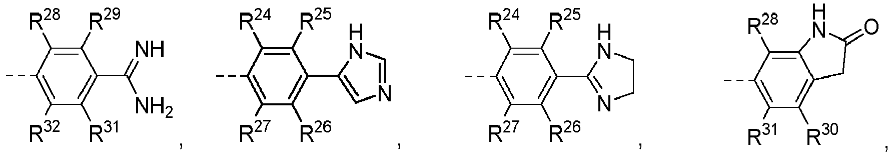 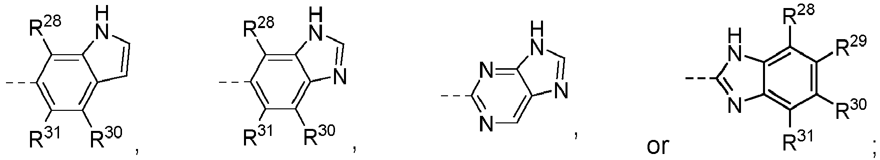 wherein **R**^{**24** -} **R³²** have the meanings as defined herein.

In an even more preferred embodiment of the present invention, **R³** represents 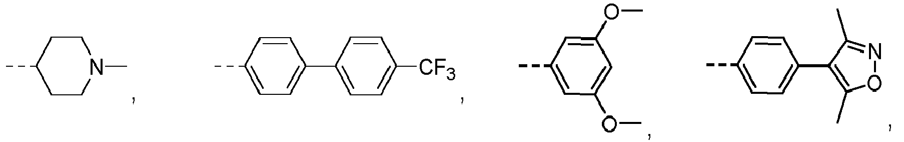 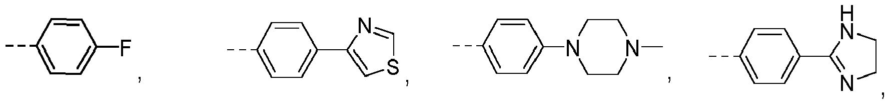 or

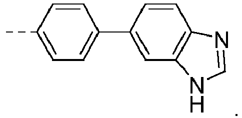

A further aspect of the present invention is directed to the compounds of the (**I-a**), (**I-b**), (**I-c**), and (**I-d**). 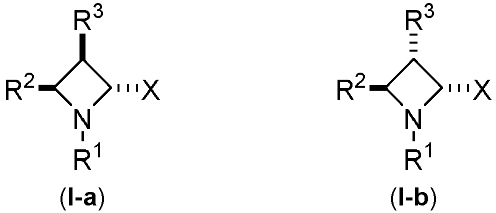 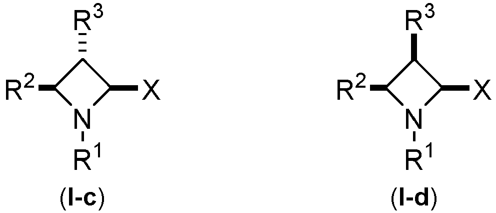 wherein **R¹**, **R²**, **R³** and **X** have the meanings as defined herein.

Preferably, in all formulae (**I-a**), (**I-b**), (**I-c**), and (**I-d**) as disclosed herein, **R¹** represents **-L¹**-**A¹** with **L¹** being -CO-.

Preferably, in all formulae (**I-a**), (**I-b**), (**I-c**), and (**I-d)**, **R²** represents **-L²**-**A²**, wherein **L²** represents -CH₂-NH-CO- , or

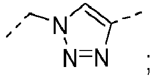

Preferably, in all formulae (**I-a**), (**I-b**), (**I-c**), and (**I-d)**, **R²** represents -N**R⁴R⁵**, wherein **R⁴** and **R⁵** independently of each other represent -CH₃, -CH₂CH₃, or -CH(CH₃)₂. More preferably, **R⁴** and **R⁵** represent -CH₃, -CH₂CH₃, or -CH(CH₃)₂. In a preferred embodiment, **R⁴** and **R⁵** represent -CH₃.

Preferably, in all formulae (**I-a**), (**I-b**), (**I-c**), and (**I-d)**, **R¹** represents **-P¹**-**Y¹**-**H¹** , wherein **Y¹** is selected from: -O-, -N**R²²**-, -CO-N**R²²**-, -N**R²²**-CO-. In a preferred embodiment, **Y¹** represents -N**R²²**-CO-. In a preferred embodiment, **Y¹** is -NH-CO-.

Preferably, in all formulae (**I-a**), (**I-b**), (**I-c**), and (**I-d)**, **R²** represents **-P²**-**Y²-H²** , wherein **Y²** is selected from: -O-, -N**R²²**-, -CO-N**R²²**-, -N**R²²**-CO-. In a preferred embodiment, **Y²** represents -N**R²²**-CO-.

Preferably, in all formulae (**I-a**), (**I-b**), (**I-c**), and (**I-d)**, **R³** represents **-P³**-**Y³**-**H³** , wherein **Y³** is selected from: -O-, -N**R²²**-, -CO-N**R²²**-, -N**R²²**-CO-. In a preferred embodiment, **Y³** represents -N**R²²**-CO-.

Preferably, in all formulae (**I-a**), (**I-b**), (**I-c**), and (**I-d)**, **R¹** represents **-P¹**-**H¹**, or **-P¹**-**Y¹**-**H¹** , wherein **P¹** is selected from: 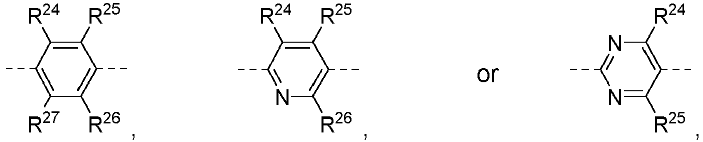 wherein **H¹**, **Y¹**, **R²⁴** - **R²⁷** have the meanings as defined herein.

More preferably, **P¹** represents:

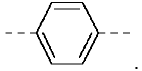

Preferably, in all formulae (**I-a**), (**I-b**), (**I-c**), and (**I-d)**, **R²** represents **-P²**-**H²**, or **-P²**-**Y²**-**H²**, wherein **P²** is selected from: 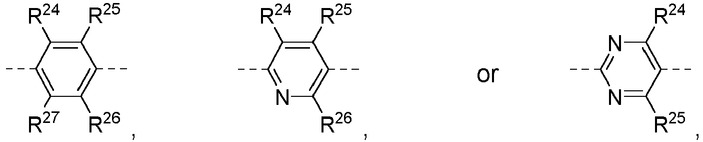 wherein **H²**, **Y²**, and **R²⁴** - **R²⁷** have the meanings as defined herein.

More preferably, **P²** represents:

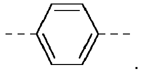

Preferably, in all formulae (**I-a**), (**I-b**), (**I-c**), and (**I-d)**, **R³** represents **-P³**-**H³**, or **-P³**-**Y³**-**H³**, wherein **P³** is selected from: 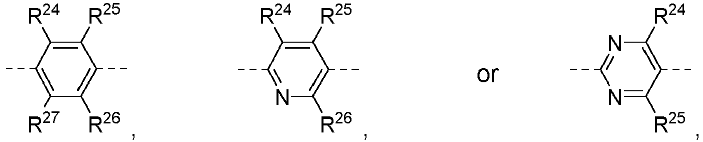 wherein **H³**, **Y³**, and **R²⁴** - **R²⁷** have the meanings as defined herein.

More preferably, **P³** represents:

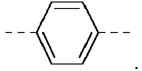

Preferably, in all formulae (**I-a**), (**I-b**), (**I-c**), and (**I-d)**, **R¹⁷** - **R²⁰** and **R²⁸-R³²** represent independently of each other -H, -F, -CI, -Br, -OH, -CH₃, -C₂H₅, -C₃H₇, -CH(CH₃)₂, -C(CH₃)₃, -NH₂, -NHCH₃, -NHC₂H₅, -N(CH₃)₂, -N(C₂H₅)₂, -OCH₃, -OC₂H₅, or

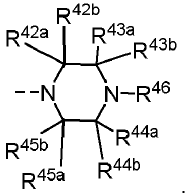

More preferably, in all formulae (**I-a**), (**I-b**), (**I-c**), and (**I-d)**, **R¹⁷** - **R²⁰** represent -H and **R²⁸**-**R³²** represent independently of each other -H, -F, -Br, -OH, -CH₃, -C₂H₅, -C₃H₇, -CH(CH₃)₂, -C(CH₃)₃, -NH₂, -N(CH₃)₂, -, -OCH₃,

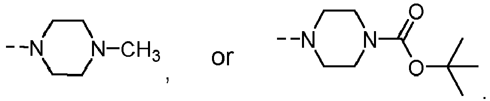

Preferably, in all formulae (**I-a**), (**I-b**), (**I-c**), and (**I-d)**, **R⁶**, **R¹¹**, **R¹²**, **R²¹** - **R²³**, **R³³** - **R³⁵**, **R⁴⁰**, **R⁴¹**, **R⁴⁶**, and **R⁴⁷** represent independently of each other -H, -CH₃, -COO-C(CH₃)₃, or -COO-CH₂Ph. Most preferably, **R⁶**, **R**^{**1**1}, **R¹²**, **R²¹** - **R²³**, **R³³** - **R³⁵**, **R⁴⁰**, **R⁴¹**, **R⁴⁶**, and **R⁴⁷** represent independently of each other -H, -CH₃, or -COO-C(CH₃)₃. In a preferred embodiment, **R⁴⁰** represents -CH₃, or -COO-C(CH₃)₃.

Preferably, Preferably, in all formulae (**I-a**), (**I-b**), (**I-c**), and (**I-d)**, **R²⁴** - **R²⁷**, **R^{36a}** - **R^{39a}**, **R^{36b}** - **R^{39b} R^{42a}** - **R^{45a}**, and **R^{42b}** - **R^{45b}** represent independently of each other H, -F, -CI, -Br, -OH, -CH₃, -C₂H₅, -C₃H₇, -CH(CH₃)₂, -C(CH₃)₃, -NH₂, -NHCH₃, -NHC₂H₅, -N(CH₃)₂, -N(C₂H₅)₂, -OCH₃, or -OC₂H₅.

More preferably, **R²⁴** - **R²⁷** represent -H. More preferably, **R^{36a} - R^{39a}**, **R^{36b}** - **R^{39b} R^{42a}** - **R^{45a}**, and **R^{42b}** - **R^{45b}** represent -H. More preferably, **R²⁴** - **R²⁷**, **R^{36a}** - **R^{39a}**, **R^{36b}** - **R^{39b} R^{42a}** - **R^{45a}**, and **R^{42b}** - **R^{45b}** represent -H.

Preferably, in all formulae (**I-a**), (**I-b**), (**I-c**), and (**I-d**) as disclosed herein, **R¹** represents -CH₂CH₂CH₃, -CH₂CH=CH₂, 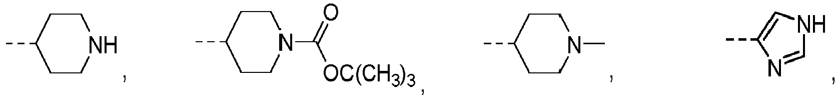 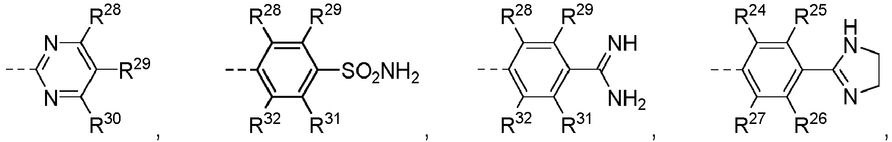 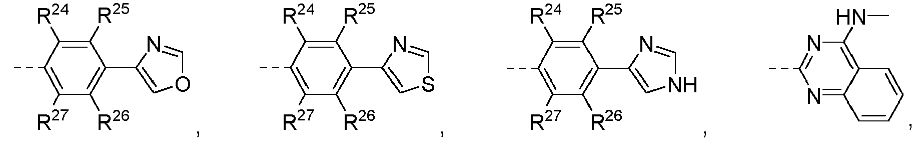 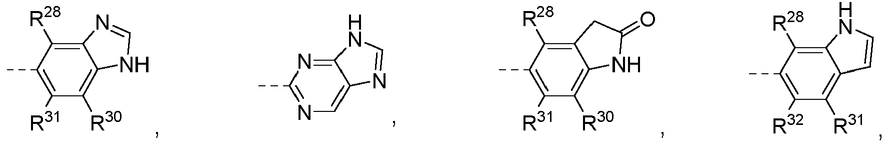 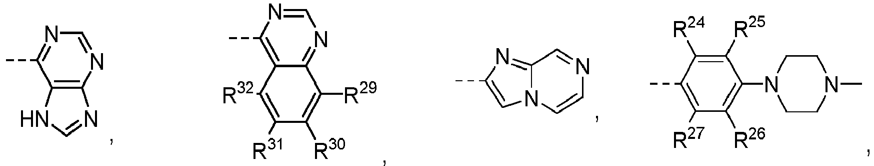 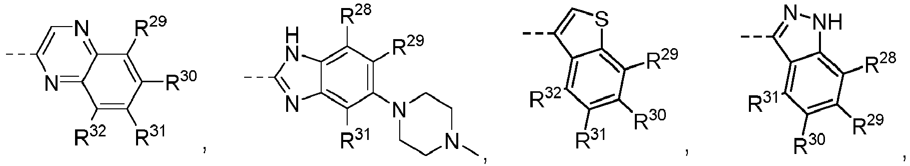 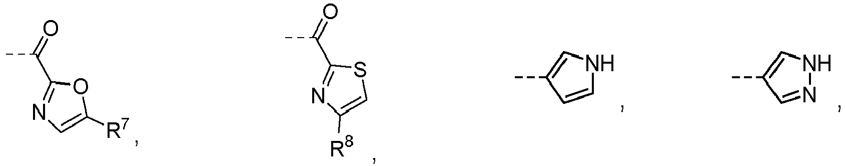 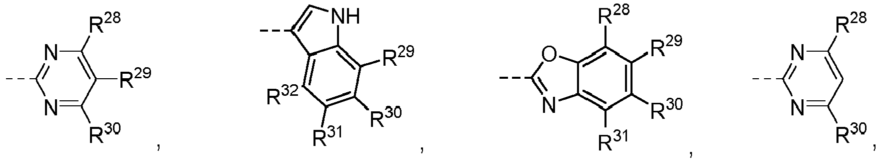 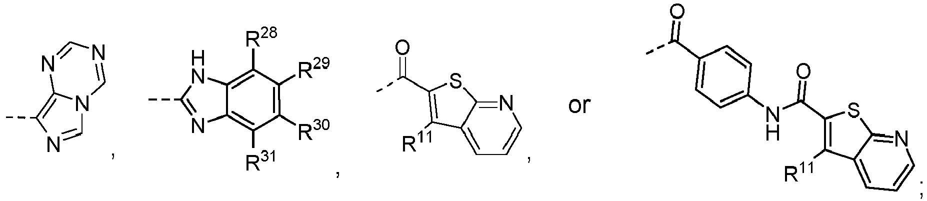 wherein **R⁷**, **R⁸**, **R¹¹** and **R**^{**24** -} **R³²** have the meanings as defined herein.

More preferably, in all formulae (**I-a**), (**I-b**), (**I-c**), and (**I-d**) as disclosed herein, **R¹** represents -CH₂CH₂CH₃, -CH₂CH=CH₂,

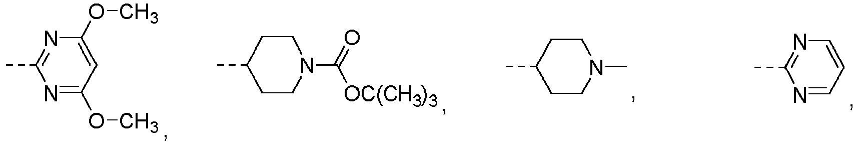

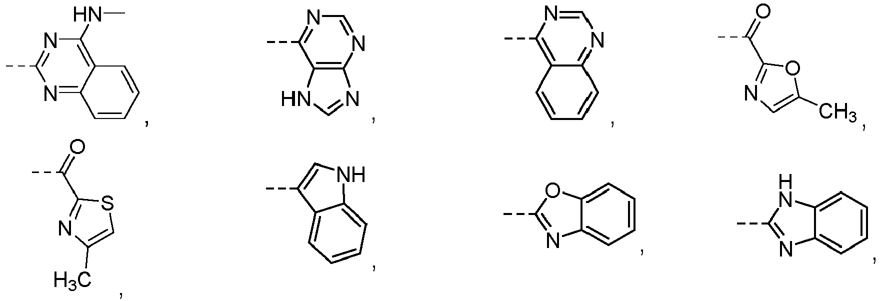

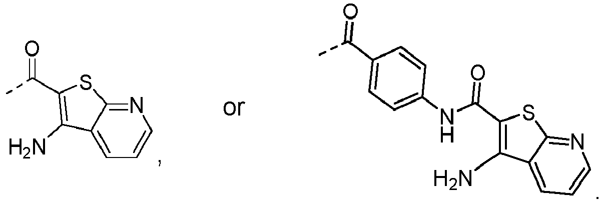

Preferably, in all formulae (**I-a**), (**I-b**), (**I-c**), and (**I-d**) as disclosed herein, **R²** represents -H, -CN, -N(CH₃)₂, 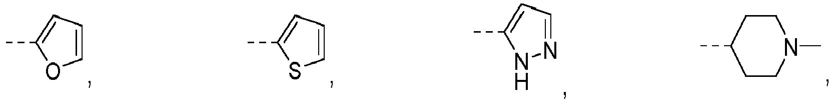 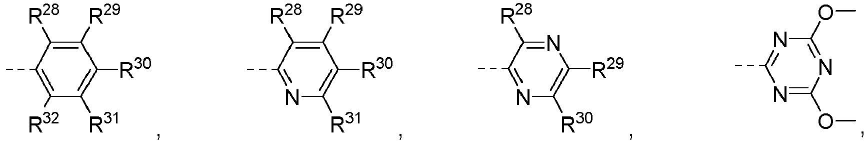 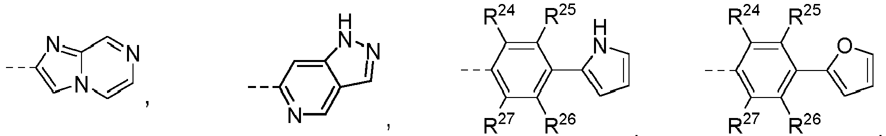 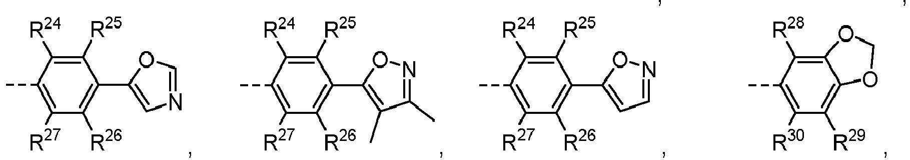 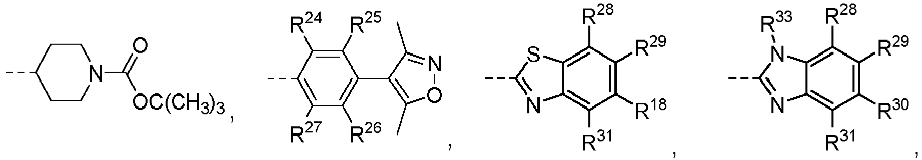 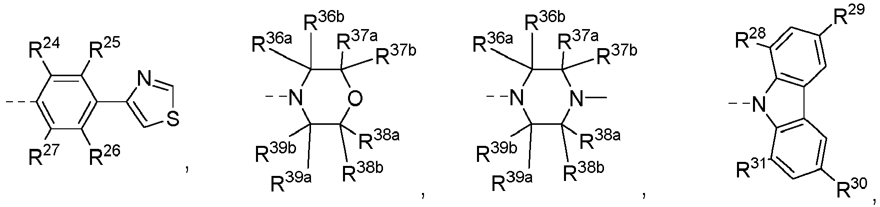 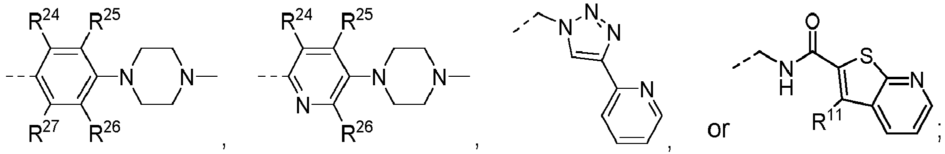 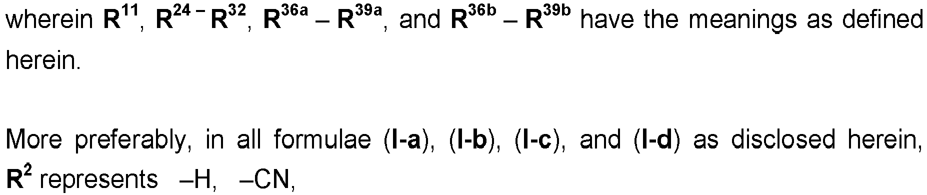 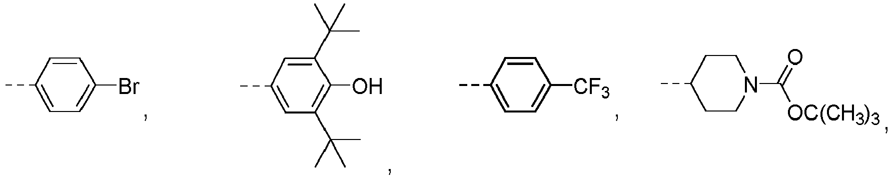 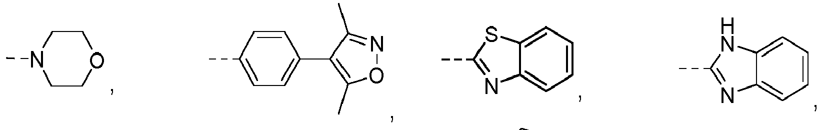 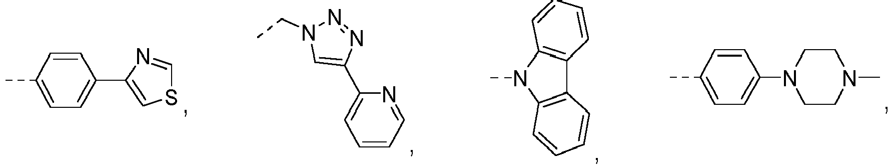 or

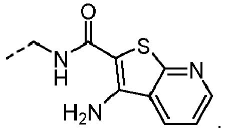

Preferably, in all formulae (**I-a**), (**I-b**), (**I-c**), and (**I-d**) as disclosed herein, **R³** represents 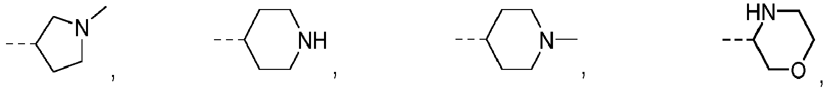 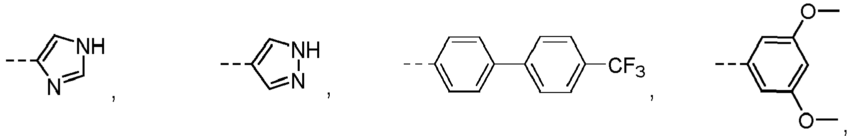 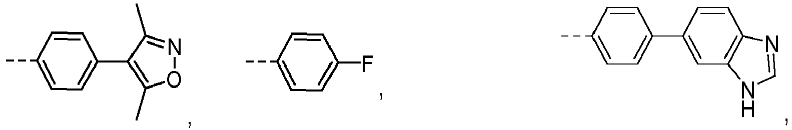 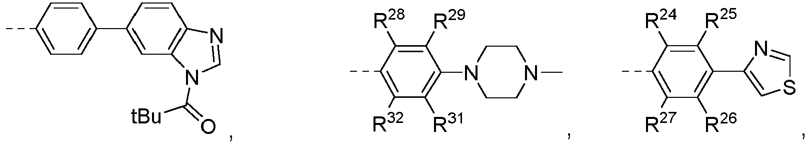 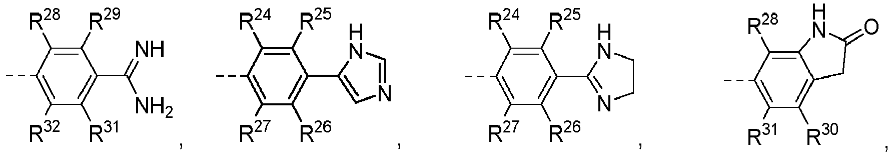 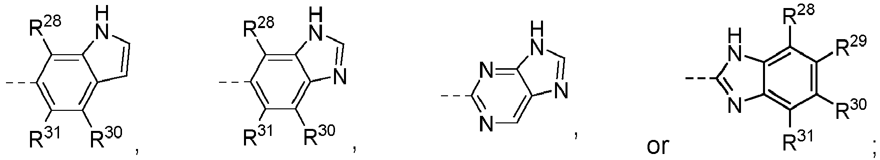 wherein **R**^{**24** -} **R³²** have the meanings as defined herein.

More preferably, in all formulae (**I-a**), (**I-b**), (**I-c**), and (**I-d**) as disclosed herein, **R³** represents 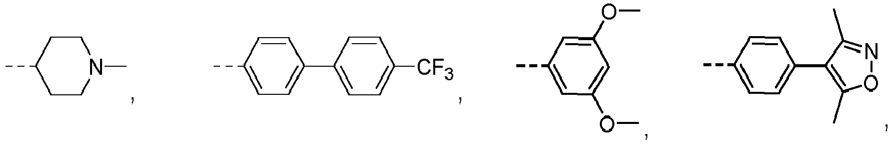 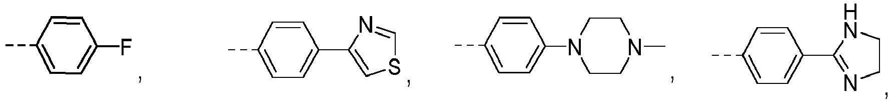 or

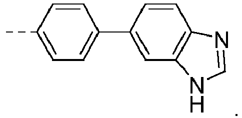

A further aspect of the present invention is directed to RNA-binding azetidine compounds the following formulae (**II-1**) to (**II-6**). 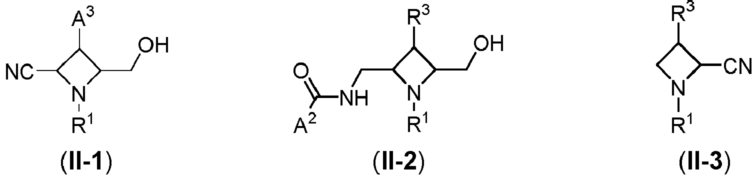 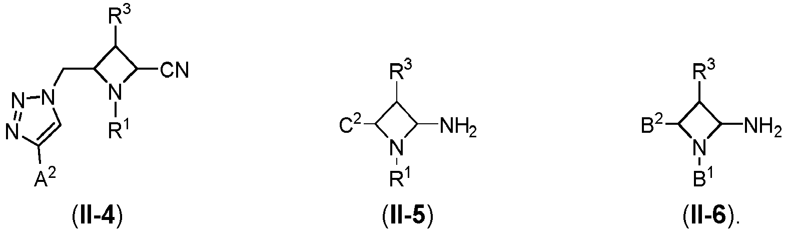 wherein **R¹**, **R³, A², A³, B¹** and **B²** have the meanings as defined herein.

Thus, in a preferred embodiment, the present invention is directed to a compound of formula (**II-1**), wherein **R¹** and **A³** have the meanings as defined herein.

Preferably, in formula (**II-1**), as disclosed herein, **R¹** represents -CH₂CH₂CH₃, -CH₂CH=CH₂, 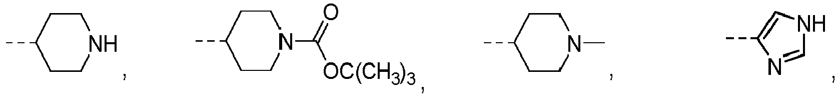 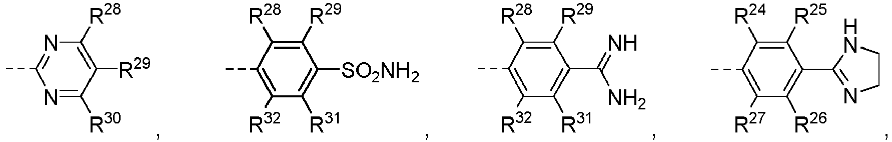 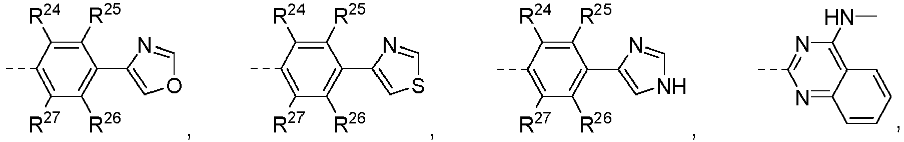 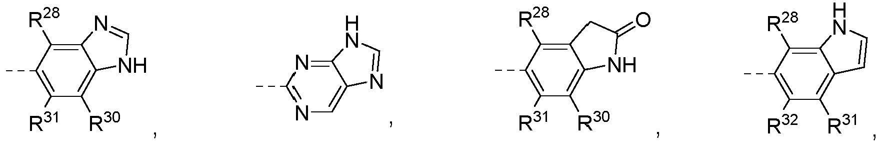 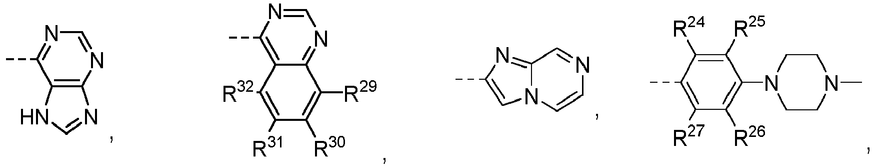 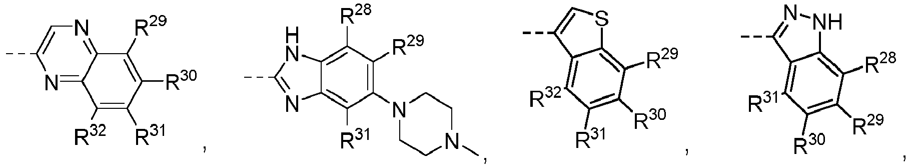 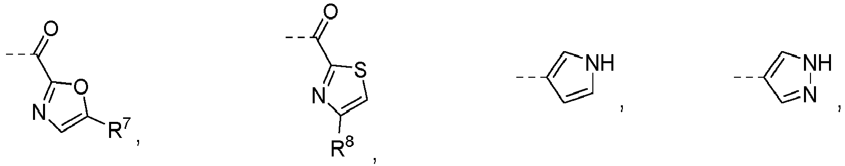 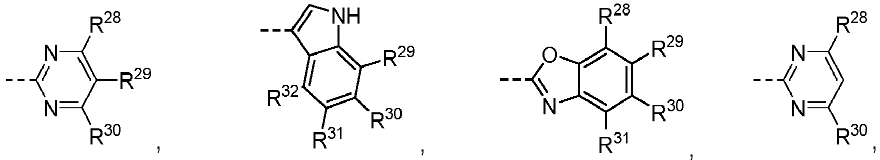 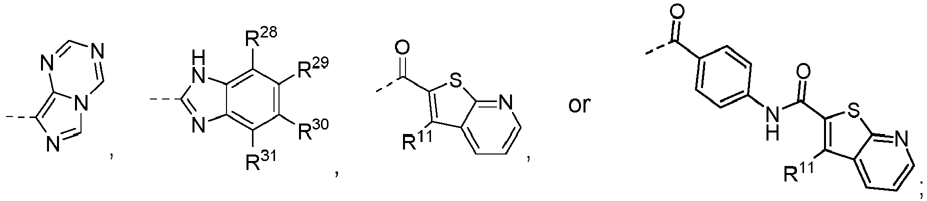 wherein **R⁷**, **R⁸**, **R¹¹** and **R**^{**24** -} **R³²** have the meanings as defined herein.

More preferably, in formula (**II-1**), as disclosed herein, **R¹** represents -CH₂CH₂CH₃, -CH₂CH=CH₂,

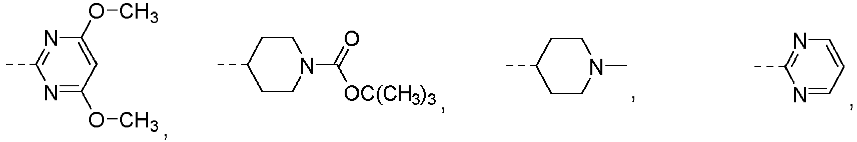

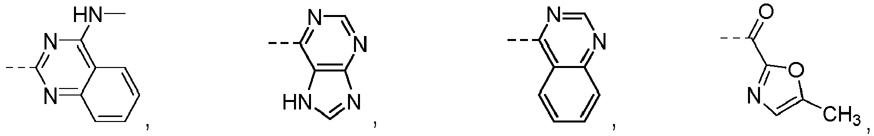

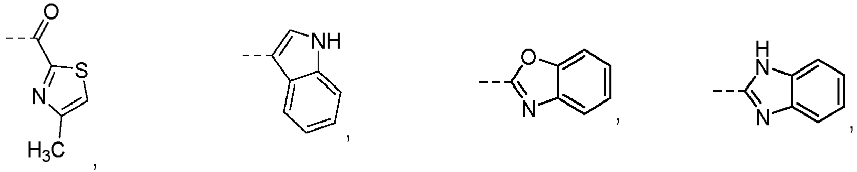

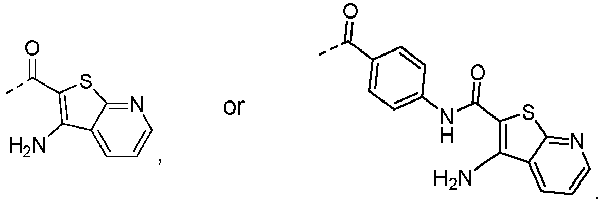

Preferably, in formula (**II-1**), as disclosed herein, **A³** represents 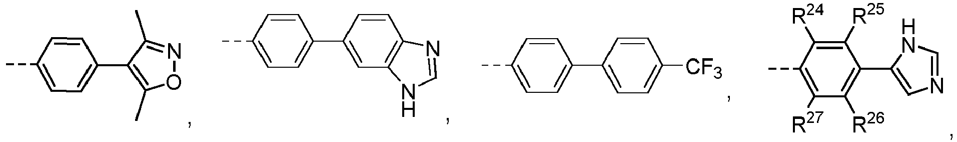 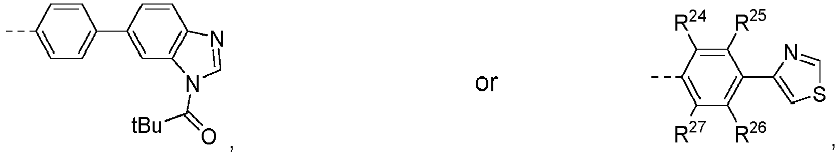 wherein **R**^{**24** -} **R³²** have the meanings as defined herein.

More preferably, in formulae (**II-1**) as disclosed herein, **A³** represents

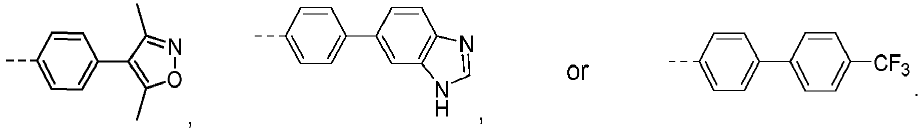

Thus, in a preferred embodiment, the present invention is directed to a compound of formula (**II-2**), 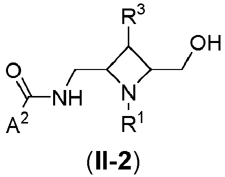 wherein **R¹**, **R³** and **A²** have the meanings as defined herein.

Preferably, in formula (**II-2**), as disclosed herein, **R¹** represents -CH₂CH₂CH₃, -CH₂CH=CH₂, 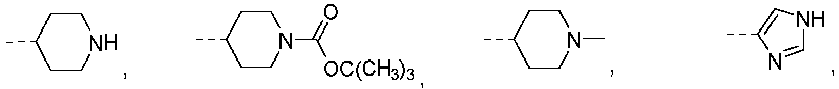 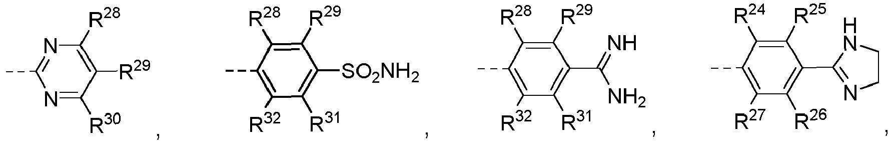 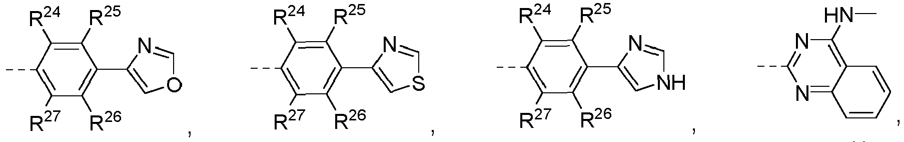 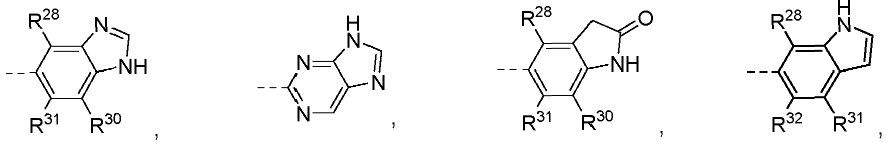 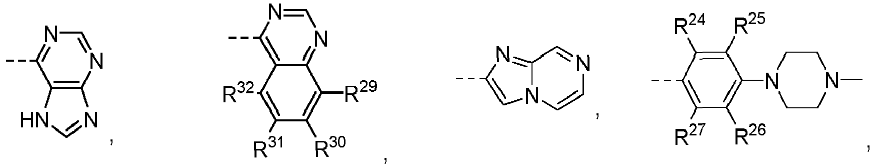 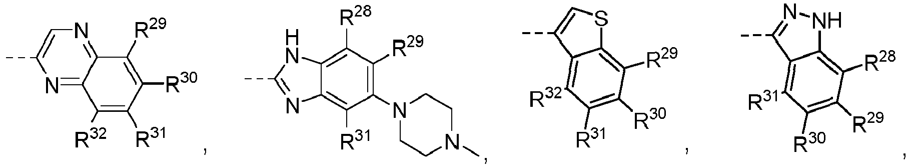 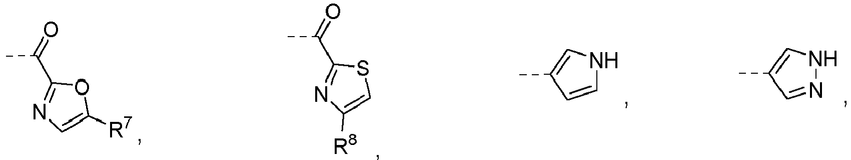 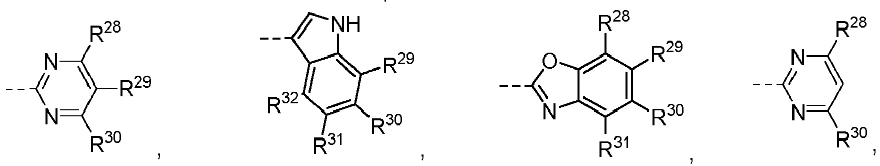 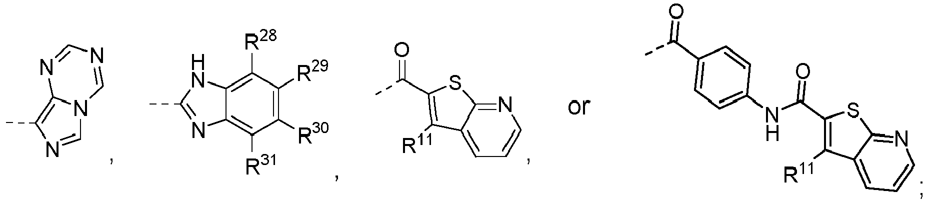 wherein **R⁷**, **R⁸**, **R¹¹** and **R^{24 -} R³²** have the meanings as defined herein.

More preferably, in formula (**II-2**), as disclosed herein, **R¹** represents -CH₂CH₂CH₃, -CH₂CH=CH₂,

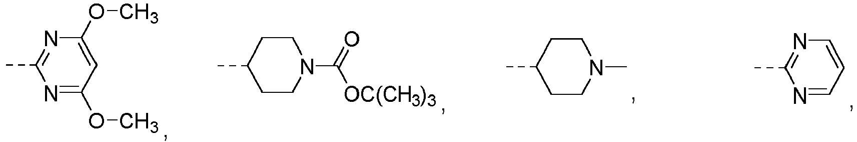

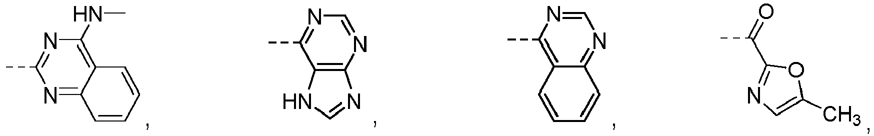

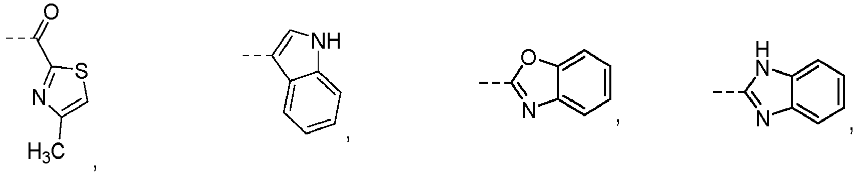

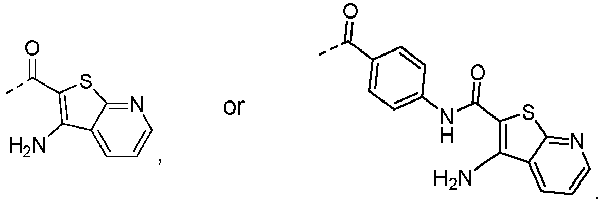

Preferably, in formula (**II-2**), as disclosed herein, **A²** represents 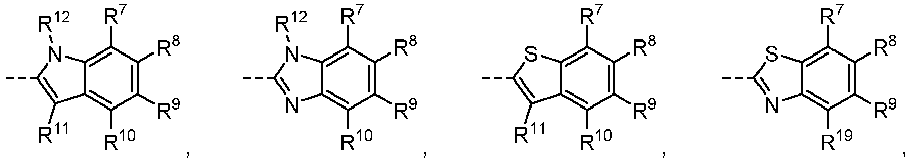 or 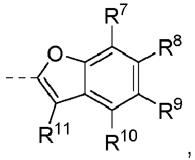 wherein **R⁷** - **R¹¹** have the meanings as defined herein.

More preferably, in formula (**II-2**), as disclosed herein, **A²** represents

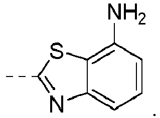

Preferably, in formula (**II-2**) as disclosed herein, **R³** represents 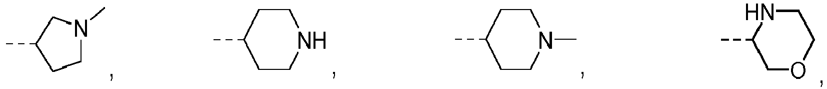 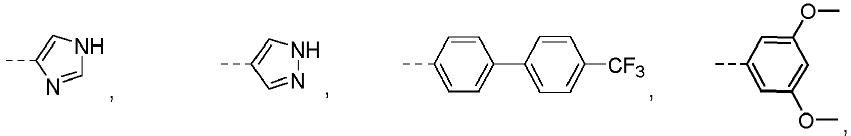 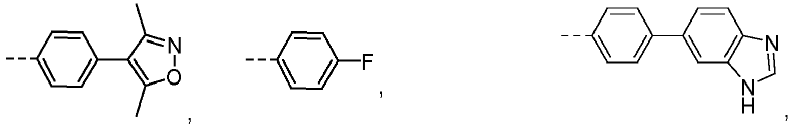 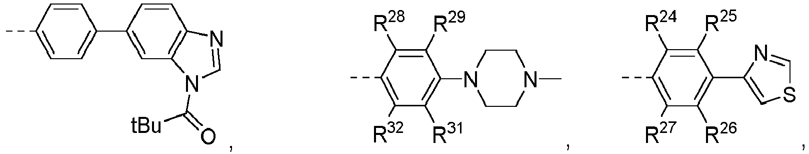 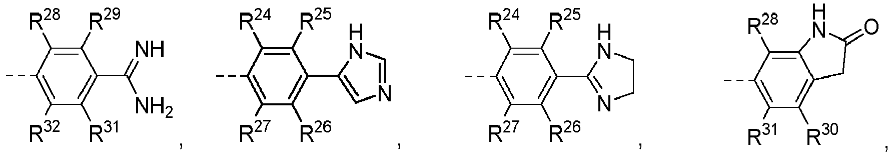 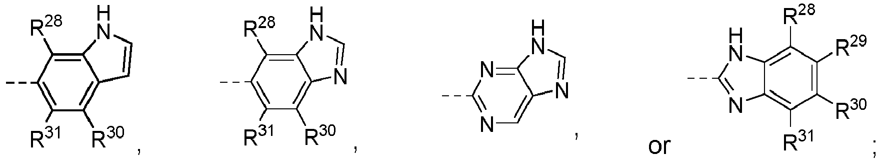 wherein **R**^{**24** -} **R³²** have the meanings as defined herein.

More preferably, in formula (**II-2**) as disclosed herein, **R³** represents 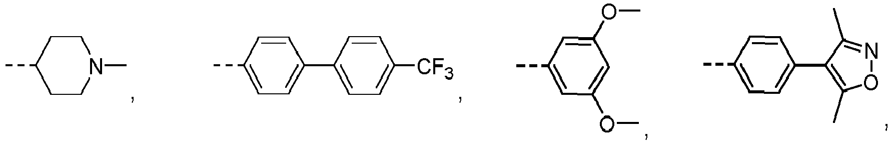 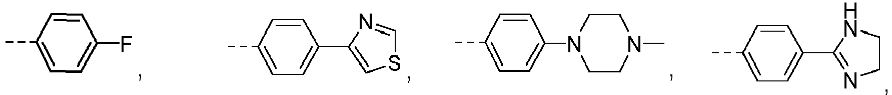 or

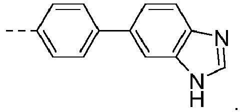

Thus, in a preferred embodiment, the present invention is directed to a compound of formula (**II-3**), 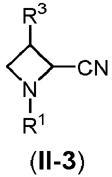 wherein **R¹** and **R³** have the meanings as defined herein.

Preferably, in formula (**II-3**), as disclosed herein, **R¹** represents -CH₂CH₂CH₃, -CH₂CH=CH₂, 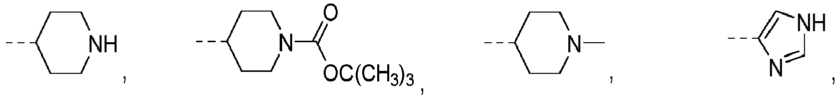 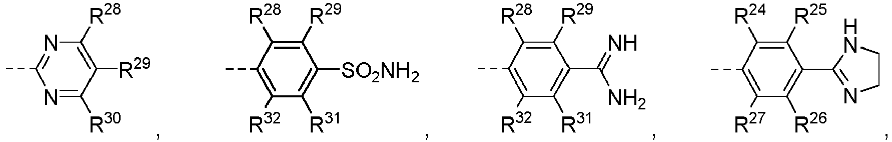 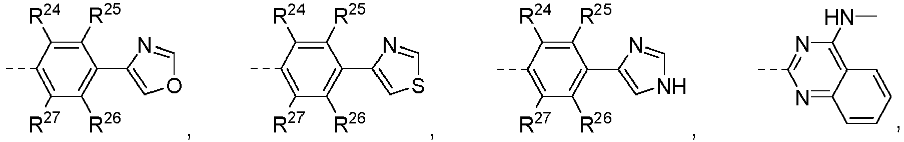 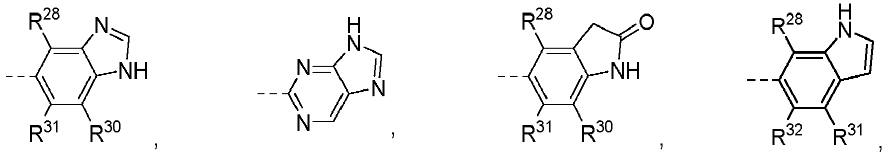 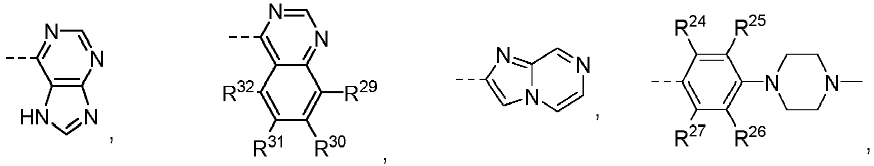 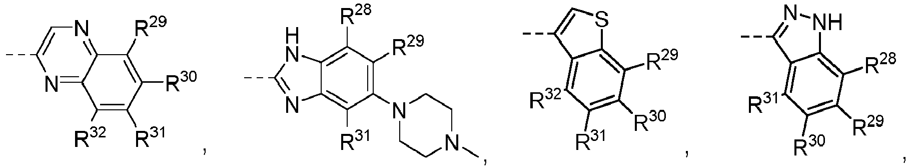 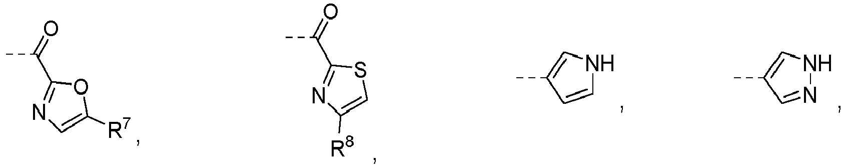 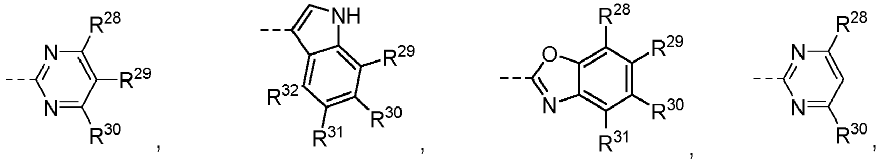 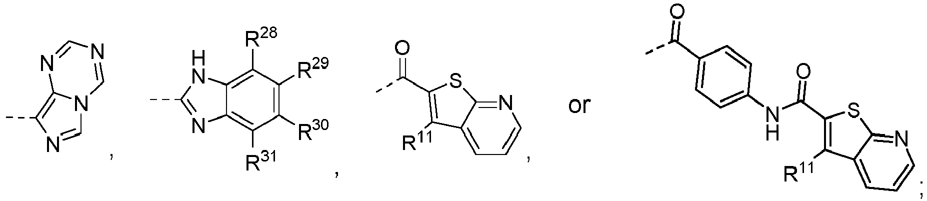 wherein **R⁷**, **R⁸**, **R¹¹** and **R**^{**24** -} **R³²** have the meanings as defined herein.

More preferably, in formula (**II-3**), as disclosed herein, **R¹** represents -CH₂CH₂CH₃, -CH₂CH=CH₂,

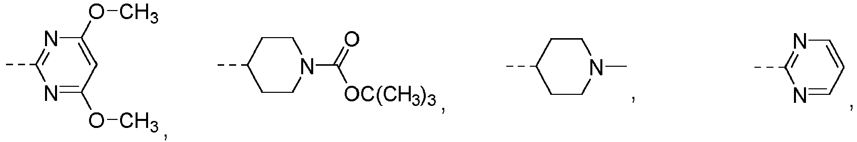

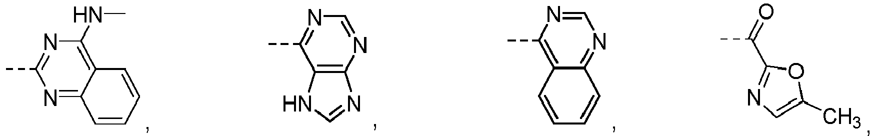

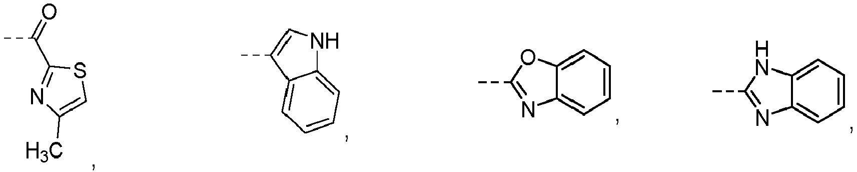

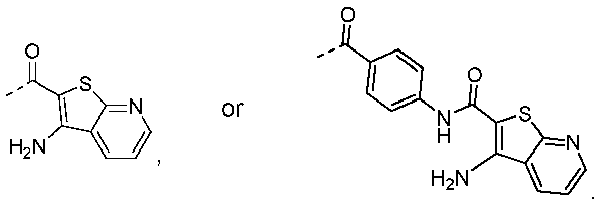

Preferably, in formula (**II-3**) as disclosed herein, **R³** represents 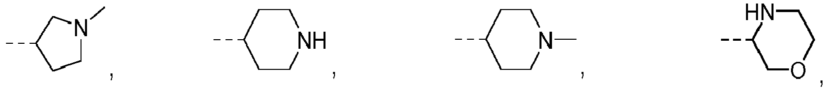 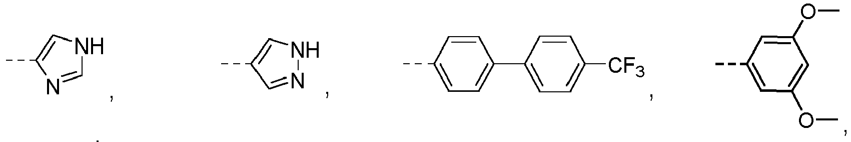 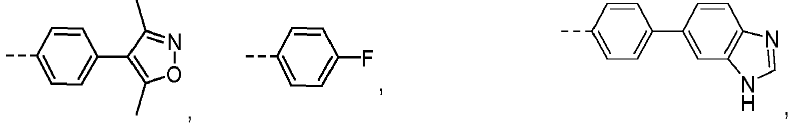 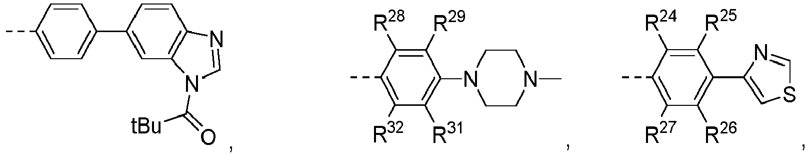 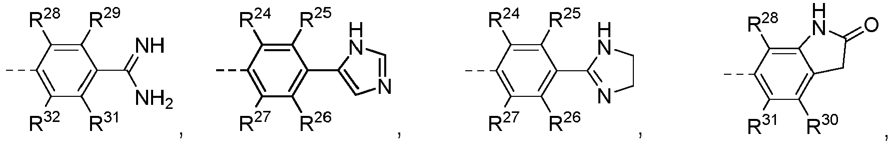 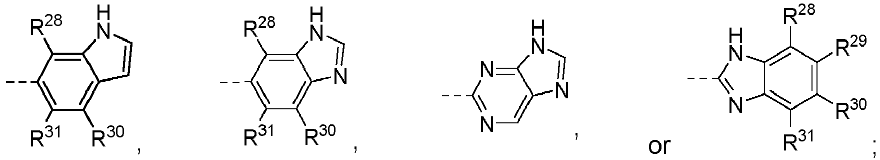 wherein **R**^{**24** -} **R³²** have the meanings as defined herein.

More preferably, in formula (**II-3**) as disclosed herein, **R³** represents 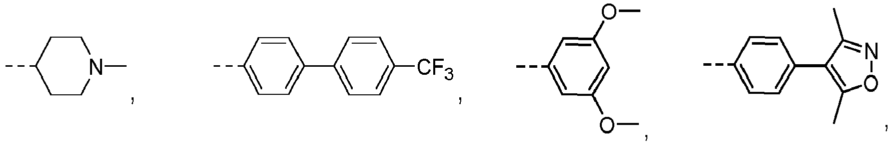 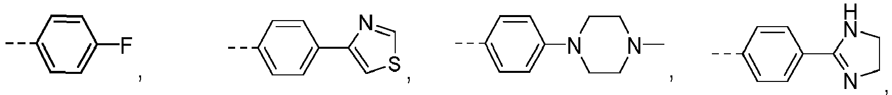 or

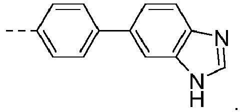

Thus, in a preferred embodiment, the present invention is directed to a compound of formula (**II-4**), 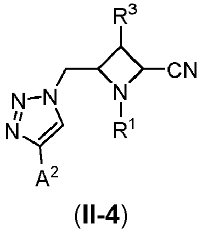 wherein **R¹**, **R³** and **A²** have the meanings as defined herein.

Preferably, in formula (**II-4**), as disclosed herein, **R¹** represents -CH₂CH₂CH₃, -CH₂CH=CH₂, 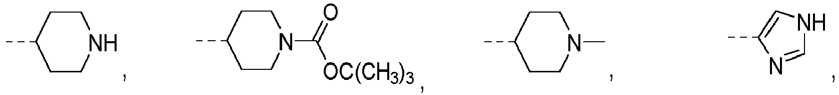 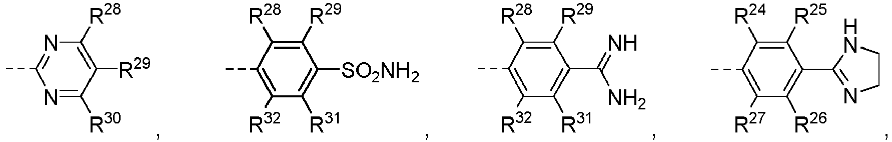 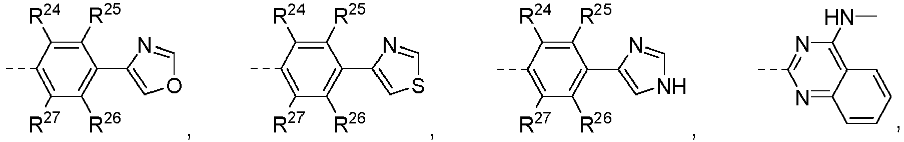 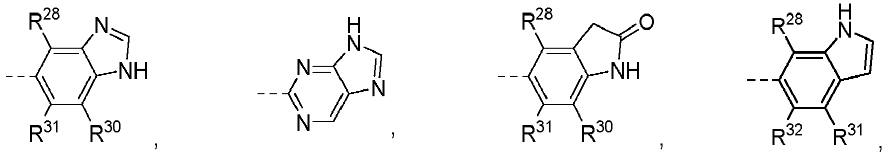 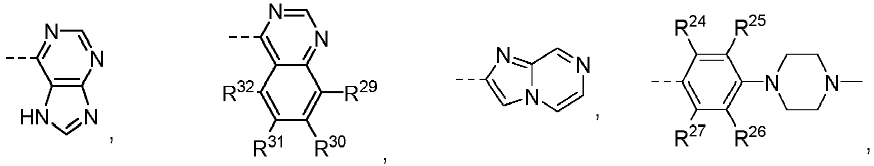 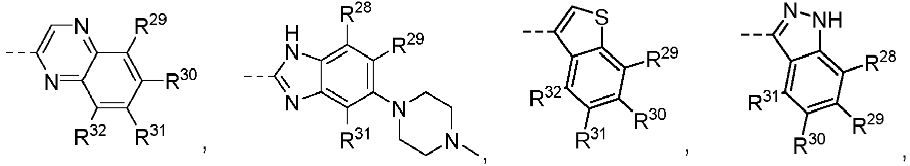 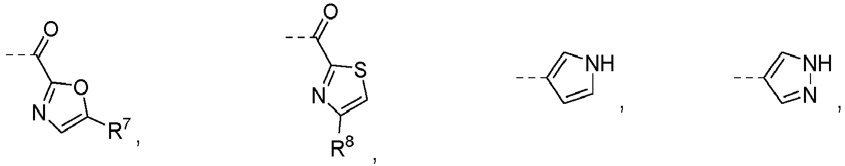 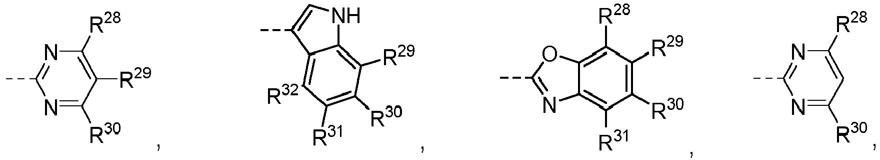 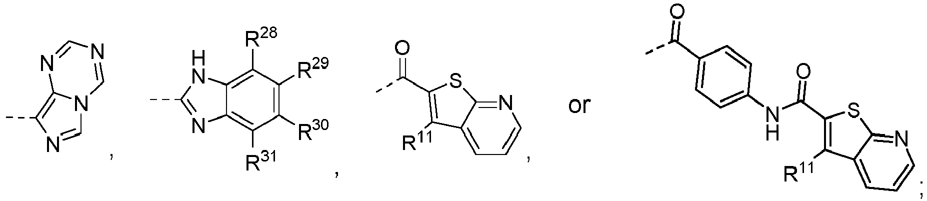 wherein **R⁷**, **R⁸**, **R¹¹** and **R**^{**24** -} **R³²** have the meanings as defined herein.

More preferably, in formula (**II-4**), as disclosed herein, **R¹** represents -CH₂CH₂CH₃, -CH₂CH=CH₂,

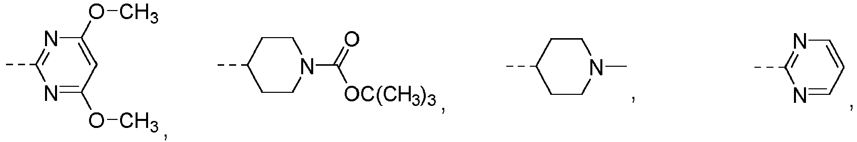

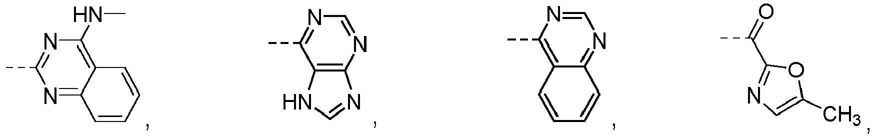

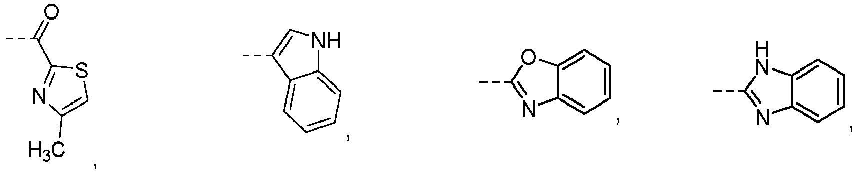

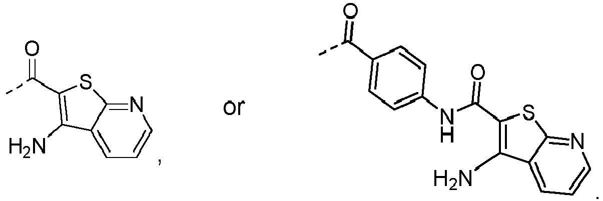

Preferably, in formula (**II-4**), as disclosed herein, **A²** represents 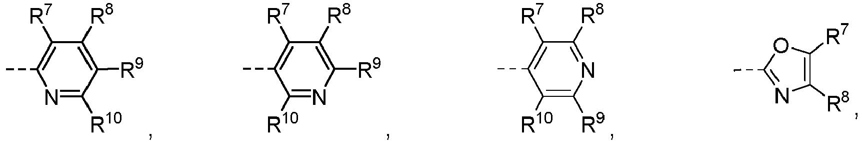 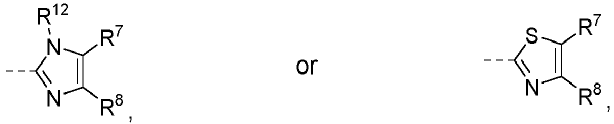 wherein **R⁷** - **R¹⁰** have the meanings as defined herein.

More preferably, in formula (**II-4**), as disclosed herein, **A²** represents

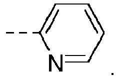

Preferably, in formula (**II-4**) as disclosed herein, **R³** represents 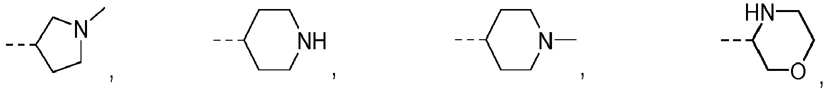 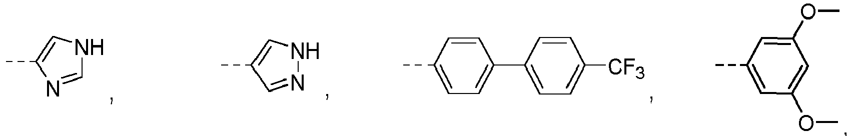 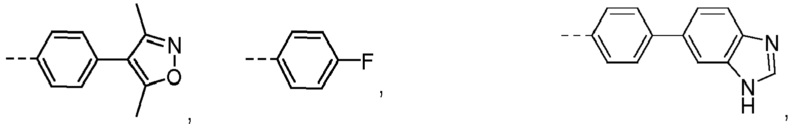 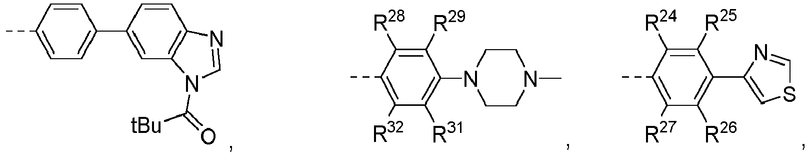 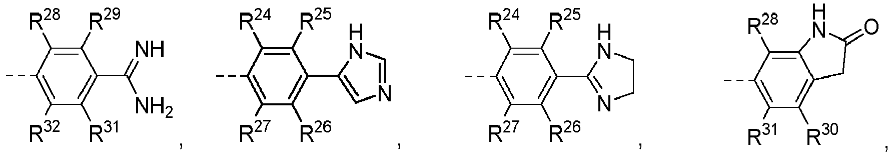 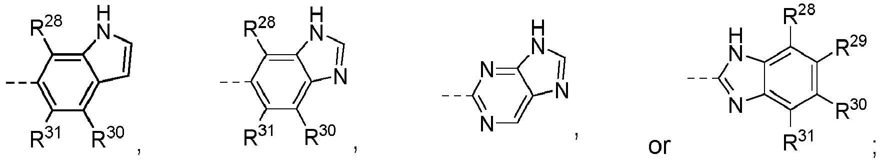 wherein **R**^{**24** -} **R³²** have the meanings as defined herein.

More preferably, in formula (**II-4**) as disclosed herein, **R³** represents 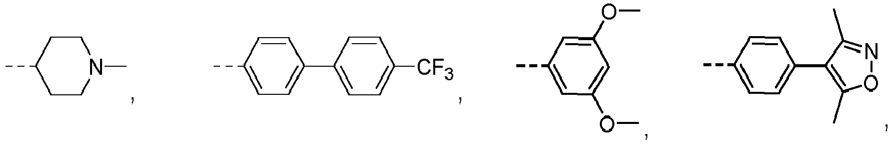 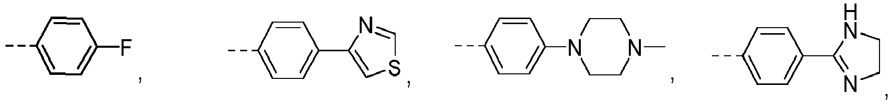 or

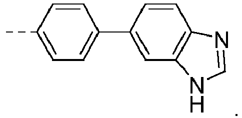

Thus, in a preferred embodiment, the present invention is directed to a compound of formula (**II-5**), 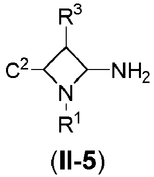 wherein **R¹**, **R³,** and **C²** have the meanings as defined herein.

Preferably, in formula (**II-5**), as disclosed herein, **R¹** represents -CH₂CH₂CH₃, -CH₂CH=CH₂, 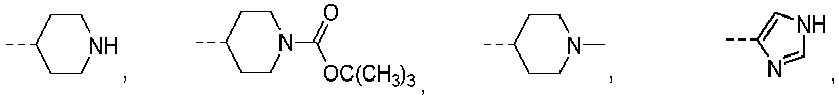 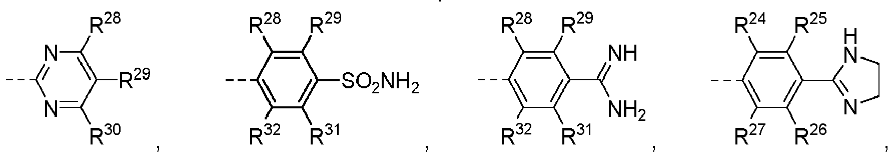 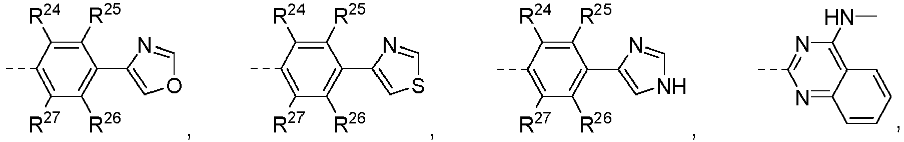 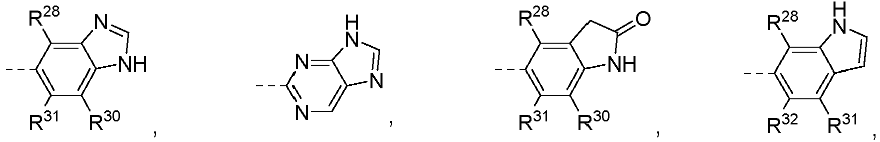 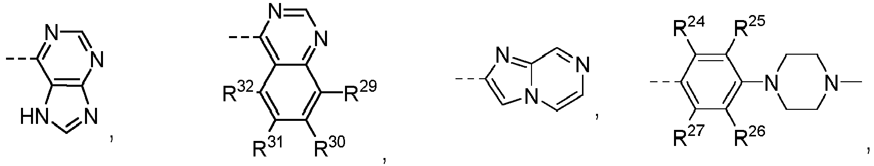 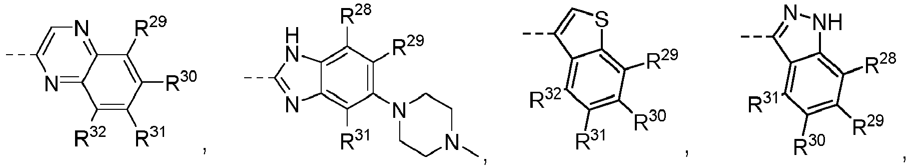 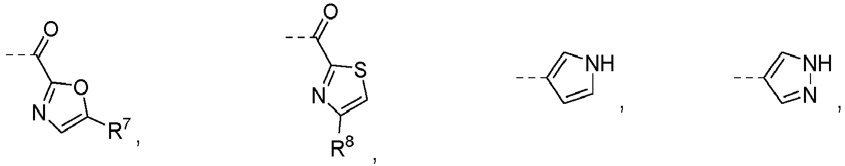 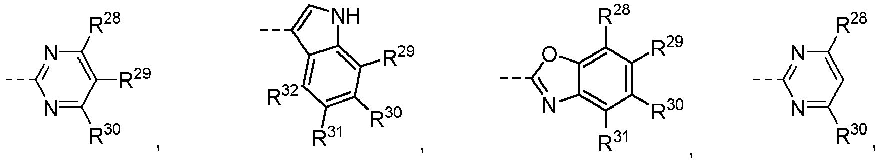 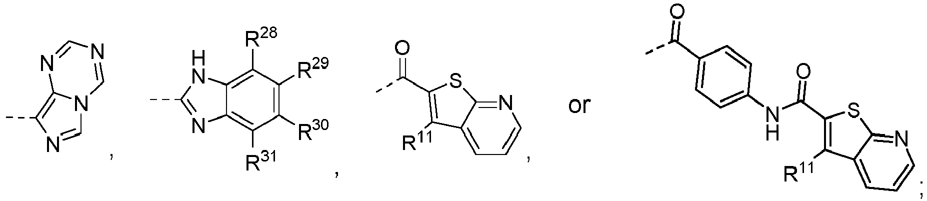 wherein **R⁷**, **R⁸**, **R¹¹** and **R**^{**24** -} **R³²** have the meanings as defined herein.

More preferably, in formula (**II-5**), as disclosed herein, **R¹** represents -CH₂CH₂CH₃, -CH₂CH=CH₂,

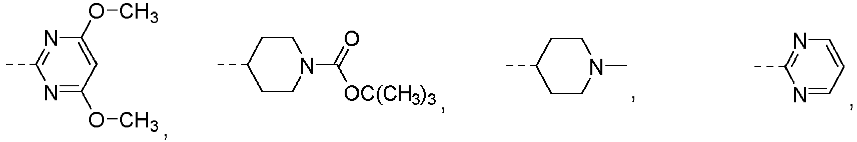

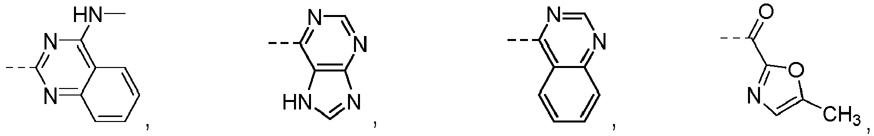

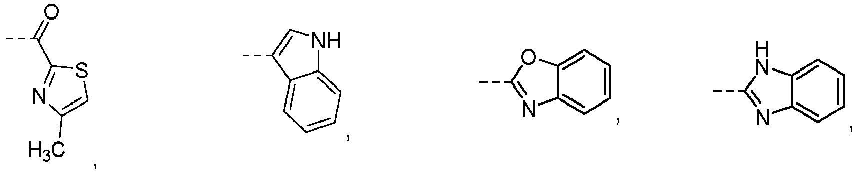

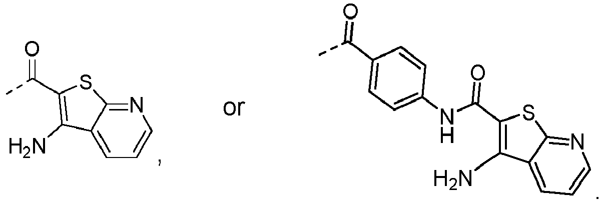

Preferably, in formula (**II-5**) as disclosed herein, **R³** represents 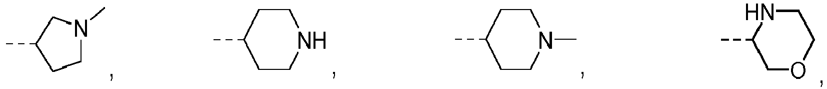 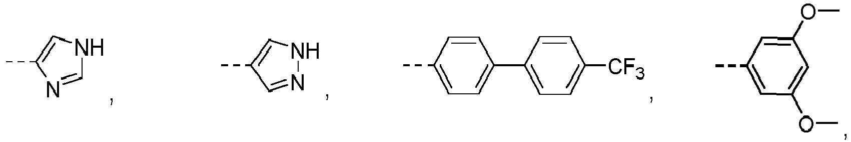 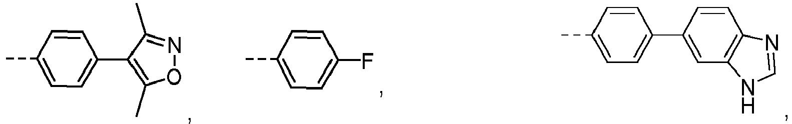 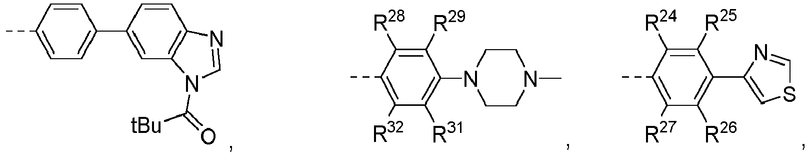 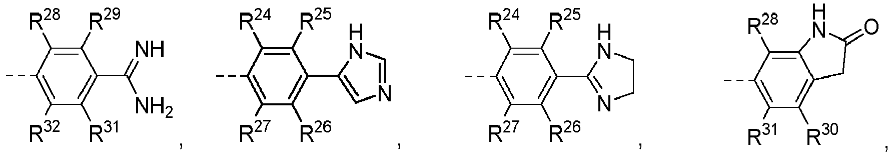 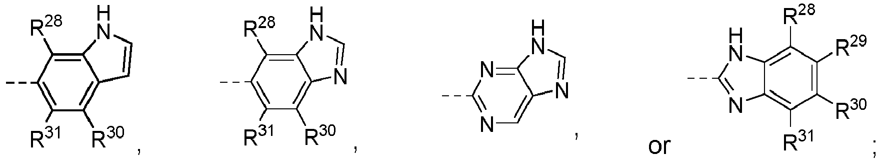 wherein **R**^{**24** -} **R³²** have the meanings as defined herein.

More preferably, in formula (**II-5**) as disclosed herein, **R³** represents 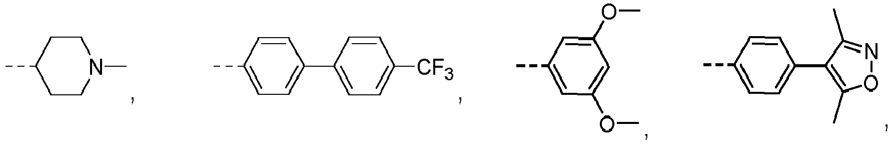 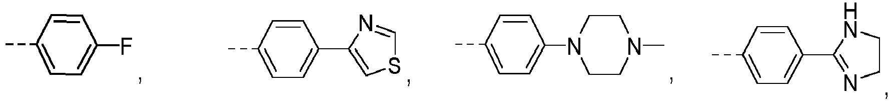 or

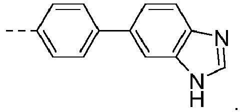

Preferably, in formula (**II-5**) as disclosed herein, **C²** represents 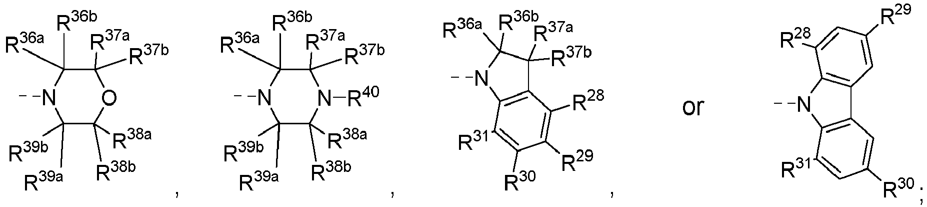 wherein **R²⁸-R³²**, **R^{36a}** - **R^{39a}**, and **R^{36b}** - **R^{39b}** have the meanings as defined herein.

More preferably, in formula (**II-5**) as disclosed herein **C²** represents

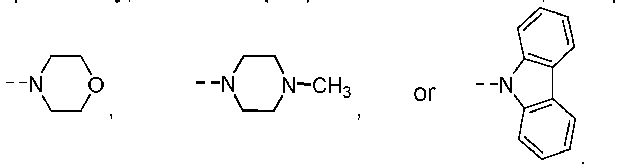

Thus, in a preferred embodiment, the present invention is directed to a compound of formula (11-6), 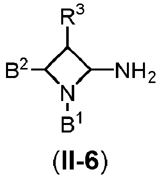 wherein **R³, B¹** and **B²** have the meanings as defined herein.

Preferably, in formula (**II-6**), as disclosed herein, **B¹** represents 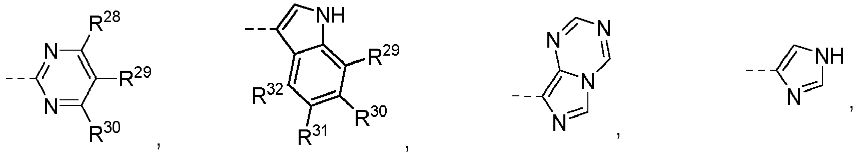 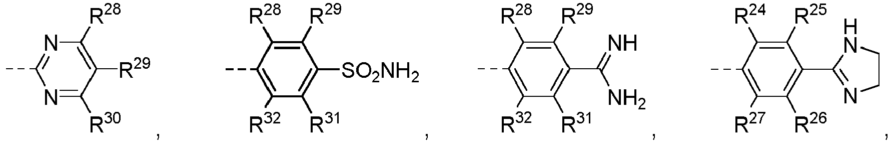 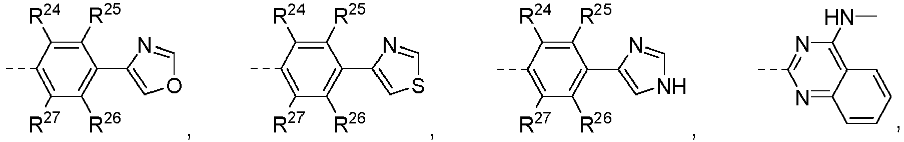 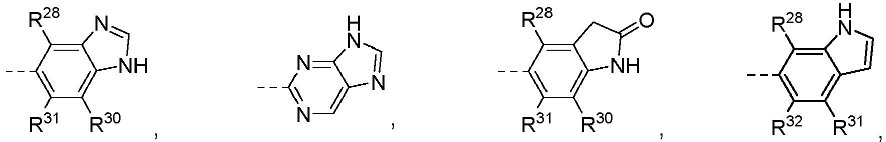 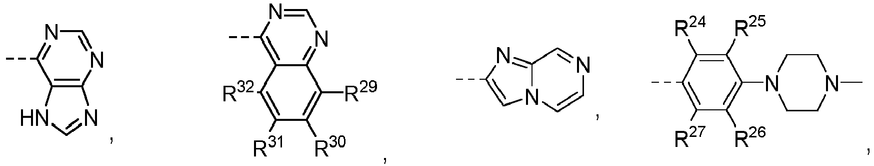 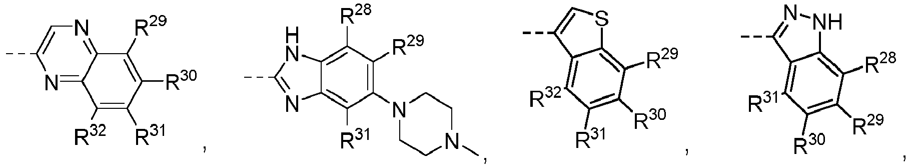 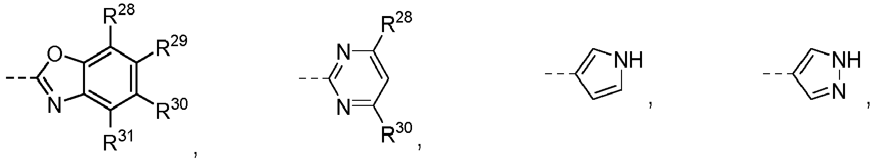 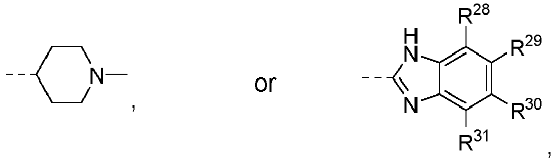 wherein **R**^{**24** -} **R³²** have the meanings as defined herein.

More preferably, in formula (11-6), as disclosed herein, **B¹** represents 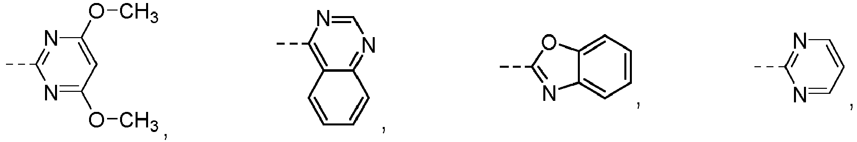 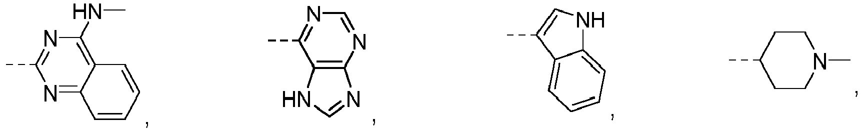 or

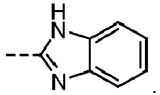

Preferably, in formula (**II-6)** as disclosed herein, **B²** represents 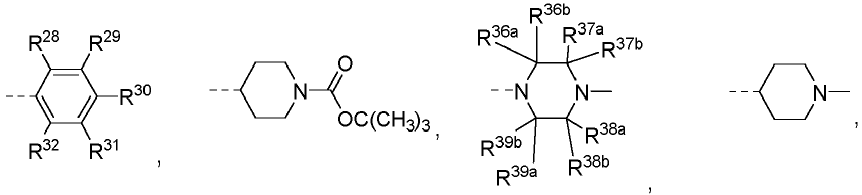 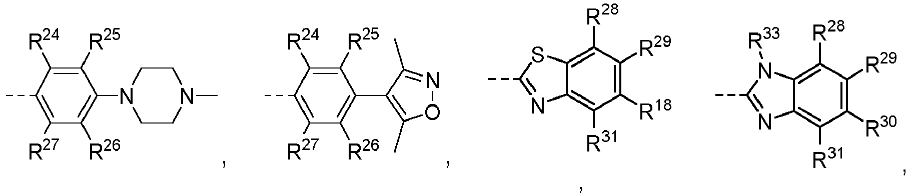 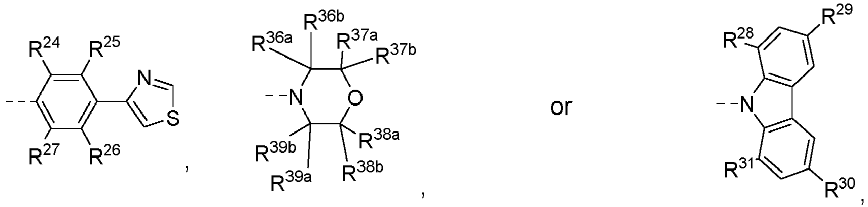 wherein **R**^{**24**-}**R³²**, **R^{36a} - R^{39a}**, and **R^{36b}** - **R^{39b}** have the meanings as defined herein.

More preferably, in formula (**II-6)** as disclosed herein, **B²** represents

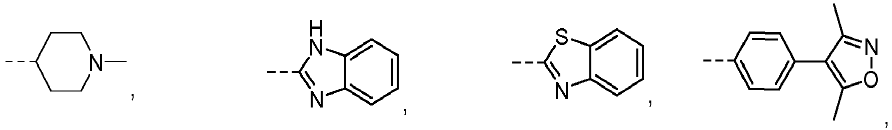

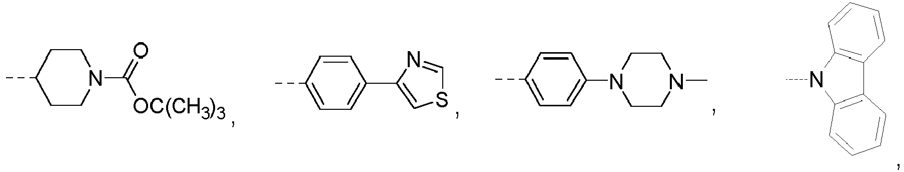

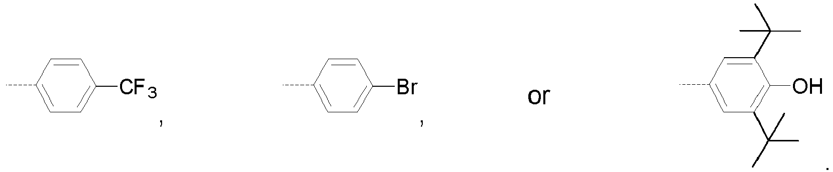

Preferably, in formula (**II-6)** as disclosed herein, **B³** represents 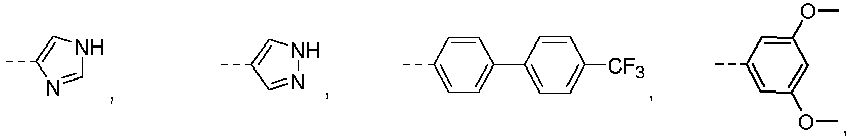 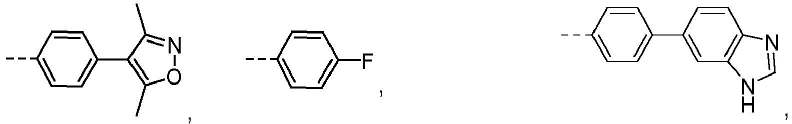 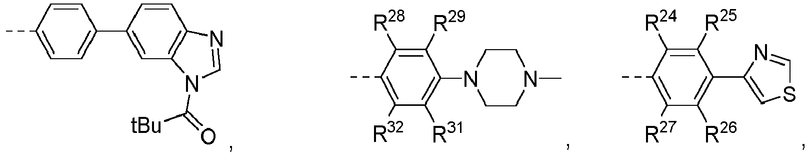 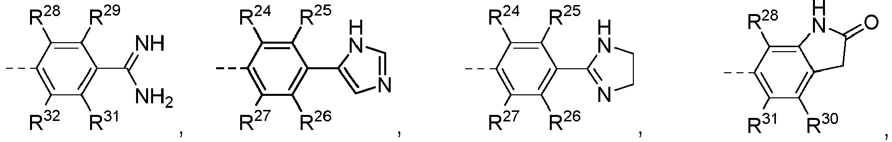 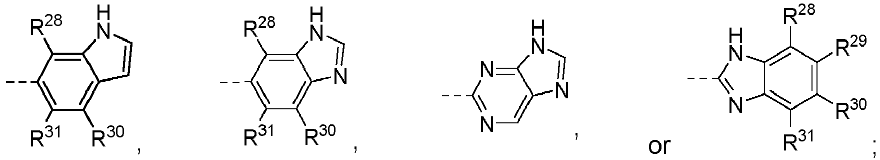 wherein **R^{24 -} R³²** have the meanings as defined herein.

More preferably, in formula (**II-6)** as disclosed herein, **B³** represents 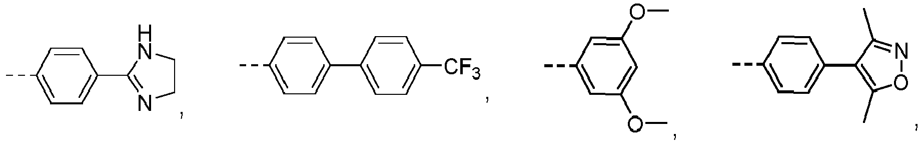 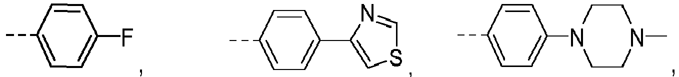 or

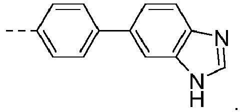

Preferably, the inventive compounds are selected from the following list
1-(3-aminothieno[2,3-b] pyridine-2-carbonyl)-4-(hydroxymethyl)-3-(4'-(trifluoromethyl)-[1,1'-biphenyl]-4-yl) azetidine-2-carbonitrile;
1-(3-aminothieno[2,3-b]pyridine-2-carbonyl)-4-(hydroxymethyl)-3-(4'-(trifluoromethyl)-[1,1 '-biphenyl]-4-yl)azetidine-2-carbonitrile;
1-(3-aminothieno[2,3-b]pyridine-2-carbonyl)-4-(hydroxymethyl)-3-(4'-(trifluoromethyl)-[1,1 '-biphenyl]-4-yl)azetidine-2-carbonitrile;
3-amino-N-((4-(hydroxymethyl)-1-propyl-3-(4'-(trifluoromethyl)-[1,1'-biphenyl]-4-yl)azetidin-2-yl)methyl)thieno[2,3-b]pyridine-2-carboxamide;
3-amino-N-((4-(hydroxymethyl)-1-propyl-3-(4'-(trifluoromethyl)-[1,1'-biphenyl]-4-yl)azetidin-2-yl)methyl)thieno[2,3-b]pyridine-2-carboxamide;
1-allyl-4-((4-(pyridin-2-yl)-1H-1,2,3-triazol-1-yl)methyl)-3-(4'-(trifluoromethyl)-[1,1'-biphenyl]-4-yl)azetidine-2-carbonitrile;
3-(4-(1H-benzo[d]imidazol-6-yl)phenyl)-1-(3-aminothieno[2,3-b]pyridine-2-carbonyl)-4-(hydroxymethyl)azetidine-2-carbonitrile;
N-((3-(4-(1H-benzo[d]imidazol-6-yl)phenyl)-4-(hydroxymethyl)-1-propylazetidin-2-yl)methyl)-3-aminothieno[2,3-b]pyridine-2-carboxamide;
N-((3-(4-(1H-benzo[d]imidazol-6-yl)phenyl)-4-(hydroxymethyl)-1-propylazetidin-2-yl)methyl)-3-aminothieno[2,3-b]pyridine-2-carboxamide;
1-allyl-4-((4-(pyridin-2-yl)-1H-1,2,3-triazol-1-yl)methyl)-3-(4'-(trifluoromethyl)-[1,1'-biphenyl]-4-yl)azetidine-2-carbonitrile;
4-(hydroxymethyl)-1-(4-methylthiazole-2-carbonyl)-3-(4'-(trifluoromethyl)-[1,1'-biphenyl]-4-yl)azetidine-2-carbonitrile;
4-(hydroxymethyl)-1-(5-methyloxazole-2-carbonyl)-3-(4'-(trifluoromethyl)-[1,1'-biphenyl]-4-yl)azetidine-2-carbonitrile;
3-(4-(3,5-dimethylisoxazol-4-yl)phenyl)-1-(4-(methylamino)quinazolin-2-yl)azetidine-2-carbonitrile;
3-(4-(3,5-dimethylisoxazol-4-yl)phenyl)-1-(4-(methylamino)quinazolin-2-yl)azetidine-2-carbonitrile;
1-(3-aminothieno[2,3-b]pyridine-2-carbonyl)-3-(4-(3,5-dimethylisoxazol-4-yl)phenyl)azetidine-2-carbonitrile;
1-(3-aminothieno[2,3-b]pyridine-2-carbonyl)-3-(4-(3,5-dimethylisoxazol-4-yl)phenyl)azetidine-2-carbonitrile;
1-(4-(methylamino)quinazolin-2-yl)-3-(4'-(trifluoromethyl)-[1,1'-biphenyl]-4-yl)azetidine-2-carbonitrile;
1-(4-(methylamino)quinazolin-2-yl)-3-(4'-(trifluoromethyl)-[1,1'-biphenyl]-4-yl)azetidine-2-carbonitrile;
1-(3-aminothieno[2,3-b]pyridine-2-carbonyl)-3-(4'-(trifluoromethyl)-[1,1'-biphenyl]-4-yl)azetidine-2-carbonitrile;
1-(3-aminothieno[2,3-b]pyridine-2-carbonyl)-3-(4'-(trifluoromethyl)-[1,1'-biphenyl]-4-yl)azetidine-2-carbonitrile;
3-(4-(1H-benzo[d]imidazol-6-yl)phenyl)-1-(4-(methylamino)quinazolin-2-yl)azetidine-2-carbonitrile;
3-(4-(1H-benzo[d]imidazol-6-yl)phenyl)-1-(4-(methylamino)quinazolin-2-yl)azetidine-2-carbonitrile;
tert-butyl 4-(2-amino-4-(4-bromophenyl)-3-(4-(4-methylpiperazin-1-yl)phenyl)azetidin-1-yl)piperidine-1-carboxylate;
tert-butyl 4-(2-amino-4-(3,5-di-tert-butyl-4-hydroxyphenyl)-3-(4-fluorophenyl)azetidin-1-yl)piperidine-1-carboxylate;
di-tert-butyl 4,4'-(4-amino-3-(4-fluorophenyl)azetidine-1,2-diyl)bis(piperidine-1-carboxylate);
di-tert-butyl 4,4'-(4-amino-3-(4-(4-methylpiperazin-1-yl)phenyl)azetidine-1,2-diyl)bis(piperidine-1-carboxylate);
tert-butyl 4-(2-amino-4-(4-bromophenyl)-3-(4-fluorophenyl)azetidin-1-yl)piperidine-1-carboxylate;
tert-butyl 4-(2-amino-4-(4-(3,5-dimethylisoxazol-4-yl)phenyl)-3-(4-fluorophenyl)-azetidin-1-yl)piperidine-1-carboxylate;
4-(benzo[d]thiazol-2-yl)-3-(1-methylpiperidin-4-yl)-1-(quinazolin-4-yl)azetidin-2 amine;
4-(benzo[d]thiazol-2-yl)-3-(1-methylpiperidin-4-yl)-1-(pyrimidin-2-yl)azetidin-2-amine;
4-(1H-benzo[d]imidazol-2-yl)-1,3-bis(1-methylpiperidin-4-yl)azetidin-2-amine;
4-(1H-benzo[d]imidazol-2-yl)-3-(3,5-dimethoxyphenyl)-1-(1H-indol-3-yl)azetidin-2-amine;
3-(1-methylpiperidin-4-yl)-1-(9H-purin-6-yl)-4-(4-(trifluoromethyl)phenyl)azetidin-2-amine;
3-(3,5-dimethoxyphenyl)-1-(1-methylpiperidin-4-yl)-4-(4-(thiazol-4-yl)phenyl)azetidin-2-amine;
1-(benzo[d]oxazol-2-yl)-3-(4-(4,5-dihydro-1H-imidazol-2-yl)phenyl)-4-(4-(4-methylpiperazin-1-yl)phenyl)azetidin-2-amine;
3-(3,5-dimethoxyphenyl)-1-(1-methylpiperidin-4-yl)-4-morpholinoazetidin-2-amine;
1-(4,6-dimethoxypyrimidin-2-yl)-4-(4-(4-methylpiperazin-1-yl)phenyl)-3-(4-(thiazol-4-yl)phenyl)azetidin-2-amine;
4-(9H-carbazol-9-yl)-1-(1-methylpiperidin-4-yl)-3-(4-(thiazol-4-yl)phenyl)azetidin-2-amine;
1-(1H-benzo[d]imidazol-2-yl)-3-(4-(3,5-dimethylisoxazol-4-yl)phenyl)-4-(4-methylpiperazin-1-yl)azetidin-2-amine;
3-(3,5-dimethoxyphenyl)-4-(4-(3,5-dimethylisoxazol-4-yl)phenyl)-1-(1-methylpiperidin-4-yl)azetidin-2-amine;
4-(benzo[d]thiazol-2-yl)-3-(3,5-dimethoxyphenyl)-1-(1-methylpiperidin-4-yl)azetidin-2-amine.

The compounds of the general formulae **(I),** (**I-a**), (**I-b**), (**I-c**), (**I-d**), (**II-1)**, (**II-2)**, (**II-3**), (**II-4**), (**II-5**), and (**II-6**) may exist in the form of optical isomers, i.e. diastereomers, enantiomers and mixtures of said isomers in all ratios, e.g. racemic mixtures. The invention includes all such forms, in particular the pure diastereomeric forms or enantiomeric forms. The different isomeric forms may be separated or resolved one from the other by conventional methods, or any given isomer may be obtained by conventional synthetic methods or by stereospecific or asymmetric syntheses.

### Medical use

Another aspect of the present invention relates to the use of the inventive RNA-binding azetidine compounds of general formulae **(I),** (**I-a**), (**I-b**), (**I-c**), (**I-d**), (**II-1)**, (**II-2)**, (**II-3**), (**II-4**), (**II-5**), and (**II-6**) as drugs, i.e. as pharmaceutically active agents applicable in medicine.

As shown by melting temperature curves determined by differential scanning fluorimetry (DSF), the compounds of general formulae (**I-a**), (**I-b**), (**I-c**), (**I-d**), (**II-1)**, (**II-2)**, (**II-3**), (**II-4**), (**II-5**), and (**II-6**) show a high ability for binding to microRNA comprising a stem loop (hairpin) structural motif. Surprisingly, it turned out that the compounds according to the general formulae (**I-a**), (**I-b**), (**I-c**), (**I-d**), (**II-1)**, (**II-2)**, (**II-3**), (**II-4**), (**II-5**), and (**II-6**) as well as pharmaceutically acceptable salts thereof exhibit antiproliferation and anticancer activity, thereby modulating the function and/or activity of the microRNA. Thus, the compounds according to general formulae ((**I-a)**, (**I-b**), (**I-c**), (**I-d**), (**II-1)**, (**II-2)**, (**II-3**), (**II-4**), (**II-5**), and (**II-6**), as well as pharmaceutically acceptable salts thereof are useful as microRNA binders.

As used herein the term **"microRNA binder"** refers to a small molecule compound that binds to one more structural motifs of an RNA molecule, including stem-loop, internal loop, bulge, three-stem junction, tetraloop, and pseudoknot.

**Misexpression,** as used herein, refers to a non-wild type pattern of gene expression. It includes: expression at non-wild type levels, i.e., over or under expression; a pattern of expression that differs from wild type in terms of the time or stage at which the microRNA is expressed, e.g., increased or decreased expression (as compared with wild type) at a predetermined developmental period or stage; a pattern of expression that differs from wild type in terms of decreased expression (as compared with wild type) in a predetermined cell type or tissue type; a pattern of expression that differs from wild type in terms of the effect of an environmental stimulus or extracellular stimulus on expression of the microRNA, e.g., a pattern of increased or decreased expression (as compared with wild type) in the presence of an increase or decrease in the strength of the stimulus.

Therefore, the present invention relates the use of the inventive compounds of formula (I) in the treatment and/or prophylaxis of a disease caused by and/or associated with microRNA expression or microRNA misexpression 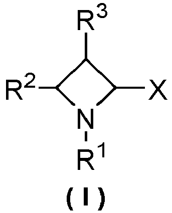 wherein
**R¹** represents -CH₃, -CH₂CH₃, -CH₂CH₂CH₃, -CH(CH₃)₂, -(CH₂)₃CH₃, -(CH₂)₄CH₃, -(CH₂)₅CH₃, -CH₂CH(CH₃)₂, -(CH₂)₂CH(CH₃)₂, -CH(CH₂CH₃)CH₃, -CH(CH₂CH₃)₂, -C(CH₃)₃, -CH₂CH=CH₂, -(CH₂)₂CH=CH₂, -(CH₂)₃CH=CH₂, -(CH₂)₄CH=CH₂, -**L¹-A¹**, or **-B¹** ;
**R²** represents -H, -CN, -**NR⁴R⁵**, -**L²**-**A²**, -**B²**, or -**C²**;
**R³** represents -**A³** or -**B³** with the proviso that when **R²** is -CN **R³** is -**A³** ;
**L¹** represents -CO- , or -SO₂- ;
**L²** represents -CH₂-**NR⁶**-CO- , -CH₂-N**R⁶**-SO₂- or 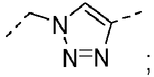
**R⁴** and **R⁵** independently of each other represent -CH₃, -CH₂CH₃, -CH(CH₃)₂, -CH₂CH₂CH₃, -CH₂CH(CH₃)₂, -C(CH₃)₃, -cyclo-C₃H₅, -cyclo-C₄H₇, -cyclo-C₅H₉, -cyclo-C₆H₁₁, -CH₂-cyclo-C₃H₅, -CH₂-cyclo-C₄H₇, -CH₂-cyclo-C₅H₉, -CH₂-cyclo-C₆H₁₁, -CH₂-Ph, -CH₂OCH₃, -CH₂OCH₂CH₃, -CH₂CH₂OCH₃, or -CH₂CH₂SCH₃;
**A¹** and **A²** are independently of each other selected from 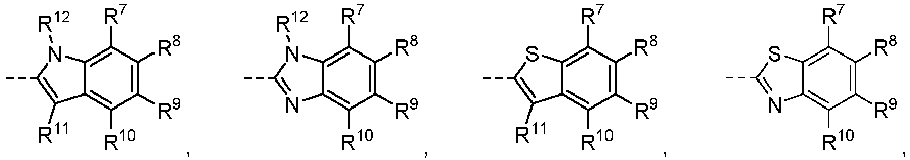 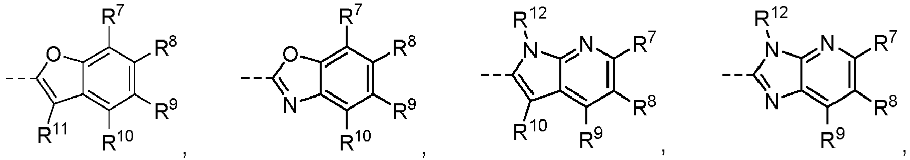 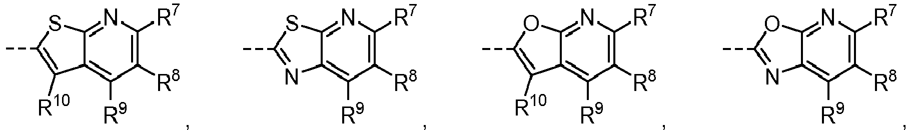 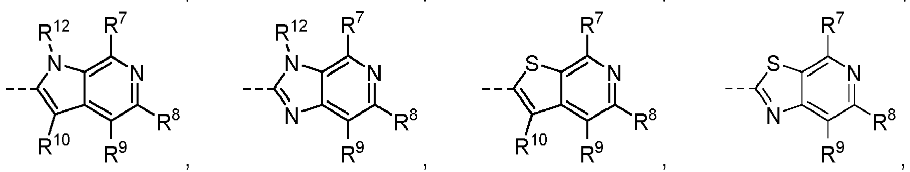 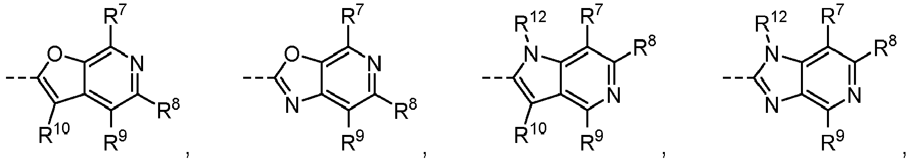 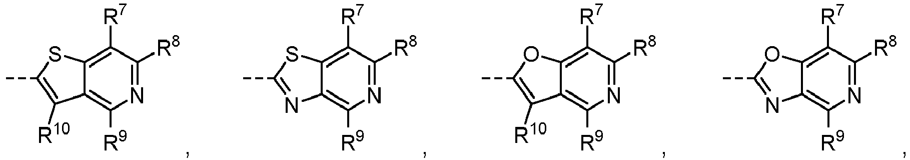 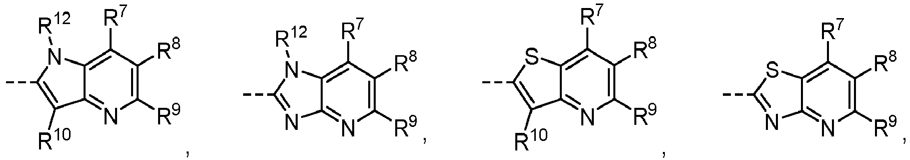 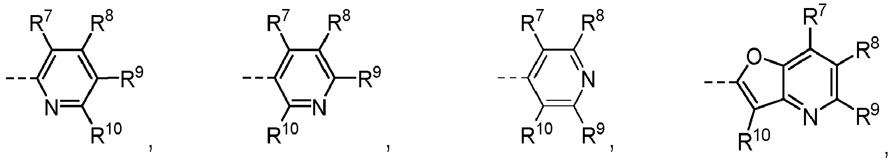 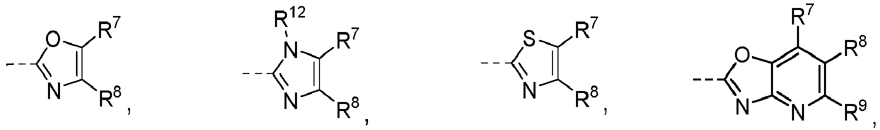 or 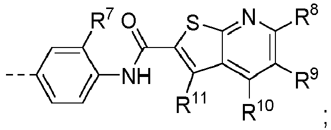
**A³** represents 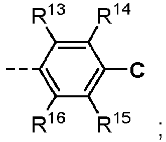 with C being selected from: 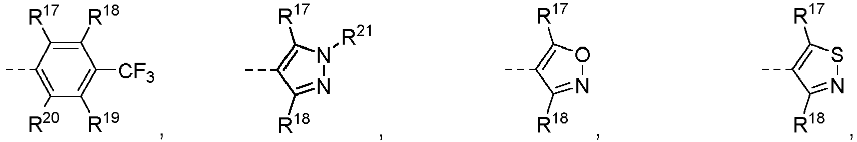 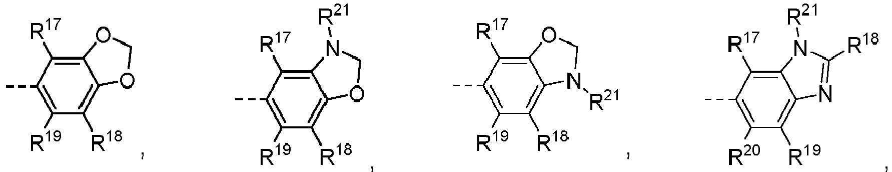 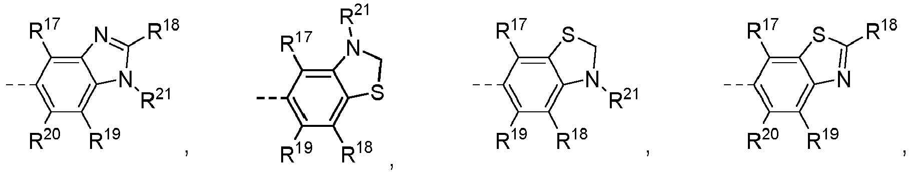 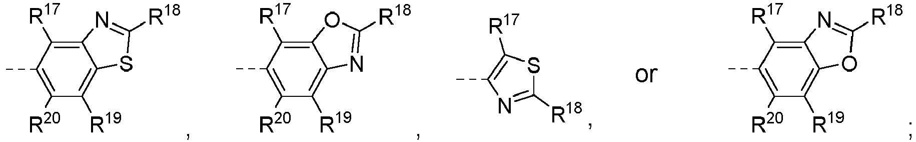
**B¹** represents **-H¹**, -**P¹**-**H¹** or -**P¹**-**Y¹**-**H¹** ;
**B²** represents -H**², -P²-H²,** or **-P²-Y²-H²;**
**B³** represents **-H³, -P³-H³,** or **-P³-Y³-H³;**
**C²** is selected from: 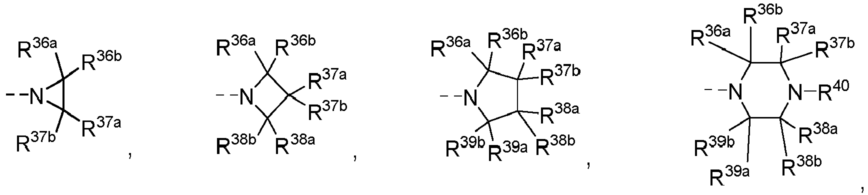 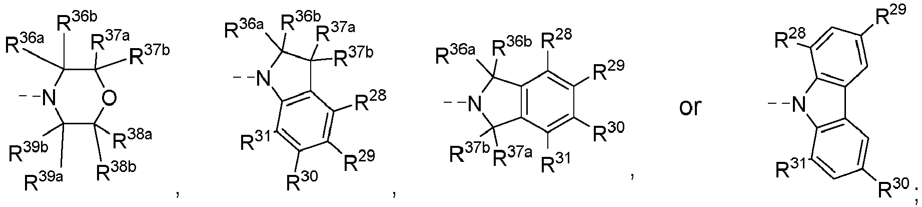
**Y¹**, **Y²**, and **Y³** are independently of each other selected from: -O-, -N**R²²**- -CO-N**R²²**-, -N**R²²**-CO-, -SO₂-N**R²²**-, -N**R²²**-SO₂-, or -N**R²²**-CO-N**R²³**-;
**P¹**, **P²**, and **P³** are independently of each other selected from: 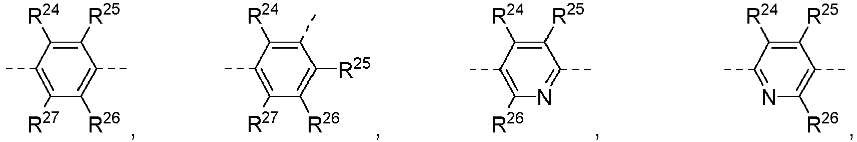 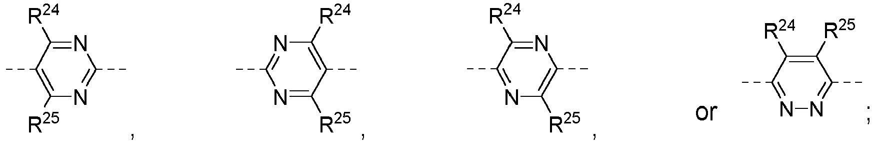
**H¹**, **H²**, and **H³** are independently of each other selected from: 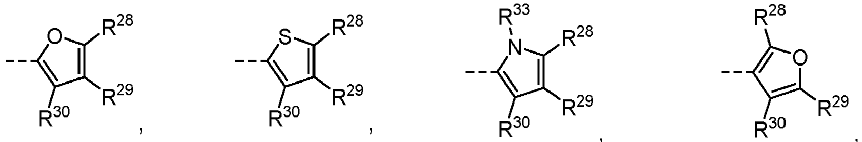 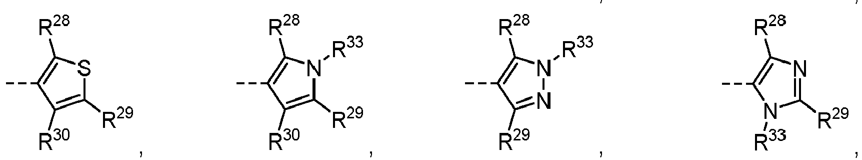 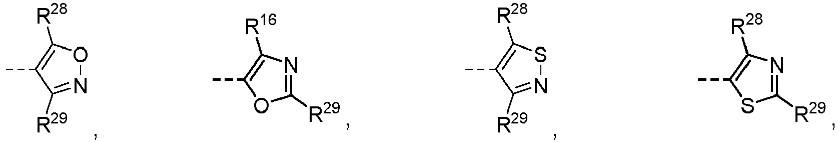 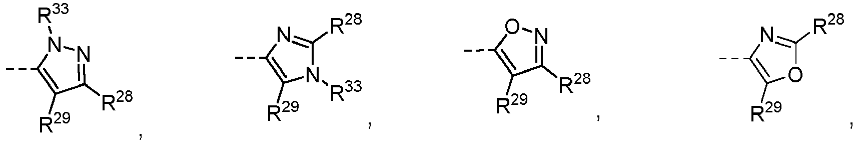 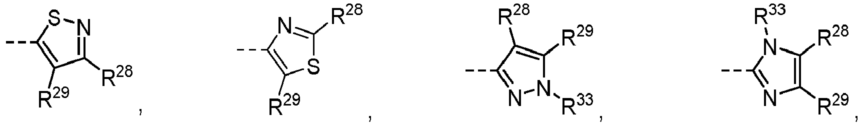 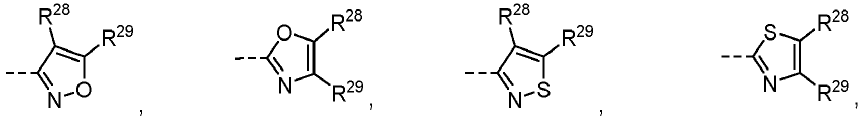 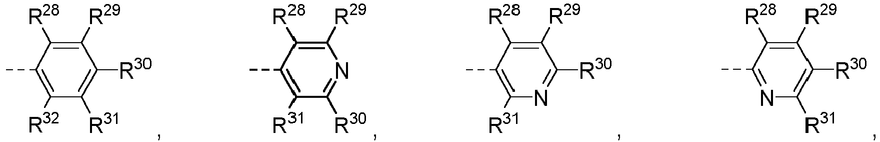 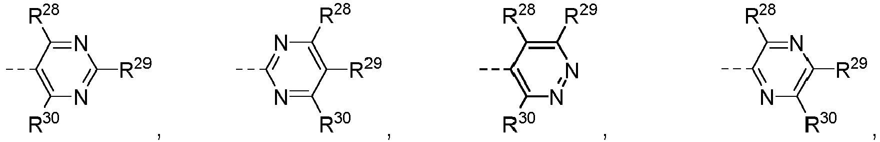 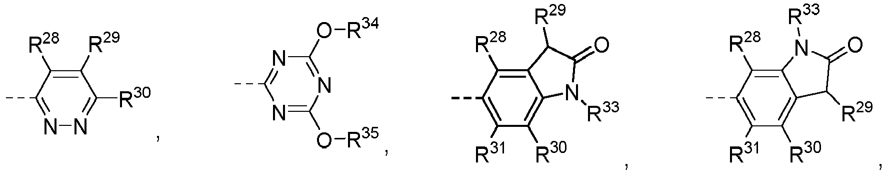 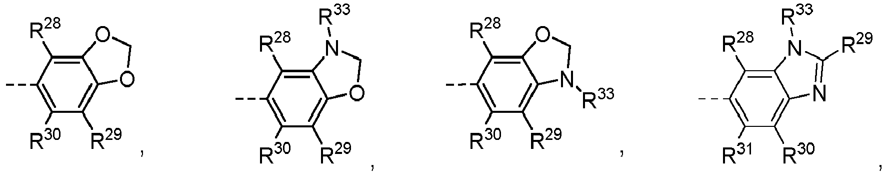 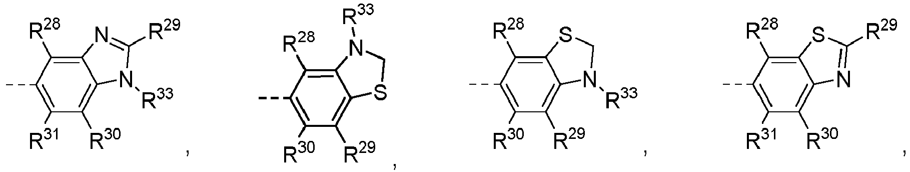 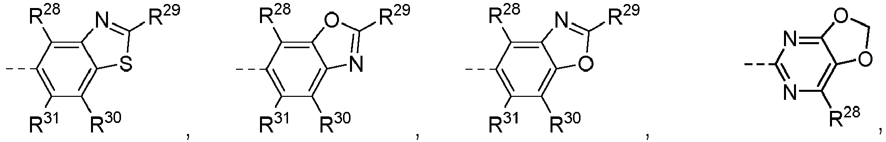 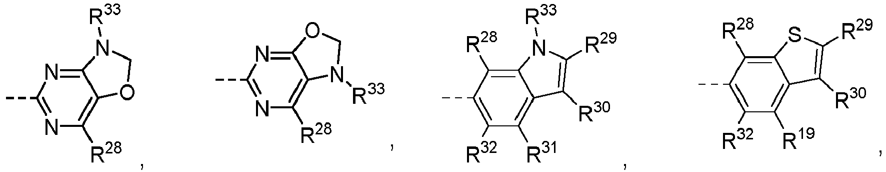 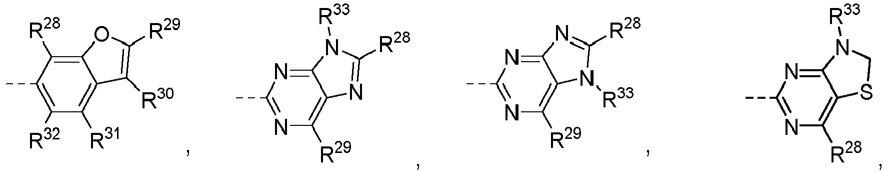 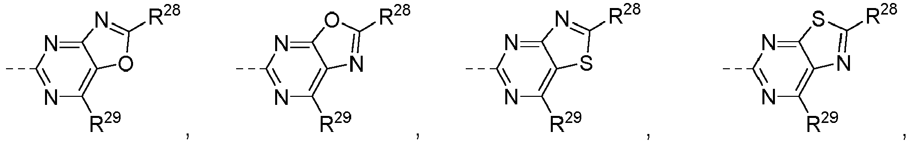 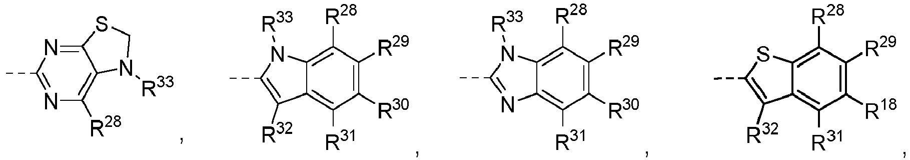 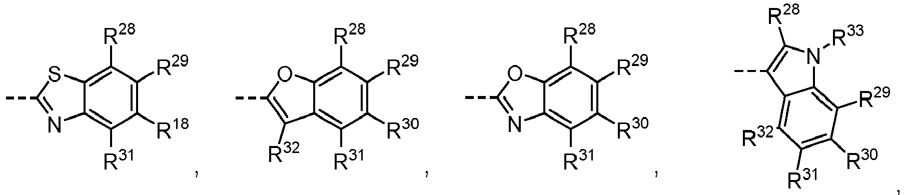 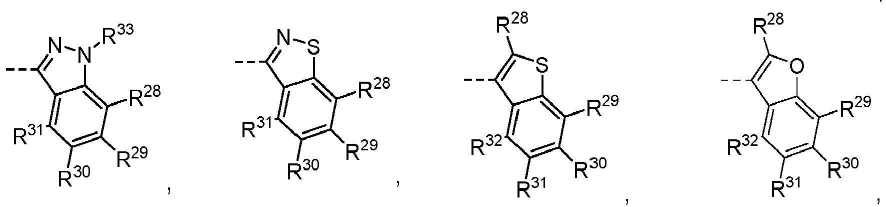 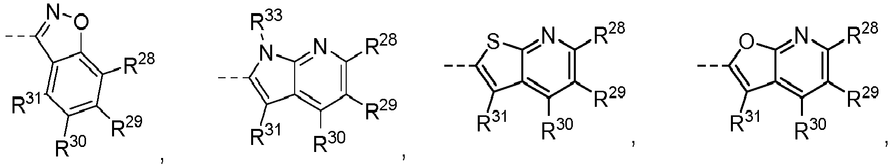 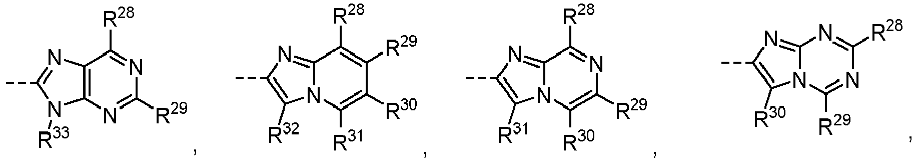 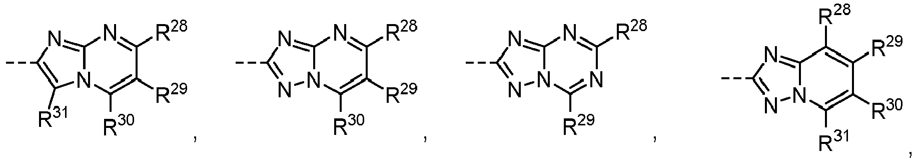 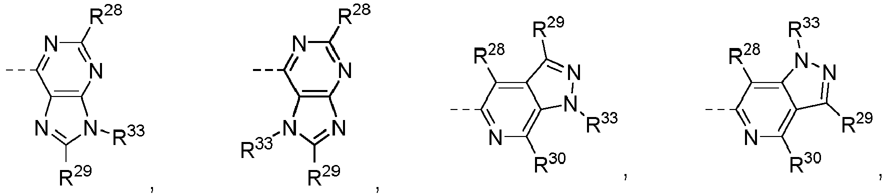 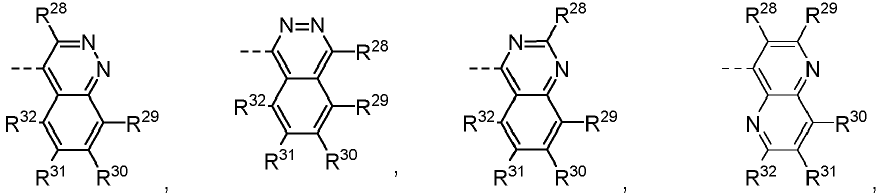 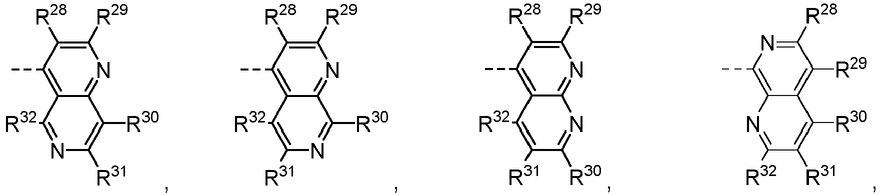 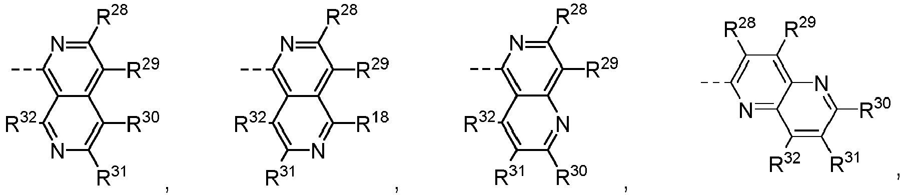 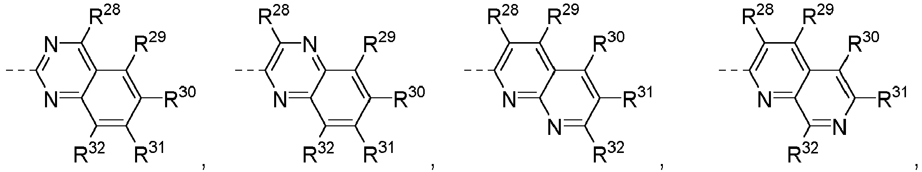 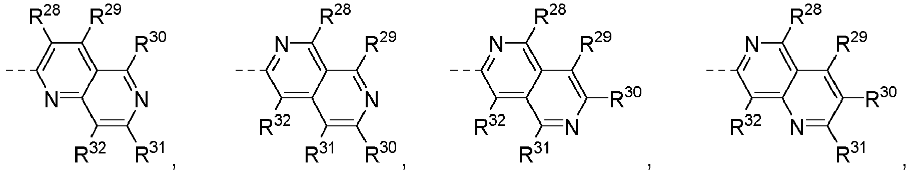 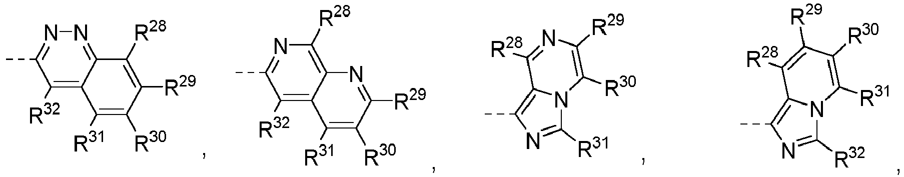 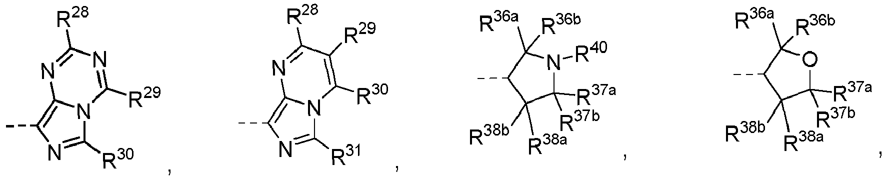 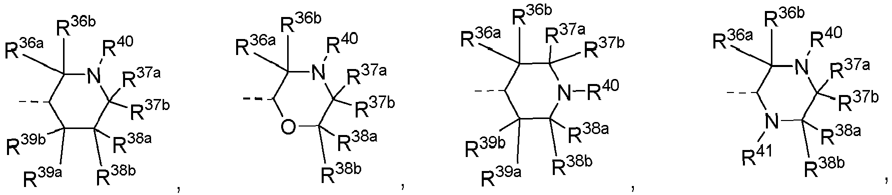 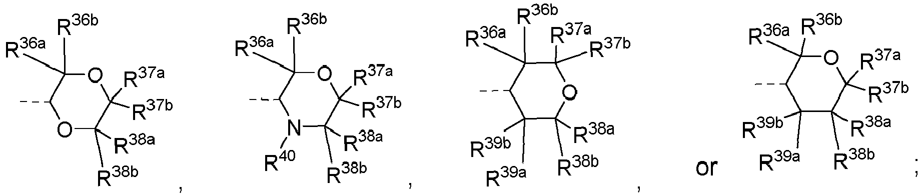
**R¹⁷** - **R²⁰** and **R²⁸-R³²** represent independently of each other -H, -F, -CI, -Br, -I, -OH, -CN, -NO₂, -CH₃, -C₂H₅, -C₃H₇, -CH(CH₃)₂, -C₄H₉, -CH₂-CH(CH₃)₂, -CH(CH₃)-C₂H₅, -C(CH₃)₃, -cyclo-C₃H₅, -CH₂-cyclo-C₃H₅, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -OCH₃, -OC₂H₅, -OC₃H₇, -OCH(CH₃)₂, -OC(CH₃)₃, -OC₄H₉, -OCHF₂, -OCF₃, -OCH₂CF₃, -OC₂F₅, -OCH₂OCH₃, -O-cyclo-C₃H₅, -OCH₂-cyclo-C₃H₅, -O-C₂H₄-cyclo-C₃H₅, -CHO, -COCH₃, -COCF₃, -COC₂H₅, -COC₃H₇, -COCH(CH₃)₂, -COC(CH₃)₃, -COOH, -COOCH₃, -COOC₂H₅, -COOC₃H₇, -COOCH(CH₃)₂, -COOC(CH₃)₃, -OOC-CH₃, -OOC-CF₃, -OOC-C₂H₅, -OOC-C₃H₇, -OOC-CH(CH₃)₂, -OOC-C(CH₃)₃, -NH₂, -NHCH₃, -NHC₂H₅, -NHC₃H₇, -NHCH(CH₃)₂, -NHC(CH₃)₃, -N(CH₃)₂, -N(C₂H₅)₂, -N(C₃H₇)₂, -N[CH(CH₃)₂]₂, -N[C(CH₃)₃]₂, -NHCOCH₃, -NHCOCF₃, -NHCOC₂H₅, -NHCOC₃H₇, -NHCOCH(CH₃)₂, -NHCOC(CH₃)₃, -CONH₂, -CONHCH₃, -CONHC₂H₅, -CONHC₃H₇, -CONHCH(CH₃)₂, -CONH-cyclo-C₃H₅, -CONHC(CH₃)₃, -CON(CH₃)₂, -CON(C₂H₅)₂, -CON(C₃H₇)₂, -CON[CH(CH₃)₂]₂, -CON[C(CH₃)₃]₂, -SO₂NH₂, -SO₂NHCH₃, -SO₂NHC₂H₅, -SO₂NHC₃H₇, -SO₂NHCH(CH₃)₂, -SO₂NH-cyclo-C₃H₅, -SO₂NHC(CH₃)₃, -SO₂N(CH₃)₂, -SO₂N(C₂H₅)₂, -SO₂N(C₃H₇)₂, -SO₂N[CH(CH₃)₂]₂, -SO₂N[C(CH₃)₃]₂, -NHSO₂CH₃, -NHSO₂CF₃, -NHSO₂C₂H₅, -NHSO₂C₃H₇, -NHSO₂CH(CH₃)₂, -NHSO₂C(CH₃)₃, -CH=CH₂, -CH₂-CH=CH₂, -C(CH₃)=CH₂, -CH=CH-CH₃, -C=CH, -C=C-CH₃, -CH₂-C=CH, -Ph, -O-Ph, -O-CH₂-Ph, -C(=NH)NH₂, or 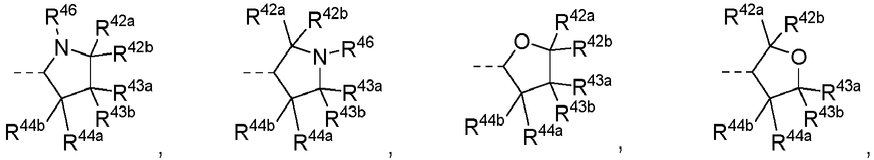 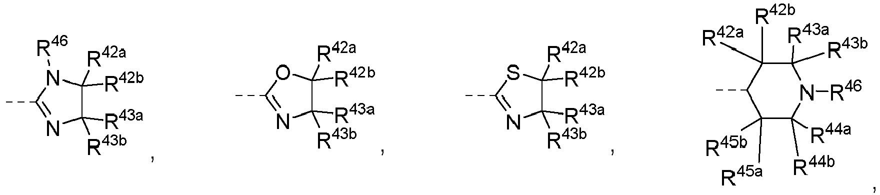 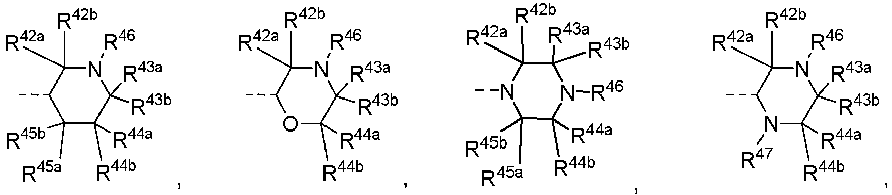 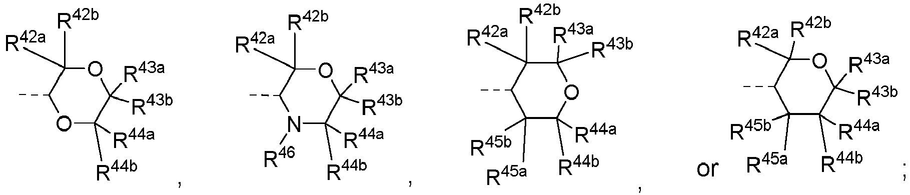
**R⁶**, **R¹¹**, **R¹²**, **R²¹** - **R²³**, **R³³** - **R³⁵**, **R⁴⁰**, **R⁴¹**, **R⁴⁶**, and **R⁴⁷** represent independently of each other -H, -CH₃, -CH₂CH₃, -CH(CH₃)₂, -CH₂CH₂CH₃, -CH₂CH(CH₃)₂, -C(CH₃)₃, -cyclo-C₃H₅, -cyclo-C₄H₇, -cyclo-C₅H₉, -cyclo-C₆H₁₁, -CH₂-cyclo-C₃H₅, -CH₂-cyclo-C₄H₇, -CH₂-cyclo-C₅H₉, -CH₂-cyclo-C₆H₁₁, -CH₂-Ph, -CH₂OCH₃, -CH₂OCH₂CH₃, -CH₂CH₂OCH₃, -COO-C(CH₃)₃, -COO-CH₂Ph, or -CH₂CH₂SCH₃;
**R²⁴ - R²⁷**, **R^{36a}** - **R^{39a}**, **R^{36b}** - **R^{39b} R^{42a}** _ **R^{45a}** and **R^{42b}** - **R**^{**45**b} represent independently of each other -H, -F, -CI, -Br, -I, -OH, -CN, -NO₂, -CH₃, -C₂H₅, -C₃H₇, -CH(CH₃)₂, -C₄H₉, -CH₂-CH(CH₃)₂, -CH(CH₃)-C₂H₅, -C(CH₃)₃, -cyclo-C₃H₅, -CH₂-cyclo-C3H₅, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -OCH₃, -OC₂H₅, -OC₃H₇, -OCH(CH₃)₂, -OC(CH₃)₃, -OC₄H₉, -OCHF₂, -OCF₃, -OCH₂CF₃, -OC₂F₅, -OCH₂OCH₃, -O-cyclo-C₃H₅, -OCH₂-cyclo-C₃H₅, -O-C₂H₄-cyclo-C₃H₅, -CHO, -COCH₃, -COCF₃, -COC₂H₅, -COC₃H₇, -COCH(CH₃)₂, -COC(CH₃)₃, -COOH, -COOCH₃, -COOC₂H₅, -COOC₃H₇, -COOCH(CH₃)₂, -COOC(CH₃)₃, -OOC-CH₃, -OOC-CF₃, -OOC-C₂H₅, -OOC-C₃H₇, -OOC-CH(CH₃)₂, -OOC-C(CH₃)₃, -NH₂, -NHCH₃, -NHC₂H₅, -NHC₃H₇, -NHCH(CH₃)₂, -NHC(CH₃)₃, -N(CH₃)₂, -N(C₂H₅)₂, -N(C₃H₇)₂, -N[CH(CH₃)₂]₂, -N[C(CH₃)₃]₂, -NHCOCH₃, -NHCOCF₃, -NHCOC₂H₅, -NHCOC₃H₇, -NHCOCH(CH₃)₂, -NHCOC(CH₃)₃, -CONH₂, -CONHCH₃, -CONHC₂H₅, -CONHC₃H₇, -CONHCH(CH₃)₂, -CONH-cyclo-C₃H₅, -CONHC(CH₃)₃, -CON(CH₃)₂, -CON(C₂H₅)₂, -CON(C₃H₇)₂, -CON[CH(CH₃)₂]₂, -CON[C(CH₃)₃]₂, -SO₂NH₂, -SO₂NHCH₃, -SO₂NHC₂H₅, -SO₂NHC₃H₇, -SO₂NHCH(CH₃)₂, -SO₂NH-cyclo-C₃H₅, -SO₂NHC(CH₃)₃, -SO₂N(CH₃)₂, -SO₂N(C₂H₅)₂, -SO₂N(C₃H₇)₂, -SO₂N[CH(CH₃)₂]₂, -SO₂N[C(CH₃)₃]₂, -NHSO₂CH₃, -NHSO₂CF₃, -NHSO₂C₂H₅, -NHSO₂C₃H₇, -NHSO₂CH(CH₃)₂, -NHSO₂C(CH₃)₃, -CH=CH₂, -CH₂-CH=CH₂, -C(CH₃)=CH₂, -CH=CH-CH₃, -C≡CH, -C≡C-CH₃, -CH₂-C≡CH, -Ph, -O-Ph, or -O-CH₂-Ph;
**X** represents -CN, -NH₂, or -CH₂OH;
or a regiomer, diastereomer, enantiomer, a mixture of regiomers, a mixture of diastereomers, a mixture of enantiomers, a prodrug, a solvate, a hydrate, or a pharmaceutically acceptable salt thereof.

Further, the present invention relates to the use of compounds according to general formulae **(I),** (**I-a**), (**I-b**), (**I-c**), (**I-d**), **(II-1),** (**II-2)**, (**II-3**), (**II-4**), (**II-5**), and (**II-6),** as well as pharmaceutically acceptable salts thereof for the prophylaxis and/or treatment of a disease caused by and/or associated with microRNA expression or microRNA misexpression. In one embodiment of the present invention compounds according to general formulae **(I),** (**I-a**), (**I-b**), (**I-c**), (**I-d**), **(II-1),** (**II-2)**, (**II-3**), (**II-4**), (**II-5**), and (**II-6**), as well as pharmaceutically acceptable salts thereof can be used for the prophylaxis and/or treatment of a disease caused by and/or associated with microRNA expression or microRNA misexpression, wherein said compound modulates the function and/or activity of the microRNA.

In one embodiment of the present invention compounds according to general formulae **(I),** (**I-a**), (**I-b**), (**I-c**), (**I-d**), **(II-1),** (**II-2)**, (**II-3**), (**II-4**), (**II-5**), and (**II-6**), as well as pharmaceutically acceptable salts thereof can be used for the prophylaxis and/or treatment of a disease caused by and/or associated with microRNA expression or microRNA misexpression, wherein said disease is selected from cancer, tumor, metastasis, virus infection, immunological disease, autoimmune disease, inflammatory disorder, diabetes, cardiovascular disease, metabolic disease, and neurodegenerative disease.

Thus, the compounds of the present invention can be used for prophylaxis and/or treatment of cancers, tumors, metastases, virus infections, immunological disorders, autoimmune diseases, inflammatory disorders, diabetes, cardiovascular diseases, metabolic diseases, and neurodegenerative diseases.

More specifically, tumours, cancers or metastases that can be treated and/or prevented by the inventive compounds are selected from the group comprising or consisting of: adenocarcinoma, choroidal melanoma, acute leukemia, acoustic neurinoma, ampullary carcinoma, anal carcinoma, astrocytoma, basal cell carcinoma, pancreatic cancer, desmoid tumor, bladder cancer, bronchial carcinoma, estrogen dependent and independent breast cancer, Burkitt's lymphoma, corpus cancer, CUP-syndrome (carcinoma of unknown primary), colorectal cancer, small intestine cancer, small intestinal tumors, ovarian cancer, endometrial carcinoma, ependymoma, epithelial cancer types, Ewing's tumors, gastrointestinal tumors, gastric cancer, gallbladder cancer, gall bladder carcinomas, uterine cancer, cervical cancer, glioblastomas, gynecologic tumors, ear, nose and throat tumors, hematologic neoplasias, hairy cell leukemia, urethral cancer, skin cancer, skin testis cancer, brain tumors (gliomas), brain metastases, testicle cancer, hypophysis tumor, carcinoids, Kaposi's sarcoma, laryngeal cancer, germ cell tumor, bone cancer, colorectal carcinoma, head and neck tumors (tumors of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's lymphomas), lymphomas, stomach cancer, malignant melanoma, malignant neoplasia, malignant tumors gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, prostate cancer, pharyngeal cancer, rectal carcinoma, retinoblastoma, vaginal cancer, thyroid carcinoma, Schneeberger disease, esophageal cancer, spinalioms, T-cell lymphoma (mycosis fungoides), thymoma, tube carcinoma, eye tumors, urethral cancer, urologic tumors, urothelial carcinoma, vulva cancer, wart appearance, soft tissue tumors, soft tissue sarcoma, Wilm's tumor, cervical carcinoma, tongue cancer, invasive ductal carcinoma, invasive lobular carcinoma, ductal carcinoma in situ, lobular carcinoma in situ, small-cell lung carcinoma, non-small-cell lung carcinoma, bronchial adenoma, pleuropulmonary blastoma, mesothelioma, brain stem glioma, hypophtalmic glioma, cerebellar astrocytoma, cerebral astrocytoma, neuroectodermal tumours, pineal tumors, sarcoma of the uterus, salivary gland cancers, anal gland adenocarcinomas, mast cell tumors, pelvis tumours, ureter tumours, hereditary papillary renal cancers, sporadic papillary renal cancers, intraocular melanoma, hepatocellular carcinoma (liver cell carcinomas with or without fibrolamellar variant), cholangiocarcinoma (intrahepatic bile duct carcinoma), mixed hepatocellular cholangiocarcinoma, squamous cell carcinoma, malignant melanoma, Merkel cell skin cancer, non-melanoma skin cancer, hypopharyngeal cancer, nasopharyngeal cancer, oropharyngeal cancer, oral cavity cancer, squamous cell cancer, oral melanoma, AIDS-related lymphoma, cutaneous T-cell lymphoma, lymphoma of the central nervous system, malignant fibrous histiocytoma, lymphosarcoma, rhabdomyosarcoma, malignant histiocytosis, fibrosarcoma, hemangiosarcoma, hemangiopericytoma, leiomyosarcoma., canine mammary carcinoma, and feline mammary carcinoma.

In preferred embodiments, the cancer is selected from breast cancer, colon cancer, pancreas cancer and prostate cancer.

Furthermore, the compounds of general formulae **(I),** (**I-a**), (**I-b**), (**I-c**), (**I-d**), **(II-1),** (**II-2)**, (**II-3**), (**II-4**), (**II-5**), and (**II-6**) are useful for the treatment and/or prophylaxis of immunological disorders and/or autoimmune diseases, wherein the immunological disorders and/or autoimmune diseases are selected from the group comprising or consisting of: asthma, diabetes, rheumatic diseases, AIDS, rejection of transplanted organs and tissues, rhinitis, chronic obstructive pulmonary diseases, osteoporosis, ulcerative colitis, sinusitis, lupus erythematosus, recurrent infections, atopic dermatitis / eczema and occupational allergies, food allergies, drug allergies, severe anaphylactic reactions, anaphylaxis, manifestations of allergic diseases, primary immunodeficiencies, antibody deficiency states, cell mediated immunodeficiencies, severe combined immunodeficiency, DiGeorge syndrome, Hyper-IgE syndrome, Wiskott-Aldrich syndrome, ataxia-telangiectasia, immune mediated cancers, white cell defects, autoimmune diseases, systemic lupus erythematosus, rheumatoid arthritis (RA), multiple sclerosis (MS), immune-mediated or Type 1 Diabetes Mellitus, immune mediated glomerulonephritis, scleroderma, pernicious anemia, alopecia, pemphigus, pemphigus vulgaris, myasthenia gravis, inflammatory bowel diseases, Crohn's disease, psoriasis, autoimmune thyroid diseases, Hashimoto's disease, dermatomyositis, goodpastture syndrome, myasthenia gravis pseudoparalytica, ophtalmia sympatica, phakogene uveitis, chronical agressive hepatitis, primary billiary cirrhosis, autoimunehemolytic anemy, and Werlof disease.

The compounds of general formulae **(I),** (**I-a**), (**I-b**), (**I-c**), (**I-d**), **(11-1),** (**II-2)**, (**II-3**), **(II-4), (II-5),** and (**II-6**) are useful for the treatment and/or prophylaxis of viral infectious diseases caused by a pathogen selected from the group comprising: Adenoviruses, Ebolavirus, Epstein-Barr-virus, Flavivirus, FSME-virus, Influenza virus, Hanta-virus, human immunodeficiency virus ("HIV"), herpes simplex virus ("HSV", type 1 or 2), human herpes virus 6 (HHV-6), human Papilloma virus ("HPV", type 16 or 18), human Cytomegalovirus ("HCMV"), human hepatitis B or C virus ("HBV", Type B; "HCV", type C), Lassavirus, Lyssavirus (EBL 1 or EBL 2), Marburgvirus, Norovirus, Parvovirus B19, Pestivirus, Poliovirus, respiratory syncytial virus, Rhinovirus, Rotaviruses, SARS-associated Coronavirus, Varicella-Zoster virus.

Additionally, the inventive compounds of general formulae **(I),** (**I-a**), (**I-b**), (**I-c**), **(I-d),** (**II-1)**, (**II-2)**, (**II-3**), (**II-4**), (**II-5**), and (**II-6**) are useful for the treatment and/or prophylaxis of inflammatory disorders, wherein the inflammatory disorders are selected from the group comprising or consisting of: abscessation, acanthameba, acanthamebiasis, acne vulgaris, actinomycosis, acute inflammatory dermatoses, acute laryngeal infections of adults, acute multifocal placoid pigmentary epitheliopathy, acute (thermal) injury, acute retinal necrosis, acute suppurative otitis media, algal disorders, allergic contact dermatitis, amyloidosis angioedema, ankylosing spondylitis, aspergillosis, atopic dermatitis, Aujeszky's disease, autoantibodies in vasculitis, babesiosis, bacterial disorders, bacterial laryngitis, bacterial meningitis, Behcet's disease, birdshot choroidopathy, blastomycosis, borna disease, brucellosis, bullous myringitis, bursitis, candidiasis, canine distemper encephalomyelitis, canine distemper encephalomyelitis in immature animals, canine ehrlichiosis, canine herpes virus encephalomyelitis, cholesteatoma, chronic (granulomatous) diseases, chronic inflammatory dermatoses, chronic relapsing encephalomyelitis, chronic suppurative otitis media, cicatricial pemphigoid, coccidiomycosis, coccidioidomycosis, common upper respiratory infection, contact ulcer and granuloma, Crohn's disease, cryptococcosis, cysticercosis, dermatomyositis, diphtheria, discoid lupus erythematosus, drug-induced vasculitis, drug or hypersensitivity reaction, encephalitozoonosis, eosinophilic meningoencephalitis, erythemal multiforme (EM minor), feline leukemia virus, feline immunodeficiency virus, feline infectious peritonitis, feline polioencephalomyelitis, feline spongiform encephalopathy, fibromyositis, Fuch's heterochromic cyclitis, gastroesophageal (laryngopharyngeal) reflux disease, giant cell arteritis, glanders, glaucomatocyclitic crisis, gonorrhea granular myringitis, granulomatous meningoencephalomyelitis, herpes simplex, histoplasmosis, idiopathic diseases, idiopathic inflammatory disorders, immune and idiopathic disorders, infections of the immunocompromised host, infectious canine hepatitis, inhalation laryngitis, interstitial nephritis, irritant contact dermatitis, juvenile rheumatoid arthritis, Kawasaki's disease, La Crosse virus encephalitis, laryngeal abscess, laryngotracheitis (croup), leishmaniasis, lens-induced uveitis, leprosy, leptospirosis, leukemia, lichen planus, lupus, lyme disease, lymphoma, meningitis, meningoencephalitis in greyhounds, miscellaneous meningitis / meningoencephalitis, microscopic polyangiitis, multifocal choroiditis, multifocal distemper encephalomyelitis in mature animals, multiple sclerosis, muscle tension dysphonias, mycotic (fungal) diseases, mycotic diseases of the CNS, necrotizing encephalitis, neosporosis, old dog encephalitis, onchocerciasis, parasitic encephalomyelitis, parasitic infections, pars planitis, parvovirus encephalitis, pediatric laryngitis, pollution and inhalant allergy, polymyositis, post-vaccinal canine distemper encephalitis, post-vaccinal rabies, prion protein induced diseases, protothecosis, protozoal encephalitis-encephalomyelitis, psoriasis, psoriatic arthritis, pug dog encephalitis, pyogranulomatous meningoencephalomyelitis, rabies, radiation injury, radiation laryngitis, radionecrosis, relapsing polychondritis, Reiters's syndrome, retinitis pigmentosa, retinoblastoma, rheumatoid arthritis, rickettsial disorders, rocky mountain spotted fever, salmon poisoning, sarcocystosis, sarcoidosis, schistosomiasis, scleroderma, scleroma, serpiginous choroiditis, shaker dog disease, Sjogren's syndrome, spasmodic croup, spirochetal (syphilis) diseases, spongiotic dermatitis, sporotrichosis, steroid responsive meningitis-arteritis, Stevens-Johnson syndrome (SJS, EM major), supraglottitis (epiglottitis), sympathetic ophthalmia, syngamus laryngeus, syphilis, systemic lupus erythematosus, systemic vasculitis in sarcoidosis, Takayasu's arteritis, tendinitis (tendonitis), thromboangiitis obliterans (Buerger's Disease), tick-borne encephalitis in dogs, toxic epidermal necrolysis (TEN), toxocariasis, toxoplasmosis, trauma, traumatic laryngitis, trichinosis, trypanosomiasis, tuberculosis, tularemia, ulcerative colitis, urticaria (hives), vasculitis, vasculitis and malignancy, vasculitis and rheumatoid arthritis, vasculitis in systemic lupus erythematosus, vasculitis in the idiopathic inflammatory myopathies, vasculitis of the central nervous system, vasculitis secondary to bacterial, fungal, and parasitic infection, viral disorders, viral laryngitis, vitiligo, vocal abuse, vocal-cord hemorrhage, Vogt Koyanagi Harada syndrome, Wegener's granulomatosis, and Whipple's disease.

Moreover, the inventive compounds of general formulae **(I),** (**I-a**), (**I-b**), (**I-c**), (**I-d**), **(II-1),** (**II-2)**, (**II-3**), (**II-4**), (**II-5**), and (**II-6**) are useful for the treatment and/or prophylaxis of cardiovascular diseases, wherein the cardiovascular diseases are selected from the group comprising or consisting of: hypercholesterolemia, diabetic dyslipidemia, hypertension, arteriosclerosis, atherosclerosis, myocardial infarction, acute coronary syndrome, angina, congestive heart failure, aortic aneurysm, aortic dissection, iliac or femoral aneurysm, pulmonary embolism, primary hypertension, atrial fibrillation, stroke, transient ischemic attack, systolic dysfunction, diastolic dysfunction, myocarditis, atrial tachycardia, ventricular fibrillation, endocarditis, arteriopathy, vasculitis, atherosclerotic plaque, vulnerable plaque, acute coronary syndrome, acute ischemic attack, sudden cardiac death, peripheral vascular disease, coronary artery disease (CAD), peripheral artery disease (PAD), and cerebrovascular disease.

Moreover, the inventive compounds of general formulae **(I),** (**I-a**), (**I-b**), (**I-c**), (**I-d**), **(II-1),** (**II-2)**, (**II-3**), (**II-4**), (**II-5**), and (**II-6**) are useful for the treatment and/or prophylaxis of metabolic diseases, wherein the metabolic diseases are selected from the group comprising or consisting of: Acid-Base Imbalance, Metabolic Bone Diseases, Metabolic Brain Diseases, Calcium Metabolism Disorders, DNA Repair-Deficiency Disorders, Glucose Metabolism Disorders, Hyperlactatemia, Iron Metabolism Disorders, Lipid Metabolism Disorders, Malabsorption Syndromes, Metabolic Syndrome, Metabolism, Inborn Errors, Mitochondrial Diseases, Phosphorus Metabolism Disorders, Porphyrias, Proteostasis Deficiencies, Skin Diseases, Metabolic, Wasting Syndrome, and Water-Electrolyte Imbalance.

Moreover, the inventive compounds of general formulae **(I),** (**I-a**), (**I-b**), (**I-c**), (**I-d**), **(II-1),** (**II-2)**, (**II-3**), (**II-4**), (**II-5**), and (**II-6**) are useful for the treatment and/or prophylaxis of neurodegenerative diseases, wherein the neurodegenerative diseases are selected from the group comprising or consisting of: ADHD, AIDS-neurological complications, absence of the Septum Pellucidum, acquired epileptiform aphasia, acute disseminated encephalomyelitis, adrenoleukodystrophy, agenesis of the Corpus Callosum, agnosia, Aicardi Syndrome, Alexander Disease, Alpers' Disease, alternating hemiplegia, Alzheimer's Disease, amyotrophic lateral sclerosis (ALS), anencephaly, aneurysm, Angelman Syndrome, angiomatosis, anoxia, aphasia, apraxia, arachnoid cysts, arachnoiditis, Arnold-Chiari Malformation, arteriovenous malformation, aspartame, Asperger Syndrome, ataxia telangiectasia, ataxia, attention deficit-hyperactivity disorder, autism, autonomic dysfunction, back pain, Barth Syndrome, Batten Disease, Behcet's Disease, Bell's Palsy, benign essential blepharospasm, benign focal amyotrophy, benign intracranial hypertension, Bernhardt-Roth Syndrome, Binswanger's Disease, blepharospasm, Bloch-Sulzberger Syndrome, brachial plexus birth injuries, brachial plexus injuries, Bradbury-Eggleston Syndrome, brain aneurysm, brain injury, brain and spinal tumors, Brown-Sequard Syndrome, bulbospinal muscular atrophy, Canavan Disease, Carpal Tunnel Syndrome, causalgia, cavernomas, cavernous angioma, cavernous malformation, central cervical cord syndrome, central cord syndrome, central pain syndrome, cephalic disorders, cerebellar degeneration, cerebellar hypoplasia, cerebral aneurysm, cerebral arteriosclerosis, cerebral atrophy, cerebral beriberi, cerebral gigantism, cerebral hypoxia, cerebral palsy, cerebro-oculo-facio-skeletal syndrome, Charcot-Marie-Tooth Disorder, Chiari Malformation, chorea, choreoacanthocytosis, chronic inflammatory demyelinating polyneuropathy (CIDP), chronic orthostatic intolerance, chronic pain, Cockayne Syndrome Type II, Coffin Lowry Syndrome, coma, including persistent vegetative state, complex regional pain syndrome, congenital facial diplegia, congenital myasthenia, congenital myopathy, congenital vascular cavernous malformations, corticobasal degeneration, cranial arteritis, craniosynostosis, Creutzfeldt-Jakob Disease, cumulative trauma disorders, Cushing's Syndrome, cytomegalic inclusion body disease (CIBD), cytomegalovirus infection, dancing eyes-dancing feet syndrome, Dandy-Walker Syndrome, Dawson Disease, De Morsier's Syndrome, Dejerine-Klumpke Palsy, dementia-multi-infarct, dementia-subcortical, dementia with Lewy Bodies, dermatomyositis, developmental dyspraxia, Devic's Syndrome, diabetic neuropathy, diffuse sclerosis, Dravet's Syndrome, dysautonomia, dysgraphia, dyslexia, dysphagia, dyspraxia, dystonias, early infantile epileptic encephalopathy, Empty Sella Syndrome, encephalitis lethargica, encephalitis and meningitis, encephaloceles, encephalopathy, encephalotrigeminal angiomatosis, epilepsy, Erb's Palsy, Erb-Duchenne and Dejerine-Klumpke Palsies, Fabry's Disease, Fahr's Syndrome, fainting, familial dysautonomia, familial hemangioma, familial idiopathic basal ganglia calcification, familial spastic paralysis, febrile seizures (e.g., GEFS and GEFS plus), Fisher Syndrome, Floppy Infant Syndrome, Friedreich's Ataxia, Gaucher's Disease, Gerstmann's Syndrome, Gerstmann-Straussler-Scheinker Disease, giant cell arteritis, giant cell inclusion disease, globoid cell leukodystrophy, glossopharyngeal neuralgia, Guillain-Barre Syndrome, HTLV-1 associated myelopathy, Hallervorden-Spatz Disease, head injury, headache, hemicrania continua, hemifacial spasm, hemiplegia alterans, hereditary neuropathies, hereditary spastic paraplegia, heredopathia atactica polyneuritiformis, Herpes Zoster Oticus, Herpes Zoster, Hirayama Syndrome, holoprosencephaly, Huntington's Disease, hydranencephaly, hydrocephalus-normal pressure, hydrocephalus (in particular TGFβ-induced hydrocephalus), hydromyelia, hypercortisolism, hypersomnia, hypertonia, hypotonia, hypoxia, immune-mediated encephalomyelitis, inclusion body myositis, incontinentia pigmenti, infantile hypotonia, infantile phytanic acid storage disease, infantile refsum disease, infantile spasms, inflammatory myopathy, intestinal lipodystrophy, intracranial cysts, intracranial hypertension, Isaac's Syndrome, Joubert Syndrome, Kearns-Sayre Syndrome, Kennedy's Disease, Kinsbourne syndrome, Kleine-Levin syndrome, Klippel Feil Syndrome, Klippel-Trenaunay Syndrome (KTS), Klüver-Bucy Syndrome, Korsakoff's Amnesic Syndrome, Krabbe Disease, Kugelberg-Welander Disease, kuru, Lambert-Eaton Myasthenic Syndrome, Landau-Kleffner Syndrome, lateral femoral cutaneous nerve entrapment, lateral medullary syndrome, learning disabilities, Leigh's Disease, Lennox-Gastaut Syndrome, Lesch-Nyhan Syndrome, leukodystrophy, Levine-Critchley Syndrome, Lewy Body Dementia, lissencephaly, locked-in syndrome, Lou Gehrig's Disease, lupus-neurological sequelae, Lyme Disease-Neurological Complications, Machado-Joseph Disease, macrencephaly, megalencephaly, Melkersson-Rosenthal Syndrome, meningitis, Menkes Disease, meralgia paresthetica, metachromatic leukodystrophy, microcephaly, migraine, Miller Fisher Syndrome, mini-strokes, mitochondrial myopathies, Mobius Syndrome, monomelic amyotrophy, motor neuron diseases, Moyamoya Disease, mucolipidoses, mucopolysaccharidoses, multi-infarct dementia, multifocal motor neuropathy, multiple sclerosis (MS), multiple systems atrophy (MSA-C and MSA-P), multiple system atrophy with orthostatic hypotension, muscular dystrophy, myasthenia-congenital, myasthenia gravis, myelinoclastic diffuse sclerosis, myoclonic encephalopathy of infants, myoclonus, myopathy-congenital, myopathy-thyrotoxic, myopathy, myotonia congenita, myotonia, narcolepsy, neuroacanthocytosis, neurodegeneration with brain iron accumulation, neurofibromatosis, neuroleptic malignant syndrome, neurological complications of AIDS, neurological manifestations of Pompe Disease, neuromyelitis optica, neuromyotonia, neuronal ceroid lipofuscinosis, neuronal migration disorders, neuropathy-hereditary, neurosarcoidosis, neurotoxicity, nevus cavernosus, Niemann-Pick Disease, O'Sullivan-McLeod Syndrome, occipital neuralgia, occult spinal dysraphism sequence, Ohtahara Syndrome, olivopontocerebellar atrophy, opsoclonus myoclonus, orthostatic hypotension, Overuse Syndrome, pain-chronic, paraneoplastic syndromes, paresthesia, Parkinson's Disease, parmyotonia congenita, paroxysmal choreoathetosis, paroxysmal hemicrania, Parry-Romberg, Pelizaeus-Merzbacher Disease, Pena Shokeir II Syndrome, perineural cysts, periodic paralyses, peripheral neuropathy, periventricular leukomalacia, persistent vegetative state, pervasive developmental disorders, phytanic acid storage disease, Pick's Disease, Piriformis Syndrome, pituitary tumors, polymyositis, Pompe Disease, porencephaly, Post-Polio Syndrome, postherpetic neuralgia, postinfectious encephalomyelitis, postural hypotension, postural orthostatic tachycardia syndrome, postural tachycardia syndrome, primary lateral sclerosis, prion diseases, progressive hemifacial atrophy, progressive locomotor ataxia, progressive multifocal leukoencephalopathy, progressive sclerosing poliodystrophy, progressive supranuclear palsy, pseudotumor cerebri, pyridoxine dependent and pyridoxine responsive siezure disorders, Ramsay Hunt Syndrome Type I, Ramsay Hunt Syndrome Type II, Rasmussen's Encephalitis and other autoimmune epilepsies, reflex sympathetic dystrophy syndrome, refsum disease-infantile, refsum disease, repetitive motion disorders, repetitive stress injuries, restless legs syndrome, retrovirus-associated myelopathy, Rett Syndrome, Reye's Syndrome, Riley-Day Syndrome, SUNCT headache, sacral nerve root cysts, Saint Vitus Dance, Salivary Gland Disease, Sandhoff Disease, Schilder's Disease, schizencephaly, seizure disorders, septo-optic dysplasia, severe myoclonic epilepsy of infancy (SMEI), shaken baby syndrome, shingles, Shy-Drager Syndrome, Sjogren's Syndrome, sleep apnea, sleeping sickness, Soto's Syndrome, spasticity, spina bifida, spinal cord infarction, spinal cord injury, spinal cord tumors, spinal muscular atrophy, spinocerebellar atrophy, Steele-Richardson-Olszewski Syndrome, Stiff-Person Syndrome, striatonigral degeneration, stroke, Sturge-Weber Syndrome, subacute sclerosing panencephalitis, subcortical arteriosclerotic encephalopathy, Swallowing Disorders, Sydenham Chorea, syncope, syphilitic spinal sclerosis, syringohydromyelia, syringomyelia, systemic lupus erythematosus, Tabes Dorsalis, Tardive Dyskinesia, Tarlov Cysts, Tay-Sachs Disease, temporal arteritis, tethered spinal cord syndrome, Thomsen Disease, thoracic outlet syndrome, thyrotoxic myopathy, Tic Douloureux, Todd's Paralysis, Tourette Syndrome, transient ischemic attack, transmissible spongiform encephalopathies, transverse myelitis, traumatic brain injury, tremor, trigeminal neuralgia, tropical spastic paraparesis, tuberous sclerosis, vascular erectile tumor, vasculitis including temporal arteritis, Von Economo's Disease, Von Hippel-Lindau disease (VHL), Von Recklinghausen's Disease, Wallenberg's Syndrome, Werdnig-Hoffinan Disease, Wernicke-Korsakoff Syndrome, West Syndrome, Whipple's Disease, Williams Syndrome, Wilson's Disease, X-Linked Spinal and Bulbar Muscular Atrophy, and Zellweger Syndrome.

Moreover, the compounds according to general formulae **(I),** (**I-a**), (**I-b**), (**I-c**), (**I-d**), **(II-1),** (**II-2)**, (**II-3**), (**II-4**), (**II-5**), and (**II-6**), as well as pharmaceutically acceptable salts thereof are useful for the prophylaxis and/or treatment of a disease caused by and/or associated with microRNA expression or microRNA misexpression, wherein the microRNA comprises a stem loop (hairpin) structure.

Moreover, the compounds according to general formulae **(I),** (**I-a**), (**I-b**), (**I-c**), (**I-d**), **(II-1),** (**II-2)**, (**II-3**), (**II-4**), (**II-5**), and (**II-6**), as well as pharmaceutically acceptable salts thereof are useful for the prophylaxis and/or treatment of a disease caused by and/or associated with microRNA expression or microRNA misexpression, wherein the microRNA is a human microRNA selected from hsa-miR-153-1, hsa-miR-153-2, hsa-miR-18a, hsa-miR-19b-2, hsa-miR-20a, hsa-miR-222, hsa-miR-3180-4, hsa-miR-4267, hsa-miR-487a, hsa-miR-539, hsa-miR-571, hsa-miR-106a, hsa-miR-1197, hsa-miR-1324, hsa-miR-27b, hsa-miR-3178, hsa-miR-548a-2, hsa-miR-658, hsa-miR-662, hsa-miR-93, hsa-miR-1307, hsa-miR-3147, hsa-miR-375, hsa-miR-378f, hsa-miR-378h, hsa-miR-422a, hsa-miR-423, hsa-miR-4673, hsa-miR-4761, hsa-let-7e, hsa-miR-150, hsa-miR-181b-2, hsa-miR-186, hsa-miR-25, hsa-miR-27a, hsa-miR-3130-2, hsa-miR-3616, hsa-miR-381, hsa-miR-382, hsa-miR-449a, hsa-miR-4739, hsa-miR-495, hsa-miR-501, hsa-miR-548g, hsa-miR-548j, hsa-miR-555, hsa-miR-660, hsa-miR-7-1, and hsa-miR-92a-1.

Moreover, the compounds according to general formulae **(I),** (**I-a**), (**I-b**), (**I-c**), (**I-d**), **(II-1),** (**II-2)**, (**II-3**), (**II-4**), (**II-5**), and (**II-6**), as well as pharmaceutically acceptable salts thereof are useful for the prophylaxis and/or treatment of a disease caused by and/or associated with microRNA expression or microRNA misexpression, wherein the microRNA comprises a stem-loop region having a sequence as set forth in SEQ ID No. 3 to 53.

### Pharmaceutical compositions

Another aspect of the present invention is directed to pharmaceutical compositions comprising at least one compound of the present invention as active agent, together with at least one pharmaceutically acceptable carrier, excipient and/or diluent. The pharmaceutical compositions of the present invention can be prepared in a conventional solid or liquid carrier or diluent and a conventional pharmaceutically-made adjuvant at suitable dosage level in a known way. The preferred preparations are adapted for oral application. These administration forms include, for example, pills, tablets, film tablets, coated tablets, capsules, powders and deposits.

Furthermore, the present invention also includes pharmaceutical preparations for parenteral application, including dermal, intradermal, intragastral, intracutan, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain at least one compound according to the present invention.

The pharmaceutical compositions according to the present invention containing at least one compound according to the present invention as active agent will typically be administered together with suitable carrier materials selected with respect to the intended form of administration, i.e. for oral administration in the form of tablets, capsules (either solid filled, semi-solid filled or liquid filled), powders for constitution, extrudates, deposits, gels, elixirs, dispersable granules, syrups, suspensions, and the like, and consistent with conventional pharmaceutical practices. For example, for oral administration in the form of tablets or capsules, the active drug component may be combined with any oral non-toxic pharmaceutically acceptable carrier, preferably with an inert carrier like lactose, starch, sucrose, cellulose, magnesium stearate, dicalcium phosphate, calcium sulfate, talc, mannitol, ethyl alcohol (liquid filled capsules) and the like. Moreover, suitable binders, lubricants, disintegrating agents and coloring agents may also be incorporated into the tablet or capsule. Powders and tablets may contain about 5 to about 95 weight % of the inventive compound of general formulae **(I),** (**I-a**), (**I-b**), (**I-c**), (**I-d**), **(11-1),** (**II-2)**, (**II-3**), (**II-4**), (**II-5**), and (**II-6**) as active agent.

Suitable binders include starch, gelatin, natural sugars, corn sweeteners, natural and synthetic gums such as acacia, sodium alginate, carboxymethylcellulose, polyethylene glycol and waxes. Among suitable lubricants there may be mentioned boric acid, sodium benzoate, sodium acetate, sodium chloride, and the like.

Suitable disintegrants include starch, methylcellulose, guar gum, and the like. Sweetening and flavoring agents as well as preservatives may also be included, where appropriate. The disintegrants, diluents, lubricants, binders etc. are discussed in more detail below.

Moreover, the pharmaceutical compositions of the present invention may be formulated in sustained release form to provide the rate controlled release of any one or more of the components or active ingredients to optimize the therapeutic effect(s), e.g. antihistaminic activity and the like. Suitable dosage forms for sustained release include tablets having layers of varying disintegration rates or controlled release polymeric matrices impregnated with the active components and shaped in tablet form or capsules containing such impregnated or encapsulated porous polymeric matrices.

Liquid form preparations include solutions, suspensions, and emulsions. As an example, there may be mentioned water or water/propylene glycol solutions for parenteral injections or addition of sweeteners and opacifiers for oral solutions, suspensions, and emulsions. Liquid form preparations may also include solutions for intranasal administration. Aerosol preparations suitable for inhalation may include solutions and solids in powder form, which may be present in combination with a pharmaceutically acceptable carrier such as an inert, compressed gas, e.g. nitrogen. For preparing suppositories, a low melting fat or wax, such as a mixture of fatty acid glycerides like cocoa butter is melted first, and the active ingredient is then dispersed homogeneously therein e.g. by stirring. The molten, homogeneous mixture is then poured into conveniently sized molds, allowed to cool, and thereby solidified.

Also included are solid form preparations that are intended to be converted, shortly before use, to liquid form preparations for either oral or parenteral administration. Such liquid forms include solutions, suspensions, and emulsions.

The compounds according to the present invention may also be delivered transdermally. The transdermal compositions may have the form of a cream, a lotion, an aerosol and/or an emulsion and may be included in a transdermal patch of the matrix or reservoir type as is known in the art for this purpose.

The term capsule as recited herein refers to a specific container or enclosure made e.g. of methyl cellulose, polyvinyl alcohols, or denatured gelatins or starch for holding or containing compositions comprising the active ingredient(s). Capsules with hard shells are typically made of blended of relatively high gel strength gelatins from bones or pork skin. The capsule itself may contain small amounts of dyes, opaquing agents, plasticisers and/or preservatives. Under tablet a compressed or molded solid dosage form is understood, which comprises the active ingredients with suitable diluents. The tablet may be prepared by compression of mixtures or granulations obtained by wet granulation, dry granulation, or by compaction well known to a person of ordinary skill in the art.

Oral gels refer to the active ingredients dispersed or solubilized in a hydrophilic semi-solid matrix. Powders for constitution refers to powder blends containing the active ingredients and suitable diluents which can be suspended e.g. in water or in juice.

Suitable diluents are substances that usually make up the major portion of the composition or dosage form. Suitable diluents include sugars such as lactose, sucrose, mannitol, and sorbitol, starches derived from wheat, corn rice, and potato, and celluloses such as microcrystalline cellulose. The amount of diluent in the composition can range from about 5 to about 95 % by weight of the total composition, preferably from about 25 to about 75 weight %, and more preferably from about 30 to about 60 weight %.

The term disintegrants refers to materials added to the composition to support break apart (disintegrate) and release the pharmaceutically active ingredients of a medicament. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures. The amount of disintegrant in the composition may range from about 2 to about 20 weight % of the composition, more preferably from about 5 to about 10 weight %.

Binders are substances which bind or "glue" together powder particles and make them cohesive by forming granules, thus serving as the "adhesive" in the formulation. Binders add cohesive strength already available in the diluent or bulking agent. Suitable binders include sugars such as sucrose, starches derived from wheat corn rice and potato, natural gums such as acacia, gelatin and tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminum silicate. The amount of binder in the composition may range from about 2 to about 20 weight % of the composition, preferably from about 3 to about 10 weight %, and more preferably from about 3 to about 6 weight %.

Lubricants refer to a class of substances, which are added to the dosage form to enable the tablet granules etc. after being compressed to release from the mold or die by reducing friction or wear. Suitable lubricants include metallic stearates such as magnesium stearate, calcium stearate, or potassium stearate, stearic acid, high melting point waxes, and other water soluble lubricants such as sodium chloride, sodium benzoate, sodium acetate, sodium oleate, polyethylene glycols and D,L-leucine. Lubricants are usually added at the very last step before compression, since they must be present at the surface of the granules. The amount of lubricant in the composition may range from about 0.2 to about 5 weight % of the composition, preferably from about 0.5 to about 2 weight %, and more preferably from about 0.3 to about 1.5 weight % of the composition.

Glidents are materials that prevent caking of the components of the pharmaceutical composition and improve the flow characteristics of granulate so that flow is smooth and uniform. Suitable glidents include silicon dioxide and talc. The amount of glident in the composition may range from about 0.1 to about 5 weight % of the final composition, preferably from about 0.5 to about 2 weight %.

Coloring agents are excipients that provide coloration to the composition or the dosage form. Such excipients can include food grade dyes adsorbed onto a suitable adsorbent such as clay or aluminum oxide. The amount of the coloring agent may vary from about 0.1 to about 5 weight % of the composition, preferably from about 0.1 to about 1 weight %.

The pharmaceutical compositions of the present invention are suitable for the treatment and/or prophylaxis of proliferative diseases, tumours, cancers, metastases, immunological disorders, autoimmune diseases and inflammatory disorders.

Another aspect of the present invention relates to drug combinations and pharmaceutical compositions comprising at least one compound of general formulae **(I),** (**I-a**), (**I-b**), (**I-c**), (**I-d**), **(11-1),** (**II-2)**, (**II-3**), (**II-4**), (**II-5**), and (**II-6**) as active agent together with at least one pharmaceutically acceptable carrier, excipient and/or diluent and one or more other anti-tumor agents or anti-retroviral drugs. As used herein the term "drug combination" refers to a combination of at least two pharmaceutically active agents or therapeutic agents with or without further ingredients, carrier, diluents and/or solvents.

Compounds of formulae **(I),** (**I-a**), (**I-b**), (**I-c**), (**I-d**), (**II-1)**, (**II-2)**, (**II-3**), (**II-4**), (**II-5**), and (**II-6**) may be administered as the sole pharmaceutical agent or in combination with one or more additional therapeutic agents, wherein the drug combination causes no unacceptable adverse effects. This combination therapy includes administration of a single pharmaceutical dosage formulation, which contains a compound of any one of formulae **(I),** (**I-a**), (**I-b**), (**I-c**), (**I-d**), (**II-1)**, (**II-2)**, (**II-3**), (**II-4),** (**II-5**), and (**II-6**) and one or more additional therapeutic agents in form of a single pharmaceutical composition, as well as administration of the compound of formulae **(I),** (**I-a**), (**I-b**), (**I-c**), (**I-d**), (**II-1)**, (**II-2)**, (**II-3**), (**II-4**), (**II-5**), and (**II-6**) and each additional therapeutic agent in its own separate pharmaceutical dosage formulation, i.e. in its own separate pharmaceutical composition. For example, a compound of any one of formula **(I),** (**I-a**), (**I-b**), (**I-c**), (**I-d**), (**II-1)**, (**II-2)**, (**II-3**), (**II-4**), (**II-5**), and (**II-6**) and a therapeutic agent may be administered to the patient together in a single oral dosage composition such as a tablet or capsule, or each agent may be administered in separate pharmaceutical compositions.

Where separate pharmaceutical compositions are used, the compound of formulae **(I),** (**I-a**), (**I-b**), (**I-c**), (**I-d**), (**II-1)**, (**II-2)**, (**II-3**), (**II-4**), (**II-5**), and (**II-6**) and one or more additional therapeutic agents may be administered at essentially the same time (e.g. concurrently) or at separately staggered times (e.g. sequentially). The compounds of the present invention may also be employed in cancer treatment in conjunction with radiation therapy and/or surgical intervention.

Furthermore, the compounds of formulae **(I),** (**I-a**), (**I-b**), (**I-c**), (**I-d**), (**II-1)**, (**II-2)**, (**II-3**), (**II-4**), (**II-5**), and (**II-6**) may be utilized, as such or in compositions, in research and diagnostics, or as analytical reference standards, and the like, which are well known in the art.

### Description of the figures:

- **Figure 1:**: shows melting temperature change for precursor microRNA-17 hairpin treated with indicated compounds (A: **A1,** B: **A3,** C: **A2,** D: **A4,** E: **A11)** in comparison with that of the DMSO control.
- **Figure 2:**: shows melting temperature change for precursor microRNA-21 hairpin treated with indicated compounds (A: **A1,** B: **A3,** C: **A2,** D: **A4,** E: **A11)** in comparison with that of the DMSO control.
- **Figure 3**: shows colony formation results for compounds **A10, A11, B2, B5, B8,** and **B9** in comparison to DMSO. Compounds **A10, A11, B2, B5, B8, B9** can inhibit MDA-MB-231 cells proliferation, **B2** and **B8** have better antiproliferation ability compare to other selected compounds.
- **Figure 4**: shows anticancer activity of the inventive compounds with IC₅₀ curves in different cancer cell lines. Most tested compounds showed potent anticancer effect in selected cancer cell lines.

The following examples are included to demonstrate preferred embodiments of the invention. It should be appreciated by those of skill in the art that the techniques disclosed in the following examples represent techniques discovered by the inventor to function well in the practice of the invention, and thus can be considered to constitute preferred modes for its practice. However, those of skill in the art should, in light of the present disclosure, appreciate that many changes can be made in the specific embodiments, which are disclosed and still obtain a like or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

### EXAMPLES

### A. Chemical Synthesis

### Abbreviations and Acronyms:

- DBU:: 1,8-Diazabicyclo[5.4.0]undec-7-ene;
- DCM:: Dichloromethane;
- DIBAL:: Diisobutylaluminium hydride;
- DIPEA:: N, N-Diisopropylethylamine;
- DMF:: Dimethylfomamide;
- Et2O:: Diethyl ether;
- EtOAc:: Ethyl acetate;
- Et3N:: Triethylamine;
- EtOH:: Ethanol;
- h:: hour;
- HOBt:: 1-Hydroxybenzotriazole;
- HPLC:: High-performance liquid chromatography;
- MeOH:: Methaol;
- min:: minute;
- MS:: Molecular Sieves;
- Ms:: Methanesulfonyl;
- MW:: Microwave;
- NIS:: N-iodosuccinimide;
- Ns:: p-nitrobenzenesulfonyl;
- PE:: Petroleumbenzin;
- pH:: potential of hydrogen;
- PyBOP:: Benzotriazol-1-yl-oxytripyrrolidinophosphonium-hexafluorophosphate;
- RM:: Reaction mixture;
- rt:: room temperature;
- TFA:: Trifluoroacetic acid;
- TfOH:: Triflic acid;
- THF:: Tetrahydrofuran;
- TMS:: Trimethylsilyl;
- TLC:: Thin layer chromatography.

### General information for chemical synthesis:

Commercial reagents purchased from Sigma Aldrich, BLD Pharmatech GmbH, TCI Deutschland GmbH were used without further purification, except where noted. Solvents were dried and redistilled prior to use in the usual way. All reactions were performed in oven-dried glassware under an inert atmosphere, unless noted otherwise. Analytical thin layer chromatography (TLC) was performed on Kieselgel 60 F254 aluminium plates pre-coated with a 0.25 mm thickness of silica gel. The TLC plates were visualized with UV light and by staining with Hanessian solution (ceric sulfate and ammonium molybdate in aqueous sulfuric acid) or sulfuric acid-ethanol solution.

Analytical UHPLC-MS and LC-MS was performed on an Agilent 1290 Infinity system equipped with a mass detector. Appropriate gradient systems were applied by mixing Water (+ 0.1% TFA) and Acetonitrile (+ 0.1%). Purification of the products was performed by either column chromatography using Fluka Kieselgel 60 (230-400 mesh) or an Agilent 1100 preparative HPLC system equipped with a mass detector. ¹H and ¹³C NMR spectra were measured with a Varian 400-MR, or Varian 700 spectrometer with Me₄Si as the internal standard. NMR chemical shifts (δ) were recorded in ppm and coupling constants (J) were reported in Hz. High-resolution mass spectra (HRMS) were recorded with an Agilent 6210 ESI-TOF mass spectrometer at the Max-Planck-Institute of Molecular Physiology

**General procedure 2 for the synthesis of imines.** Rigorously dried 4Å molecular sieves (1.0 g per mmol) were added to a solution of amine in DCM (5 mL per mmol) under N₂ at rt. After a period of 5 min, carbonyl compound (1.0 equiv.) was added to the reaction mixture, which was left stirred overnight at rt before filtration through celite and removal of solvents in *vacuo,* to yield the crude imine. Imines was judged >95% pure by ¹H NMR, and used in the next step without further purification.

**General procedure 3 for the synthesis of sulfonyl azetidinimines**. To a solution of sulfonyl azide (1.0 mmol) in DCM (2 mL) at rt was treated sequentially with Et₃N (2.0 mmol), the alkyne (1.0 mmol), the imine (1.2 mmol), and Cul (0.1 mmol). The reaction mixture was then stirred at room temperature under N₂ for 3-5 h, solvent was removed *in vacuo,* and the product was purified by silica gel column chromatography or prep-HPLC.

**General procedure 4 for the de-protection of the Ns functional group from sulfonyl azetidinimines.** To a solution of p-nitrobenzenesulfonyl azetidinimine (0.1 mmol) in DMF was added K₂CO₃ (0.5 mmol, 5.0 equiv.) followed by benzene thiol (0.25 mmol, 2.5 equiv.). The resulting suspension was stirred at rt for 3 h before the solvent was removed *in vacuo.* The crude product was purified by silica gel column chromatography or prep-HPLC to afford the pure product.

### Example A.1: Synthesis of 1-(3-aminothieno[2,3-b] pyridine-2-carbonyl)-4-(hydroxymethyl)-3-(4'-(trifluoromethyl)-[1,1'-biphenyl]-4-yl) azetidine-2-carbonitrile (A1)

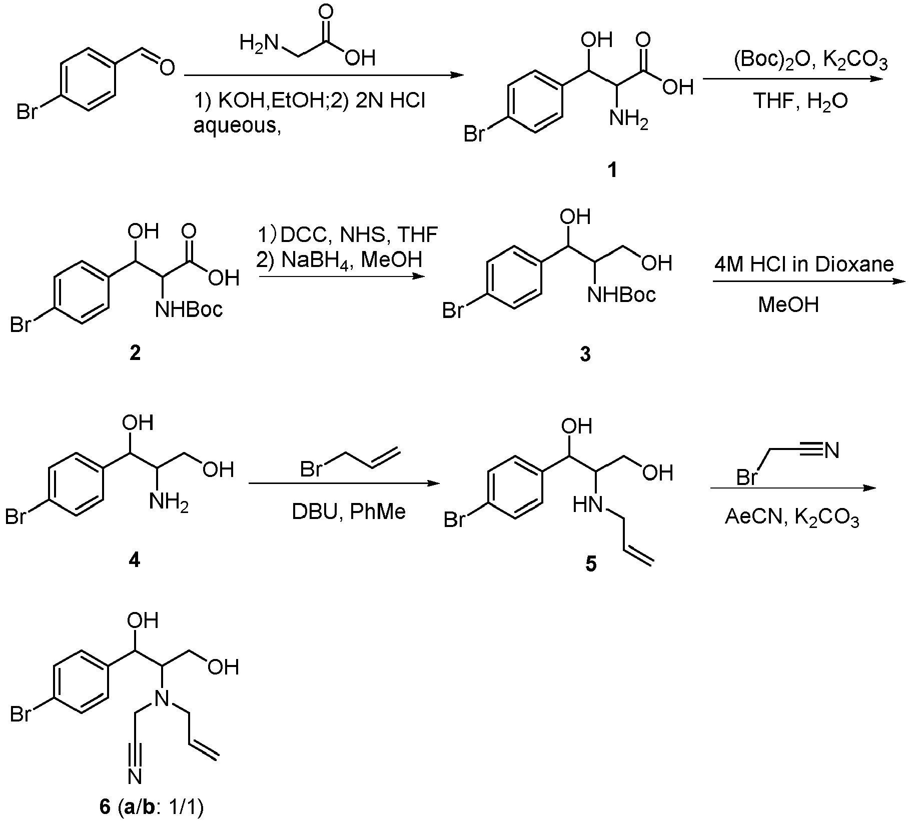

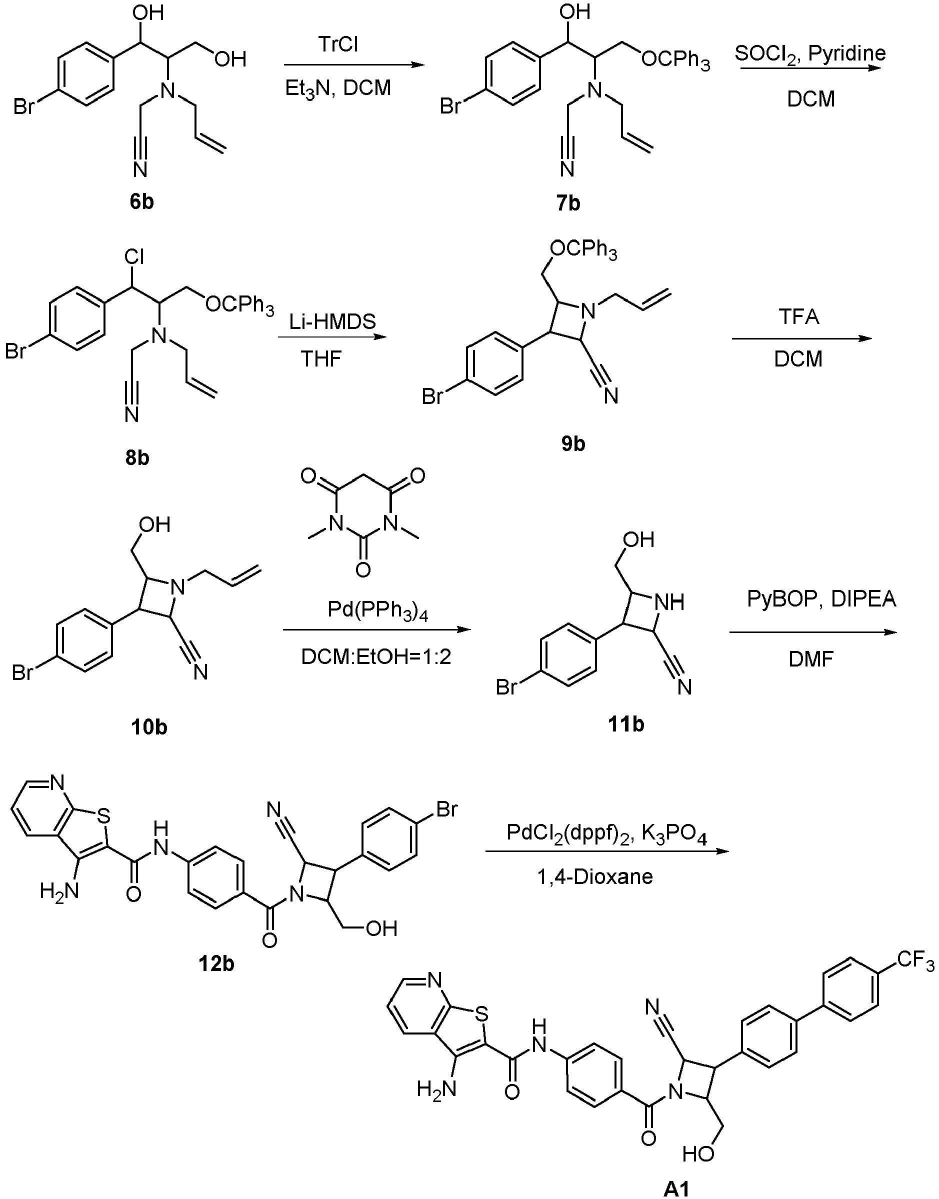

### Intermediate 1: Synthesis of 2-amino-3-(4-bromophenyl)-3-hydroxypropanoic acid

4-Bromobenzaldehyde (100 g, 0.54 mol) was dissolved in ethanol (EtOH) in a 2 L round-bottom flask. To the solvent glycine (20.30 g, 0.27 mol) in ice bath, was added KOH (30.30 g, 0.54 mol). It is essential to add the KOH all at once. After ~15 min, a thick white precipitate began to form. Then, the reaction mixture was stirred for 12 h at rt under N₂. 2N HCI aqueous solution was added (400 mL) to adjust the pH of the solution to ~4. The mixture was then stirred at 60 °C until it became a homogeneous yellow solution. The solvent was removed under vacuum to give a suspension with white precipitate. The precipitate was filtered and the remaining aqueous solution was washed with EtOAc (200 mL) three times. The pH of the aqueous solution was then basified to 9 with concentrated ammonia solution, and excess ammonia was removed *in vacuo.* Evaporation of the reaction mixture to one-quarter of the volume when the formation of the precipitate was first observed. Filter the mixture and the obtained filter cake was dried *in vacuo* to yield the intermediate **1** (51.30 g, 0.2 mol).

¹H NMR (400 MHz, CD₃OD): δ 3.64 (d, 1H, J = 3.6 Hz), 5.25 (d, 1H, J = 3.6Hz), 7.41 (d, 2H, J = 8.4 Hz), 7.53 (d, 2H, J = 8.4 Hz).

### Intermediate 2: Synthesis of 3-(4-bromophenyl)-2-((tert-butoxycarbonyl) amino)-3-hydroxypropanoic acid.

Intermediate **1** (32.60 g, 12.53 mmol) was dissolved in 10% K₂CO₃ (10% w/v; 500 mL) aqueous solution. Di-tert-butyl dicarbonate (34.20 g, 157.00 mmol) was dissolved in 1,4-dioxane (500 mL) and added to the aqueous solution, before the mixture was stirred at rt for 48 h. The mixture was concentrated *in vacuo* and taken up in 1 N NaOH aqueous solution (100 mL) and washed with Et₂O (100 mL) twice. The aqueous layer was acidified with 1 N HCI aqueous solution and the product was extracted with EtOAc (200 mL) three times. The organic phase was dried over anhydrous MgSO₄, filtered, and concentrated *in vacuo.* Intermediate **2** (26.70 g, 74.0 mmol) was isolated and used in the next step without further purification.

¹H NMR (400 MHz, CD₃OD): δ 1.32 (s, 9H), 4.37 (d, 1H, J = 2.7 Hz), 5.23 (d, 1H, J = 2.7 Hz), 7.32 (d, 2H, 8.4 Hz), 7.46 (d, 2H, J = 8.4 Hz).

### Intermediate 3: tert-butyl (1-(4-bromophenyl)-1,3-dihydroxypropan-2-yl) carbamate

Intermediate **2** (5.29 g, 14.70 mmol) and *N*-hydroxy-succinimide (1.69 g, 14.70 mmol) were dissolved in EtOAc (200 mL) and the mixture cooled to 0 °C. *N, N*'-dicyclo-hexyl-carbodiimide (3.04 g, 14.70 mmol) was added and the reaction mixture was stirred for 30 min, warmed to rt and stirred for another 30 min. It was then cooled to 0 °C and filtered to remove the precipitated *N*, *N*'-dicyclo-hexylurea by-product. The filtrate was concentrated *in vacuo* and the residue dissolved in THF (100 mL). The solution was cooled to 0 °C and added NaBH₄(5.60 g, 150.00 mmol). The mixture was stirred for 2 min and then water was added to the mixture dropwise until bubbling stopped, followed by the addition of equal volume of THF in a duration of 30 min. The mixture was warmed to rt and stirred for 5 h. The dioxane was removed *in vacuo* and EtOAc (200 mL) was added, followed by gradual acidification of the aqueous layer with 1 N HCI aqueous solution. The EtOAc layer was washed with brine and dried over anhydrous Mg₂SO₄. Filtered the mixture and the filtrate was concentrated *in vacuo* to get crude product (4.56 g, 12.66 mmol). The crude product was purified with silica gel column chromatography, eluting with EtOAc/PE (1:4) to get the Intermediate 3 as a white solid (4.31 g, 12.4 mmol).

¹H NMR (400 MHz, CD₃OD): δ 1.32 (s, 9H), 3.46-3.51 (m, 1H), 3.63-3.73 (m, 2H), 4.88 (br s, 1H), 7.29 (d, 2H, J = 8.3 Hz), 7.45 (d, 2H, J = 8.3 Hz).

### Intermediate 4: Synthesis of 2-amino-1-(4-bromophenyl) propane-1,3-diol

Intermediate **3** (4.31 g, 12.4 mmol) was dissolved in 4M HCI in dioxane. The mixture was stirred for 2 h at rt and concentrated under vacuum. The HCI salt was partitioned between 50 mL of 2 N NaOH aqueous solution and an equal volume of EtOAc. The aqueous layer was washed with EtOAc (50 mL). The combined EtOAc fractions were washed with brine, dried over anhydrous MgSO₄ and concentrated under vacuum to give a waxy solid (2.72 g, 11.1 mmol), which was used without further purification.

¹H NMR (400 MHz, CD₃OD): δ 2.83-2.90 (m, 1H), 3.30-3.35 (m, 1H), 3.46 (dd, 1H, J = 10.8Hz, 4.8 Hz), 4.56 (d, 1H, J = 6.6 Hz), 7.29 (d, 2H, J = 8.6 Hz), 7.49 (d, 2H, J = 8.6 Hz).

### Intermediate 5: Synthesis of 2-(allylamino)-1-(4-bromophenyl) propane-1,3-diol

Intermediate **4** (2.72 g, 11.1 mmol) was dissolved in PhMe (50 mL). DBU (1.68 g, 11.1 mmol) was added to the reaction mixture, which was stirred for 10min at rt. 3-Bromoprop-1-ene (1.34 g, 11.10 mmol) was then added to the reaction mixture, followed by stirring at 40 °C for 2 h. Water (100 mL) was then added to the reaction solution followed by EtOAc (50 mL). The aqueous phase was extracted by EtOAc (100 mL) three times. The combined organic phase was washed with brine and dried over MgSO₄. Filtered the mixture and the filtrate was concentrated *in vacuo* to get the crude product. Purified by silica gel column chromatography to get the intermediate **5** (1.59 g, 5.6 mmol).

### Intermediates 6a and 6b: Synthesis of 2-(allyl(1-(4-bromophenyl)-1,3-dihydroxypropan-2-yl) amino) acetonitrile

Intermediate **5** (1.59 g, 5.6 mmol) was dissolved in acetonitrile (50 mL). To this solution, potassium carbonate (1.54 g, 11.11 mmol) and 2-bromoacetonitrile (2.00 g, 16.67 mmol) were added and the resulting heterogeneous mixture was heated to 85 °C. After stirring the mixture for 3 h, the reaction mixture was concentrated under reduced pressure. The resulting residue was dissolved in water and EtOAc. Extract the aqueous layer two additional times with EtOAc. The combined EtOAc was washed with brine and dried by MgSO₄. The combined organic phases were dried over MgSO₄, filtered and the filtrate was concentrated under reduced pressure to obtain the crude product, which was purified by chromatography over silica gel to obtain intermediate **6a** (0.76 g, 2.3 mmol) and **6b** (0.72 g, 2.2 mmol).

### Intermediate 7b: Synthesis of 2-(allyl(1-(4-bromophenyl)-1-hydroxy-3-(trityloxy) propan-2-yl) amino) acetonitrile

Intermediate **6b** (0.72 g, 2.2 mmol) was dissolved in DCM (50 mL). Triethylamine (0.67 g, 6.6 mmol) was added to the solution of intermediate **7b.** Cooled the solution to 0 °C. Added the trityl chloride (0.93 g, 3.3 mmol). The mixture was stirred to slowly warm to rt for overnight. The reaction mixture was quenched with saturated NH₄Cl aqueous solution, extracted with DCM twice, the combined organic phase dried over MgSO₄, filtered and the filtrate was concentrated in *vacuo* to obtain the crude product, which was purified by silica gel column chromatography, eluting with EtOAc/PE (gradient: 1:1) to obtain the intermediate **7b** (0.78 g, 1.37 mmol)

### Intermediate 8b: Synthesis of 2-(allyl(1-(4-bromophenyl)-1-chloro-3-(trityloxy) propan-2-yl) amino) acetonitrile

To a solution of pyridine (0.54 g, 6.9 mmol) in DCM (30 mL) was added thionyl chloride (0.33 g, 2.8 mmol) at rt. Cooled the solution to 0 °C. Intermediate **7b** was dissolved in DCM (5 mL) and added to the reaction solution dropwise over 3min. The mixture was stirred for additional 15 min at 0 °C. The mixture was neutralized by the addition of saturated sodium bicarbonate aqueous solution. The mixture was extracted two additional times with DCM. Dried the combined organic phase over MgSO₄. Filter the mixture and the filtrate was concentrated *in vacuo* to obtain the crude product. Purify the residue by silica gel column chromatography, eluting with 9:91 EtOAc:PE to obtain the intermediate **8b** (0.58 g, 1.0 mmol).

### Intermediate 9b: Synthesis of 1-allyl-3-(4-bromophenyl)-4-((trityloxy)methyl) azetidine-2-carbonitrile

The intermediate **8b** (0.55 g 0.98 mmol) was dissolved in THF (30 mL). Stirred and Cooled the solution to -78 °C. The lithium bis(trimethylsilyl) amide (1M solution in THF, 1.5 mL, 1.5 mmol) was added dropwise to the mixture over 3 min.The resulting reaction mixture was stirred for 1 h at -78 °C. The reaction mixture was quenched with saturated NH₄Cl aqueous solution. The product was extracted three times with EtOAc (30 mL). The combined organic phases were dried over MgSO₄, filtered, and the obtained filtrate was concentrated *in vacuo* to give the crude product, which was purified by silica gel column chromatography eluting with 9:91 EtOAc/PE to obtain the intermediate **9b** (0.45 g, 0.82 mmol).

### Intermediate 10b: Synthesis of 1-allyl-3-(4-bromophenyl)-4-(hydroxymethyl) azetidine-2-carbonitrile

The intermediate **9b** (0.40 g, 0.73 mmol) was dissolved in DCM (20 mL). Cooled the reaction solution to 0 °C. TFA (0.42 g, 3.65 mmol) was added to the mixture. The mixture was stirred to slowly warm to rt for overnight. The reaction mixture was quenched with saturated Na₂CO₃ aqueous solution. The product was extracted three times with EtOAc (30 mL), the combined organic phases were dried over MgSO₄, filtered, and the filtrate was concentrated to obtain the crude product, which was purified by silica gel column chromatography, eluting with EtOAc/PE (gradient: 1:4-1:1) to obtain the intermediate **10b** (0.21 g, 0.68 mmol).

### Intermediate 11b: Synthesis of 3-(4-bromophenyl)-4-(hydroxymethyl) azetidine-2-carbonitrile

Intermediate **10b** (100.0 mg, 0.33 mmol) was dissolved in DCM/EtOH (v/v 1:2, 12 mL). To the reaction mixture were added 1,3-dimethylbarbituric (76.3 mg, 0.49 mmol) and Pd(PPh₃)₄ (18.8 mg, 0.016 mmol) under N₂ protection. The mixture was heated to 40 °C overnight. The solvent was removed *in vacuo.* The residue was purified by silica gel column chromatography, eluting with 40:1 DCM/MeOH to obtain the intermediate **11b** (70.23 mg, 0.26 mmol).

### Intermediate 12b: Synthesis of 3-amino-N-(4-(3-(4-bromophenyl)-2-cyano-4-(hydroxymethyl) azetidine-1-carbonyl) phenyl) thieno[2,3-b] pyridine-2-carboxamide

4-(3-aminothieno[2,3-b]pyridine-2-carboxamido)benzoic acid (80.9 mg, 258.2 µmol) was dissolved in DMF (10 mL), DIPEA (100.0 mg, 770.2 µmol) and PyBOP (147 mg, 386.0 µmol) was added to the solution. The mixture was stirred at rt for 10 min. Intermediate **11b** (68.8 mg, 257.7 µmol) was dissolved in DMF (1 mL) dropwise to the reaction solution. The mixture was stirred at rt for overnight. The solvent was removed *in vacuo.* The residue was purified by silica gel column chromatography, eluting with 40:1 DCM/MeOH to obtain the intermediate **12b** (118.1 mg, 0.21 mmol).

### Synthesis of A1: Synthesis of 1-(3-aminothieno[2,3-b] pyridine-2-carbonyl)-4-(hydroxymethyl)-3-(4'-(trifluoromethyl)-[1,1'-biphenyl]-4-yl) azetidine-2-carbonitrile

**General procedure 1:** Intermediate **12b** (12.7 mg, 22.6 µmol) was dissolved in 1,4-dioxane. (4-(trifluoromethyl) phenyl) boronic acid (11.8 mg, 33.8 µmol) and K₃PO₄ (14.4 mg, 67.7 µmol)were added to the solution under N₂ protection. The mixture was heated to 90 °C overnight. The solvent was removed *in vacuo.* The residue was purified by prep-HPLC to obtain **A1,** white solid (5.6 mg, 9.0 µmol).

### Example A.2: Synthesis of 1-(3-aminothieno[2,3-b] pyridine-2-carbonyl)-4-(hydroxymethyl)-3-(4'-(trifluoromethyl)-[1,1'-biphenyl]-4-yl) azetidine-2-carbonitrile (A2)

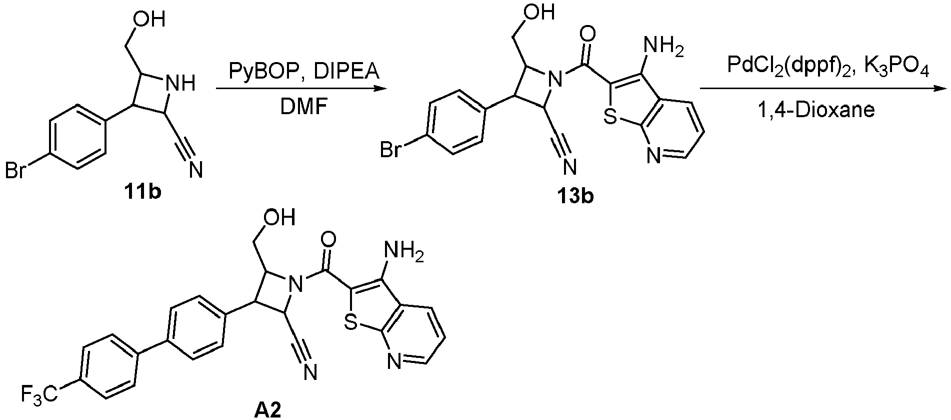

### Intermediate 13b: Synthesis of 1-(3-aminothieno[2,3-b] pyridine-2-carbonyl)-3-(4-bromophenyl)-4-(hydroxymethyl) azetidine-2-carbonitrile

3-Aminothieno[2,3-b]pyridine-2-carboxylic acid (50.0 mg, 258.2 µmol) was dissolved in DMF (10 mL), DIPEA (100.0 mg, 770.2 µmol) and PyBOP (147.0 mg, 386.0 µmol) was added to the reaction mixture. The mixture was stirred at rt for 10 min. Intermediate **11b** (68.8 mg, 257.7 µmol) was dissolved in DMF (1 mL) and added dropwise to the reaction mixture. The mixture was stirred at rt for overnight. The mixture was removed *in vacuo.* The residue was purified by silica gel column chromatography, eluting with 40:1 DCM/MeOH to obtain the intermediate **12b** (93.1 mg, 210.3 µmol).

### Synthesis of A2: Synthesis of 1-(3-aminothieno[2,3-b] pyridine-2-carbonyl)-4-(hydroxymethyl)-3-(4'-(trifluoromethyl)-[1,1'-biphenyl]-4-yl) azetidine-2-carbonitrile

The corresponding product was obtained following the general procedure 1 for **A1.** The residue was purified by prep-HPLC to obtain **A2** (2.68 mg, 5.63 µmol).

### Example A.3: Synthesis of 1-(3-aminothieno[2,3-b] pyridine-2-carbonyl)-4-(hydroxymethyl)-3-(4'-(trifluoromethyl)-[1,1'-biphenyl]-4-yl) azetidine-2-carbonitrile (A3)

**Synthesis of Intermediates 7a, 8a, 9a, 10a, 11a, 12a** were obtained following the procedure for Intermediate **7b, 8b, 9b, 10b, 11b, 12b.**

### Synthesis of A3: Synthesis of 1-(3-aminothieno[2,3-b] pyridine-2-carbonyl)-4-(hydroxymethyl)-3-(4'-(trifluoromethyl)-[1,1'-biphenyl]-4-yl) azetidine-2-carbonitrile

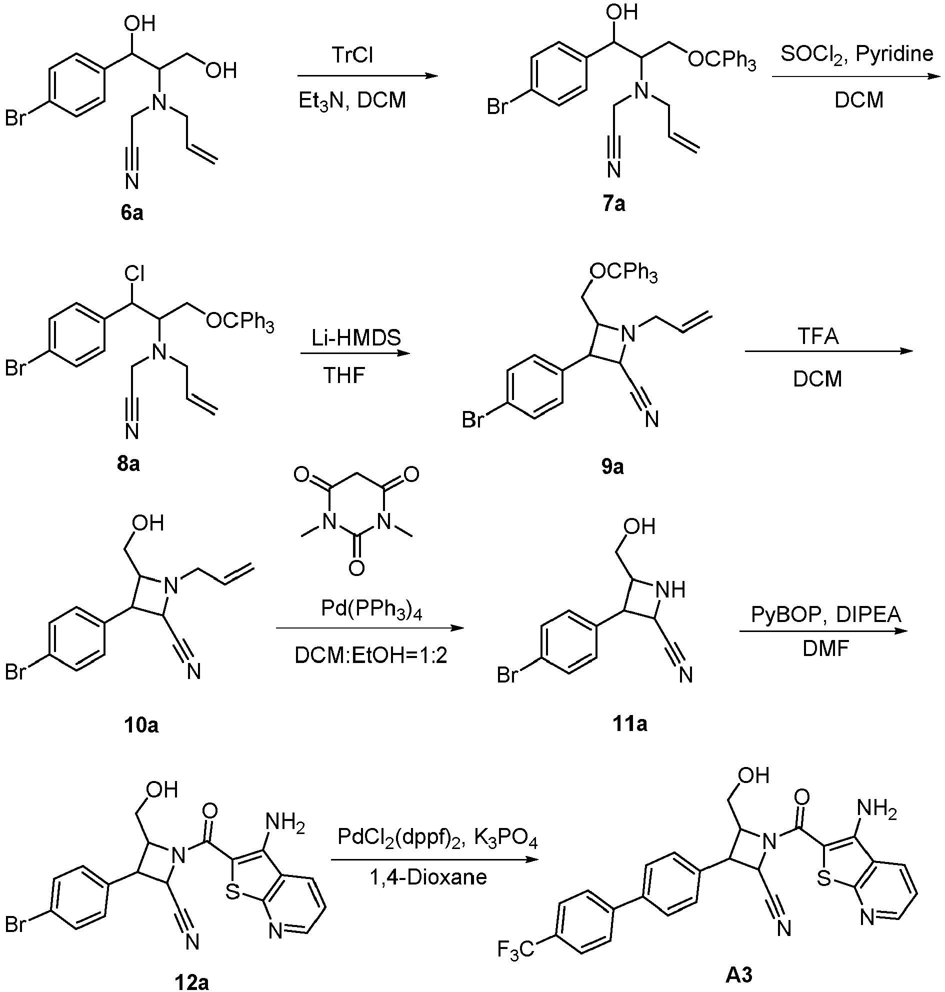

The corresponding product was obtained following the general procedure 1 for **A1.** The residue was purified by prep-HPLC to obtain **A3** (3.56 mg, 7.42 µmol)

### Example A.4: Synthesis of 3-amino-N-((4-(hydroxymethyl)-1-propyl-3-(4'-(trifluoromethyl)-[1,1'-biphenyl]-4-yl) azetidin-2-yl) methyl) thieno[2,3-b] pyridine-2-carboxamide (A5)

### Intermediate 11c: Synthesis of 3-(4-bromophenyl)-4-(hydroxymethyl)-1-propylazetidine-2-carbonitrile

Intermediate **10b** (100.0 mg, 325.7 µmol)was dissolved in MeOH (10 mL), and Pd/C (10.0 mg) was added under H₂ atmosphere. The mixture was stirred at rt for 15 min. Pd/C was filtered by diatomaceous earth. The filtrate was concentrated *in vacuo* to give the crude intermediate **11c** (96.0 mg, 321.5 µmol), which was used in the next step without further purification.

### Intermediate 12c: Synthesis of (4-(aminomethyl)-3-(4-bromophenyl)-1-propylazetidin-2-yl) methanol

Intermediate **11c** (96.0 mg, 321.5 µmol) was dissolved in DCM (20 mL) and the reaction mixture was cooled to 0 °C. DIBAL (1.3 mL, 1.9 mmol, 2.5 M in tolune) was added to the reaction mixture over 3 min, and the reaction mixture was allowed to slowly warm to rt and stirred for 1 h. The reaction mixture was quenched by slowly addition of methanol until gas evolution stopped. The saturated solution of sodium potassium tartrate was added to the mixture. The gel-like solution was then stirred until the separation of two layers. The aqueous layer was extracted twice with DCM (10 mL). The combined organic phases were dried over MgSO₄ and the filtrate was concentrated *in vacuo* to give the crude product, which was purified by silica gel column chromatography eluting with DCM: MeOH=20:1 to yield the intermediate **12c** (73.0 mg, 234.7 µmol).

### Intermediate 13c: Synthesis of 3-amino-N-((3-(4-bromophenyl)-4-(hydroxymethyl)-1- propylazetidin-2-yl) methyl) thieno[2,3-b] pyridine-2-carboxamide

3-Aminothieno[2,3-b]pyridine-2-carboxylic acid (40.0 mg, 206.6 µmol)was dissolved in DMF (10 mL), and DIPEA (100.0mg, 770.2 µmol)and PyBOP (147.0 mg, 386.0 µmol)were added to the reaction mixture, which was stirred at rt for 10 min. Intermediate **12c** (64.7 mg, 206.6 µmol ) dissolved in DMF (1 mL) was added dropwise to the reaction mixture, which was stirred at rt overnight. The solvent was removed *in vacuo.* The residue was purified by silica gel column chromatography, eluting with DCM: MeOH=40:1 to obtain the intermediate **13c** (56.3 mg, 115.0 µmol).

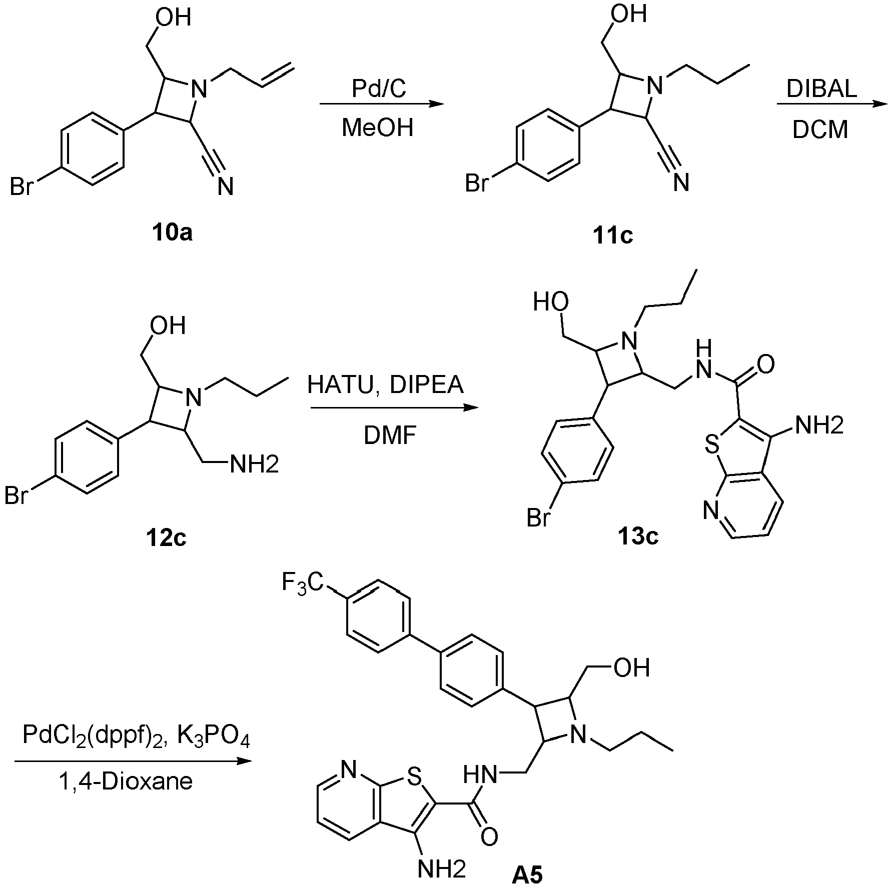

### Synthesis of A5: 3-amino-N-((4-(hydroxymethyl)-1-propyl-3-(4'-(trifluoromethyl)-[1,1'-biphenyl]-4-yl) azetidin-2-yl) methyl) thieno[2,3-b] pyridine-2-carboxamide

The corresponding product was obtained following the general procedure 1 for **A1.** The residue was purified by Prep-HPLC to obtain **A5** (3.86 mg, 6.92 µmol)

¹H NMR (700 MHz, DMSO-*d*6) δ 9.90 - 9.73 (m, 1H), 8.65 (ddd, *J* = 11.7, 4.6, 1.7 Hz, 1H), 8.44 (ddd, *J* = 20.0, 8.1, 1.6 Hz, 1H), 7.92 (dd, *J* = 23.3, 8.2 Hz, 2H), 7.86 - 7.79 (m, 4H), 7.63 - 7.58 (m, 2H), 7.46 (ddd, *J* = 14.8, 8.2, 4.5 Hz, 1H), 7.21 (s, 2H), 5.72 (d, *J* = 51.7 Hz, 1H), 5.12 - 5.07 (m, 1H), 4.67 (tt, *J* = 9.7, 4.5 Hz, 1H), 4.45 (t, *J* = 9.4 Hz, 1H), 4.30 (t, *J* = 9.8 Hz, 1H), 4.01 (dd, *J* = 13.9, 6.4 Hz, 1H), 3.87 - 3.80 (m, 2H), 3.57 - 3.53 (m, 1H), 3.39 - 3.35 (m, 1H), 3.16 (dtt, *J* = 18.2, 6.7, 3.7 Hz, 2H), 1.78 - 1.61 (m, 1H), 1.56 (tdt, *J* = 10.5, 7.5, 5.2 Hz, 1H), 0.91 (dt, *J* = 10.3, 7.3 Hz, 3H).

### Example A.5: Synthesis of N-((3-(4-(1H-benzo[d]imidazol-6-yl) phenyl)-4-(hydroxymethyl)-1- propylazetidin-2-yl) methyl)-3-aminothieno[2,3-b] pyridine-2-carboxamide (A9)

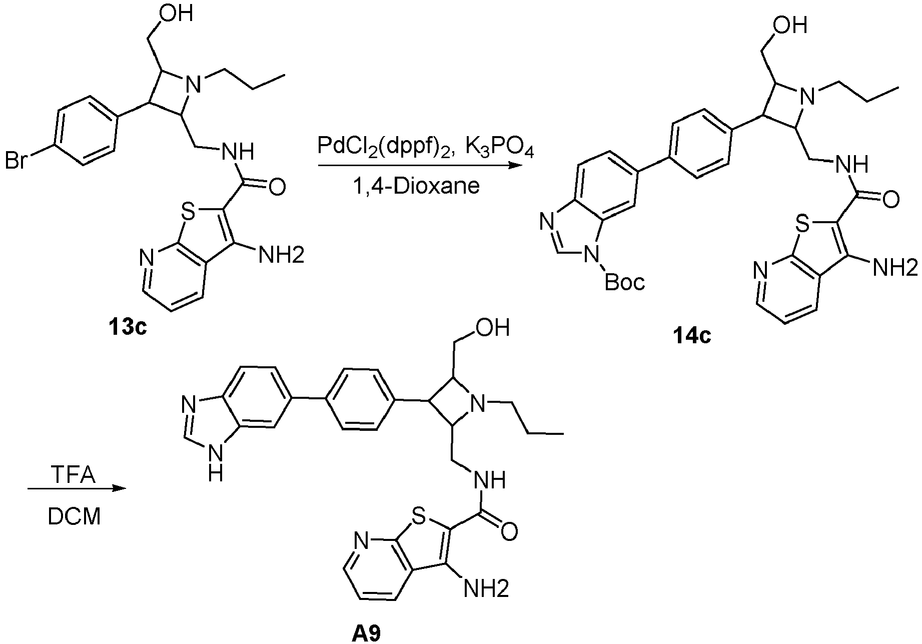

### Synthesis of A9: Synthesis of N-((3-(4-(1H-benzo[d]imidazol-6-yl)phenyl)-4-(hydroxymethyl)-1-propylazetidin-2-yl)methyl)-3-aminothieno[2,3-b]pyridine-2-carboxamide

Intermediate **13c** (100.0 mg, 0.2 mmol) was dissolved in 1,4-dioxane (10 mL), The corresponding product was obtained following the general procedure 1 for **A1.** The residue was purified by silica gel column chromatography eluting with DCM/MeOH (v/v 10:1) to obtain **14c** (58.2 mg, 0.09 mmol), which was then dissolved in DCM (15 mL), followed by the addition of 1 mL TFA and stirring at rt for 3 h. The solvent was removed and the residue was purified by prep-HPLC to obtain the product **A9** (8.69 mg, 16.52 µmol).

¹H NMR (700 MHz, DMSO-*d*6) δ 9.82 (d, *J* = 62.7 Hz, 1H), 9.16 (s, 1H), 8.65 (ddd, *J* = 11.7, 4.6, 1.7 Hz, 1H), 8.45 (ddd, *J* = 17.5, 8.2, 1.6 Hz, 1H), 8.02 (dd, *J* = 11.4, 1.7 Hz, 1H), 7.89 - 7.75 (m, 5H), 7.59 (dd, *J* = 11.0, 8.1 Hz, 2H), 7.46 (ddd, *J* = 14.6, 8.1, 4.5 Hz, 1H), 7.27 (d, *J* = 63.9 Hz, 2H), 5.72 (d, *J* = 54.9 Hz, 1H), 5.13 - 5.08 (m, 1H), 4.89 (d, *J* = 13.0 Hz, 1H), 4.67 (tt, *J* = 9.8, 4.4 Hz, 1H), 4.45 (t, *J =* 9.4 Hz, 1H), 4.30 (t, *J* = 9.8 Hz, 1H), 4.05 - 4.01 (m, 1H), 3.95 - 3.90 (m, 1H), 3.83 (d, *J* = 3.2 Hz, 1H), 3.39 - 3.34 (m, 1H), 3.15 (ddt, *J* = 28.1, 14.9, 4.2 Hz, 2H), 1.78 - 1.51 (m, 2H), 0.91 (dt, *J* = 10.1, 7.4 Hz, 3H).

### Example A.6: Synthesis of 1-allyl-4-((4-(pyridin-2-yl)-1H-1,2,3-triazol-1-yl) methyl)-3-(4'-(trifluoromethyl)-[1,1'-biphenyl]-4-yl) azetidine-2-carbonitrile (A10)

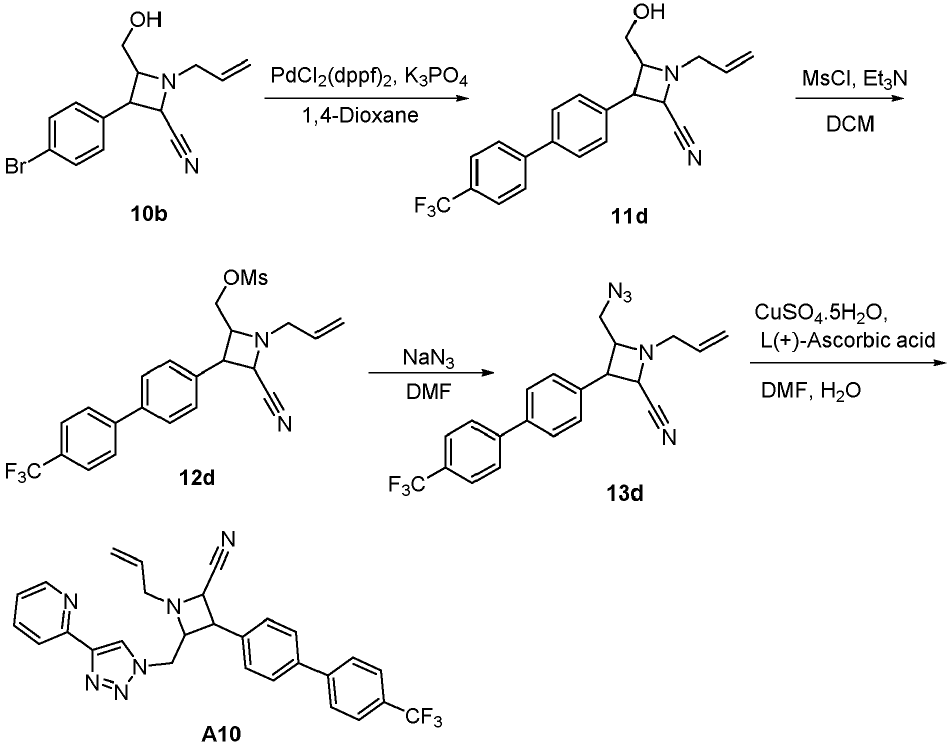

### Intermediate 11d: Synthesis of 1-allyl-4-(hydroxymethyl)-3-(4'-(trifluoromethyl)-[1,1'-biphenyl]-4-yl) azetidine-2-carbonitrile

Intermediate **10b** (50.0 mg, 162.9 µmol) was dissolved in 1,4-dioxane. The corresponding product was obtained following the general procedure 1 for **A1.** The residue was purified by silica gel column chromatography, eluting with EtOAc/PE(gradient: 1:6-1:4) to obtain the intermediate **11d** (46.02 mg, 123.73 µmol).

### Intermediate 12d: Synthesis of (1-allyl-4-cyano-3-(4'-(trifluoromethyl)-[1,1'-biphenyl]-4-yl) azetidin-2-yl) methyl methanesulfonate

Intermediate **11d** (40.0 mg, 135.0 µmol) was dissolved in DCM (10 mL) and cooled to 0 °C, MsCl (30.9 mg, 270.0 µmol) and Et₃N (41.0 mg, 405.1 µmol) were added to the reaction mixture, which was stirred at rt for 4 h. The reaction mixture was then quenched with saturated Na₂CO₃ aqueous solution and diluted with DCM. The organic phase was washed by brine and dried over MgSO₄. The filtrate was concentrated *in vacuo,* and the residue was purified by silica gel column chromatography, eluting with EtOAc/PE (gradient: 1:9) to obtain the intermediate **12d** (48.2 mg, 107.0 µmol).

### Intermediate 13d: Synthesis of 1-allyl-4-(azidomethyl)-3-(4'-(trifluoromethyl)-[1,1'-biphenyl]-4-yl) azetidine-2-carbonitrile

Intermediate **12d** (45.0 mg, 120.3 µmol) was dissolved in DMF (3 mL). NaN₃ (46.9 mg, 601.5 µmol) was added to the mixture. The mixture was heated to 60 °C for 5 h. The solvent was removed *in vacuo.* The residue was purified by silica gel column chromatography eluting with EtOAc/PE (gradient: 1:19) to obtain the intermediate **13d** (23.1 mg, 57.9 µmol).

### Synthesis of A10: Synthesis of 1-allyl-4-((4-(pyridin-2-yl)-1H-1,2,3-triazol-1-yl) methyl)-3-(4'-(trifluoromethyl)-[1,1'-biphenyl]-4-yl) azetidine-2-carbonitrile

Intermediate **13d** (4.0 mg, 10.1 µmol) was dissolved in DMF (5 mL). The mixture was stirred at rt. 2-Ethynylpyridine (2.1 mg, 20.2 µmol) was added to the solution followed by CuSO₄.5H₂O (5.0 mg, 20.2 µmol) and L (+)-Ascorbic acid (3.5 mg, 20.2 µmol). The mixture was stirred at rt overnight. The solvent was removed in *vacuo.* Purified the residue by prep-HPLC to obtain the final product **A10** (2.5 mg, 5.0 µmol).

¹H NMR (500 MHz, DMSO-*d*₆) δ 8.66 (s, 1H), 8.60 (d, *J* = 4.8 Hz, 1H), 8.03 (d, *J* = 7.9 Hz, 1H), 7.95 - 7.86 (m, 3H), 7.80 (d, *J* = 8.3 Hz, 2H), 7.77 - 7.70 (m, 2H), 7.39 (d, *J* = 8.2 Hz, 2H), 7.35 (dd, *J* = 7.5, 5.0 Hz, 1H), 5.78 (dddd, *J* = 17.1, 10.3, 6.7, 5.1 Hz, 1H), 5.24 (dq, *J* = 17.1, 1.6 Hz, 1H), 5.16 (dt, *J* = 10.4, 1.5 Hz, 1H), 4.82 (d, *J* = 8.1 Hz, 1H), 4.80 - 4.69 (m, 2H), 4.27 (dt, *J* = 8.8, 4.4 Hz, 1H), 3.77 (t, *J* = 8.5 Hz, 1H), 3.42 (ddt, *J* = 14.1, 5.2, 1.7 Hz, 1H), 3.18 (dd, *J* = 14.1, 6.7 Hz, 1H). MS⁺: Calculated for (C₂₈H₂₃F₃N₆)⁺, 501.53, Found: 501.49.

### Example A.7: Synthesis of 3-(4-(3,5-dimethylisoxazol-4-yl) phenyl)-1-(4-(methylamino) quinazolin-2-yl) azetidine-2-carbonitrile (B1)

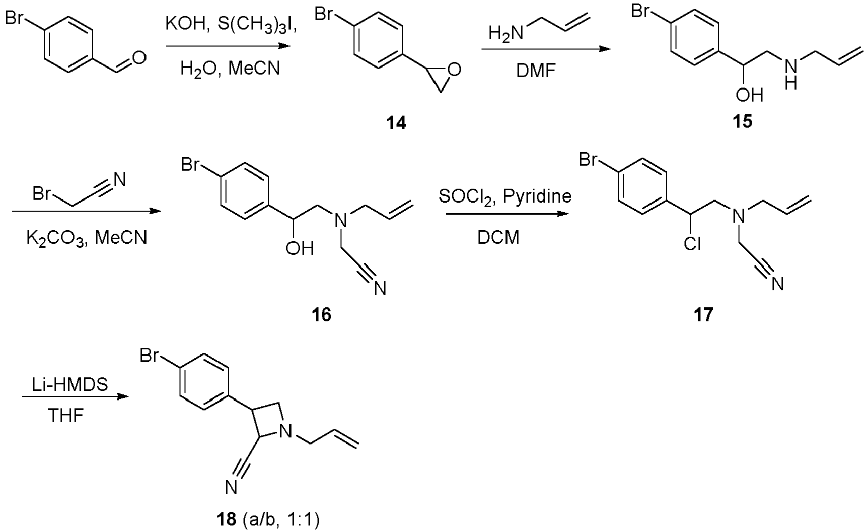

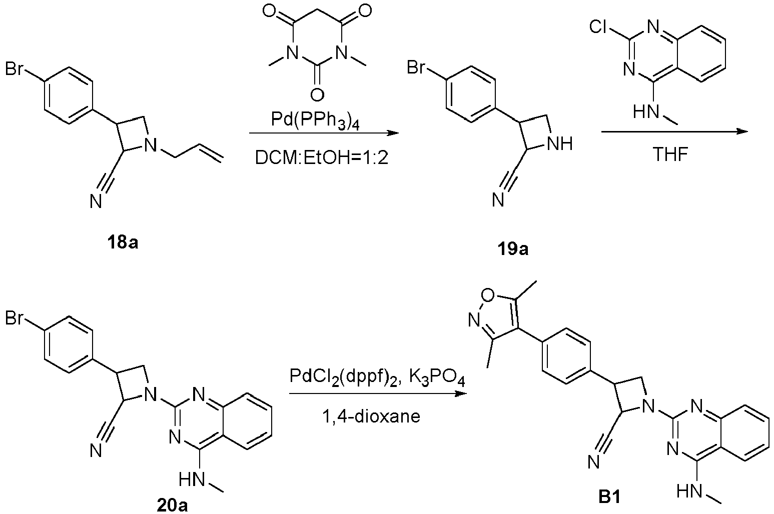

### Intermediate 14: Synthesis of 2-(4-bromophenyl) oxirane

A reaction mixture of 4-bromobenzaldehyde (5.0 g, 27.0 mmol), trimethylsulfonium iodide (6.1 g, 29.8 mmol), water (1.26 mL, 70.3 mmol) and potassium hydroxide (3.0 g, 54.1 mmol) in acetonitrile (50 mL) was stirred at 55 °C for 4 h. The mixture was then partitioned between water and diethyl ether, and the organic phase was washed with water, diluted with hydrochloric acid, washed with brine, and dried over MgSO₄. Crude product of intermediate **14** (5.28 g 26.53 mmol) was obtained by removal of organic phase *in vacuo.* The obtained crude was used in the next step without further purification.

### Intermediate 15: Synthesis of 2-(allylamino)-1-(4-bromophenyl) ethan-1-ol

Intermediate **14** (4.4 g, 22.1 mmol) was dissolved in DMF (100 mL). 2-Propen-1-amine (5.1 g, 88.4 mmol) was added to the reaction mixture, which was stirred at 60 °C overnight. The solvent was then removed *in vacuo.* The obtained residue was purified by silica gel column chromatography eluting with EtOAc/hexanes (gradient: 2:1) to obtain the intermediate **15** (4.1 g, 16.0 mmol).

### Intermediate 16: Synthesis of 2-(allyl(2-(4-bromophenyl)-2-hydroxyethyl) amino) acetonitrile

Intermediate **15** (1.70 g, 6.6 mmol) was dissolved in acetonitrile (20 mL). Added potassium carbonate (1.84 g, 13.3 mmol) and 2-bromoacetonitrile (2.38 g, 19.9 mmol) to the reaction mixture, which was stirred at 85 °C for 3 h. The residue obtained after evaporation of solvent *in vacuo* was added water, and the aqueous layer was extracted twice with EtOAc (30 mL). The combined organic phase was washed with brine and dried by MgSO₄. Filtered the reaction mixture and the obtained filtrate was concentrated *in vacuo* to give the crude product, which was purified by silica gel column chromatography eluting with EtOAc/hexanes (gradient: 1:1) to obtain the intermediate **16** (1.56 g, 5.34 mmol)

### Intermediate 17: Synthesis of 2-(allyl(2-(4-bromophenyl)-2-chloroethyl) amino) acetonitrile

Thionyl chloride (1.05 g, 8.82 mmol) was added to a solution of pyridine (1.74 g, 22.0 mmol) in DCM (30 mL) at rt. Cooled the solution to 0 °C. Intermediate **16** was dissolved in DCM (10 mL) and added to the reaction mixture dropwise over 8 min. Stirred the mixture for additional 15 min at 0 °C. The mixture was neutralized by the addition of saturated sodium bicarbonate aqueous solution. The aqueous phase was extracted twice with DCM (50 mL). The combined organic phases were dried over MgSO₄, filtered, and the filtrate was concentrated *in vacuo* to obtain the crude product. The residue was purified by by silica gel column chromatography eluting with EtOAc/PE (9:91) to obtain the intermediate **17** (1.32 g, 4.13 mmol).

### Intermediates 18a and 18 b: Synthesis of 1-allyl-3-(4-bromophenyl) azetidine-2-carbonitrile

Intermediate **17** (1.0 g, 3.23 mmol) was dissolved in THF (30 mL). Stirred and Cooled the solution to -78 °C. Thelithium bis(trimethylsilyl) amide (1 M solution in THF, 4.8 mL, 4.8 mmol) was added dropwise to the mixture over 3 min.

The reaction mixture was stirred for an additional 1 h at -78 °C. The reaction mixture was quenched with aqueous saturated NH₄Cl solution, extracted three times with EtOAc (20 mL), dried the combined organic phases over MgSO₄, filtered, and concentrated the filtrate *in vacuo* to obtain the crude product. The residue was purified by silica gel column chromatography, eluting with EtOAc/hexanes (gradient: 9:91) to obtain intermediate **18a** (0.43 g, 1.62 mmol) and **18b** (0.40 g, 1.43 mmol). The first product collected was intermediate **18a** and **18b** was followed.

### Intermediate 19a: Synthesis of 3-(4-bromophenyl) azetidine-2-carbonitrile

Intermediate **18a** (340.0 mg, 1.23 mmol) was dissolved in DCM: EtOH=1:2 (18 mL). Added 1,3-dimethylbarbituric (287.3 mg, 1.84 mmol), Pd (PPh3)₄ (70.9 mg, 61.3 µmol) under N₂ protection. The mixture was heated to 40 °C overnight. The solvent was removed *in vacuo.* The residue was purified by silica gel column chromatography, eluting with DCM: MeOH (gradient: 40:1) to obtain the intermediate **19** (286.0 mg, 1.2 mmol).

### Intermediate 20a: Synthesis of 3-(4-bromophenyl)-1-(4-(methylamino) quinazolin-2-yl) azetidine-2-carbonitrile

Intermediate **19a** (80.0 mg, 337.4 µmol)was dissolved in THF (10 mL), the solution was allowed to stir at rt. 2-chloro-N-methylquinazolin-4-amine (65.3 mg, 337.4 µmol) was added to the solution. The mixture was stirred and heated to 80 °C for overnight. The product was precipitated and the reaction mixture was filter. THF washed the residue three times and then dried the residue *in vacuo* to obtain the intermediate **20a** (89.0 mg, 225.7 µmol).

### Synthesis of B1: Synthesis of 3-(4-(3,5-dimethylisoxazol-4-yl) phenyl)-1-(4-(methylamino) quinazolin-2-yl) azetidine-2-carbonitrile

Intermediate **20a** (20.0 mg, 50.7 µmol)was dissolved in 1,4-dioxane (10 mL). The corresponding product was obtained following the general procedure 1 for **A1.** The residue was purified by prep-HPLC to obtain the B1 (10.3 mg, 24.1 µmol).

¹H NMR (500 MHz, DMSO-*d*₆) δ 12.79 (s, 1H), 9.82 (s, 1H), 8.27 (d, *J* = 8.1 Hz, 1H), 7.89 (t, *J* = 7.8 Hz, 1H), 7.71 - 7.66 (m, 2H), 7.63 (d, *J* = 8.4 Hz, 1H), 7.55 (t, *J* = 7.8 Hz, 1H), 7.49 - 7.42 (m, 2H), 5.47 (d, *J* = 7.0 Hz, 1H), 4.77 (t, *J* = 8.5 Hz, 1H), 4.62 - 4.53 (m, 1H), 4.44 (t, *J* = 7.8 Hz, 1H), 3.15 (d, *J* = 4.5 Hz, 3H), 2.41 (s, 3H), 2.24 (s, 3H).

¹³C NMR (176 MHz, DMSO) δ 165.8, 160.3, 158.8, 158.6, 158.4, 158.2, 137.0, 135.9, 130.2, 129.7, 128.7, 125.8, 124.5, 117.8, 117.7, 116.0, 110.7, 57.2, 55.5, 40.5, 40.4, 40.3, 40.1, 40.0, 39.9, 39.8, 39.7, 28.9, 11.8, 10.9.

MS⁺: Calculated for (C₂₄H₂₂N₆O) ⁺, 411.48, Found: 411.46.

### Example A.8: Synthesis of 3-(4-(3,5-dimethylisoxazol-4-yl) phenyl)-1-(4-(methylamino) quinazolin-2-yl) azetidine-2-carbonitrile (B2)

Synthesis of intermediates **19b** and **20b, B2** (5.15 mg, 12.1 µmol) were obtained following the procedure for Intermediate **19a, 20a, B1.**

¹H NMR (700 MHz, DMSO-*d*₆) δ 9.73 (s, 1H), 8.24 (d, *J* = 8.2 Hz, 1H), 7.86 (d, *J* = 8.0 Hz, 1H), 7.70 - 7.66 (m, 2H), 7.63 (d, *J* = 8.4 Hz, 1H), 7.55 - 7.47 (m, 3H), 5.83 (d, *J* = 8.9 Hz, 1H), 4.72 (d, *J* = 9.4 Hz, 1H), 4.54 (s, 1H), 4.43 (q, *J* = 8.0 Hz, 1H), 3.13 (d, *J* = 4.5 Hz, 3H), 2.43 (s, 3H), 2.26 (s, 3H).

¹³C NMR (176 MHz, DMSO) δ 165.7, 160.3, 158.6, 158.4, 158.2, 130.2, 129.5, 129.4, 124.4, 118.4, 116.0, 110.7, 56.1, 55.8, 55.8, 40.5, 40.4, 40.4, 40.3, 40.1, 40.0, 39.9, 39.8, 39.7, 37.3, 28.8, 11.9, 11.0.

MS⁺: Calculated for (C₂₄H₂₂N₆O) ⁺, 411.48, Found: 411.46.

### Example A.9: Synthesis of 1-(3-amino-7I4-thieno[2,3-b] thiopyran-2-carbonyl)-3-(4-(3,5-dimethylisoxazol-4-yl) phenyl) azetidine-2-carbonitrile (B3)

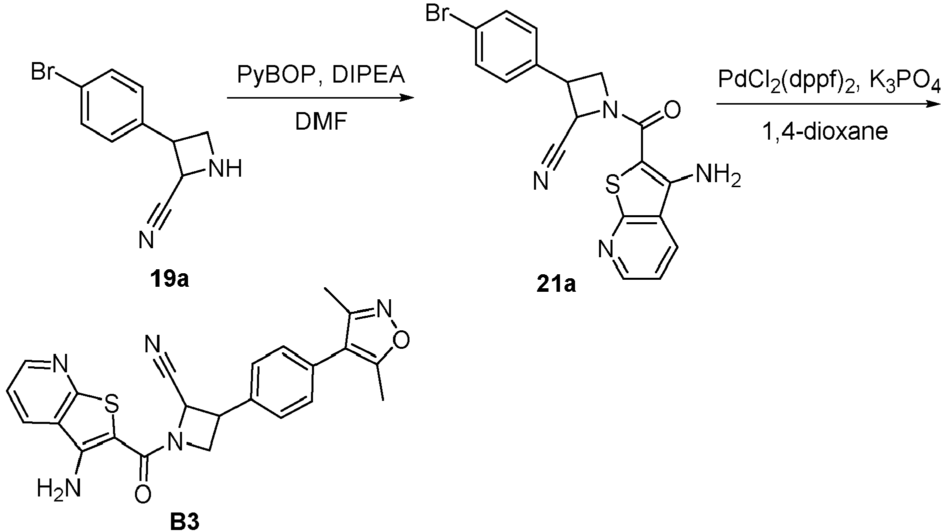

3-Aminothieno[2,3-b] pyridine-2-carboxylic acid (100.0 mg, 0.5 mmol) was dissolved in DMF (20 mL), Et₃N (156.3 mg, 1.5 mmol) and PyBOP (234.94 mg, 617.9 µmol) was added to the reaction mixture, which was stirred at rt for 30 min. Intermediate **19a** (146.5 mg, 0.6 mmol) was dissolved in DMF (3 mL) dropwise to the reaction solution. The mixture was stirred at rt overnight. The solvent was removed *in vacuo.* The residue was purified by silica gel column chromatography eluting with DCM: MeOH (gradient: 40:1) to obtain the intermediate **21a** (185.1 mg, 447.9 µmol).

### Synthesis of B3: Synthesis of 1-(3-amino-7l4-thieno[2,3-b] thiopyran-2-carbonyl)-3-(4-(3,5-dimethylisoxazol-4-yl) phenyl) azetidine-2-carbonitrile

Intermediated **21a** (20.0 mg, 48.4 µmol) was dissolved in 1,4-dioxane (10 mL), The corresponding product was obtained following the general procedure 1 for **A1.** The residue was purified by prep-HPLC to obtain **B3** (8.36 mg, 19.5 µmol).

### Example A.10: Synthesis of 1-(4-(methylamino) quinazolin-2-yl)-3-(4'-(trifluoromethyl)-[1,1'-biphenyl]-4-yl) azetidine-2-carbonitrile (B5)

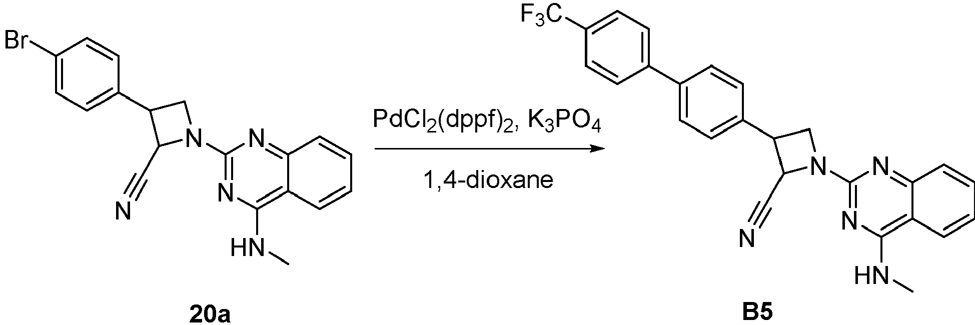

Intermediate **20a** (20.0 mg, 50.7 µmol) was dissolved in 1,4-dioxane (10 mL). The corresponding product was obtained following the general procedure 1 for **A1.** Purify the residue by prep-HPLC to obtain **B5** (4.56 mg, 9.9 µmol).

¹H NMR (500 MHz, DMSO-*d*₆): δ 12.79 (s, 1H), 9.80 (s, 1H), 8.24 (d, *J* = 8.0 Hz, 1H), 7.91 (d, *J* = 8.2 Hz, 2H), 7.85 - 7.78 (m, 5H), 7.70 (m, *J* = 7.9 Hz, 2H), 7.60 (d, *J* = 8.3 Hz, 1H), 7.51 (d, *J* = 7.9 Hz, 1H), 5.44 (d, *J* = 8.6 Hz , 1H), 4.74 (t, *J* = 8.9 Hz, 1H), 4.56 (d, *J* = 8.4 Hz, 1H), 4.41 (q, *J* = 8.0 Hz, 1H), 3.13 (d, *J* = 4.5 Hz, 3H). MS⁺: Calculated for (C₂₆H₂₀F₃N₅) ⁺, 460.17, Found: 460.23.

### Example A.11: Synthesis of 1-(4-(methylamino) quinazolin-2-yl)-3-(4'-(trifluoromethyl)-[1,1'-biphenyl]-4-yl) azetidine-2-carbonitrile (B6)

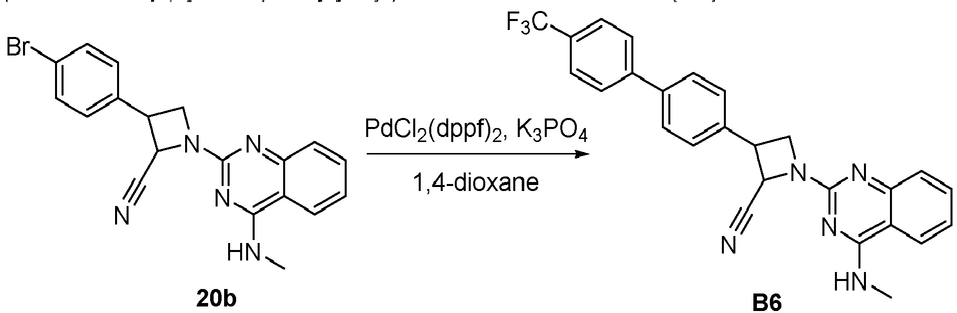

Intermediate **20b** (20.0 mg, 50.7 µmol) was dissolved in 1,4-dioxane (10 mL). The corresponding product was obtained following the general procedure 1 for **A1.** Purify the residue by prep-HPLC to obtain **B6** (6.89 mg, 15.0 µmol).

¹H NMR (500 MHz, DMSO-*d*₆): δ 12.82 (s, 1H), 9.77 (s, 1H), 8.25 (d, *J* = 8.2 Hz, 1H), 7.96 (d, *J* = 8.1 Hz, 2H), 7.92 - 7.80 (m, 5H), 7.75 - 7.69 (m, 2H), 7.63 (d, *J* = 8.3 Hz, 1H), 7.52 (d, *J* = 7.9 Hz, 1H), 5.85 (d, *J* = 8.6 Hz, 1H), 4.72 (t, *J* = 8.9 Hz, 1H), 4.55 (s, 1H), 4.44 (q, *J* = 8.0 Hz, 1H), 3.12 (d, *J* = 4.5 Hz, 3H).

¹³C NMR (176 MHz, DMSO) δ 160.3, 158.4, 158.3, 144.0, 138.7, 137.6, 129.7, 128.6, 128.4, 128.0, 127.7, 126.4, 126.3, 126.3, 126.3, 125.7, 124.4, 124.1, 118.3, 110.6, 56.1, 55.8, 55.8, 40.5, 40.4, 40.3, 40.1, 40.0, 39.9, 39.8, 39.7, 37.2, 28.8. MS⁺: Calculated for (C₂₆H₂₀F₃N₅) ⁺, 460.17, Found: 460.26.

### Example A.12: Synthesis of 1-(3-aminothieno[2,3-b] pyridine-2-carbonyl)-3-(4'-(trifluoromethyl)-[1,1'-biphenyl]-4-yl) azetidine-2-carbonitrile (B7)

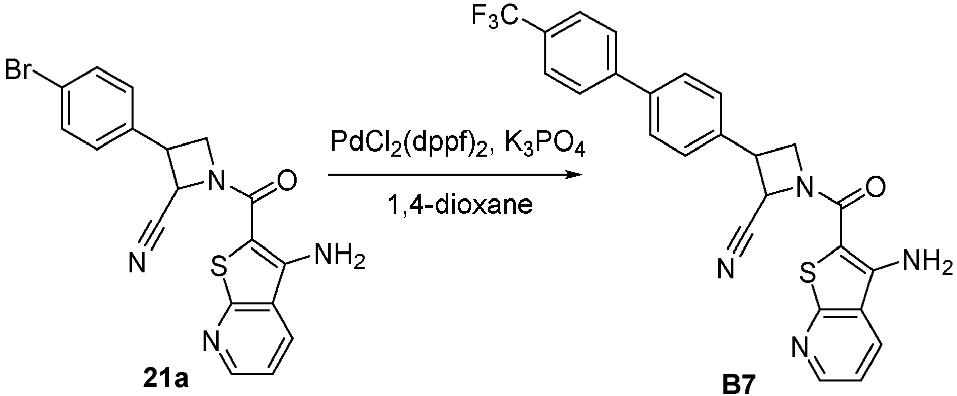

Intermediate 21a (20.0 mg, 48.4 µmol) was dissolved in 1,4-dioxane (10 mL). The corresponding product was obtained following the general procedure 1 for A1. Purify the residue by prep-HPLC to obtain the B7 (5.26 mg, 11.0 µmol).

¹H NMR (500 MHz, DMSO-*d*₆) δ 8.69 (dd, *J* = 4.6, 1.6 Hz, 1H), 8.56 (dd, *J* = 8.1, 1.6 Hz, 1H), 7.91 (d, *J* = 8.2 Hz, 2H), 7.81 (d, *J* = 8.3 Hz, 2H), 7.79 - 7.75 (m, 2H), 7.65 - 7.62 (m, 2H), 7.49 (dd, *J* = 8.1, 4.6 Hz, 1H), 5.25 (d, *J* = 7.0 Hz, 1H), 4.88 (dd, *J* = 8.9, 7.3 Hz, 1H), 4.49 (t, *J* = 7.2 Hz, 1H), 4.41 (dt, *J* = 9.0, 7.1 Hz, 1H).

MS⁺: Calculated for (C₂₅H₁₇F₃N₄OS) ⁺, 479.11, Found: 479.16.

### Example A.13: Synthesis of 3-(4-(1H-benzo[d]imidazol-6-yl) phenyl)-1-(4-(methylamino) quinazolin-2-yl) azetidine-2-carbonitrile (B9)

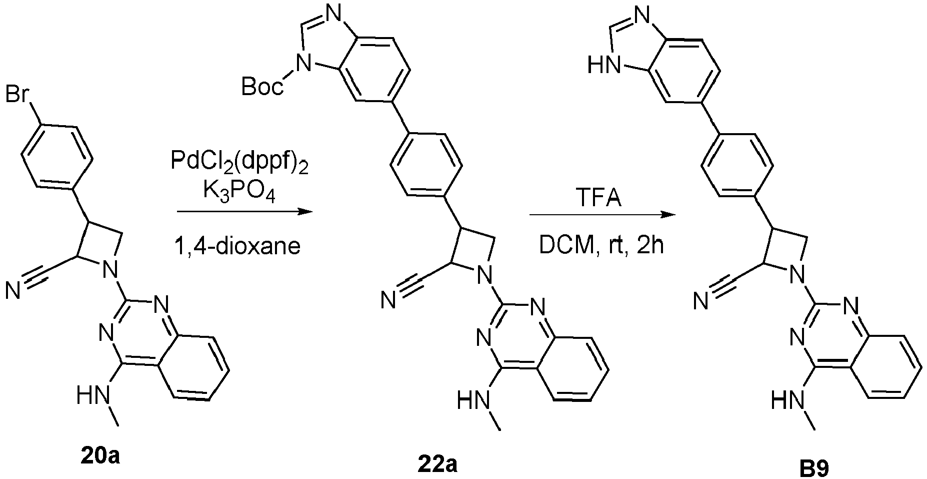

Intermediate **20a** (20.0 mg, 50.7 µmol) was dissolved in 1,4-dioxane (10 mL). The corresponding product was obtained following the general procedure 1 for **A1.** Purify the residue by prep-HPLC to obtain intermediate **22a** (7.89 mg, 14.9 µmol). Dissolving **22a** in 20 mL DCM, add 1 mL TFA, stirring the reaction under the rt for 2 h. Removing the solvent, Purify the residue by prep-HPLC to obtain **B9** (3.28 mg, 7.6 µmol).

¹H NMR (700 MHz, DMSO-*d*₆) δ 9.96 (s, 1H), 8.90 (s, 1H), 8.24 (d, *J* = 8.2 Hz, 1H), 7.95 (d, *J* = 1.7 Hz, 1H), 7.86 (t, *J* = 7.8 Hz, 1H), 7.80 (d, *J* = 8.5 Hz, 1H), 7.78 - 7.75 (m, 2H), 7.72 - 7.68 (m, 1H), 7.69 - 7.66 (m, 2H), 7.61 (d, *J* = 8.2 Hz, 1H), 7.51 (t, *J* = 7.8 Hz, 1H), 5.43 (d, *J* = 7.1 Hz, 1H), 4.75 (t, *J* = 8.5 Hz, 1H), 4.56 (q, *J* = 7.4 Hz, 1H), 4.41 (t, *J* = 7.7 Hz, 1H), 3.14 (d, *J* = 4.5 Hz, 3H).

MS⁺: Calculated for (C₂₆H₂₁N₇) ⁺, 431.19, Found: 431.19.

### Example A.14: Synthesis of 3-(4-(1H-benzo[d]imidazol-6-yl) phenyl)-1-(4-(methylamino) quinazolin-2-yl) azetidine-2-carbonitrile (B10)

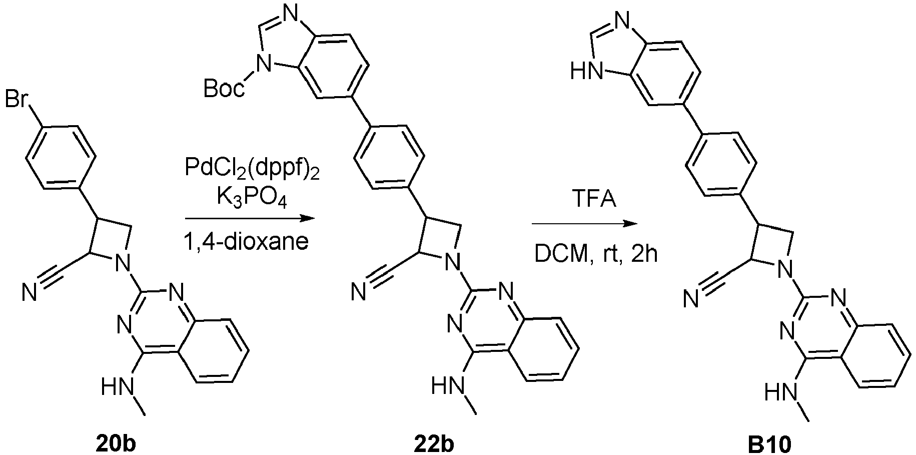

Intermediate **20b** (20.0 mg, 50.7 µmol) was dissolved in 1,4-dioxane (10 mL). The corresponding product was obtained following the general procedure 1 for **A1.** Purify the residue by prep-HPLC to obtain the intermediate **22b** (8.96 mg, 16.9 µmol). Dissolving **22b** in 20 mL DCM, add 1 mL TFA, stirring the reaction at rt for 2 h. The solvent was removed and the residue was purified by prep-HPLC to obtain **B10** (3.89 mg, 9.0 µmol).

¹H NMR (700 MHz, DMSO-*d*₆) δ 9.72 (s, 1H), 9.06 (s, 1H), 8.26 (d, *J* = 8.7 Hz, 1H), 8.04 (d, *J* = 1.6 Hz, 1H), 7.92 - 7.79 (m, 5H), 7.71 (dd, *J* = 8.3, 4.5 Hz, 2H), 7.65 (d, *J* = 8.2 Hz, 1H), 7.53 (t, *J* = 7.7 Hz, 1H), 5.87 (d, *J* = 8.8 Hz, 1H), 4.75 (t, *J* = 8.8 Hz, 1H), 4.61 - 4.55 (m, 1H), 4.45 (d, *J* = 5.9 Hz, 1H), 3.14 (d, *J* = 4.6Hz, 3H).

MS⁺: Calculated for (C₂₆H₂₁N₇) ⁺, 431.19, Found: 431.21.

### Example A.15: Synthesis of tert-butyl 4-(2-amino-4-(4-bromophenyl)-3-(4-(4-methylpiperazin-1-yl)phenyl)azetidin-1-yl)piperidine-1-carboxylate (C1)

### Intermediate C1-01: Synthesis of 4-nitrobenzenesulfonyl azide

To a solution of the 4-nitrobenzenesulfonyl chloride (5.0 g, 22.56 mmol) in acetone (30 mL) and water (30 mL) was added the NaN₃ (2.93 g, 45.12 mmol) after cooling the reaction solution on ice bath for 5 min, followed by stirring at rt for 3 h. The solvent was concentrated *in vacuo* and the residue was washed with water and extracted with ethyl acetate (50 mL) three times. The combined organic layer was washed with brine, dried over MgSO₄, filtered, and the obtained solvent was evaporated *in vacuo.* The crude product was purified by silica gel column chromatograph (gradient: PE/EtOAc 8:1 (v/v)) to afford the title product (4.56 g, 19.98 mmol) as a yellow solid.

¹H NMR (400 MHz, CDCl₃-*d*): δ 8.44 - 8.36 (m, 2H), 8.13 - 8.07 (m, 2H).

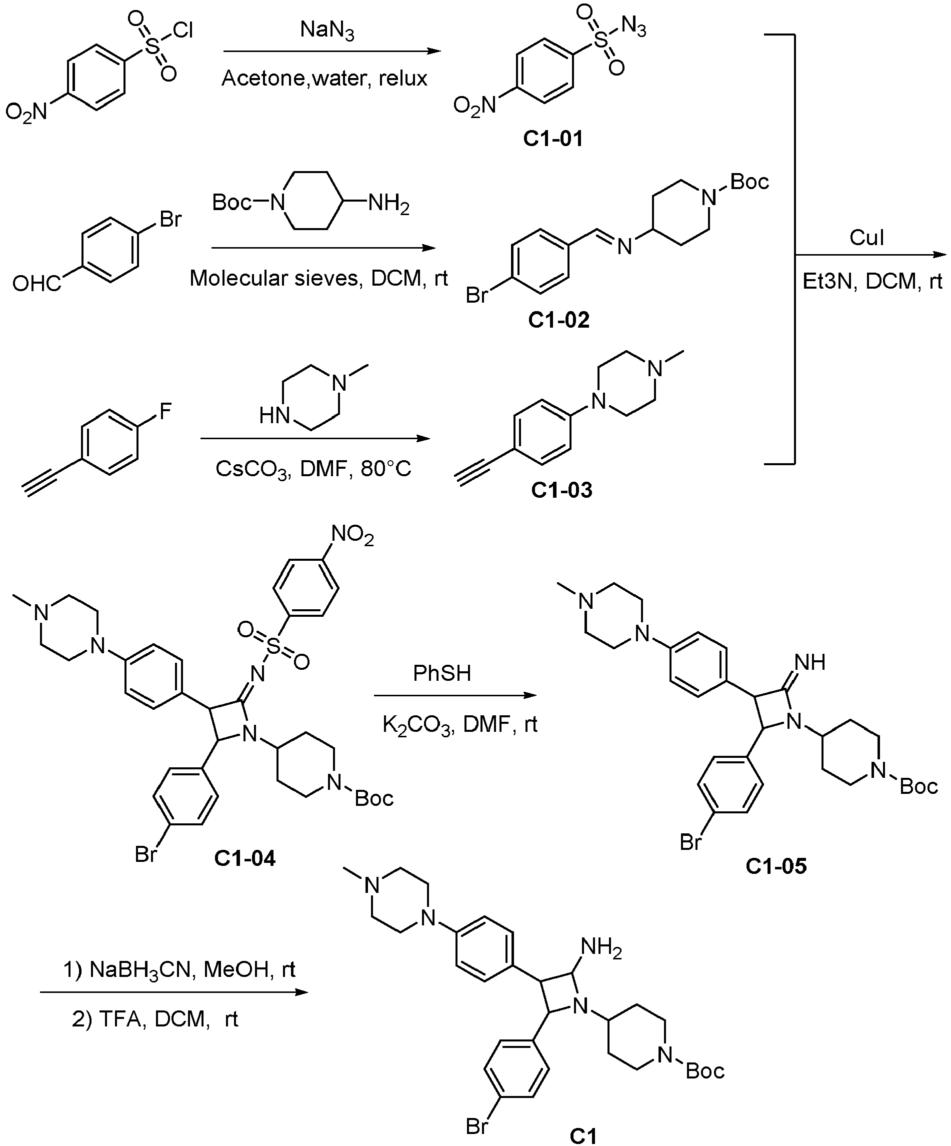

### Intermediate C1-02: Synthesis of tert-butyl-4-((4-bromobenzylidene) amino) piperidine-1-carboxylate

Synthesis of C1-02 was obtained following the **General procedure 2 for synthesis of imine.**

Rigorously dried 4Å molecular sieves (540.0 mg) were added to a solution of tert-butyl 4-aminopiperidine-1-carboxylate (108.24 mg, 0.54 mmol) in DCM (2.5 mL) under N₂ at rt. After a period of 5 min, 4-bromobenzaldehyde (100.0 mg, 0.54 mmol) was added. The mixture was stirred at rt overnight before filtration through celite and removal of solvents *in vacuo* to afford the crude imine (200.0 mg, 0.54 mmol) as a colorless oil.

¹H NMR (400 MHz, CDCl₃-*d*): δ 8.22 (s, 1H), 7.54 (d, *J* = 7.8 Hz, 2H), 7.47 (d, *J* = 8.4 Hz, 2H), 4.00 (d, *J* = 13.3 Hz, 2H), 3.32 (p, *J* = 7.7, 6.9 Hz, 1H), 2.93 (dt, *J* = 13.3, 6.8 Hz, 2H), 1.70 - 1.62 (m, 4H), 1.41 (s, 9H).

### Intermediate C1-03: Synthesis of 1-(4-ethynylphenyl)-4-methylpiperazine

To a solution of 1-ethynyl-4-fluorobenzene (1.00 g, 8.32 mmol) in DMF (30 mL) was added Cs₂CO₃ (4.07 g.12.49 mmol, 1.50 equiv.) and 1-methylpiperazine (1.25 g, 12.49 mmol, 1.5 equiv.). The reaction mixture was stirred at 80 °C for 12h and then diluting with water and extract with EtOAc (50 mL) three times. The organic phases were combined and dried with MgSO4, filtered, and the solvent was removed *in vacuo.* The crude product was purified by silica column chromatography (gradient: DCM: Methanol=20:1) to afford the title product (52 mg, 0.26 mmol) as a light-yellow solid.

### Intermediate C1-04: Synthesis of tert-butyl 4-(2-(4-bromophenyl)-3-(4-(4-methylpiperazin-1-yl) phenyl)-4-(((4- nitrophenyl) sulfonyl) imino) azetidin-1-yl) piperidine-1-carboxylate.

Synthesis of **C1-04** was obtained following the **General procedure 3 for synthesis of sulfonyl azetidinimines.**

To a solution of sulfonyl azide (34.18 mg ,0.15 mmol) in DCM (2 mL) at rt was treated sequentially with Et₃N (23.70 mg, 0.30 mmol), 1-(4-ethynylphenyl)-4-methylpiperazine (30 mg, 0.15 mmol), tert-butyl-4-((4-bromobenzylidene) amino) piperidine-1-carboxylate (55.10 mg ,0.15 mmol), and finally Cul (2.85 mg, 0.015 mmol). The reaction mixture was stirred at rt under N₂ atmosphere for 3-5 h and the solvent was removed *in vacuo.* The crude was purified by prep-HPLC to obtain the title compound (8.63 mg, 11.24 µmol) as a white solid.

### Intermediate C1-05: Synthesis of tert-butyl-4-(2-(4-bromophenyl)-4-imino-3-(4-(4-methylpiperazin-1-yl) phenyl) azetidin-1-yl) piperidine-1-carboxylate

Synthesis of C1-05 was obtained following the **General procedure 4.**

To a solution of Intermediate C1-04 (8.00 mg, 11.0 µmol) in DMF was added K₂CO₃ (55.0 µmol, 5.0 equiv.) followed by benzene thiol (25.0 µmol, 2.5 equiv.). The resulting suspension was stirred at rt for 3 h before being removed the solvent *in vacuo.* The crude product was purified by prep-HPLC to yield the pure compound (3.56 mg, 6.1 µmol) as a white solid.

### Synthesis of C1: Synthesis of tert-butyl-4-(2-amino-4-(4-bromophenyl)-3-(4-(4-methylpiperazin-1-yl) phenyl) azetidin-1-yl) piperidine-1-carboxylate (C1)

To a solution of intermediate C1-05 (3.56 mg, 7.2 µmol) in MeOH (1 mL) was added NaBH₃CN (0.47 mg, 7.5 µmol, 1.5 equiv.). The reaction mixture was stirred at rt for 1 h before removing the solvent *in vacuo.* The crude was purified by prep-HPLC to obtain the **C1** (2.38 mg, 4.1 µmol) as a white solid.

### Example A.16: Synthesis of tert-butyl 4-(2-(3,5-di-tert-butyl-4-hydroxyphenyl)-3-(4-fluorophenyl)-4-iminoazetidin-1-yl) piperidine-1-carboxylate (C2)

### Intermediate C2-01: Synthesis of tert-butyl-4-((3,5-di-tert-butyl-4-hydroxybenzylidene) amino) piperidine-1-carboxylate

3,5-di-tert-butyl-4-hydroxybenzaldehyde (100.0 mg, 0.43 mmol), tert-butyl 4-aminopiperidine-1-carboxylate (85.35 mg, 0.43 mmol, 1 equiv.) were treated according to the **General procedure 2** to obtain the title compound (174.23 mg, 0.42 mmol) that was used in the next step without further purification.

### Intermediate C2-02: Synthesis of tert-butyl 4-(2-(3,5-di-tert-butyl-4-hydroxyphenyl)-3-(4-fluorophenyl)-4-(((4-nitrophenyl) sulfonyl)imino)azetidin-1-yl)piperidine-1-carboxylate

1-ethynyl-4-fluorobenzene (40.84 mg, 0.34 mmol), 4-nitrobenzenesulfonyl azide (77.58 mg, 0.34 mmol, 1.0 equiv.), intermediate C2-01 (170.00 mg, 0.41 mmol, 1.2 equiv.) were treated according to the **General procedure 3** to obtained the title compound (purified by HPLC, 79.23 mg, 0.11 mmol).

**Intermediate C2-03:** Synthesis of tert-butyl 4-(2-(3,5-di-tert-butyl-4-hydroxyphenyl)-3-(4-fluorophenyl)-4-iminoazetidin-1-yl)piperidine-1-carboxylate Intermediate C2-02 (70.0 mg, 95.00 µmol) was treated according to the **General procedure** 4 to obtain the title compound (purified through prep-HPLC, 36.86 mg, 66.80 µmol).

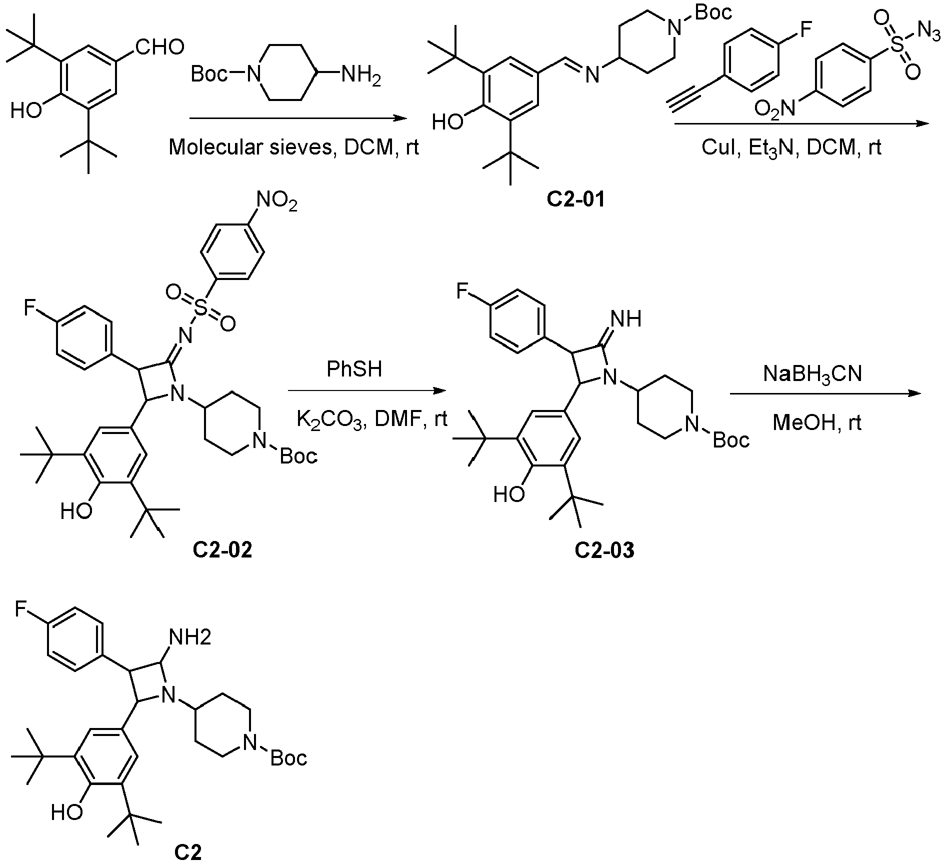

### Synthesis of C2: Synthesis of tert-butyl 4-(2-(3,5-di-tert-butyl-4-hydroxyphenyl)-3-(4-fluorophenyl)-4-iminoazetidin-1-yl)piperidine-1-carboxylate (C2)

To a solution of intermediate C2-03 (36.86 mg, 66.80 µmol) in MeOH (1 mL) was added NaBH₃CN (6.30 mg, 100.20 µmol, 1.5 equiv.). The reaction mixture was stirred at rt for 1 h before removing the solvent *in vacuo.* The crude was purified by prep-HPLC to obtain **C2** (16.54 mg, 29.87 µmol).

### Example A.17: Synthesis of 3-(4-fluorophenyl)-4-(4-methylpiperazin-1-yl)-1-(1-methylpiperidin-4-yl) azetidin-2-amine (C3)

### Intermediate C3-01: Synthesis of 1-(4-methylpiperazin-1-yl)-N-(1-methylpiperidin-4-yl) methanimine

Synthesis of intermediate C3-01 was obtained following the **General procedure 2** for synthesis of imines.

4-methylpiperazine-1-carbaldehyde (100.00 mg, 0.78 mmol), 1-methylpiperidin-4-amine (89.00 mg, 0.78 mmol, 1 equiv.) were treated accordingly to the **General procedure 2** to obtain the title compound (179.30 mg, 0.80 mmol) that was used in the next step without further purification.

### Intermediate C3-02: Synthesis of N-(3-(4-fluorophenyl)-4-(4-methylpiperazin-1-yl)-1-(1-methylpiperidin-4-yl) azetidin-2-ylidene)-4-nitrobenzenesulfonamide

1-Ethynyl-4-fluorobenzene (80.49 mg, 0.67 mmol), 4-nitrobenzenesulfonyl azide (152.12 mg, 0.67 mmol, 1.0 equiv.), intermediate C3-01(179.30 mg, 0.80 mmol, 1.2 equiv.) was treated by the **General procedure 3** to obtain the title compound (purified by HPLC, 56.29 mg, 0.10 mmol) as a white solid.

### Intermediate C3-03: Synthesis of 3-(4-fluorophenyl)-4-(4-methylpiperazin-1-yl)-1-(1-methylpiperidin-4-yl) azetidin-2-imine

Intermediate C3-02 (56.29 mg, 0.10 mmol) was treated by the **General procedure 4** to obtain the title compound (purified through prep-HPLC, 26.85 mg, 74.27 µmol) as a light-yellow solid.

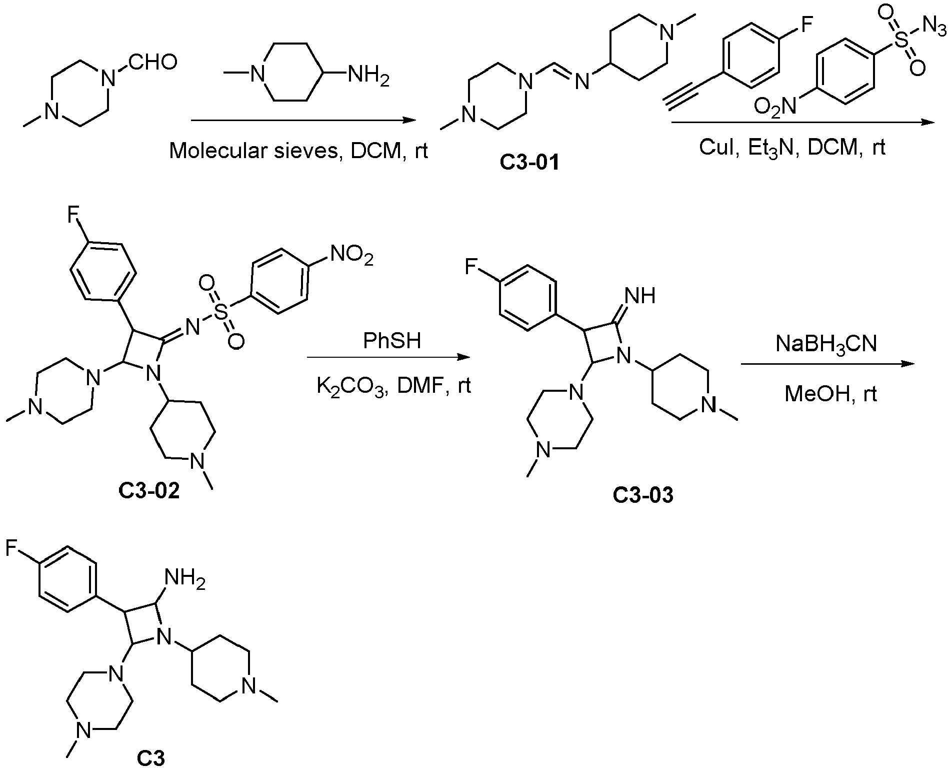

### Synthesis of C3: Synthesis of 3-(4-fluorophenyl)-4-(4-methylpiperazin-1-yl)-1-(1-methylpiperidin-4-yl) azetidin-2-amine (C3)

To a solution of intermediate C3-03 (26.85 mg, 74.69 µmol) in MeOH (1 mL) was added NaBH₃CN (7.04 mg, 112.03 µmol, 1.5 equiv.). The reaction mixture was stirred at rt for 1 h before removing the solvent *in vacuo.* The crude was purified by prep-HPLC to obtain the **C3** (18.56 mg, 51.40 µmol) as a white solid.

### Example A.18: Synthesis of tert-butyl 4-(4-amino-1-(1-(tert-butoxycarbonyl) piperidin-4-yl)-3-(4-(4-methylpiperazin-1-yl) phenyl) azetidin-2-yl) piperazine-1-carboxylate (C4)

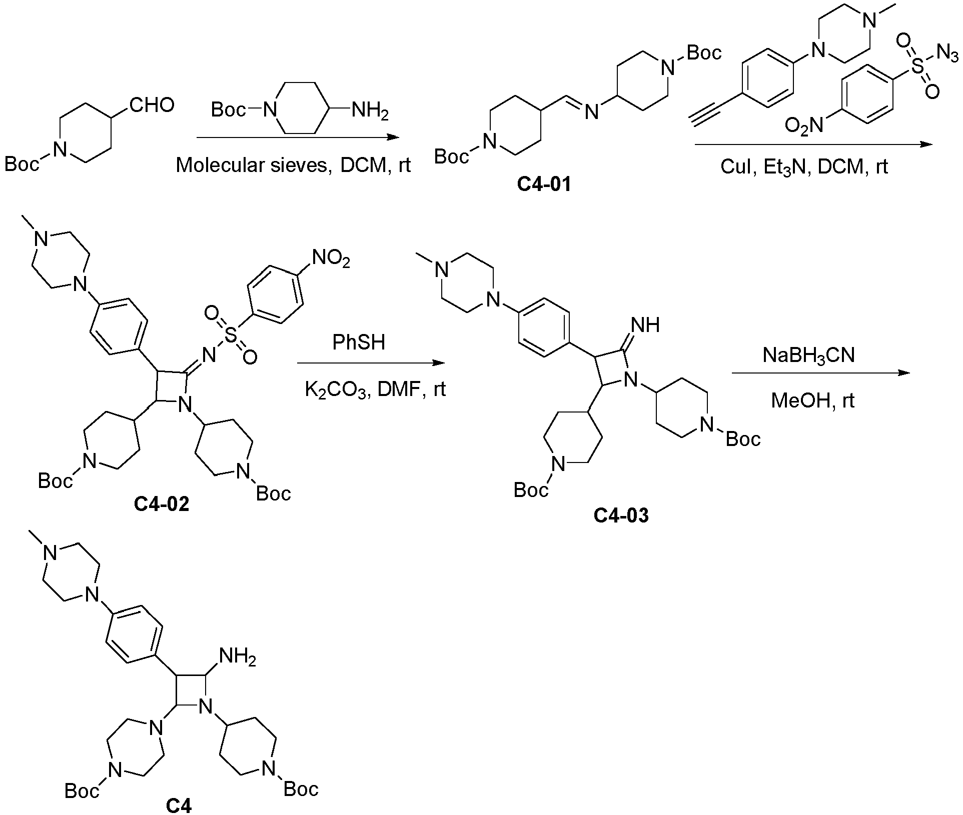

### Intermediate C4-01: Synthesis of tert-butyl-4-(((1-(tert-butoxycarbonyl) piperidin-4-yl) imino) methyl) piperidine-1-carboxylate

### Synthesis of intermediate C4-01 was obtained following the General procedure 2 for synthesis of imines.

tert-butyl 4-formylpiperidine-1-carboxylate (100.00 mg, 0.47 mmol), tert-butyl 4-aminopiperidine-1-carboxylate (94.13 mg, 0.47 mmol, 1.0 equiv.) was treated by the **General procedure 2** to obtain the title compound (186.56 mg, 0.47 mmol) as colorless oil which was used to the next step without further purification.

¹H NMR (400 MHz, CDCl₃-*d*): δ 7.51 (d, *J* = 4.9 Hz, 1H), 3.98 (s, 4H), 3.04 (dq, *J* = 14.1, 7.0 Hz, 1H), 2.90 - 2.67 (m, 4H), 2.25 (tq, *J* = 11.7, 4.2 Hz, 1H), 1.73 - 1.63 (m, 2H), 1.53 (dt, *J* = 7.3, 4.3 Hz, 4H), 1.45 - 1.30 (m, 20H).

### Intermediate C4-02: Synthesis of di-tert-butyl 4,4'-(3-(4-(4-methylpiperazin-1-yl)phenyl)-4-(((4-nitrophenyl)sulfonyl)imino)azetidine-1,2-diyl) bis(piperidine-1-carboxylate)

1-(4-ethynylphenyl)-4-methylpiperazine (68.10 mg, 0.34 mmol), 4-nitrobenzenesulfonyl azide (77.58 mg, 0.34 mmol, 1.0 equiv.), intermediate C4-01 (160.00 mg, 0.40 mmol, 1.2 equiv.) was treated by the **General procedure** 3 to obtained the title compound (purified by HPLC, 46.53 mg, 58.45 µmol)

### Intermediate C4-03: Synthesis of di-tert-butyl 4,4'-(4-imino-3-(4-(4-methylpiperazin-1-yl)phenyl)azetidine-1,2-diyl)bis(piperidine-1-carboxylate) Intermediate C4-02 (40.00 mg, 50.25 µmol) was treated by the General procedure 4 to obtain the title compound (purified through prep-HPLC, 18.57 mg, 30.40 µmol) as light brown solid

### Synthesis of C4: Synthesis of tert-butyl 4-(4-amino-1-(1-(tert-butoxycarbonyl) piperidin-4-yl)-3-(4-(4-methylpiperazin-1-yl) phenyl) azetidin-2-yl) piperazine-1-carboxylate (C4)

To a solution of intermediate C4-03 (18.57 mg, 30.40 µmol) in MeOH (1 mL) NaBH₃CN (2.87 mg, 45.60 µmol, 1.5 equiv.) was added. The reaction mixture was stirred at rt for 1 h before removing the solvent *in vacuo.* Purify the crude product through prep-HPLC to obtain the **C4** (8.96 mg, 14.62 µmol) as light brown solid.

### Example A.19: Synthesis of tert-butyl 4-(4-amino-1-(1-(tert-butoxycarbonyl) piperidin-4-yl)-3-(4-(4-methylpiperazin-1-yl) phenyl) azetidin-2-yl) piperazine-1-carboxylate (C5)

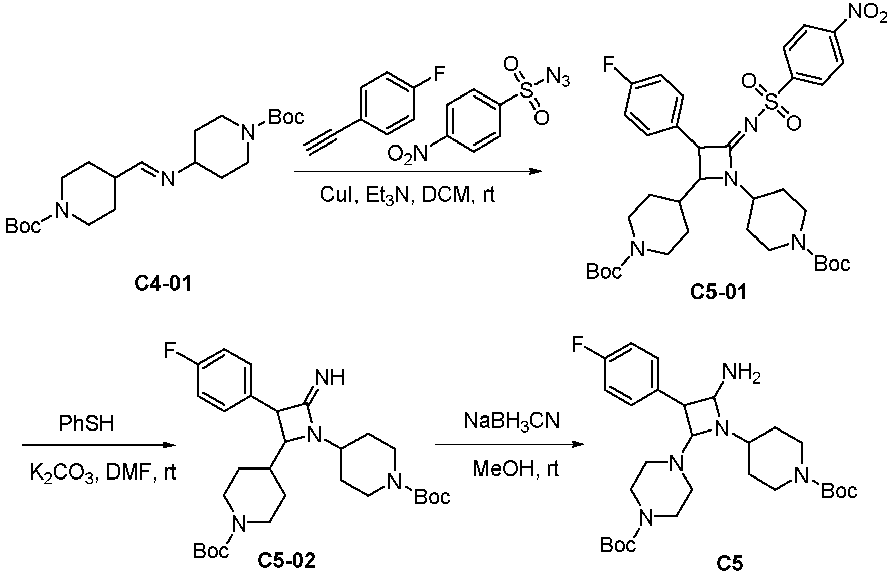

### Intermediate C5-01: Synthesis of di-tert-butyl 4,4'-(3-(4-fluorophenyl)-4-(((4-nitrophenyl) sulfonyl) imino) azetidine-1,2-diyl) bis(piperidine-1-carboxylate)

1-Ethynyl-4-fluorobenzene (45.65 mg, 0.38 mmol), 4-nitrobenzenesulfonyl azide (86.71 mg, 0.38 mmol, 1.0 equiv.), intermediate C4-01 (synthesized by example 18, 180 mg, 0.45 mmol, 1.2 equiv.) was treated by the **General procedure 3** to obtained the title compound (purified by HPLC, 72.56 mg, 0.10 mmol) as light yellow solid.

### Intermediate C5-02: Synthesis of di-tert-butyl 4,4'-(4-imino-3-(4-(4-methylpiperazin-1-yl)phenyl)azetidine-1,2-diyl)bis(piperidine-1-carboxylate)

Intermediate C5-01 (50.00 mg, 69.84 µmol) was treated by the **General procedure 4** to obtain the title compound (purified through prep-HPLC, 27.41 mg, 51.65 µmol).

### Synthesis of C5: Synthesis of tert-butyl 4-(4-amino-1-(1-(tert-butoxycarbonyl) piperidin-4-yl)-3-(4-(4-methylpiperazin-1-yl) phenyl) azetidin-2-yl) piperazine-1-carboxylate (C5)

To a solution of intermediate C5-02 (27.41 mg, 51.65 µmol) in MeOH (1 mL) was added NaBH₃CN (4.87 mg, 77.48 µmol, 1.5 equiv.). The reaction mixture was stirred at rt for 1 h before removing the solvent *in vacuo.* The crude was purified through prep-HPLC to obtain **C5** (18.59 mg, 34.90 µmol) as a white solid.

### Example A.20: Synthesis of 4-(benzo[d]thiazol-2-yl)-3-(1-methylpiperidin-4-yl)-1-(pyrimidin-2-yl) azetidin-2-amine (C8)

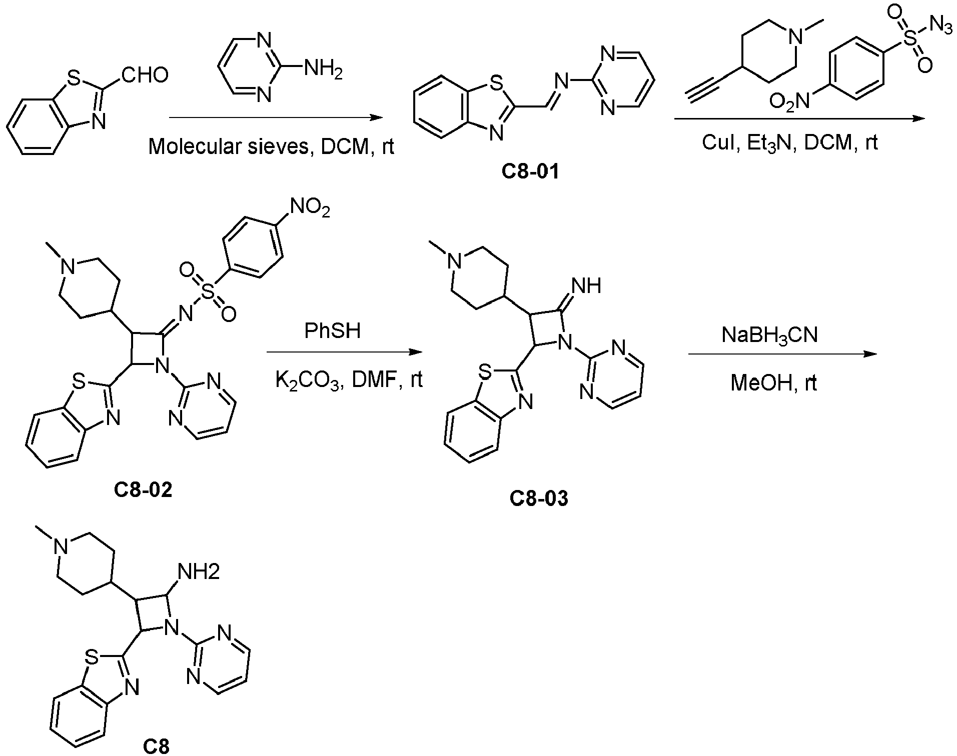

### Intermediate C8-01: Synthesis of 1-(benzo[d]thiazol-2-yl)-N-(pyrimidin-2-yl) methanimine

Benzo[d]thiazole-2-carbaldehyde (100.00 mg, 0.61 mmol) and pyrimidin-2-amine (58.27 mg, 0.61 mmol, 1.0 equiv.) were treated according to the **General procedure** 2 to obtained the title compound (142.32 mg, 0.59 mmol) that was used in the next step without further purification.

### Intermediate C8-02: Synthesis of N-(4-(benzo[d]thiazol-2-yl)-3-(1-methylpiperidin-4-yl)-1-(pyrimidin-2-yl)azetidin-2-ylidene)-4-nitrobenzenesulfonamide

4-ethynyl-1-methylpiperidine (66.53 mg, 0.54 mmol), 4-nitrobenzenesulfonyl azide (130 mg, 0.54 mmol, 1.0 equiv.), intermediate C8-01 (147.86 mg, 0.65 mmol, 1.2 equiv.) was treated by the **General procedure 3** to obtained the title compound (purified by HPLC, 35.21 mg, 62.47 µmol) as light yellow solid.

### Intermediate C8-03: Synthesis of 4-(benzo[d]thiazol-2-yl)-3-(1-methylpiperidin-4-yl)-1-(pyrimidin-2-yl) azetidin-2-imine

Intermediate C8-02 (30.00 mg, 53.22 µmol) was treated by the **General procedure 4** to obtain the title compound (purified through prep-HPLC, 18.52 mg, 48.93 µmol) as white solid.

### Synthesis of C8: 4-(benzo[d]thiazol-2-yl)-3-(1-methylpiperidin-4-yl)-1-(pyrimidin-2-yl) azetidin-2-amine (C8)

To a solution of intermediate C8-03 (18.52 mg, 48.93 µmol) in MeOH (1 mL) NaBH₃CN (4.61 mg, 73.40 µmol, 1.5 equiv.) was added and the reaction mixture was stirred at rt for 1 h before removing the solvent *in vacuo.* The crude product was purified by prep-HPLC to obtain the **C8** (12.53 mg, 32.93 µmol) as white solid.

### Example A.21: Synthesis of 3-(3,5-dimethoxyphenyl)-4-(4-(3,5-dimethylisoxazol-4-yl)phenyl)-1-(1-methylpiperidin-4-yl)azetidin-2-amine (C18)

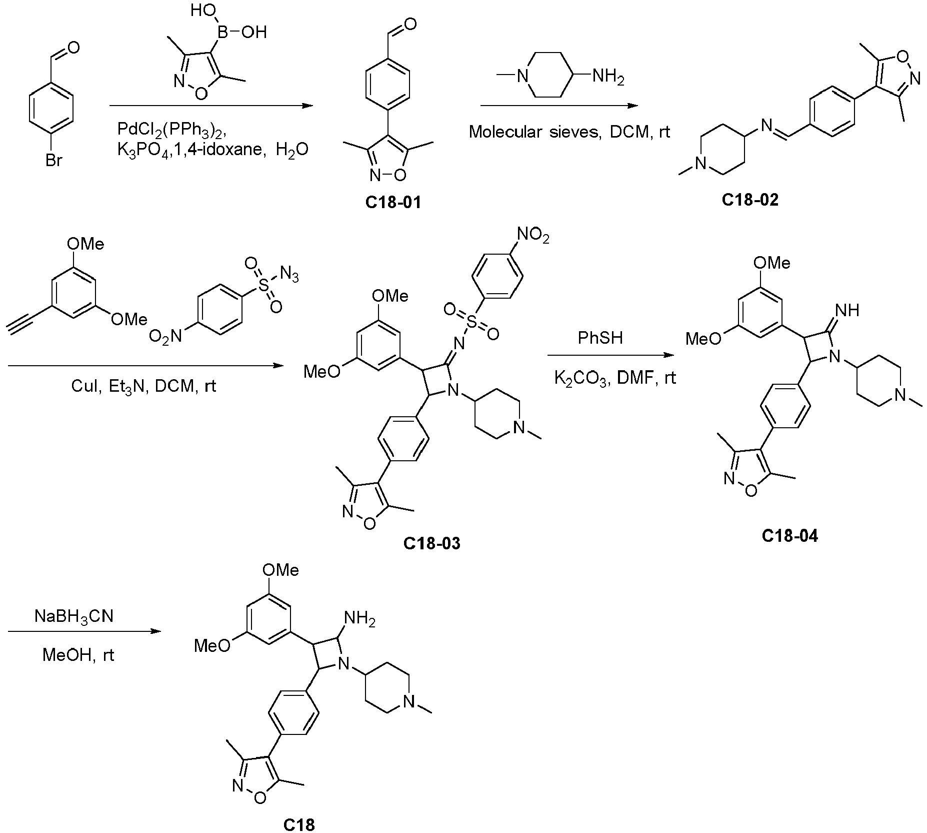

### Intermediate C18-01: Synthesis of 4-(3,5-dimethylisoxazol-4-yl)benzaldehyde.

To a solution of 4-bromobenzaldehyde (200.0 mg, 1.08 mmol) in 1,4-idoxane (10 mL) was added (3,5-dimethylisoxazol-4-yl)boronic acid (182.81 mg, 1.30 mmol, 1.20 equiv.), K₃PO₄ (344.19 mg, 1.62 mmol, 1.50 equiv.) dissolved in water (1 mL), and PdCl₂(PPh₃)₂ (29.96 mg, 54.05 µmol, 5 mol%). The reaction mixture was stirred at 90°C under N₂ protection overnight. The solvent was removed *in vacuo* and the residue was washed with water (20 mL) and extracted with EtOAc (30 mL) three times. The combined organic phases were washed with brine and dried over MgSO₄. The mixture was filtered and the filtrate was removed *in vacuo* to give the crude product, which was purified by silica gel column chromatography eluting with PE:EtOAc (gradient: 4:1-2:1) to yield the intermediate C8-01 (179.26 mg, 0.89 mmol).

### Intermediate C18-02: Synthesis of 1-(4-(3,5-dimethylisoxazol-4-yl)phenyl)-N-(1-methylpiperidin-4-yl)methanimine.

Synthesis of intermediate C18-02 was obtained according to the **General procedure 2** for synthesis of imines.

1-methylpiperidin-4-amine (100.00 mg, 0.86mmol), Intermediate C18-01 (176.22 mg, 0.86 mmol, 1.00 equiv.) was treated according to the **General procedure 2** to obtain the title compound (226.38 mg, 0.76 mmol) as a light-yellow solid that was used in the next step without further purification.

### Intermediate C18-03: Synthesis of N-(3-(3,5-dimethoxyphenyl)-4-(4-(3,5-dimethylisoxazol-4-yl)phenyl)-1-(1-methylpiperidin-4-yl)azetidin-2-ylidene)-4-nitrobenzenesulfonamide

1-Ethynyl-3,5-dimethoxybenzene (110.00 mg, 0.68 mmol), 4-nitrobenzenesulfonyl azide (154.77 mg, 0.68 mmol, 1.00 equiv.), intermediate C18-03(201.70 mg, 0.68 mmol, 1.20 equiv.) were treated accordingly to the **General procedure 3** to obtain the title compound (purified by HPLC, 126.76 mg, 0.19 mmol).

### Intermediate C18-04: Synthesis of 3-(3,5-dimethoxyphenyl)-4-(4-(3,5-dimethylisoxazol-4-yl)phenyl)-1-(1-methylpiperidin-4-yl)azetidin-2-imine.

### Synthesis of C18: 3-(3,5-dimethoxyphenyl)-4-(4-(3,5-dimethylisoxazol-4-yl)phenyl)-1-(1-methylpiperidin-4-yl)azetidin-2-amine

**C18** was prepared according to the synthesis of **C8.**

### Example A.22: Synthesis of 4-(benzo[d]thiazol-2-yl)-3-(3,5-dimethoxyphenyl)-1-(1-methylpiperidin-4-yl)azetidin-2-amine (C19)

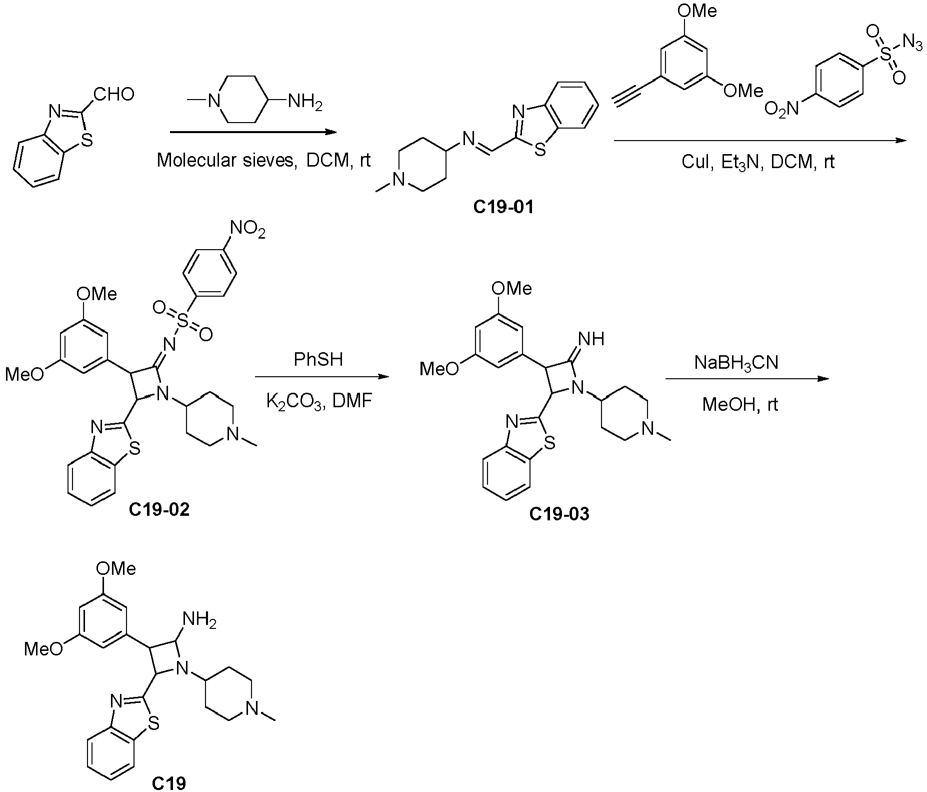

### Intermediate C19-01: Synthesis of 1-(benzo[d]thiazol-2-yl)-N-(1-methylpiperidin-4-yl)methanimine.

Synthesis of intermediate **C19-01** was performed accordingly to the **General procedure 2** for the synthesis of imines.

1-Methylpiperidin-4-amine (70.0 mg, 0.61mmol) and benzo[d]thiazole-2-carbaldehyde (100.0 mg, 0.61 mmol, 1.00 equiv.) were treated accordingly to the **General procedure 2** to obtain the title compound (167.92 mg, 0.65 mmol) as a light-yellow solid that was used in the next step without further purification.

### Intermediate C19-02: Synthesis of N-(4-(benzo[d]thiazol-2-yl)-3-(3,5-dimethoxyphenyl)-1-(1-methylpiperidin-4-yl)azetidin-2-ylidene)-4-nitrobenzenesulfonamide

1-Ethynyl-3,5-dimethoxybenzene (105.0 mg, 0.65 mmol), 4-nitrobenzenesulfonyl azide (147.7 mg, 0.65 mmol, 1.0 equiv.), intermediate C19-01 (167.9 mg, 0.65 mmol, 1.2 equiv.) were treated accordingly to the **General procedure 3** to obtain the title compound (purified by HPLC, 156.86 mg, 0.25 mmol).

¹H NMR (700 MHz, CDCl3-*d*) δ 8.01 - 7.97 (m, 2H), 7.83 (d, *J* = 8.1 Hz, 1H), 7.61 (dd, J = 8.2, 1.2 Hz, 1H), 7.54 - 7.49 (m, 2H), 7.35 (ddd, J = 8.3, 7.1, 1.2 Hz, 1H), 7.25 (ddd, *J* = 8.0, 7.1, 1.1 Hz, 1H), 6.04 (t, *J* = 2.3 Hz, 1H), 5.89 (s, 2H), 5.80 (d, *J* = 5.3 Hz, 1H), 5.23 (d, *J* = 5.3 Hz, 1H), 4.11 (tt, *J* = 11.9, 4.1 Hz, 1H), 3.66 - 3.59 (m, 2H), 3.38 (s, 7H), 2.84 (s, 1H), 2.73 (s, 5H), 2.63 - 2.54 (m, 1H), 2.38 (tt, *J* = 28.0, 12.5 Hz, 4H).

### Intermediate C19-03: Synthesis of 4-(benzo[d]thiazol-2-yl)-3-(3,5-dimethoxyphenyl)-1-(1-methylpiperidin-4-yl)azetidin-2-imine.

### Synthesis of C19: 4-(benzo[d]thiazol-2-yl)-3-(3,5-dimethoxyphenyl)-1-(1-methylpiperidin-4-yl)azetidin-2-amine

**C19** was prepared according to the synthesis of **C8.**

### Example B: Binding activity of the towards microRNAs

### Differential Scanning Fluorimetry (DSF):

To measure the binding between compounds and precursor microRNA-21 hairpin (pre-miR-21 hp) or precursor microRNA-17 hairpin (pre-miR-17 hp), DSF was performed as previously described with modification (*A*CS Chem. Biol. 2017, 12, 2, 435-443). Precursor microRNA hairpin was dissolved in PBS and annealed by briefly heating to 95 °C for 3 min and slowly cooling to rt. Assay was performed in duplicates using white qPCR 8-strips (VWR) with a total volume of 10 µL in PBS. The total reaction consisted of 1 µM precursor microRNA hairpin, 1% test compound (DMSO dilution) and 2×SYBR Green II (Thermo Fisher Scientific). Compounds were incubated with precursor microRNA hairpin at for 10 min at rt, then SYBR GreenII was added to each well, mixed and incubated 10 min at rt. The strips were spined and loaded to the RT-PCR detection system (BIO-RAD CFX Connect), the fluorescence intensity was obtained every 1 °C for 30s in the range of 20 °C to 80 °C.

### Data analysis:

The melting temperature (Tm) was determined by the maximum of the negative of the first derivative of the fluorescence vs temperature melting curves. The ΔTm was calculated by the Tm value of compound subtract the Tm value of the DMSO control.

### Results:

Part of tested compounds showed ability to modulate tested microRNA melting temperature, indicating that the compound may bind to tested microRNA hairpin to influence RNA thermostability, those being small molecule RNA binders.

The DSF results are summarized in Table 1 (ND, no data).

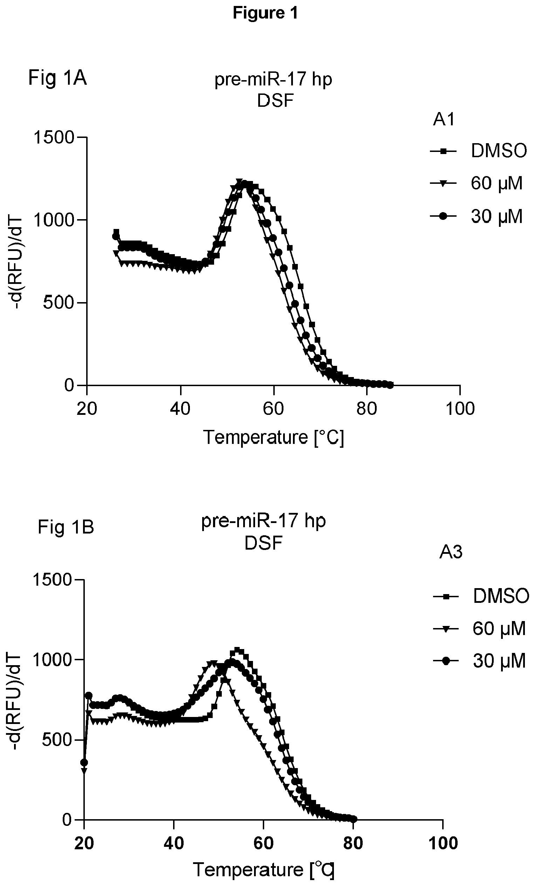

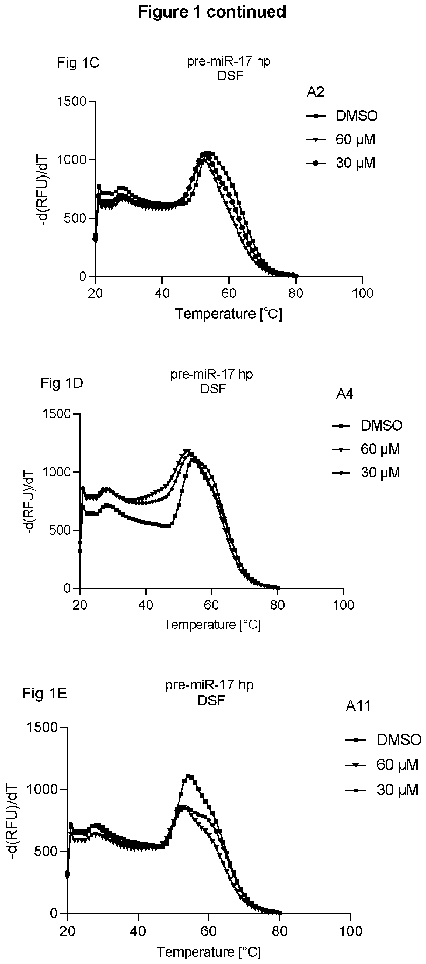

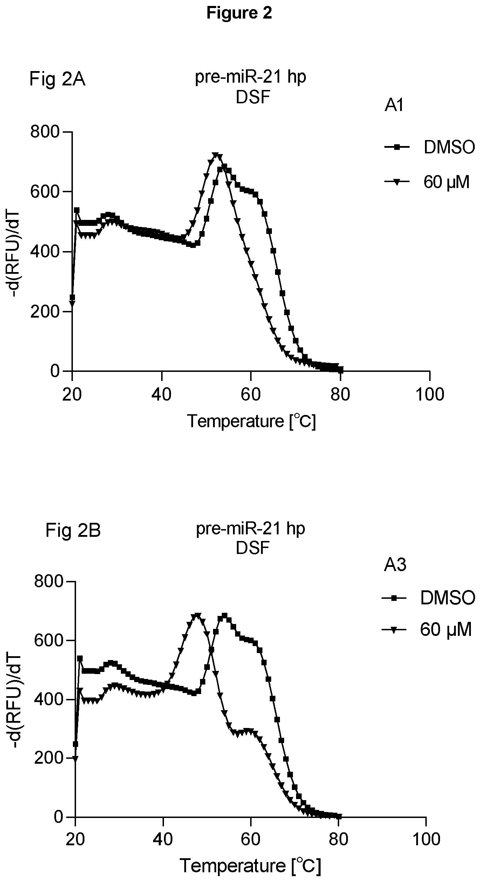

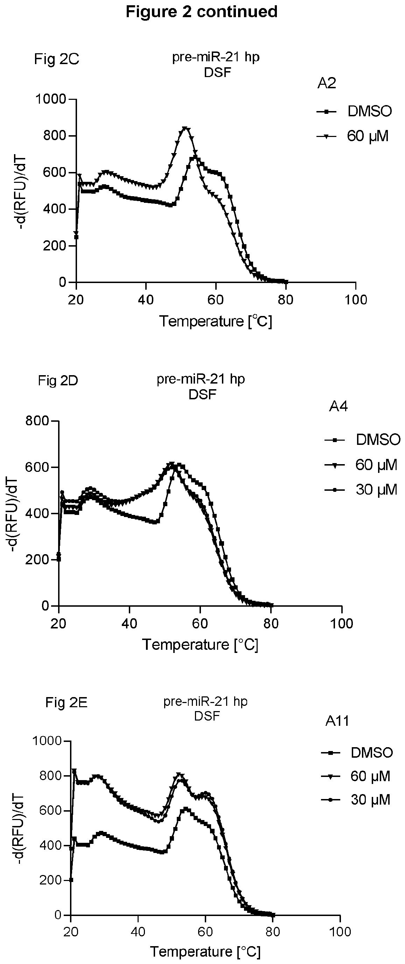

The graphical results of selected compounds towards pre-miR-17 hp are shown in Figure 1A-1E. Graphical results of selected compounds towards pre-miR-21 hp are shown in Figure 2A-2E.

**Table 1**

| **Compound** | **ΔTm (pre-miR-17 hp) @60uM [°C]** | **ΔTm (pre-miR-21 hp) @60uM [°C]** |
|---|---|---|
| **A1** | -3 | -2 |
| **A**3 | - 6.5±0.5 | -6 |
| **A2** | -2 | -2.5 ±0.5 |
| **A9** | 0 | 0 |
| **A4** | -1 | -2 |
| **A7** | 0 | 0 |
| **A11** | -1 | -2 |
| **A12** | -1 | -1.5 |
| **B6** | 1.25 | ND |
| **B5** | 0 | 0 |
| **B7** | 1.7 | 4.5 |
| **B9** | -1 | -1 |
| **B10** | 0 | 0 |
| **A10** | 0 | 0 |
| **B1** | 0 | 0 |
| **B7** | 0 | 0 |
| **B3** | 0 | 0 |
| **B4** | 0 | 0 |

### Example C: Determination of anticancer activity of the inventive compounds

### Cell culture.

MAD-MB-231, MCF-7 and JAR cells were cultured in Dulbecco's modified Eagle's (DMEM) medium supplemented with 10% Fetal Bovine Serum (FBS), 100u/mL penicillin-streptomycin. Jurkat, Molt-4 and Ramos cells were cultured in Roswell Park Memorial Institute (RPMI) 1640 medium with 10% Fetal Bovine Serum (FBS), 100u/mL penicillin-streptomycin. All cell lines grown in an incubator with humidified 5% CO₂ at 37 °C.

### Colony formation assay.

MDA-MB-231 cells were collected and seeded into 24-well plates with the density of 1000 cells per well and cultured overnight. The medium was exchanged and supplemented with different compounds and concentrations every three days. 6 days later, cells were washed with PBS and fixed by 4% paraformaldehyde solution. After 15 min fix, the paraformaldehyde solution was washed with PBS and was then stained with 0.1% (w/v) crystal violet. After 15 min, the cells were washed and photographed.

### Results

Selected compounds **A10, A11, B2, B5, B8, B9** exhibited antiproliferation activity against MDA-MB-231 cell line in a dose-dependent manner. Among those selected compounds, **B2, B5, B8** showed the most potent antiproliferation activity. At the concentration of 10 µM, **B2, B5,** and **B8** fully inhibited MDA-MB-231 cell proliferation.

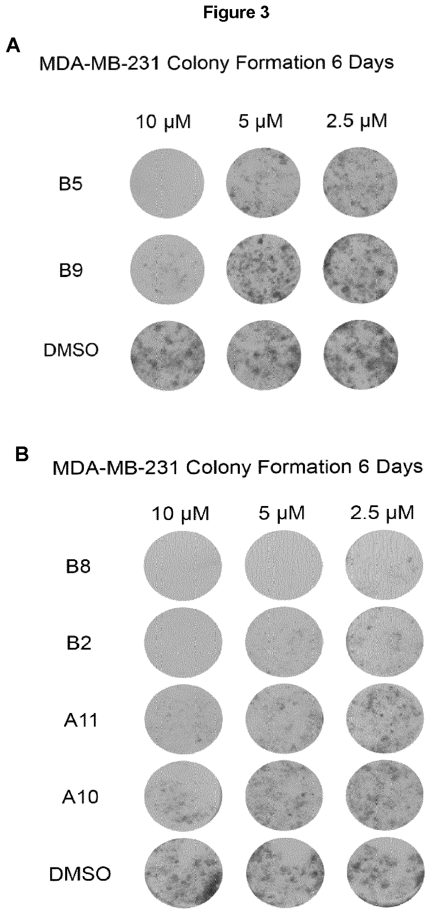

The graphical results of selected compounds are showed in Figure 3A-3B.

### MTT assay.

Jurkat, Molt-4 and Ramos cells were collected and seeded into 96-well plates at a density of 8000-10000 per well and treated with compounds. MCF-7, MDA-MB-231 and JAR cells were collected and seeded into 96-well plates at a density of 2000-3000 per well and cultured overnight to adherent before treat with compounds. After exposed to increasing concentrations of compounds for 72 h, the cells in each well were mixed with 20 µL of 5 mg/ml MTT (3-(4,5-dimethylthiazol-2-yl)-2,5-diphenyltetrazolium bromide) for 2 h, then add 50 µL of 20% SDS/0.01M HCl incubated overnight, and detected the absorbance at a wavelength of 570 nm.

### Data analysis:

Inhibition rate was calculated using the equation: $inhibition rate = 100 % \frac{Control - X}{Control - Blank}$. The IC₅₀ values were calculated by using GraphPad Prism 9 software fitting to a dose-response inhibition equation.

### Results:

Most of the tested compounds showed anticancer effect in selected cancer cell lines including 1 Burkitt lymphoma cell line (Ramos), 2 acute leukemia cell lines (Molt-4 and Jurkat), 2 breast cancer cell line (MCF-7 and MDA-MB-231) and 1 choriocarcinoma cell line (JAR).

The anticancer results are summarized in tables below. Results for Ramos, Molt-4, and Jurkat cell lines are shown in Table 2. Results for MCF-7, MDA-MB-231 and JAR cell lines are shown in Table 3 (ND, no data).

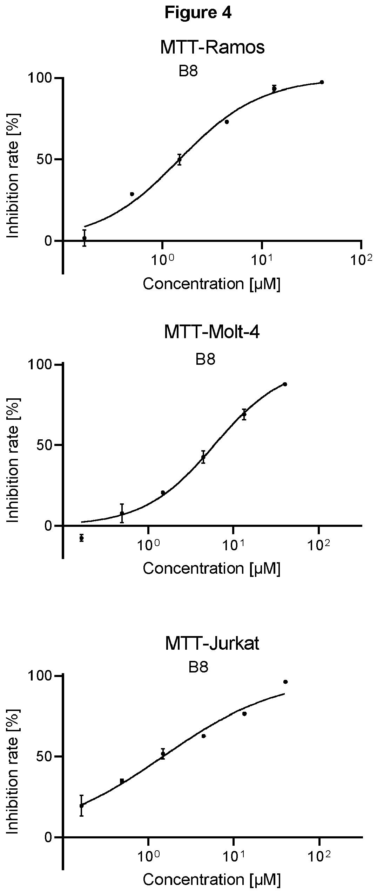

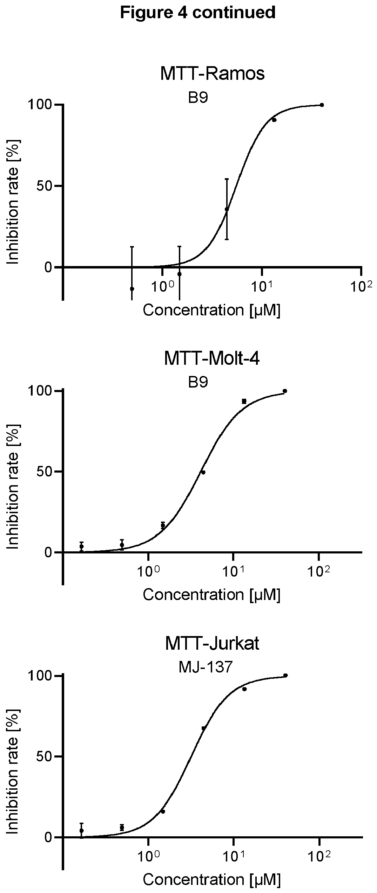

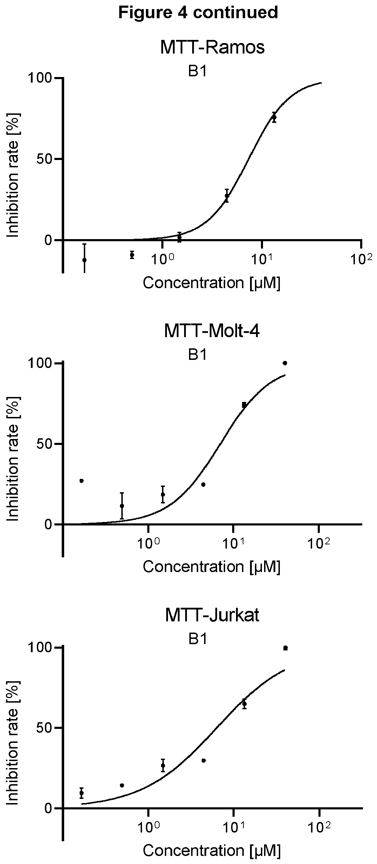

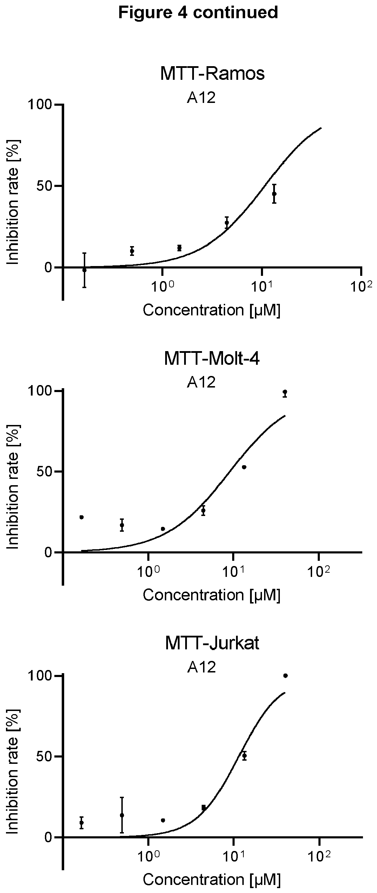

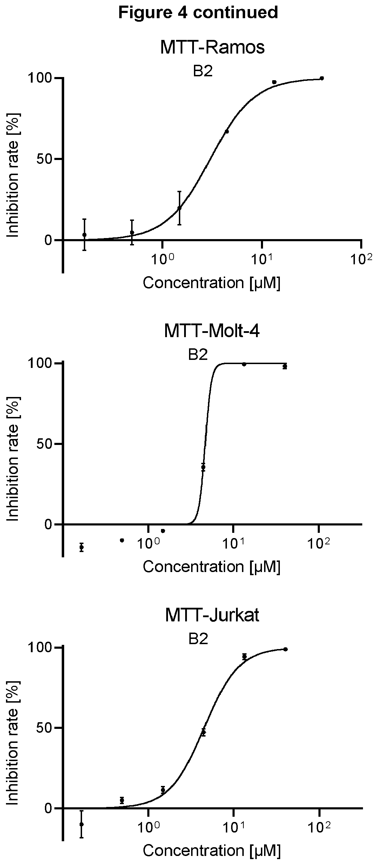

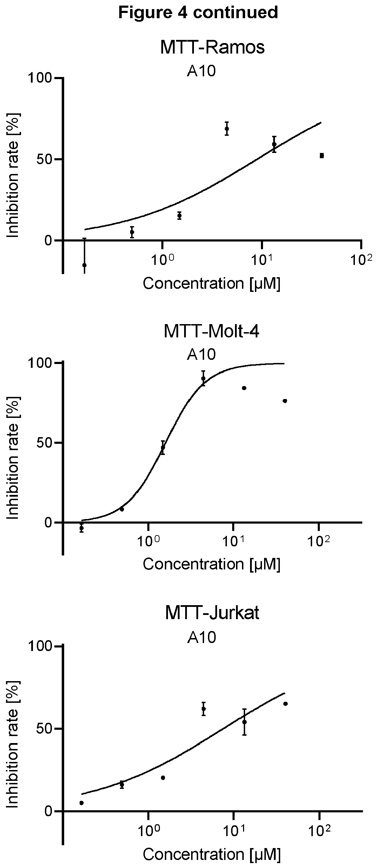

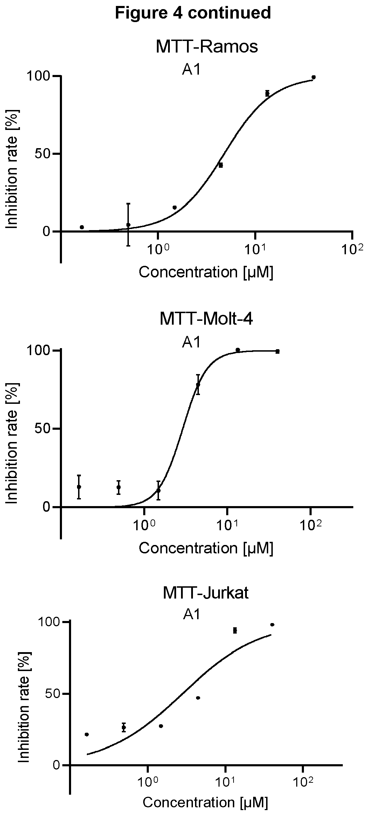

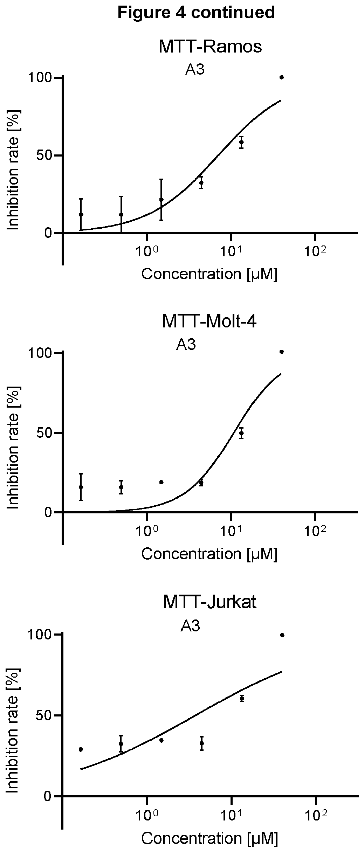

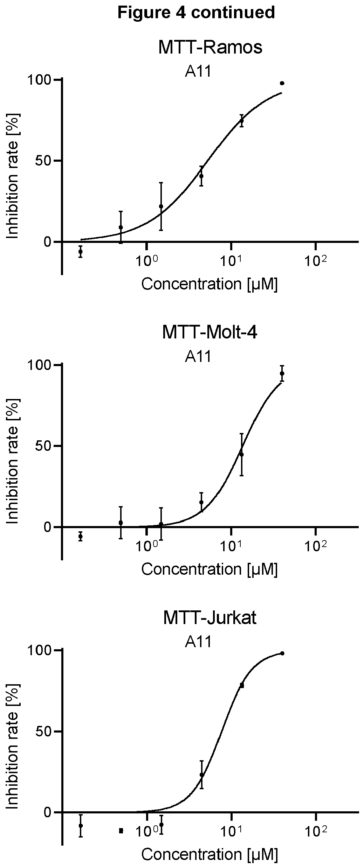

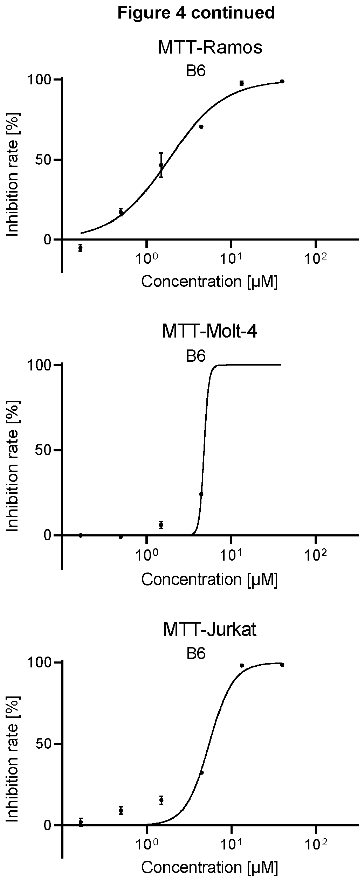

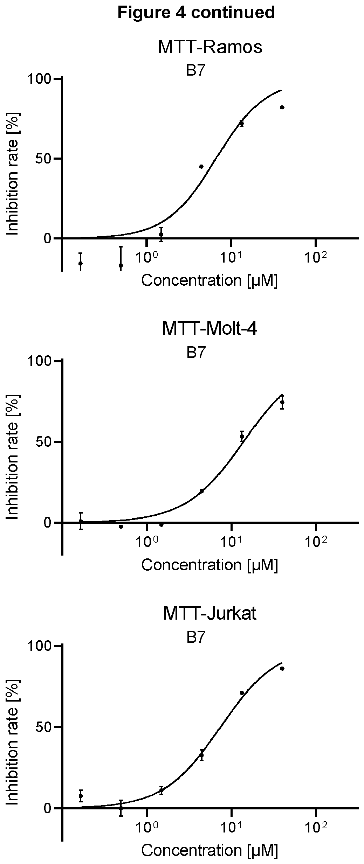

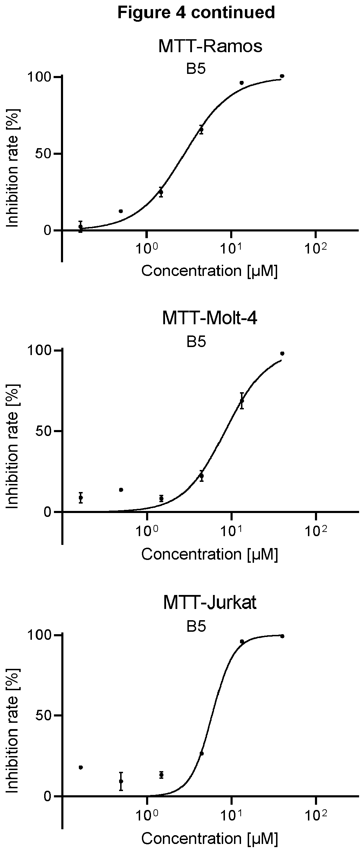

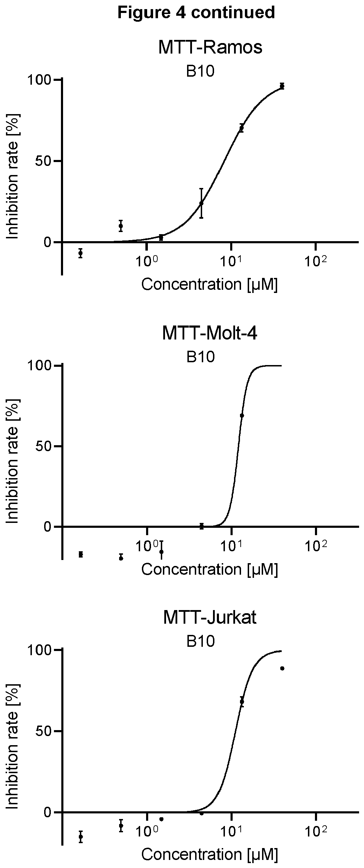

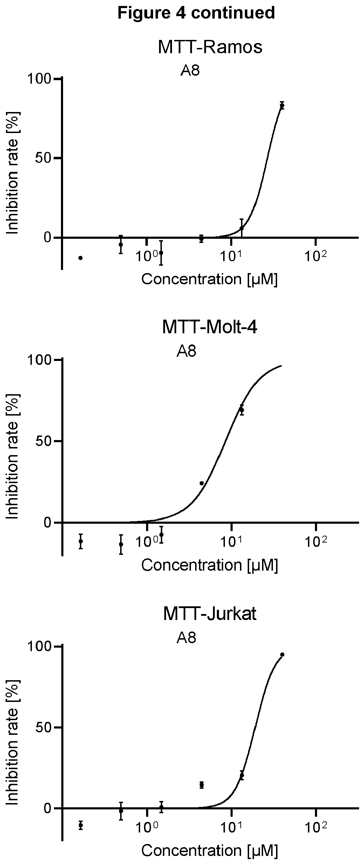

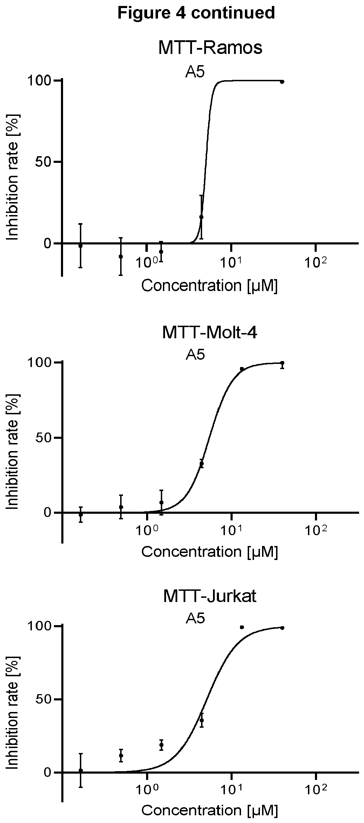

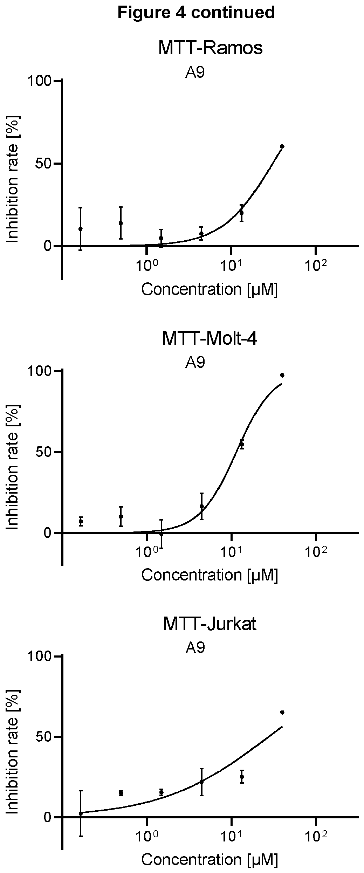

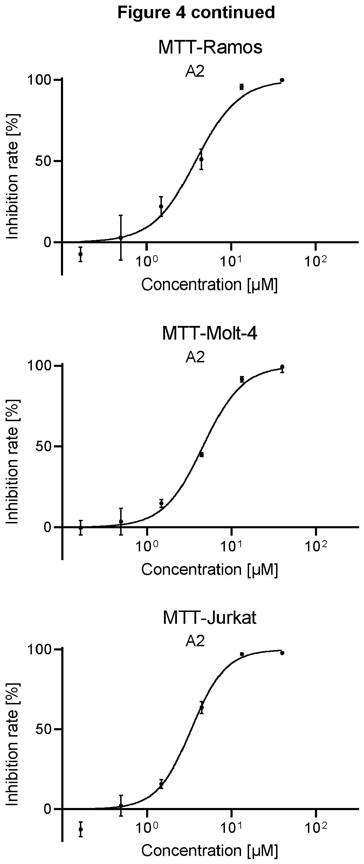

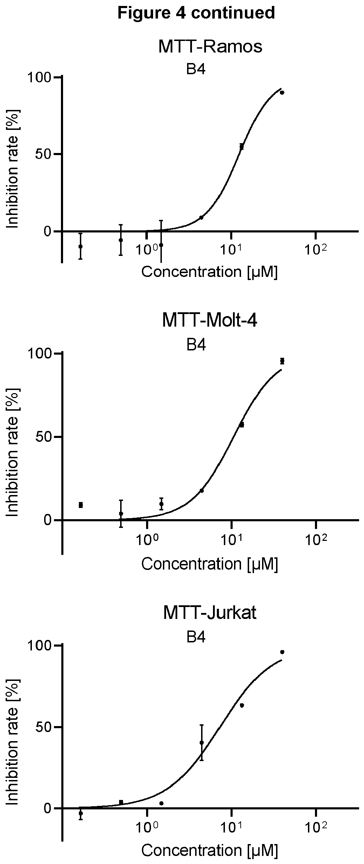

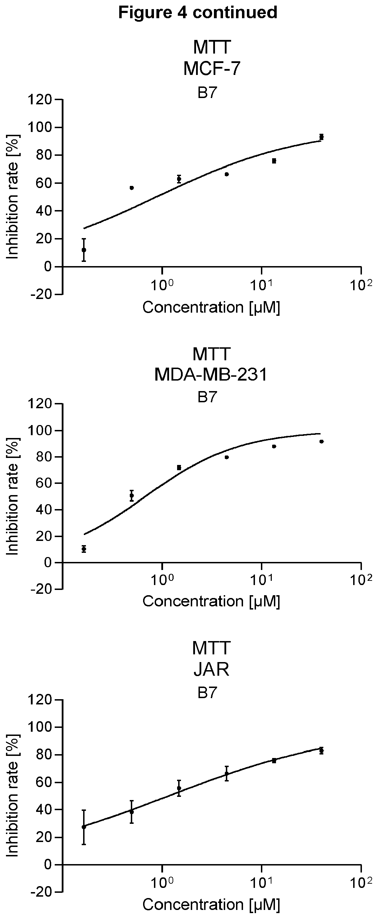

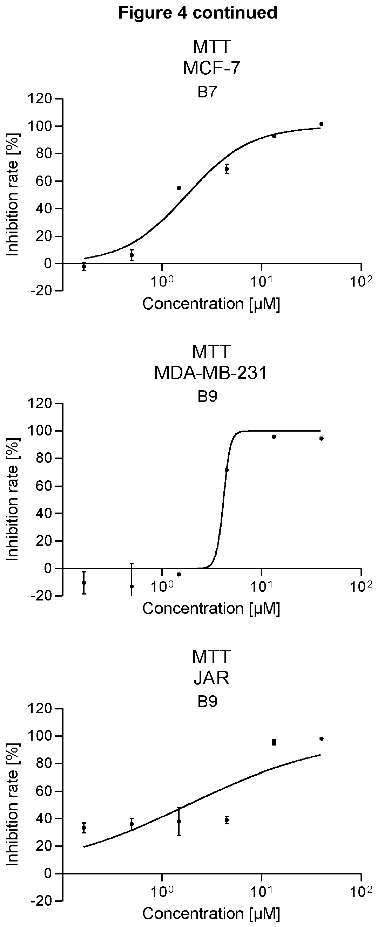

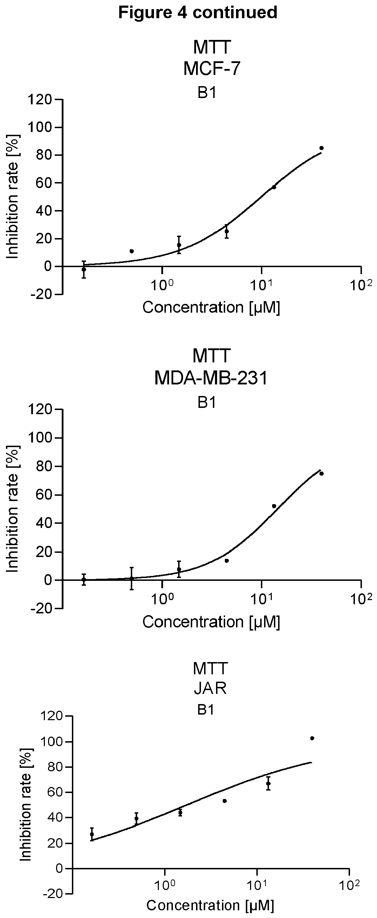

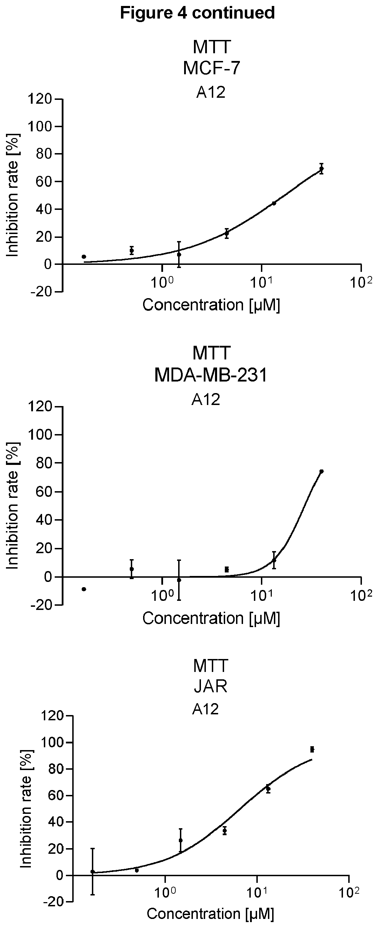

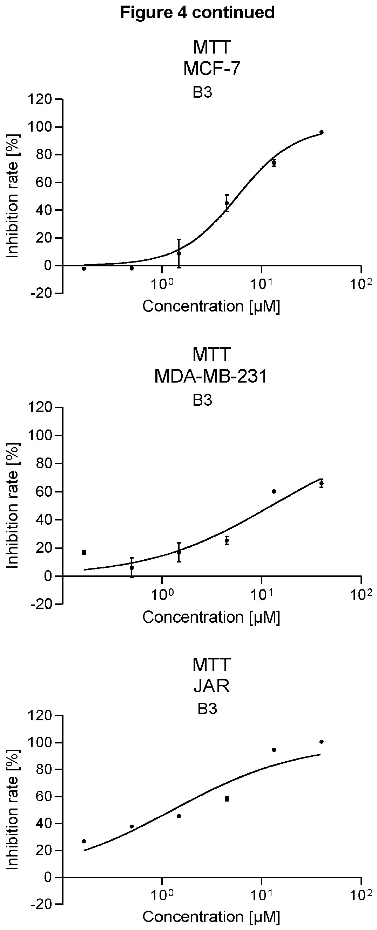

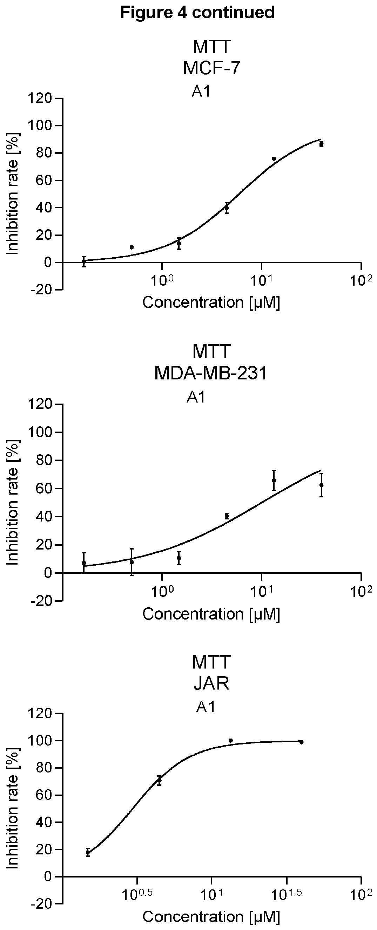

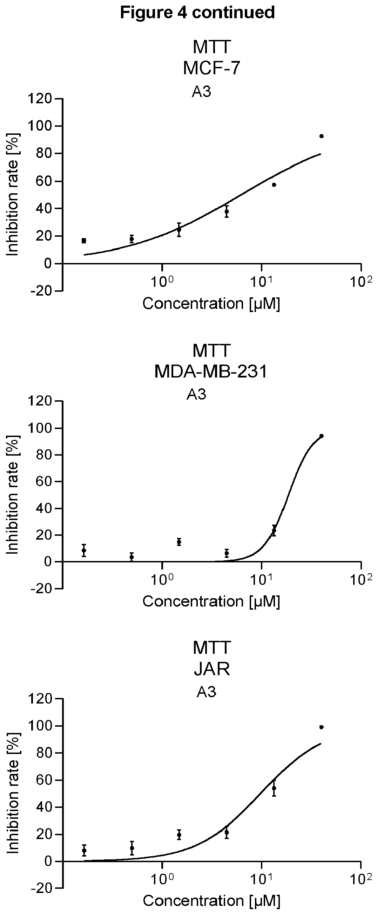

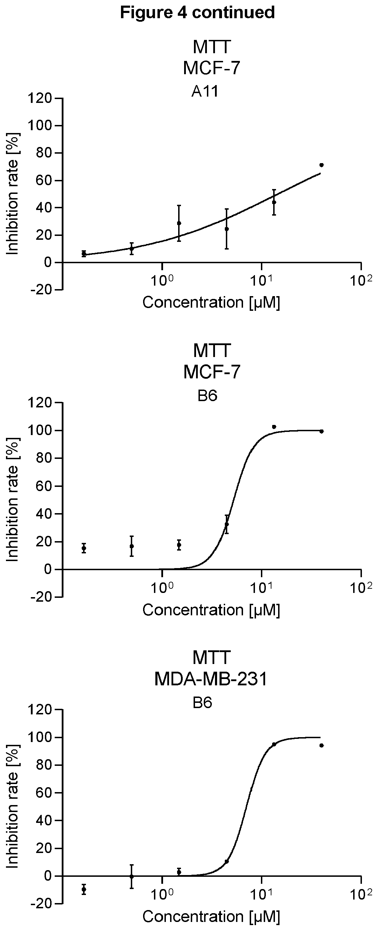

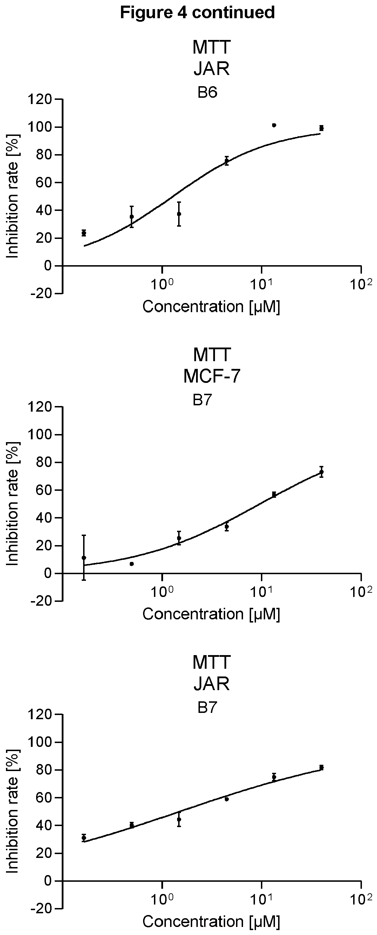

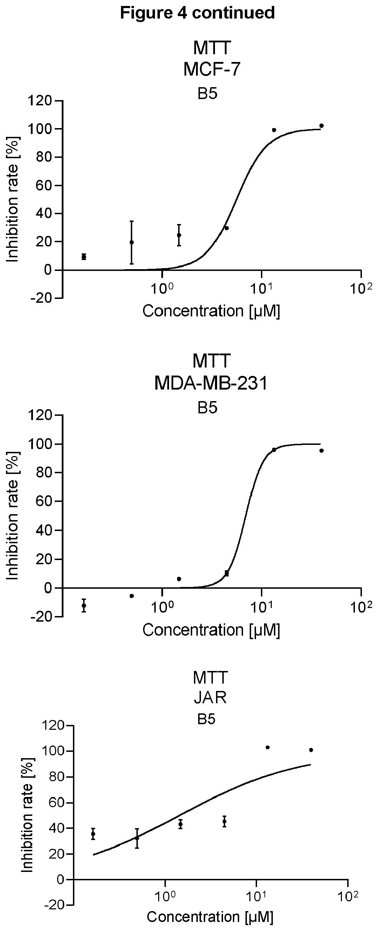

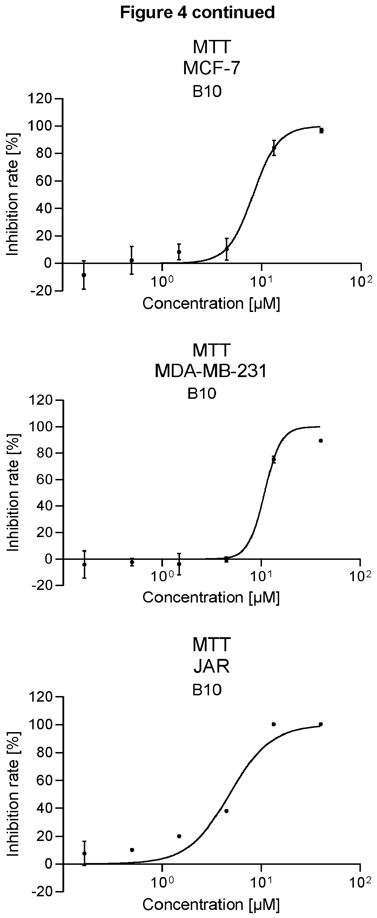

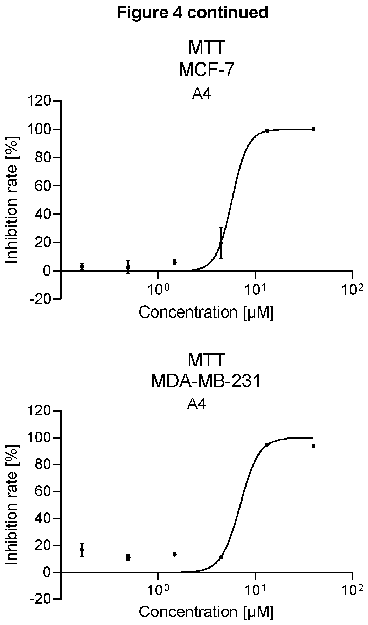

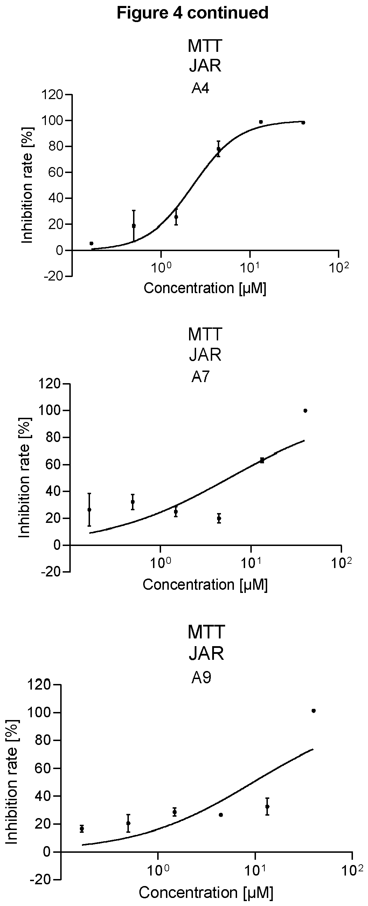

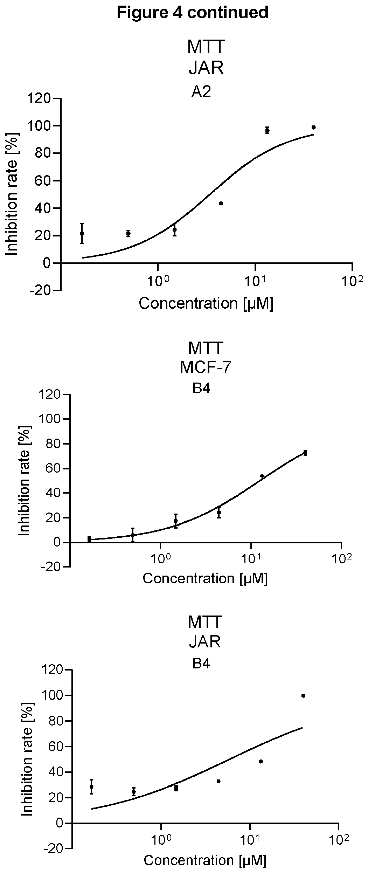

The IC₅₀ curves are shown in Figure 4.

**Table 2**

| **Compound ID** | **Ramos** | **Cell lines IC₅₀ [µM] Molt-4** | **Jurkat** |
|---|---|---|---|
| **B8** | 1.48 | 5.95 | 1.48 |
| **B9** | 5.54 | 4.14 | 3.17 |
| **B1** | 7.35 | 7.01 | 6.31 |
| **A12** | 10.66 | 9.05 | 11.45 |
| **B2** | 3.03 | 4.67 | 4.49 |
| **A10** | 8.87 | 1.60 | 7.51 |
| **A1** | 4.81 | 2.91 | 2.81 |
| **A3** | 7.03 | 10.8 | 3.73 |
| **A11** | 5.29 | 13.69 | 7.65 |
| **B6** | 1.82 | 4.79 | 5.47 |
| **B7** | 6.61 | 13.87 | 7.42 |
| **B5** | 2.80 | 8.42 | 5.90 |
| **B10** | 8.27 | 12.12 | 11.19 |
| **A8** | 26.80 | 8.50 | 19.08 |
| **A5** | 5.05 | 5.49 | 5.13 |
| **A9** | >20 | 11.44 | >20 |
| **A2** | 3.80 | 4.61 | 3.35 |
| **B4** | 12.43 | 10.61 | 7.30 |

**Table 3**

| **Compound ID** | **MCF-7** | **Cell lines IC₅₀ [µM] MDA-MB-231** | **JAR** |
|---|---|---|---|
| **B7** | 0.87 | 0.68 | 1.16 |
| **B9** | 1.78 | 4.12 | 1.82 |
| **B1** | 9.92 | 14.43 | 1.74 |
| **A12** | 16.85 | >20 | 6.58 |
| **B3** | 5.72 | 12.49 | 1.28 |
| **A10** | >20 | >20 | >20 |
| **A1** | 5.95 | 10.02 | 2.95 |
| **A3** | 6.22 | 18.62 | 9.75 |
| **A11** | 14.55 | >20 | ND |
| **B6** | 5.23 | 7.07 | 1.29 |
| **B7** | 9.62 | >20 | 1.5 |
| **B5** | 5.61 | 6.95 | 1.44 |
| **B10** | 8.24 | 10.82 | 4.78 |
| **A7** | >20 | >20 | 5.74 |
| **A4** | 5.79 | 7.02 | 2.30 |
| **A9** | >20 | >20 | 9.56 |
| **A2** | ND | ND | 3.41 |
| **B4** | 12.53 | >20 | 5.92 |

**Table 4 Sequence Listing.**

| **SEQ ID No.** | **description** | **SEQ ID No.** | **description** |
|---|---|---|---|
| 1 | microRNA 17 hairpin probe | 28 | hsa-miR-422a |
| 2 | microRNA 21 hairpin probe | 29 | hsa-miR-423 |
| 3 | hsa-miR-153-1 | 30 | hsa-miR-4673 |
| 4 | hsa-miR-153-2 | 31 | hsa-miR-4761 |
| 5 | hsa-miR-18a | 32 | hsa-let-7e |
| 6 | hsa-miR-19b-2 | 33 | hsa-miR-150 |
| 7 | hsa-miR-20a | 34 | hsa-miR-181b-2 |
| 8 | hsa-miR-222 | 35 | hsa-miR-186 |
| 9 | hsa-miR-3180-4 | 36 | hsa-miR-25 |
| 10 | hsa-miR-4267 | 37 | hsa-miR-27a |
| 11 | hsa-miR-487a | 38 | hsa-miR-3130-2 |
| 12 | hsa-miR-539 | 39 | hsa-miR-3616 |
| 13 | hsa-miR-571 | 40 | hsa-miR-381 |
| 14 | hsa-miR-106a | 41 | hsa-miR-382 |
| 15 | hsa-miR-1197 | 42 | hsa-miR-449a |
| 16 | hsa-miR-1324 | 43 | hsa-miR-4739 |
| 17 | hsa-miR-27b | 44 | hsa-miR-495 |
| 18 | hsa-miR-3178 | 45 | hsa-miR-501 |
| 19 | hsa-miR-548a-2 | 46 | hsa-miR-548g |
| 20 | hsa-miR-658 | 47 | hsa-miR-548j |
| 21 | hsa-miR-662 | 48 | hsa-miR-555 |
| 22 | hsa-miR-93 | 49 | hsa-miR-660 |
| 23 | hsa-miR-1307 | 50 | hsa-miR-7-1 |
| 24 | hsa-miR-3147 | 51 | hsa-miR-92a-1 |
| 25 | hsa-miR-375 | 52 | hsa-miR-17 stem-loop sequence |
| 26 | hsa-miR-378f | 53 | hsa-miR-21 stem-loop sequence |
| 27 | hsa-miR-378h | | |

## Claims

1. A compound of general formula (I) 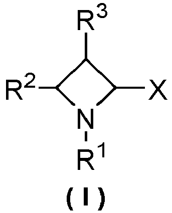 wherein
**R¹** represents -CH₃, -CH₂CH₃, -CH₂CH₂CH₃, -CH(CH₃)₂, -(CH₂)₃CH₃, -(CH₂)₄CH₃, -(CH₂)₅CH₃, -CH₂CH(CH₃)₂, -(CH₂)₂CH(CH₃)₂, -CH(CH₂CH₃)CH₃, -CH(CH₂CH₃)₂, -C(CH₃)₃, -CH₂CH=CH₂, -(CH₂)₂CH=CH₂, -(CH₂)₃CH=CH₂, -(CH₂)₄CH=CH₂, -**L¹-A¹**, or -**B¹** ;
**R²** represents -H, -CN, -N**R⁴R⁵**, -**L²⁻A²**, -**B²**, or -**C²**;
**R³** represents -**A³** or -**B³** with the proviso that when **R²** is -CN **R³** is -**A³**;
**L¹** represents -CO-, or -SO₂- ;
**L²** represents -CH₂-N**R⁶**-CO-, -CH₂-N**R⁶**-SO₂- or 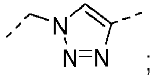
**R⁴** and **R⁵** independently of each other represent -CH₃, -CH₂CH₃, -CH(CH₃)₂, -CH₂CH₂CH₃, -CH₂CH(CH₃)₂, -C(CH₃)₃, -cyclo-C₃H₅, -cyclo-C₄H₇, -cyclo-C₅H₉, -cyclo-C₆H₁₁, -CH₂-cyclo-C₃H₅, -CH₂-cyclo-C₄H₇, -CH₂-cyclo-C₅H₉, -CH₂-cyclo-C₆H₁₁, -CH₂-Ph, -CH₂OCH₃, -CH₂OCH₂CH₃, -CH₂CH₂OCH₃, or -CH₂CH₂SCH₃;
**A¹** and **A²** are independently of each other selected from 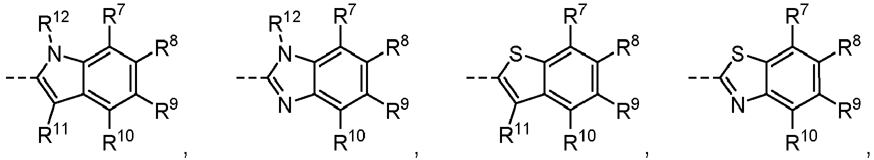 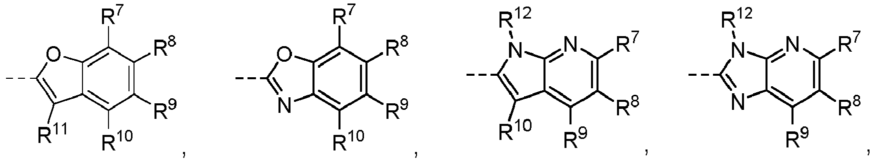 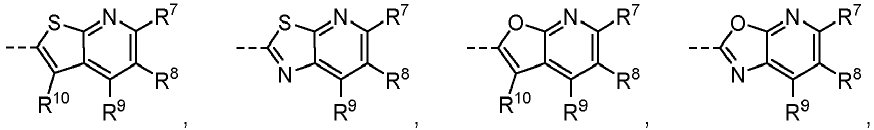 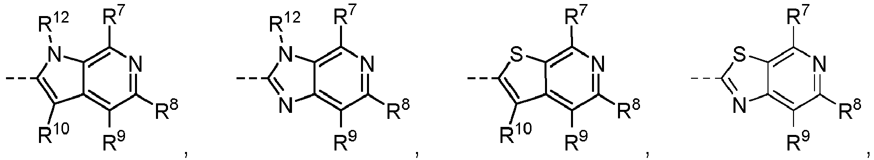 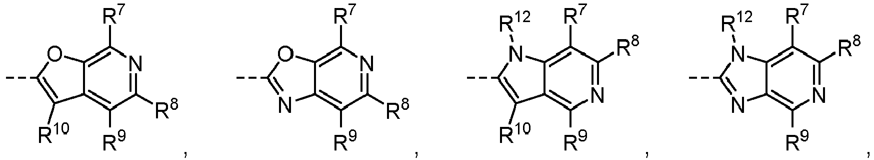 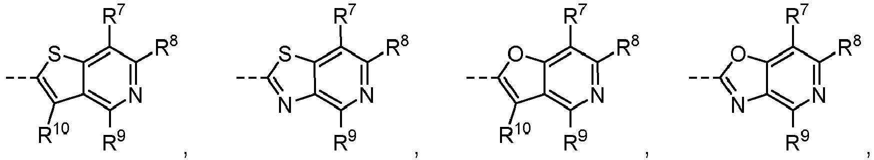 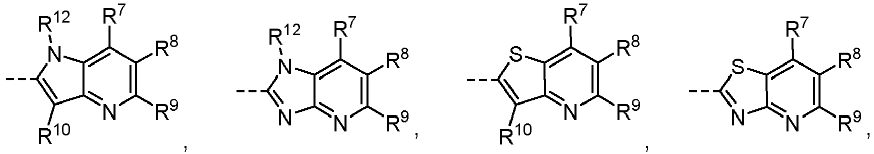 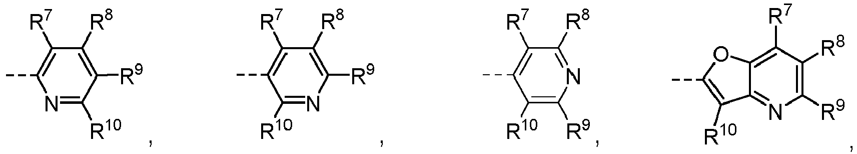 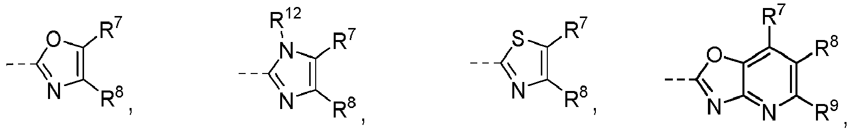 or 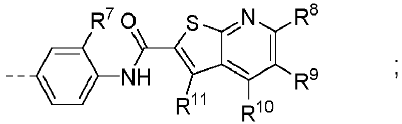
**A³** represents 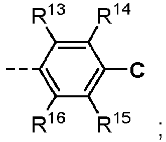 with C being selected from: 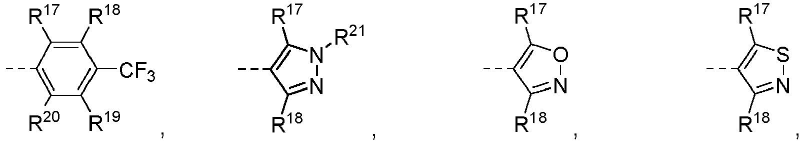 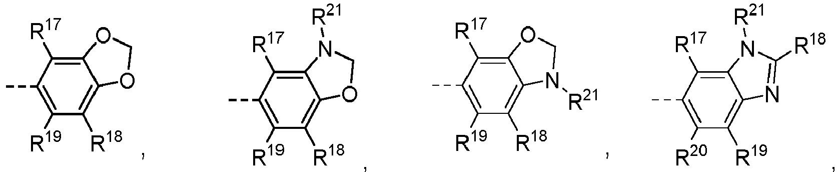 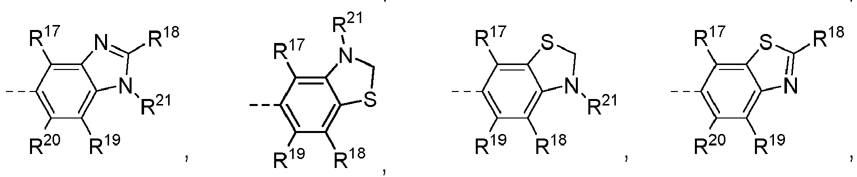 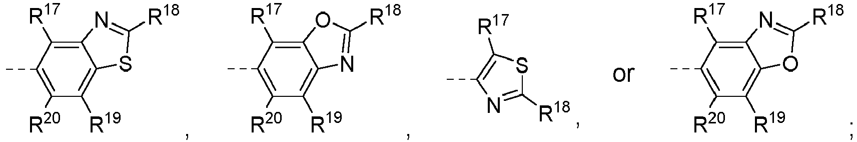
**B¹** represents -**H¹**, -**P¹**-**H¹** or -**P¹-Y¹-H¹** ;
**B²** represents **-H²**, **-P²-H²**, or **-P²-Y²-H²**;
**B³** represents **-H³**, **-P³-H³**, or **-P³-Y³-H³**;
**C²** is selected from: 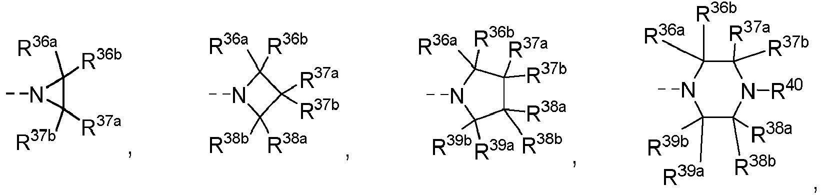 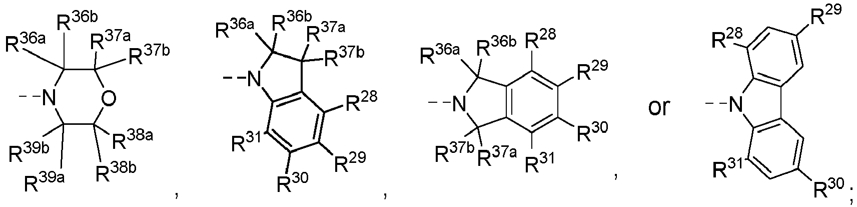
**Y¹**, **Y²**, and **Y³** are independently of each other selected from: -O-, -N**R²²-,** -CO-N**R²²**-, -N**R²²**-CO-, -SO₂-N**R²²**-, -N**R²²**-SO₂-, or -N**R²²**-CO-N**R²³**-;
**P¹**, **P²**, and **P³** are independently of each other selected from: 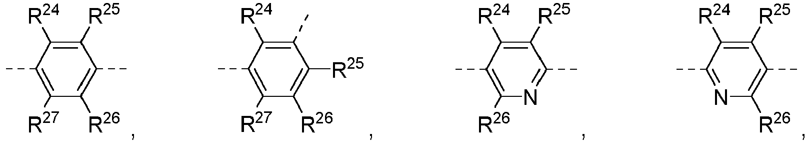 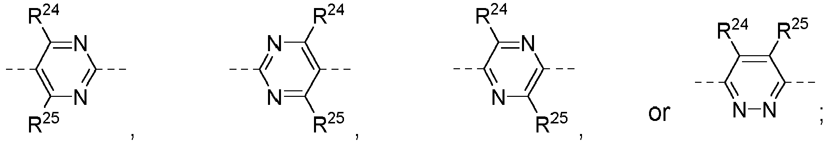
**H¹**, **H²**, and **H³** are independently of each other selected from: 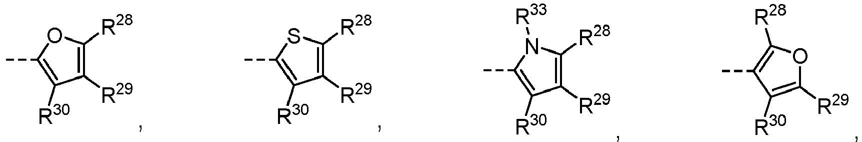 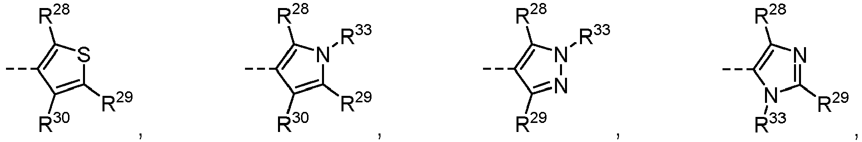 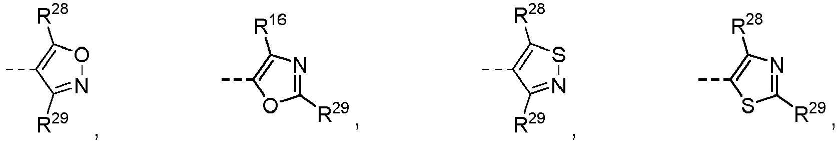 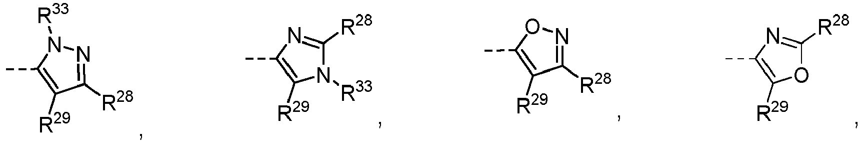 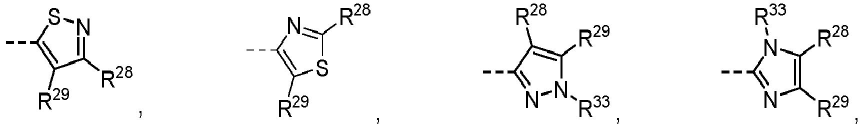 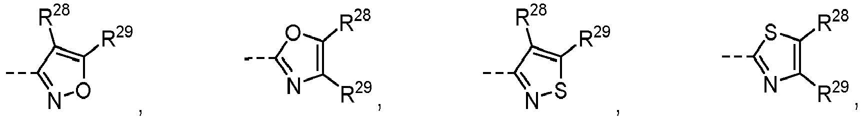 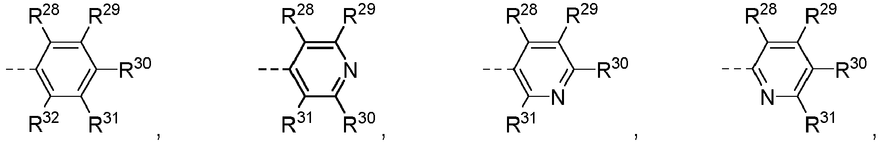 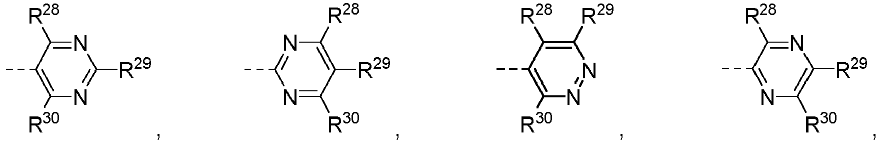 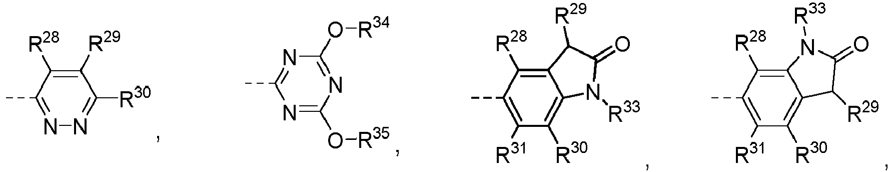 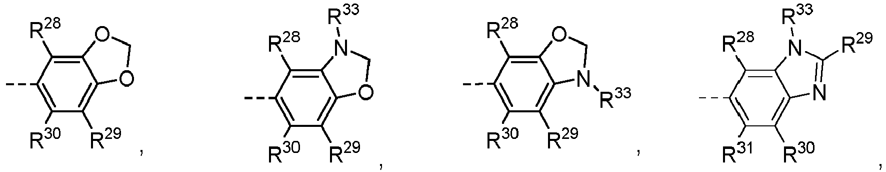 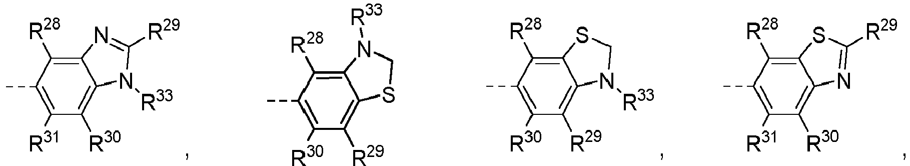 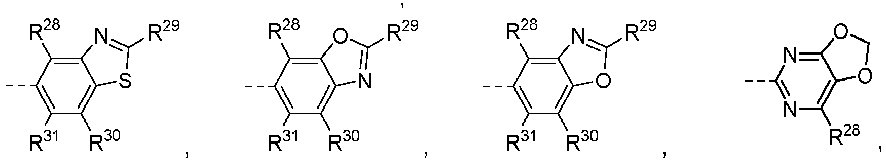 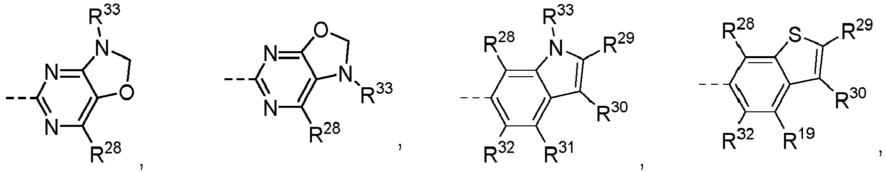 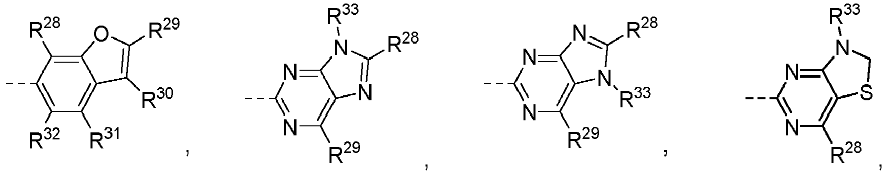 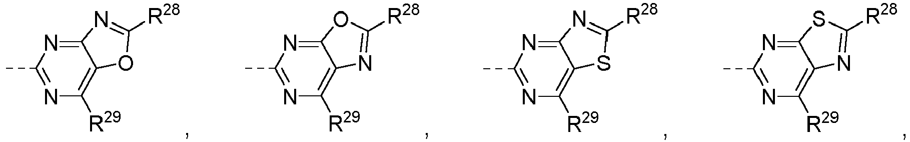 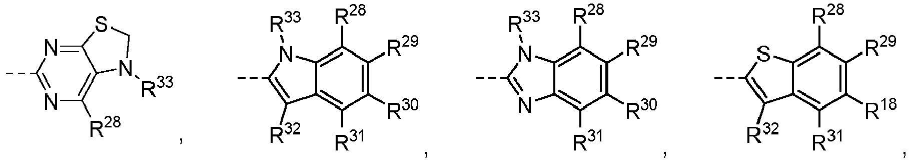 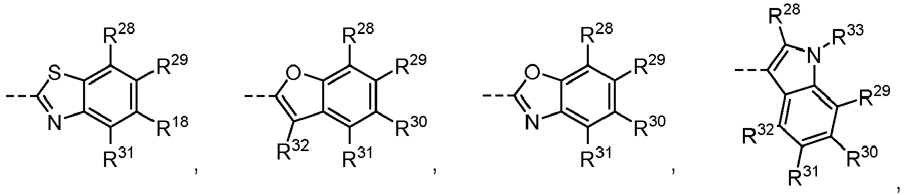 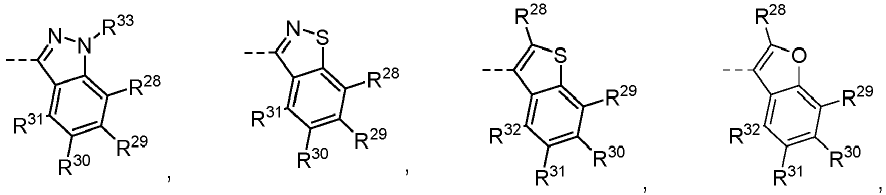 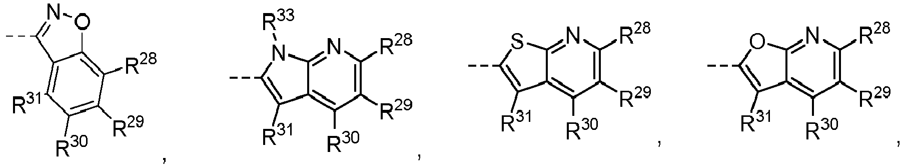 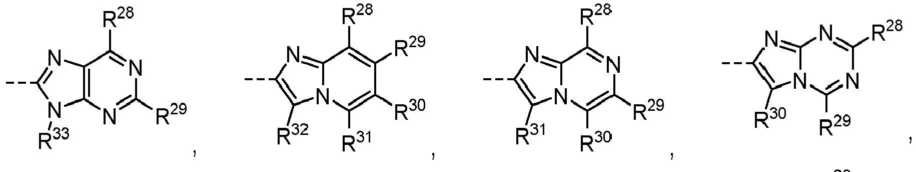 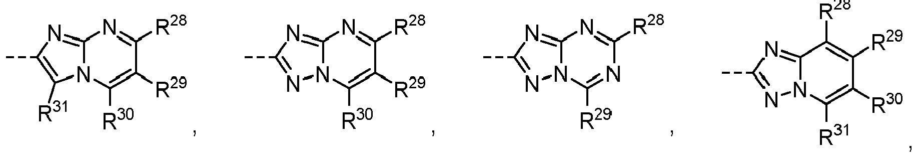 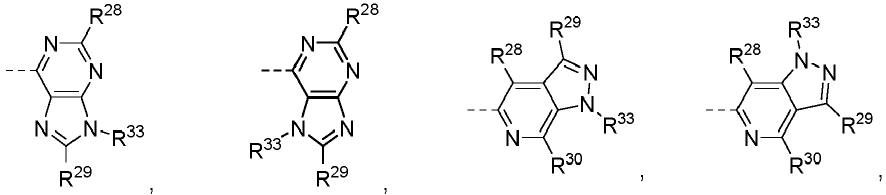 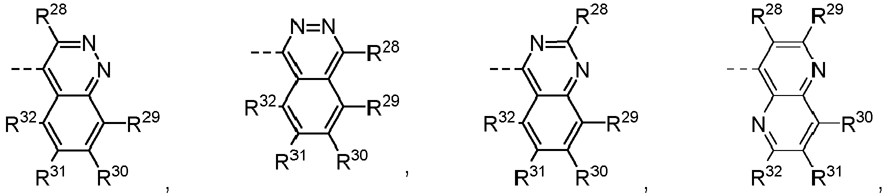 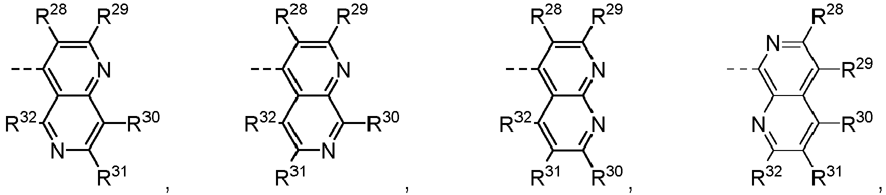 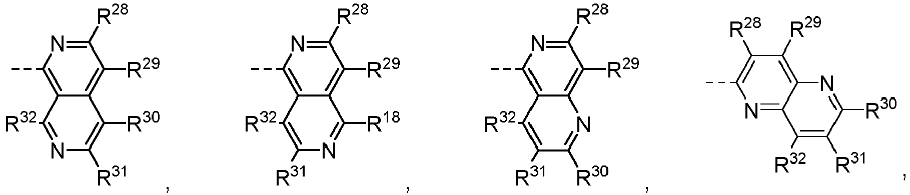 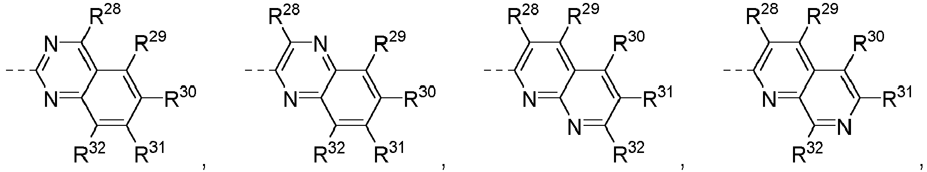 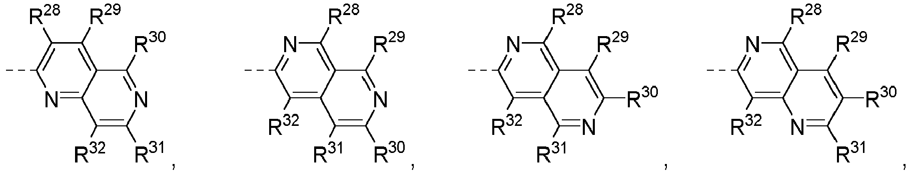 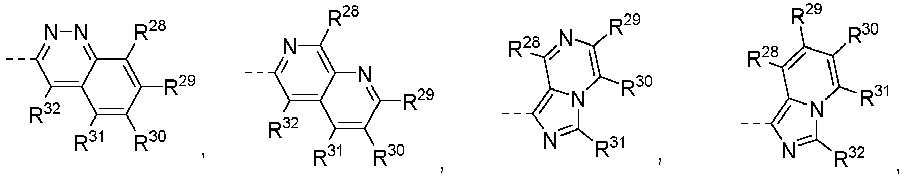 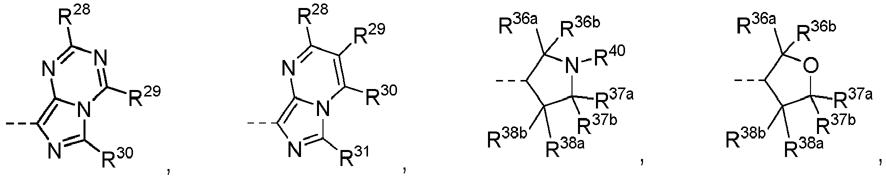 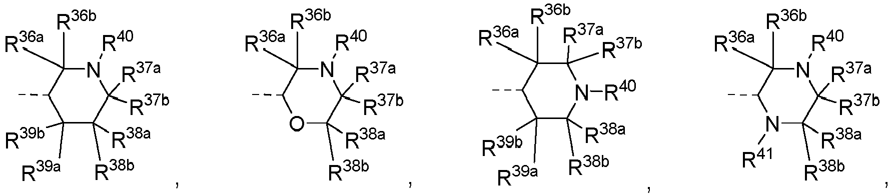 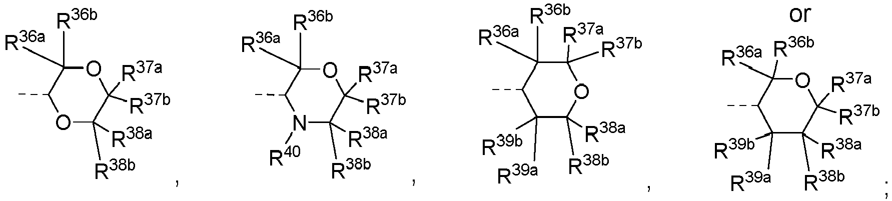
**R¹⁷** - **R²⁰** and **R²⁸**-**R³²** represent independently of each other -H, -F, -Cl, -Br, -I, -OH, -CN, -NO₂, -CH₃, -C₂H₅, -C₃H₇, -CH(CH₃)₂, -C₄H₉, -CH₂-CH(CH₃)₂, -CH(CH₃)-C₂H₅, -C(CH₃)₃, -cyclo-C₃H₅, -CH₂-cyclo-C₃H₅, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -OCH₃, -OC₂H₅, -OC₃H₇, -OCH(CH₃)₂, -OC(CH₃)₃, -OC₄H₉, -OCHF₂, -OCF₃, -OCH₂CF₃, -OC₂F₅, -OCH₂OCH₃, -O-cyclo-C₃H₅, -OCH₂-cyclo-C₃H₅, -O-C₂H₄-cyclo-C₃H₅, -CHO, -COCH₃, -COCF₃, -COC₂H₅, -COC₃H₇, -COCH(CH₃)₂, -COC(CH₃)₃, -COOH, -COOCH₃, -COOC₂H₅, -COOC₃H₇, -COOCH(CH₃)₂, -COOC(CH₃)₃, -OOC-CH₃, -OOC-CF₃, -OOC-C₂H₅, -OOC-C₃H₇, -OOC-CH(CH₃)₂, -OOC-C(CH₃)₃, -NH₂, -NHCH₃, -NHC₂H₅, -NHC₃H₇, -NHCH(CH₃)₂, -NHC(CH₃)₃, -N(CH₃)₂, -N(C₂H₅)₂, -N(C₃H₇)₂, -N[CH(CH₃)₂]₂, -N[C(CH₃)₃]₂, -NHCOCH₃, -NHCOCF₃, -NHCOC₂H₅, -NHCOC₃H₇, -NHCOCH(CH₃)₂, -NHCOC(CH₃)₃, -CONH₂, -CONHCH₃, -CONHC₂H₅, -CONHC₃H₇, -CONHCH(CH₃)₂, -CONH-cyclo-C₃H₅, -CONHC(CH₃)₃, -CON(CH₃)₂, -CON(C₂H₅)₂, -CON(C₃H₇)₂, -CON[CH(CH₃)₂]₂, -CON[C(CH₃)₃]₂, -SO₂NH₂, -SO₂NHCH₃, -SO₂NHC₂H₃, -SO₂NHC₃H₇, -SO₂NHCH(CH₃)₂, -SO₂NH-cyclo-C₃H₃, -SO₂NHC(CH₃)₃, -SO₂N(CH₃)₂, -SO₂N(C₂H₃)₂, -SO₂N(C₃H₇)₂, -SO₂N[CH(CH₃)₂]₂, -SO₂N[C(CH₃)₃]₂, -NHSO₂CH₃, -NHSO₂CF₃, -NHSO₂C₂H₃, -NHSO₂C₃H₇, -NHSO₂CH(CH₃)₂, -NHSO₂C(CH₃)₃, -CH=CH₂, -CH₂-CH=CH₂, -C(CH₃)=CH₂, -CH=CH-CH₃, -C≡CH, -C≡C-CH₃, -CH₂-C≡CH, -Ph, -O-Ph, -O-CH₂-Ph, -C(=NH)NH₂, or 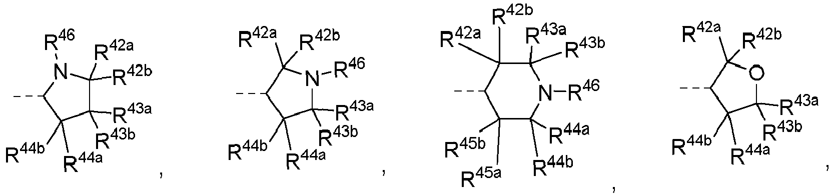 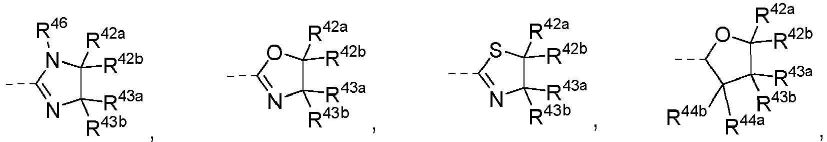 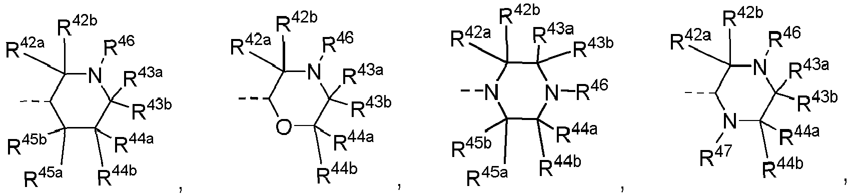 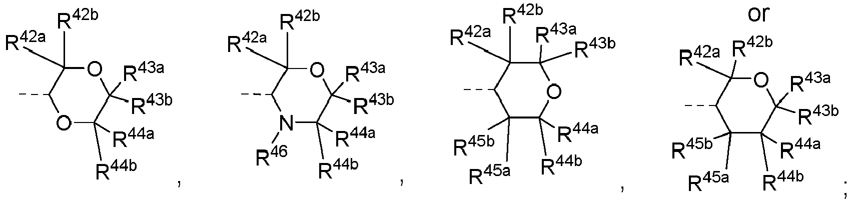
**R⁶**, **R¹¹**, **R¹²**, **R²¹** - **R²³**, **R³³** - **R³⁶**, **R⁴⁰**, **R⁴¹**, **R⁴⁶**, and **R⁴⁷** represent independently of each other -H, -CH₃, -CH₂CH₃, -CH(CH₃)₂, -CH₂CH₂CH₃, -CH₂CH(CH₃)₂, -C(CH₃)₃, -cyclo-C₃H₅, -cyclo-C₄H₇, -cyclo-C₅H₉, -cyclo-C₆H₁₁, -CH₂-cyclo-C₃H₅, -CH₂-cyclo-C₄H₇, -CH₂-cyclo-C₅H₉, -CH₂-cyclo-C₆H₁₁, -CH₂-Ph, -CH₂OCH₃, -CH₂OCH₂CH₃, -CH₂CH₂OCH₃, -COO-C(CH₃)₃, -COO-CH₂Ph, or -CH₂CH₂SCH₃;
**R²⁴** - **R²⁷**, **R^{36a}** - **R^{39a}**, **R^{36b}** - **R^{39b} R^{42a}** - **R^{45a}**, and **R^{42b}** - **R^{45b}** represent independently of each other -H, -F, -Cl, -Br, -I, -OH, -CN, -NO₂, -CH₃, -C₂H₅, -C₃H₇, -CH(CH₃)₂, -C₄H₉, -CH₂-CH(CH₃)₂, -CH(CH₃)-C₂H₅, -C(CH₃)₃, -cyclo-C₃H₅, -CH₂-cyclo-C₃H₅, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -OCH₃, -OC₂H₅, -OC₃H₇, -OCH(CH₃)₂, -OC(CH₃)₃, -OC₄H₉, -OCHF₂, -OCF₃, -OCH₂CF₃, -OC₂F₅, -OCH₂OCH₃, -O-cyclo-C₃H₅, -OCH₂-cyclo-C₃H₅, -O-C₂H₄-cyclo-C₃H₅, -CHO, -COCH₃, -COCF₃, -COC₂H₅, -COC₃H₇, -COCH(CH₃)₂, -COC(CH₃)₃, -COOH, -COOCH₃, -COOC₂H₅, -COOC₃H₇, -COOCH(CH₃)₂, -COOC(CH₃)₃, -OOC-CH₃, -OOC-CF₃, -OOC-C₂H₅, -OOC-C₃H₇, -OOC-CH(CH₃)₂, -OOC-C(CH₃)₃, -NH₂, -NHCH₃, -NHC₂H₅, -NHC₃H₇, -NHCH(CH₃)₂, -NHC(CH₃)₃, -N(CH₃)₂, -N(C₂H₅)₂, -N(C₃H₇)₂, -N[CH(CH₃)₂]₂, -N[C(CH₃)₃]₂, -NHCOCH₃, -NHCOCF₃, -NHCOC₂H₅, -NHCOC₃H₇, -NHCOCH(CH₃)₂, -NHCOC(CH₃)₃, -CONH₂, -CONHCH₃, -CONHC₂H₅, -CONHC₃H₇, -CONHCH(CH₃)₂, -CONH-cyclo-C₃H₅, -CONHC(CH₃)₃, -CON(CH₃)₂, -CON(C₂H₅)₂, -CON(C₃H₇)₂, -CON[CH(CH₃)₂]₂, -CON[C(CH₃)₃]₂, -SO₂NH₂, -SO₂NHCH₃, -SO₂NHC₂H₃, -SO₂NHC₃H₇, -SO₂NHCH(CH₃)₂, -SO₂NH-cyclo-C₃H₃, -SO₂NHC(CH₃)₃, -SO₂N(CH₃)₂, -SO₂N(C₂H₃)₂, -SO₂N(C₃H₇)₂, -SO₂N[CH(CH₃)₂]₂, -SO₂N[C(CH₃)₃]₂,-NHSO₂CH₃, -NHSO₂CF₃, -NHSO₂C₂H₃, -NHSO₂C₃H₇, -NHSO₂CH(CH₃)₂, -NHSO₂C(CH₃)₃, -CH=CH₂, -CH₂-CH=CH₂, -C(CH₃)=CH₂, -CH=CH-CH₃, -C≡CH, -C≡C-CH₃, -CH₂-C≡CH, -Ph, -O-Ph, or -O-CH₂-Ph;
**X** represents -CN, -NH₂, or -CH₂OH;
with the proviso that **X** and **R²** cannot be simultaneously -CN;
or a regiomer, diastereomer, enantiomer, a mixture of regiomers, a mixture of diastereomers, a mixture of enantiomers, a prodrug, a solvate, a hydrate, or a pharmaceutically acceptable salt thereof.

2. The compound according to claim 1, wherein the compound has any one of the following formulae (**I-a**), (**I-b**), (**I-c**), or (**I-d**).

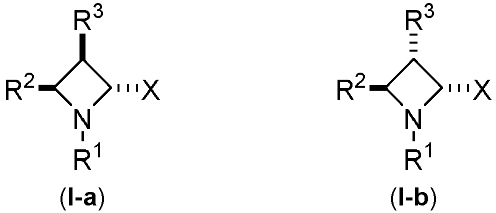

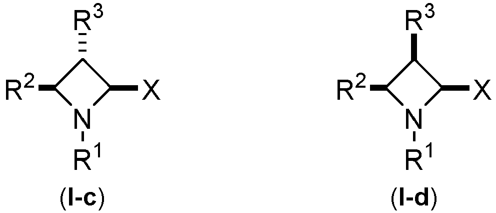

3. The compound according to claim 1, wherein the compound has any one of the following formulae (**II-1**) to (**II-6**) 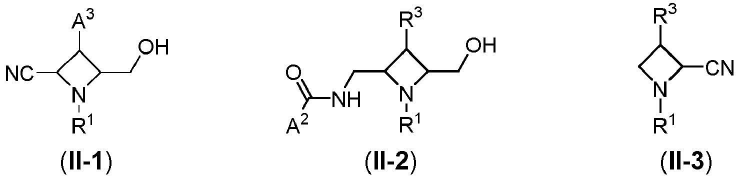 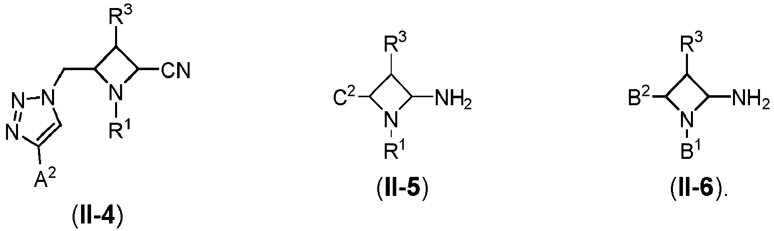 wherein **R¹**, **R³**, **A²**, **A³**, **B¹** and **B²** have the meanings as defined in claim 1.

4. The compound according to any one of claims 1 to 3, wherein **R¹** represents -CH₂CH₂CH₃, -CH₂CH=CH₂, 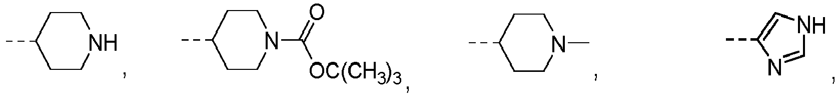 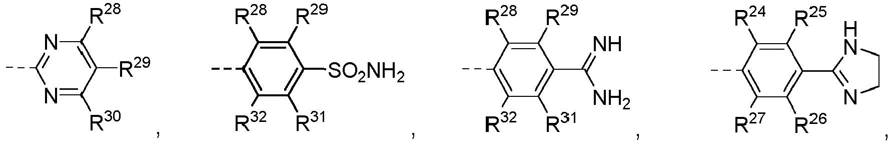 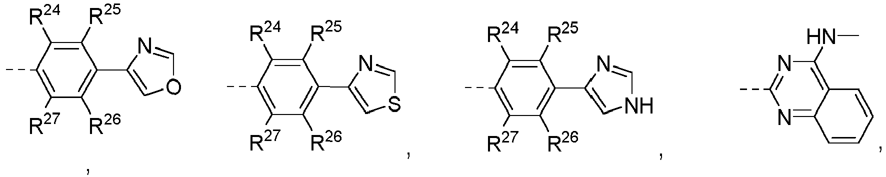 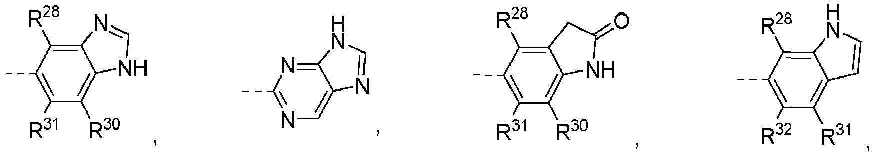 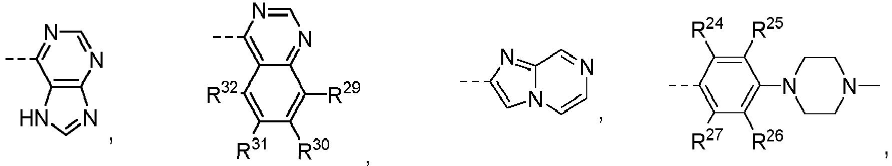 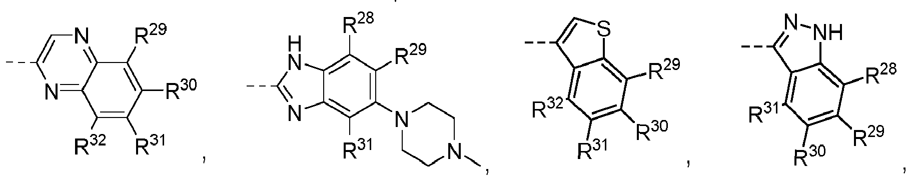 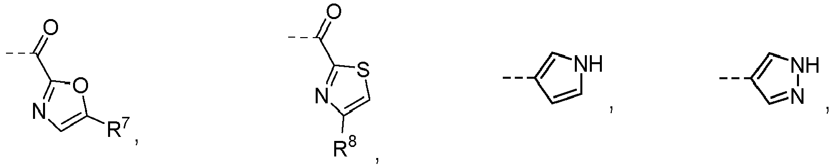 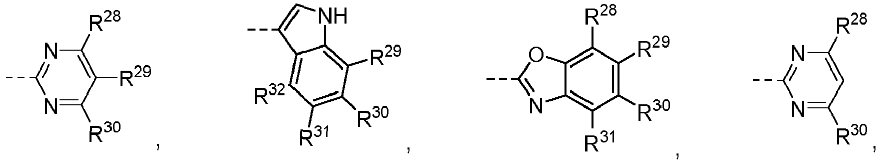 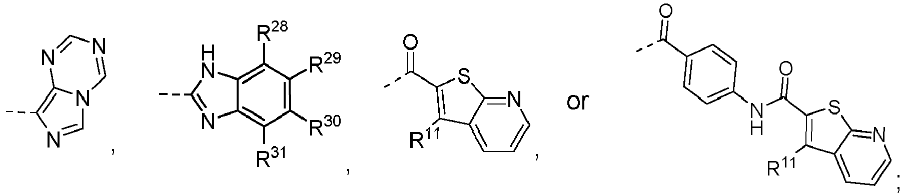 wherein **R⁷**, **R⁸**, **R**^{**1**1} and **R**^{**24** -} **R³²** have the meanings as defined in claim 1.

5. The compound according to any one of claims 1 to 4, wherein **R²** represents -H, -CN, -N(CH₃)₂, 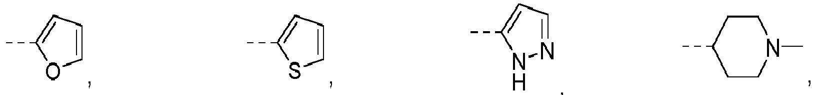 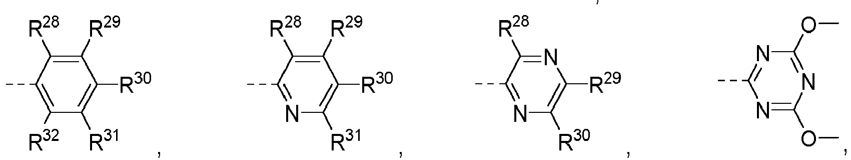 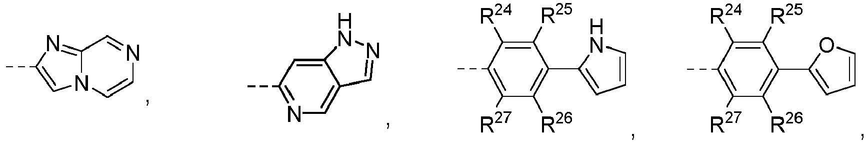 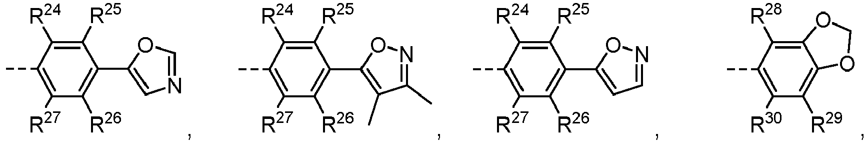 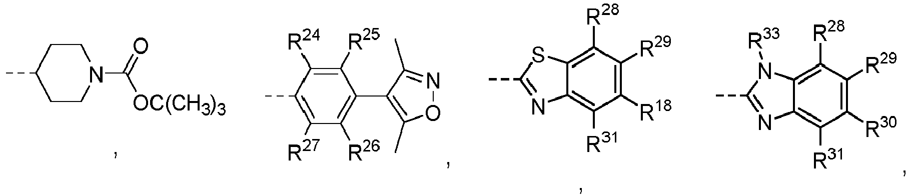 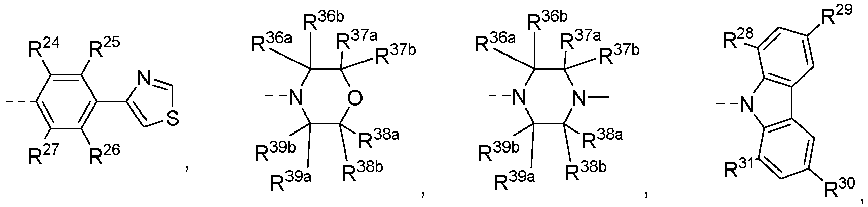 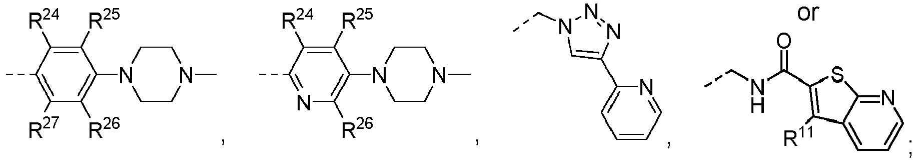 wherein **R¹¹**, **R**^{**24** -} **R³²**, **R^{36a}** - **R^{39a}**, and **R^{36b}** - **R^{39b}** have the meanings as defined in claim 1.

6. The compound according to any one of claims 1 to 5, wherein **R³** represents 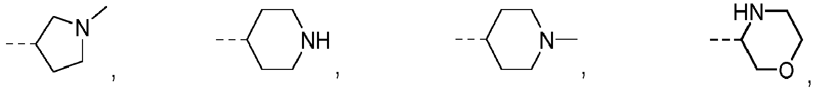 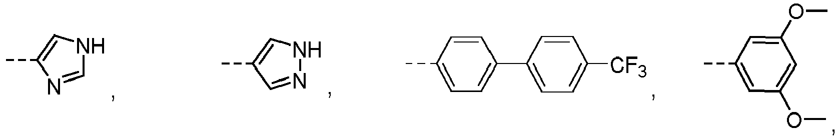 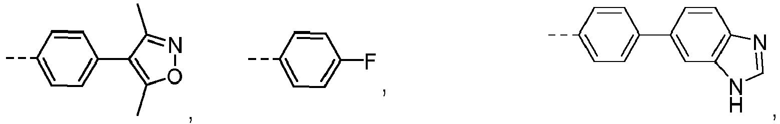 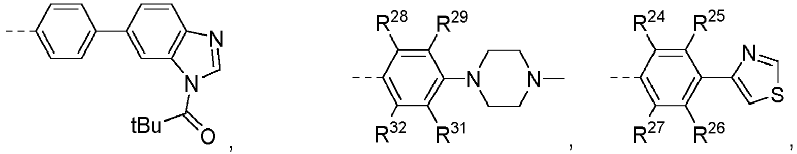 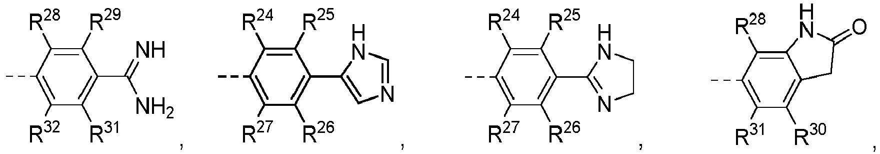 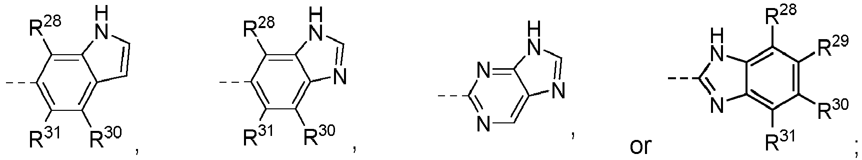 wherein **R**^{**24** -} **R³²** have the meanings as defined in claim 1.

7. The compound according to claim 1 selected from the following list
1-(3-aminothieno[2,3-b] pyridine-2-carbonyl)-4-(hydroxymethyl)-3-(4'-(trifluoromethyl)-[1 ,1'-biphenyl]-4-yl) azetidine-2-carbonitrile;
1-(3-aminothieno[2,3-b]pyridine-2-carbonyl)-4-(hydroxymethyl)-3-(4'-(trifluoromethyl)-[1,1'-biphenyl]-4-yl)azetidine-2-carbonitrile;
1-(3-aminothieno[2,3-b]pyridine-2-carbonyl)-4-(hydroxymethyl)-3-(4'-(trifluoromethyl)-[1,1'-biphenyl]-4-yl)azetidine-2-carbonitrile;
3-amino-N-((4-(hydroxymethyl)-1-propyl-3-(4'-(trifluoromethyl)-[1,1'-biphenyl]-4-yl)azetidin-2-yl)methyl)thieno[2,3-b]pyridine-2-carboxamide;
3-amino-N-((4-(hydroxymethyl)-1-propyl-3-(4'-(trifluoromethyl)-[1,1'-biphenyl]-4-yl)azetidin-2-yl)methyl)thieno[2,3-b]pyridine-2-carboxamide;
1-allyl-4-((4-(pyridin-2-yl)-1H-1,2,3-triazol-1-yl)methyl)-3-(4'-(trifluoromethyl)-[1,1'-biphenyl]-4-yl)azetidine-2-carbonitrile;
3-(4-(1H-benzo[d]imidazol-6-yl)phenyl)-1-(3-aminothieno[2,3-b]pyridine-2-carbonyl)-4-(hydroxymethyl)azetidine-2-carbonitrile;
N-((3-(4-(1H-benzo[d]imidazol-6-yl)phenyl)-4-(hydroxymethyl)-1-propylazetidin-2-yl)methyl)-3-aminothieno[2,3-b]pyridine-2-carboxamide;
N-((3-(4-(1H-benzo[d]imidazol-6-yl)phenyl)-4-(hydroxymethyl)-1-propylazetidin-2-yl)methyl)-3-aminothieno[2,3-b]pyridine-2-carboxamide;
1-allyl-4-((4-(pyridin-2-yl)-1H-1,2,3-triazol-1-yl)methyl)-3-(4'-(trifluoromethyl)-[1,1'-biphenyl]-4-yl)azetidine-2-carbonitrile;
4-(hydroxymethyl)-1-(4-methylthiazole-2-carbonyl)-3-(4'-(trifluoromethyl)-[1,1'-biphenyl]-4-yl)azetidine-2-carbonitrile;
4-(hydroxymethyl)-1-(5-methyloxazole-2-carbonyl)-3-(4'-(trifluoromethyl)-[1,1'-biphenyl]-4-yl)azetidine-2-carbonitrile;
3-(4-(3,5-dimethylisoxazol-4-yl)phenyl)-1-(4-(methylamino)quinazolin-2-yl)azetidine-2-carbonitrile;
3-(4-(3,5-dimethylisoxazol-4-yl)phenyl)-1-(4-(methylamino)quinazolin-2-yl)azetidine-2-carbonitrile;
1-(3-aminothieno[2,3-b]pyridine-2-carbonyl)-3-(4-(3,5-dimethylisoxazol-4-yl)phenyl)azetidine-2-carbonitrile;
1-(3-aminothieno[2,3-b]pyridine-2-carbonyl)-3-(4-(3,5-dimethylisoxazol-4-yl)phenyl)azetidine-2-carbonitrile;
1-(4-(methylamino)quinazolin-2-yl)-3-(4'-(trifluoromethyl)-[1,1'-biphenyl]-4-yl)azetidine-2-carbonitrile;
1-(4-(methylamino)quinazolin-2-yl)-3-(4'-(trifluoromethyl)-[1,1'-biphenyl]-4-yl)azetidine-2-carbonitrile;
1-(3-aminothieno[2,3-b]pyridine-2-carbonyl)-3-(4'-(trifluoromethyl)-[1,1'-biphenyl]-4-yl)azetidine-2-carbonitrile;
1-(3-aminothieno[2,3-b]pyridine-2-carbonyl)-3-(4'-(trifluoromethyl)-[1,1'-biphenyl]-4-yl)azetidine-2-carbonitrile;
3-(4-(1H-benzo[d]imidazol-6-yl)phenyl)-1-(4-(methylamino)quinazolin-2-yl)azetidine-2-carbonitrile;
3-(4-(1H-benzo[d]imidazol-6-yl)phenyl)-1-(4-(methylamino)quinazolin-2-yl)azetidine-2-carbonitrile;
tert-butyl 4-(2-amino-4-(4-bromophenyl)-3-(4-(4-methylpiperazin-1-yl)phenyl)azetidin-1-yl)piperidine-1-carboxylate;
tert-butyl 4-(2-amino-4-(3,5-di-tert-butyl-4-hydroxyphenyl)-3-(4-fluorophenyl)azetidin-1-yl)piperidine-1-carboxylate;
di-tert-butyl 4,4'-(4-amino-3-(4-fluorophenyl)azetidine-1,2-diyl)bis(piperidine-1-carboxylate);
di-tert-butyl 4,4'-(4-amino-3-(4-(4-methylpiperazin-1-yl)phenyl)azetidine-1,2-diyl)bis(piperidine-1-carboxylate);
tert-butyl 4-(2-amino-4-(4-bromophenyl)-3-(4-fluorophenyl)azetidin-1-yl)piperidine-1-carboxylate;
tert-butyl 4-(2-amino-4-(4-(3,5-dimethylisoxazol-4-yl)phenyl)-3-(4-fluorophenyl)azetidin-1-yl)piperidine-1-carboxylate;
4-(benzo[d]thiazol-2-yl)-3-(1-methylpiperidin-4-yl)-1-(quinazolin-4-yl)azetidin-2-amine;
4-(benzo[d]thiazol-2-yl)-3-(1-methylpiperidin-4-yl)-1-(pyrimidin-2-yl)azetidin-2-amine;
4-(1H-benzo[d]imidazol-2-yl)-1,3-bis(1-methylpiperidin-4-yl)azetidin-2-amine;
4-(1H-benzo[d]imidazol-2-yl)-3-(3,5-dimethoxyphenyl)-1-(1H-indol-3-yl)azetidin-2-amine;
3-(1-methylpiperidin-4-yl)-1-(9H-purin-6-yl)-4-(4-(trifluoromethyl)phenyl)azetidin-2-amine;
3-(3,5-dimethoxyphenyl)-1-(1-methylpiperidin-4-yl)-4-(4-(thiazol-4-yl)phenyl)azetidin-2-amine;
1-(benzo[d]oxazol-2-yl)-3-(4-(4,5-dihydro-1H-imidazol-2-yl)phenyl)-4-(4-(4-methylpiperazin-1-yl)phenyl)azetidin-2-amine;
3-(3,5-dimethoxyphenyl)-1-(1-methylpiperidin-4-yl)-4-morpholinoazetidin-2-amine;
1-(4,6-dimethoxypyrimidin-2-yl)-4-(4-(4-methylpiperazin-1-yl)phenyl)-3-(4-(thiazol-4-yl)phenyl)azetidin-2-amine;
4-(9H-carbazol-9-yl)-1-(1-methylpiperidin-4-yl)-3-(4-(thiazol-4-yl)phenyl)azetidin-2-amine;
1-(1H-benzo[d]imidazol-2-yl)-3-(4-(3,5-dimethylisoxazol-4-yl)phenyl)-4-(4-methylpiperazin-1-yl)azetidin-2-amine;
3-(3,5-dimethoxyphenyl)-4-(4-(3,5-dimethylisoxazol-4-yl)phenyl)-1-(1-methylpiperidin-4-yl)azetidin-2-amine; and
4-(benzo[d]thiazol-2-yl)-3-(3,5-dimethoxyphenyl)-1-(1-methylpiperidin-4-yl)azetidin-2-amine.

8. A pharmaceutical composition comprising a carrier and a compound according to any one of claims 1 to 7.

9. A compound of formula (I) for use in the treatment and/or prophylaxis of a disease caused by and/or associated with microRNA expression or microRNA misexpression, 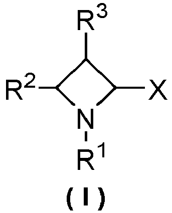 wherein
**R¹** represents -CH₃, -CH₂CH₃, -CH₂CH₂CH₃, -CH(CH₃)₂, -(CH₂)₃CH₃, -(CH₂)₄CH₃, -(CH₂)₅CH₃, -CH₂CH(CH₃)₂, -(CH₂)₂CH(CH₃)₂, -CH(CH₂CH₃)CH₃, -CH(CH₂CH₃)₂, -C(CH₃)₃, -CH₂CH=CH₂, -(CH₂)₂CH=CH₂, -(CH₂)₃CH=CH₂, -(CH₂)₄CH=CH₂, -**L¹-A¹**, or -**B¹** ;
**R²** represents -H, -CN, -N**R⁴R⁵**, -**L²-A²**, -**B²**, or -**C²**;
**R³** represents -**A³** or -**B³** with the proviso that when **R²** is -CN **R³** is -**A³**;
**L¹** represents -CO-, or -SO₂- ;
**L²** represents -CH₂-N**R⁶**-CO- , -CH₂-N**R⁶**-SO₂- or 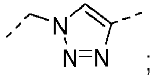
**R⁴** and **R⁵** independently of each other represent -CH₃, -CH₂CH₃, -CH(CH₃)₂, -CH₂CH₂CH₃, -CH₂CH(CH₃)₂, -C(CH₃)₃, -cyclo-C₃H₅, -cyclo-C₄H₇, -cyclo-C₅H₉, -cyclo-C₆H₁₁, -CH₂-cyclo-C₃H₅, -CH₂-cyclo-C₄H₇, -CH₂-cyclo-C₅H₉, -CH₂-cyclo-C₆H₁₁, -CH₂-Ph, -CH₂OCH₃, -CH₂OCH₂CH₃, -CH₂CH₂OCH₃, or -CH₂CH₂SCH₃;
**A¹** and A**²** are independently of each other selected from 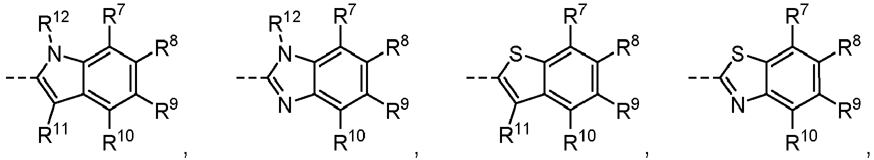 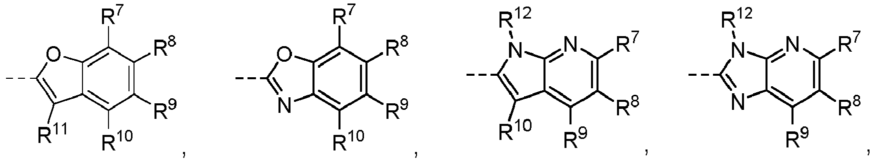 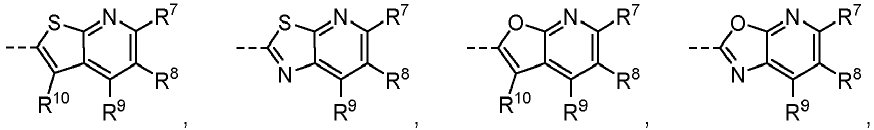 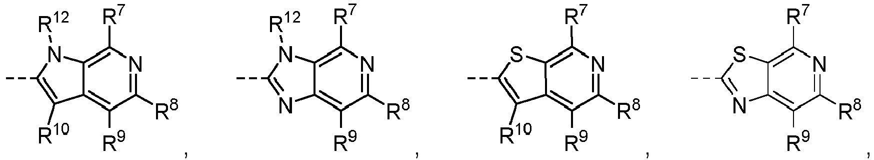 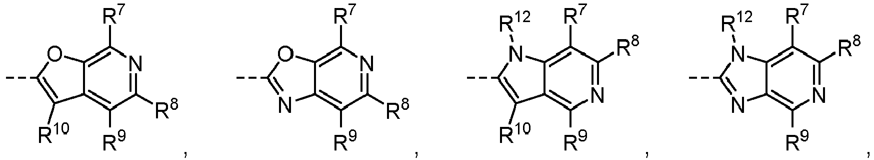 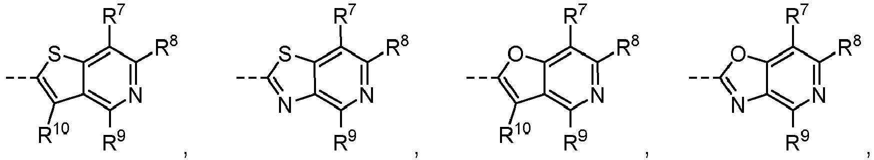 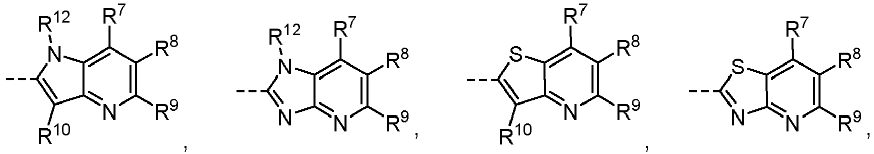 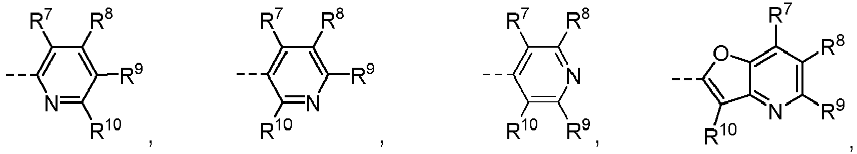 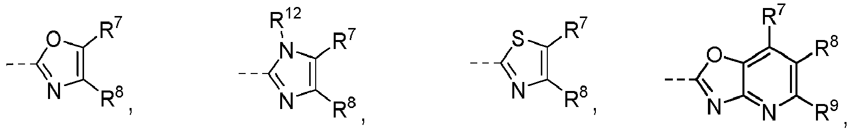 or 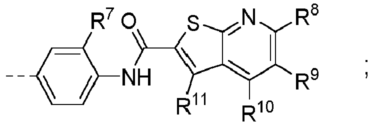
**A³** represents 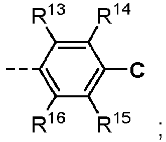 with C being selected from: 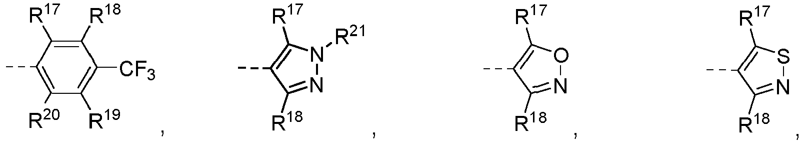 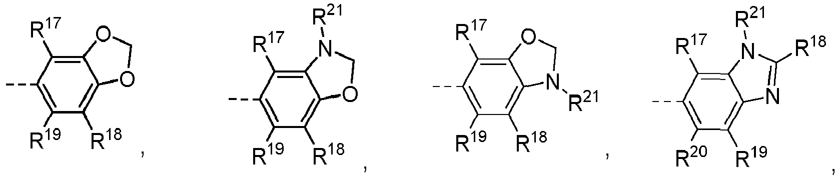 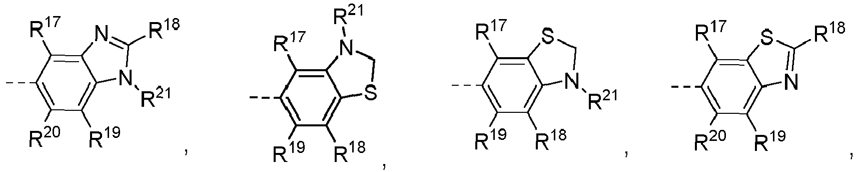 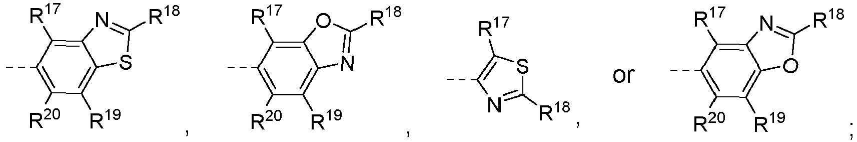
**B¹** represents -**H¹**, -**P¹-H¹**, or -**P¹-Y¹-H¹** ;
**B²** represents -**H²**, -**P²-H²**, or -**P²-Y²-H²** ;
**B³** represents -**H³**, -**P³-H³**, or -**P³**-**Y³**-**H³**;
**C²** is selected from: 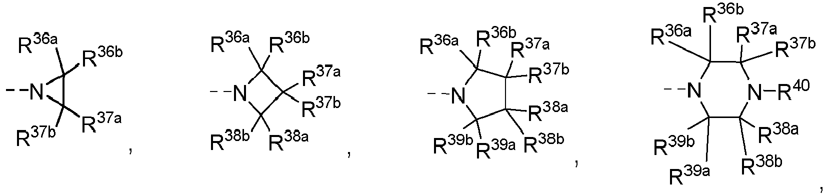 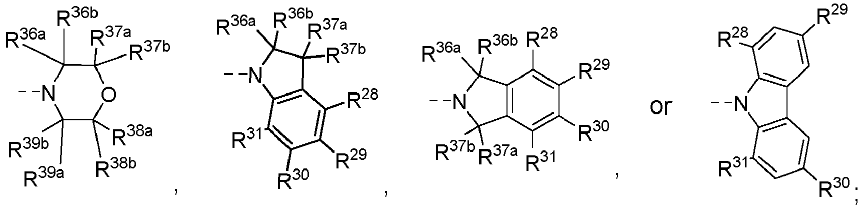
**Y¹**, **Y²**, and **Y³** are independently of each other selected from: -O-, -N**R²²**-, -CO-N**R²²**-, -N**R²²**-CO-, -SO₂-N**R²²**-, -N**R²²**-SO₂-, or -N**R²²**-CO-N**R²³**-;
**P¹**, **P²**, and **P³** are independently of each other selected from: 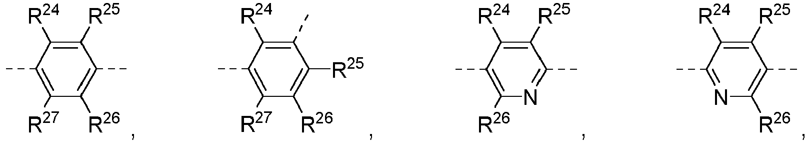 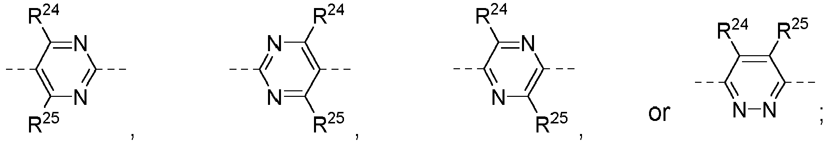
**H¹**, **H²**, and **H³** are independently of each other selected from: 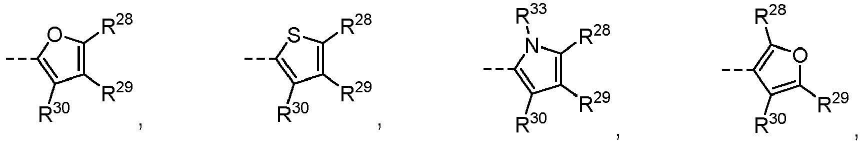 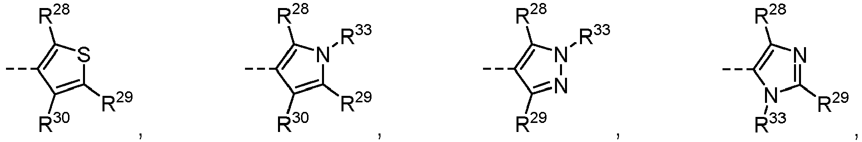 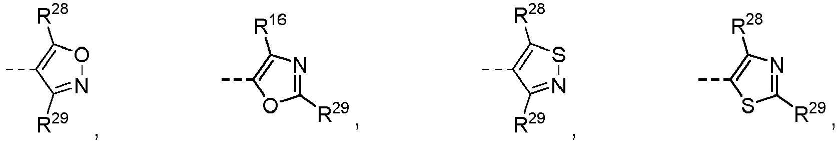 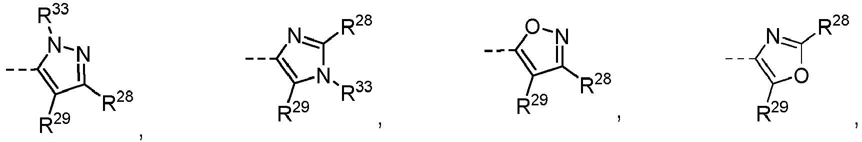 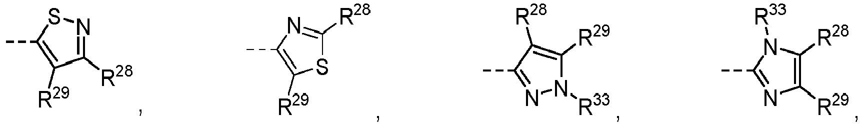 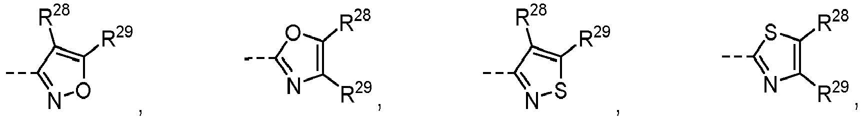 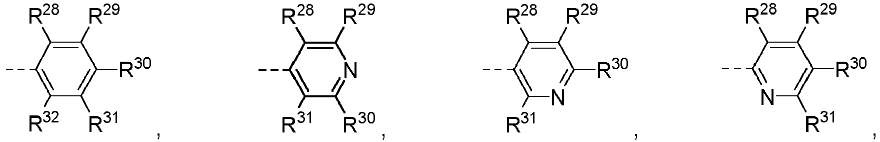 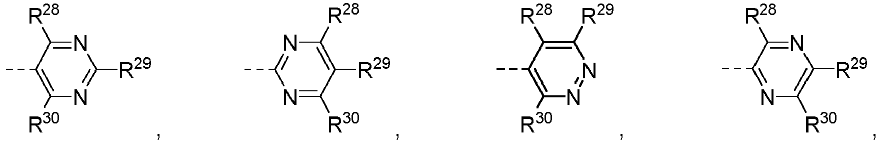 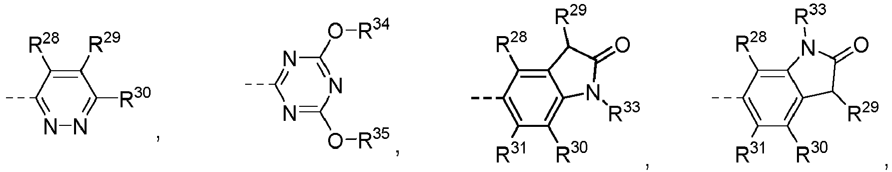 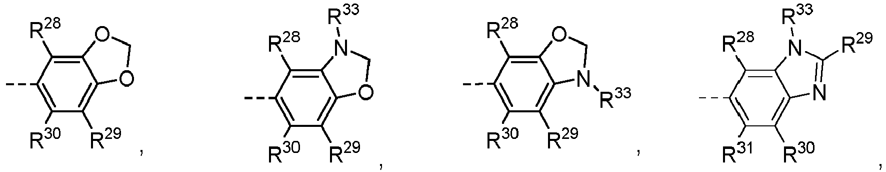 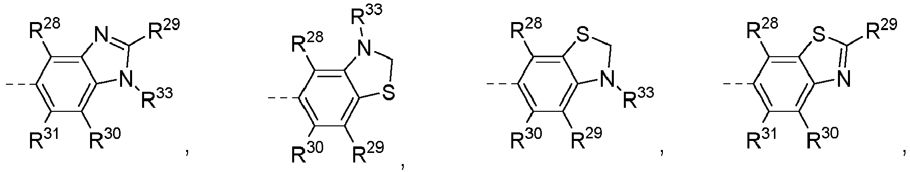 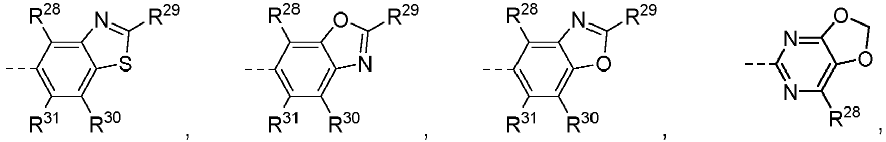 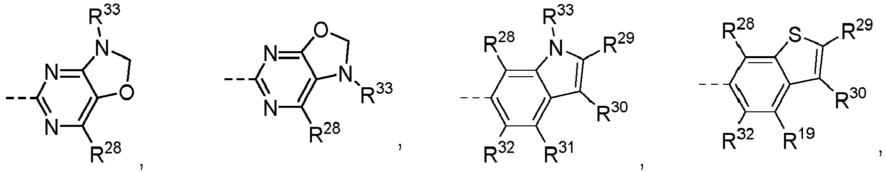 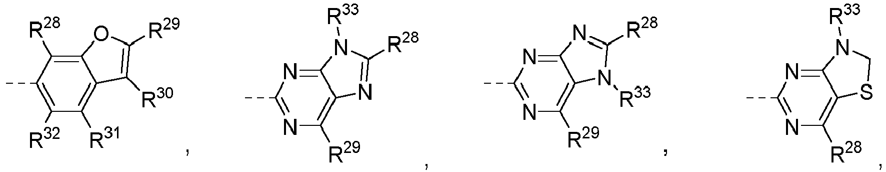 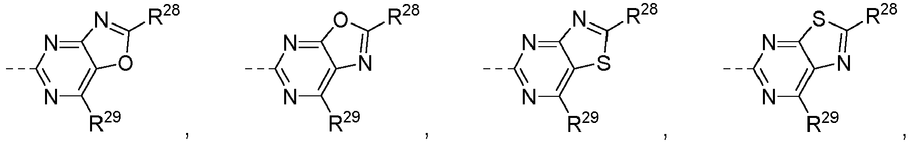 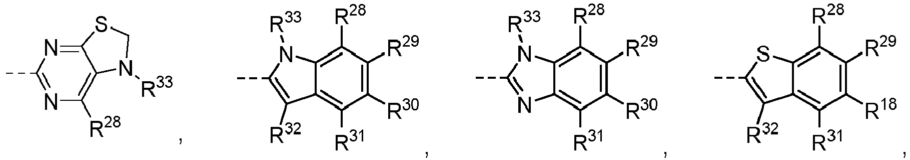 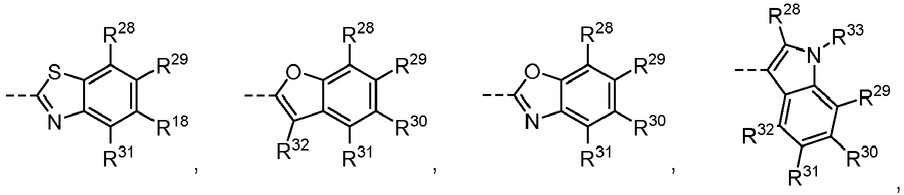 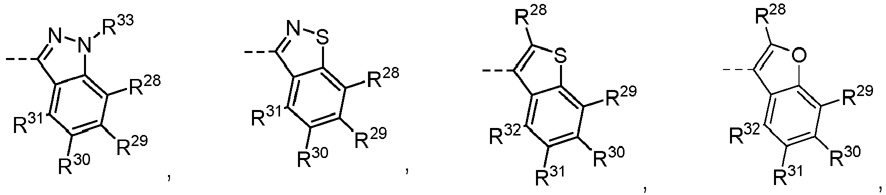 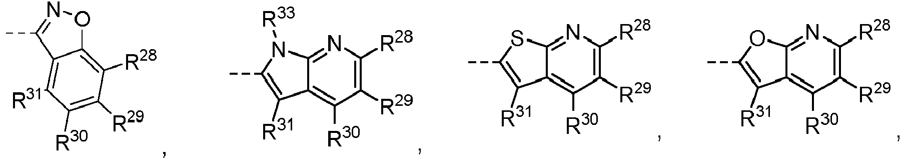 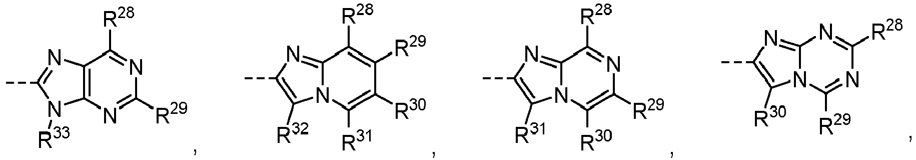 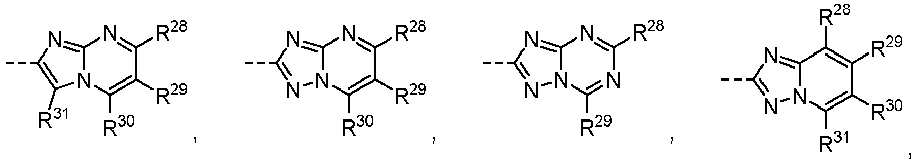 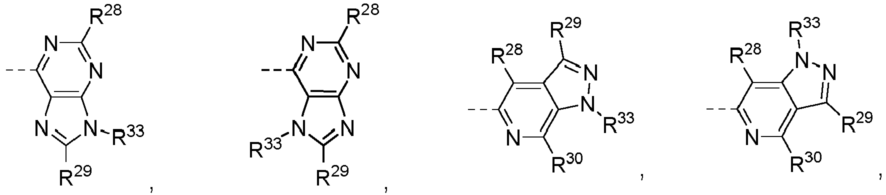 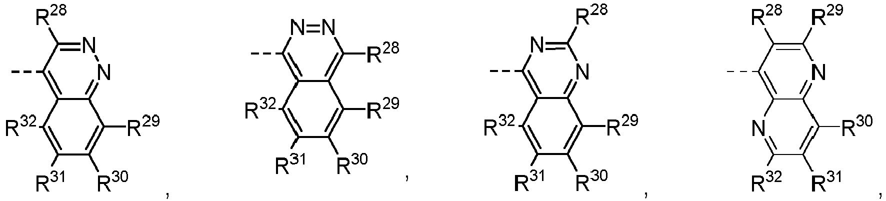 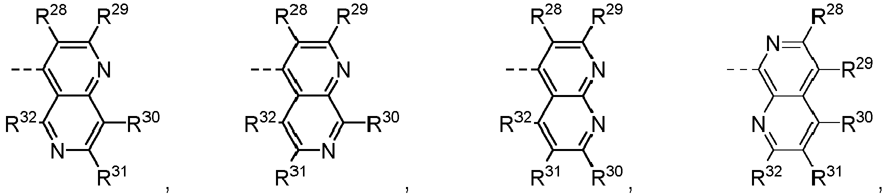 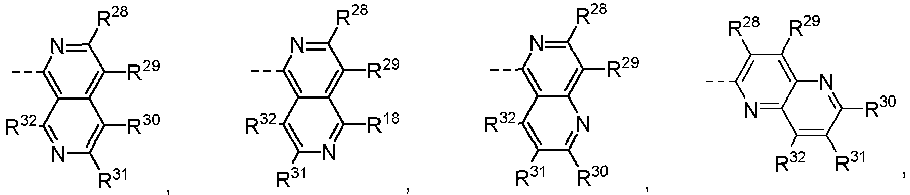 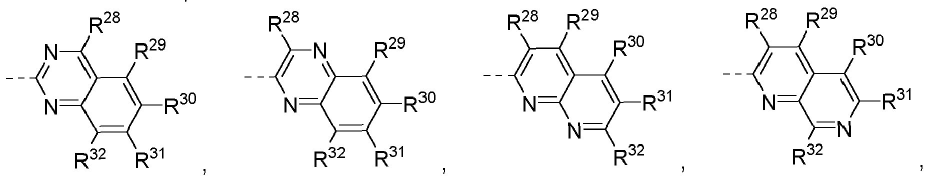 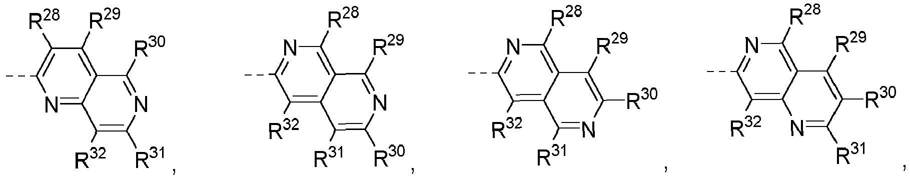 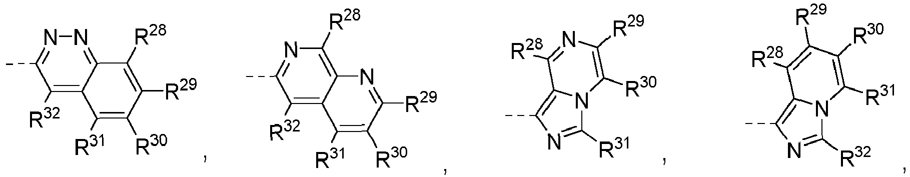 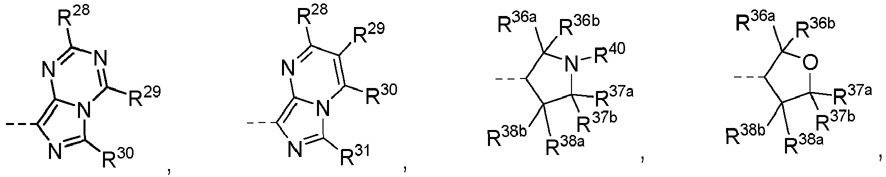 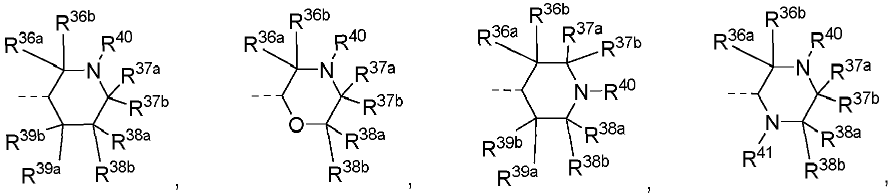 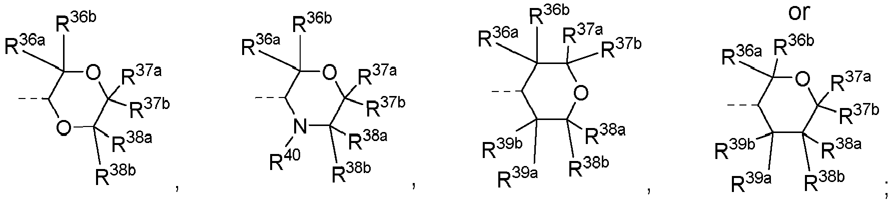
**R¹⁷** - **R²⁰** and **R²⁸**-**R³²** represent independently of each other -H, -F, -Cl, -Br, -I, -OH, -CN, -NO₂, -CH₃, -C₂H₅, -C₃H₇, -CH(CH₃)₂, -C₄H₉, -CH₂-CH(CH₃)₂, -CH(CH₃)-C₂H₅, -C(CH₃)₃, -cyclo-C₃H₅, -CH₂-cyclo-C₃H₅, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -OCH₃, -OC₂H₅, -OC₃H₇, -OCH(CH₃)₂, -OC(CH₃)₃, -OC₄H₉, -OCHF₂, -OCF₃, -OCH₂CF₃, -OC₂F₅, -OCH₂OCH₃, -O-cyclo-C₃H₅, -OCH₂-cyclo-C₃H₅, -O-C₂H₄-cyclo-C₃H₅, -CHO, -COCH₃, -COCF₃, -COC₂H₅, -COC₃H₇, -COCH(CH₃)₂, -COC(CH₃)₃, -COOH, -COOCH₃, -COOC₂H₅, -COOC₃H₇, -COOCH(CH₃)₂, -COOC(CH₃)₃, -OOC-CH₃, -OOC-CF₃, -OOC-C₂H₅, -OOC-C₃H₇, -OOC-CH(CH₃)₂, -OOC-C(CH₃)₃, -NH₂, -NHCH₃, -NHC₂H₅, -NHC₃H₇, -NHCH(CH₃)₂, -NHC(CH₃)₃, -N(CH₃)₂, -N(C₂H₅)₂, -N(C₃H₇)₂, -N[CH(CH₃)₂]₂, -N[C(CH₃)₃]₂, -NHCOCH₃, -NHCOCF₃, -NHCOC₂H₅, -NHCOC₃H₇, -NHCOCH(CH₃)₂, -NHCOC(CH₃)₃, -CONH₂, -CONHCH₃, -CONHC₂H₅, -CONHC₃H₇, -CONHCH(CH₃)₂, -CONH-cyclo-C₃H₅, -CONHC(CH₃)₃, -CON(CH₃)₂, -CON(C₂H₅)₂, -CON(C₃H₇)₂, -CON[CH(CH₃)₂]₂, -CON[C(CH₃)₃]₂, -SO₂NH₂, -SO₂NHCH₃, -SO₂NHC₂H₅, -SO₂NHC₃H₇, -SO₂NHCH(CH₃)₂, -SO₂NH-cyclo-C₃H₃, -SO₂NHC(CH₃)₃, -SO₂N(CH₃)₂, -SO₂N(C₂H₃)₂, -SO₂N(C₃H₇)₂, -SO₂N[CH(CH₃)₂]₂, -SO₂N[C(CH₃)₃]₂, -NHSO₂CH₃, -NHSO₂CF₃, -NHSO₂C₂H₃, -NHSO₂C₃H₇, -NHSO₂CH(CH₃)₂, -NHSO₂C(CH₃)₃, -CH=CH₂, -CH₂-CH=CH₂, -C(CH₃)=CH₂, -CH=CH-CH₃, -C=CH, -C≡C-CH₃, -CH₂-C≡CH, -Ph, -O-Ph, -O-CH₂-Ph, -C(=NH)NH₂, or 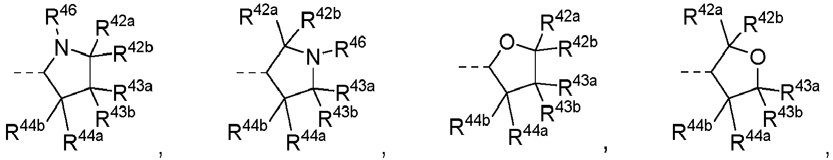 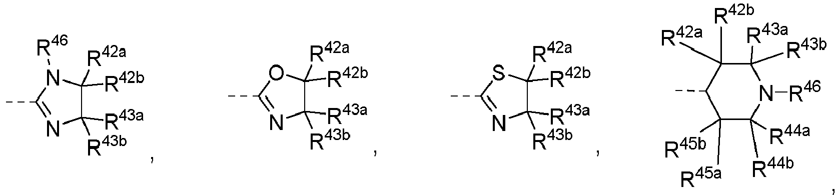 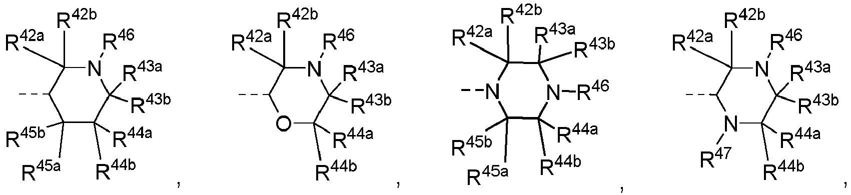 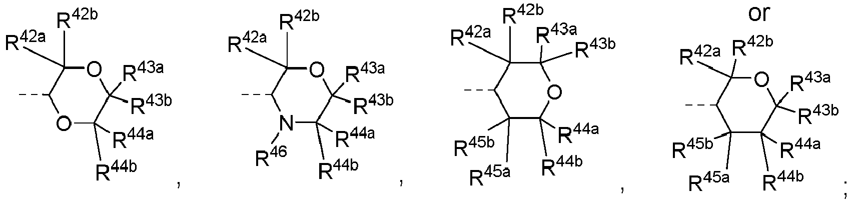
**R⁶, R¹¹, R¹², R²¹ - R²³, R³³ - R³⁶, R⁴⁰, R⁴¹, R⁴⁶,** and **R⁴⁷** represent independently of each other -H, -CH₃, -CH₂CH₃, -CH(CH₃)₂, -CH₂CH₂CH₃, -CH₂CH(CH₃)₂, -C(CH₃)₃, -cyclo-C₃H₃, -cyclo-C₄H₇, -cyclo-C₅H₉, -cyclo-C₆H₁₁, -CH₂-cyclo-C₃H₅, -CH₂-cyclo-C₄H₇, -CH₂-cyclo-C₃H₉, -CH₂-cyclo-C₆H₁₁, -CH₂-Ph, -CH₂OCH₃, -CH₂OCH₂CH₃, -CH₂CH₂OCH₃, -COO-C(CH₃)₃, -COO-CH₂Ph, or -CH₂CH₂SCH₃;
**R²⁴ - R²⁷, R^{36a} - R^{39a} R^{36b} - R^{39b} R^{42a} - R^{45a}** and **R^{42b} - R^{45b}** represent independently of each other -H, -F, -Cl, -Br, -I, -OH, -CN, -NO₂, -CH₃, -C₂H₅, -C₃H₇, -CH(CH₃)₂, -C₄H₉, -CH₂-CH(CH₃)₂, -CH(CH₃)-C₂H₅, -C(CH₃)₃, -cyclo-C₃H₃, -CH₂-cyclo-C₃H₅, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -OCH₃, -OC₂H₅, -OC₃H₇, -OCH(CH₃)₂, -OC(CH₃)₃, -OC₄H₉, -OCHF₂, -OCF₃, -OCH₂CF₃, -OC₂F₅, -OCH₂OCH₃, -O-cyclo-C₃H₅, -OCH₂-cyclo-C₃H₅, -O-C₂H₄-cyclo-C₃H₅, -CHO, -COCH₃, -COCF₃, -COC₂H₅, -COC₃H₇, -COCH(CH₃)₂, -COC(CH₃)₃, -COOH, -COOCH₃, -COOC₂H₅, -COOC₃H₇, -COOCH(CH₃)₂, -COOC(CH₃)₃, -OOC-CH₃, -OOC-CF₃, -OOC-C₂H₅, -OOC-C₃H₇, -OOC-CH(CH₃)₂, -OOC-C(CH₃)₃, -NH₂, -NHCH₃, -NHC₂H₅, -NHC₃H₇, -NHCH(CH₃)₂, -NHC(CH₃)₃, -N(CH₃)₂, -N(C₂H₅)₂, -N(C₃H₇)₂, -N[CH(CH₃)₂]₂, -N[C(CH₃)₃]₂, -NHCOCH₃, -NHCOCF₃, -NHCOC₂H₅, -NHCOC₃H₇, -NHCOCH(CH₃)₂, -NHCOC(CH₃)₃, -CONH₂, -CONHCH₃, -CONHC₂H₅, -CONHC₃H₇, -CONHCH(CH₃)₂, -CONH-cyclo-C₃H₅, -CONHC(CH₃)₃, -CON(CH₃)₂, -CON(C₂H₅)₂, -CON(C₃H₇)₂, -CON[CH(CH₃)₂]₂, -CON[C(CH₃)₃]₂, -SO₂NH₂, -SO₂NHCH₃, -SO₂NHC₂H₅, -SO₂NHC₃H₇, -SO₂NHCH(CH₃)₂, -SO₂NH-cyclo-C₃H₃, -SO₂NHC(CH₃)₃, -SO₂N(CH₃)₂, -SO₂N(C₂H₃)₂, -SO₂N(C₃H₇)₂, -SO₂N[CH(CH₃)₂]₂, -SO₂N[C(CH₃)₃]₂, -NHSO₂CH₃, -NHSO₂CF₃, -NHSO₂C₂H₅, -NHSO₂C₃H₇, -NHSO₂CH(CH₃)₂, -NHSO₂C(CH₃)₃, -CH=CH₂, -CH₂-CH=CH₂, -C(CH₃)=CH₂, -CH=CH-CH₃, -C≡CH, -C≡C-CH₃, -CH₂-C≡CH, -Ph, -O-Ph, or -O-CH₂-Ph;
**X** represents -CN, -NH₂, or -CH₂OH;
or a regiomer, diastereomer, enantiomer, a mixture of regiomers, a mixture of diastereomers, a mixture of enantiomers, a prodrug, a solvate, a hydrate, or a pharmaceutically acceptable salt thereof.

10. The compound for use according to claim 9, wherein the compound modulates the function and/or activity of the microRNA.

11. The compound for use according to claim 9 or 10, wherein the disease is selected from cancer, tumor, metastasis, virus infection, immunological disease, autoimmune disease, inflammatory disorder, diabetes, cardiovascular disease, metabolic disease, and neurodegenerative disease.

12. The compound for use according to any one of claims 9 to 11, wherein the cancer is selected from breast cancer, colon cancer, pancreas cancer and prostate cancer.

13. The compound for use according to any one of claims 9 to 12, wherein the microRNA comprises a stem-loop region having a sequence as set forth in SEQ ID No. 3 to 53.
